# EUROPEAN PATENT APPLICATION

(11) **EP 4 596 550 A1**
(43) Date of publication of application: **06.08.2025**
(21) Application number: 23896679.0
(22) Date of filing: 24.11.2023
(51) Int. Cl.: C07D 487/04, C07D 271/107, C07D 403/14, A61K 45/00, A61P 37/02, A61P 35/00

(54) **COMPOUND USABLE IN CONJUGATION REACTION AND CONJUGATE THEREOF**

(30) Priority: 29.11.2022 CN 202211509417; 02.12.2022 CN 202211536550; 08.12.2022 CN 202211573193
(71) Applicant: Sichuan Kelun-Biotech Biopharmaceutical Co., Ltd., Chengdu, Sichuan 611138 (CN)
(72) Inventor: TIAN, Qiang, Chengdu, Sichuan 611138 (CN); ZHANG, Yitao, Chengdu, Sichuan 611138 (CN); LONG, Dong, Chengdu, Sichuan 611138 (CN); LIAO, Menchang, Chengdu, Sichuan 611138 (CN); ZHOU, Qin, Chengdu, Sichuan 611138 (CN); SONG, Hongmei, Chengdu, Sichuan 611138 (CN); GE, Junyou, Chengdu, Sichuan 611138 (CN)
(74) Representative: Meissner Bolte Partnerschaft mbB
(86) International application number: PCT/CN2023/134003
(87) International publication number: WO 2024/114528

(57) **Abstract**

The present application provides a compound usable in a coupling reaction and a conjugate thereof. Further provided in the present application are a synthetic intermediate of the compound and the conjugate thereof, a synthesis method, and a use of the conjugate in preparing a drug for the treatment or prevention of an oncological disease.

## Description

The present disclosure is based on and claims priority to CN patent application No. 202211509417.1, filed on November 29, 2022, CN patent application No. 202211536550.6, filed on December 2, 2022, and CN patent application No. 202211573193.0, filed on December 8, 2022. The entire content of each of these applications is hereby incorporated by reference.

### Field of the Invention

The present application relates to a class of compounds usable in conjugation reactions and conjugates thereof, methods for the preparation of the compounds and conjugates, pharmaceutical compositions of the conjugates, and use of the conjugates in the treatment of tumor-related diseases.

### Background of the Invention

In recent years, antibody-drug conjugates are one of the hot directions of tumor precision therapy, bringing hope for tumor treatment. Antibody-drug conjugates (ADCs), which are obtained by conjugating monoclonal antibody drugs targeting specific antigens and small cytotoxic drugs by linkers, combine the powerful killing effect of traditional small molecule chemotherapy with the tumor targeting of antibody drugs. As of Zynlonta's approval in April 2021, twelve ADCs have been approved worldwide, including seven for hematological tumors and five for solid tumors.

An antibody-drug conjugate consists of an antibody, a linker and a payload. The methods for conjugation of an antibody and a payload-linker are mainly divided into non-site-specific conjugation and site-specific conjugation. In the early days, the non-site-specific conjugation methods, mainly referring to lysine conjugation and cysteine conjugation, were used, where the drug was directly conjugated to amino acid residues on the antibody by chemical methods, without involving modification of the antibody. The number of cytotoxin molecules and the conjugating site could not be determined, resulting in poor homogeneity. At present, the commonly used site-specific conjugation method is specific conjugation through genetic engineering sites or special linkers to achieve more homogeneous conjugation, thereby realizing the connection of cytotoxin at a specific site. The ADCs produced by site-specific conjugation can reduce fluctuations in pharmacodynamics, pharmacokinetics and CMC quality control caused by different conjugating sites and number.

At present, the common site-specific conjugation methods are THIOMAB technology, unnatural amino acid conjugation technology, glutaminase-induced conjugation technology, Sortase transpeptidase conjugation technology and ThioBridge technology, etc. Among them, the modification of antibodies by antibody engineering or enzyme-induced conjugation may have a certain impact on the structural stability of antibodies and have certain requirements for CMC. In addition, some ThioBridge techniques based on chemical conjugation also have certain defects, such as DBM (dibromomaleimides) linkers, which are unstable in plasma, can react with other bio-groups containing sulfhydryl, resulting in reduced efficacy and increased toxicity and side effects (Chem.-Eur. J., 2019, 25, 43-59.). Therefore, the development of novel linker substructures is still important for the development of antibody-drug conjugates with good efficacy and safety.

### Summary of the Invention

One of the objectives of the present application is to provide a novel linker for chemical conjugation, which features high reactivity, mild conjugation conditions, simple operation and can achieve site-specific conjugation, and a bioactive conjugate obtained therefrom has good homogeneity and plasma stability, and clear efficacy in vivo and in vitro.

### Compound

In a first aspect, the present disclosure provides a compound or a pharmaceutically acceptable salt, ester, stereoisomer, tautomer, polymorph, solvate, N-oxide or isotope labeled compound thereof, wherein the compound has a structure of formula I: 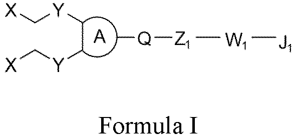 wherein
X is a leaving group, such as F, Cl, Br, I, OMs, OTs, OTf, p-nitrophenol ester, fluorophenol ester, C₁₋₆ alkyl sulfonyl or 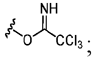
Y is absent or is selected from substituted or unsubstituted C₁₋₆ alkylene, sulfonyl and carbonyl, and when being substituted, the C₁₋₆ alkylene is substituted by substituent(s) selected from hydrogen, halogen, hydroxyl, -CN, -C₁₋₆ alkyl, -C₁₋₆ haloalkyl, -OC₁₋₆ alkyl, and -OC₁₋₆ haloalkyl;
Ring A is selected from substituted or unsubstituted C₆₋₁₀ aromatic ring, substituted or unsubstituted 5- to 12-membered heteroaromatic ring and substituted or unsubstituted 5- to 12-membered heterocycle, and when being substituted, each of the C₆₋₁₀ aromatic ring, the 5- to 12-membered heteroaromatic ring and the 5- to 12-membered heterocycle is substituted by substituent(s) independently selected from hydrogen, halogen, hydroxyl, -CN, -C₁₋₆ alkyl, -C₁₋₆ haloalkyl, -OC₁₋₆ alkyl, -OC₁₋₆ haloalkyl, carboxyl, polyethylene glycol, amino acid, phosphoric acid, sulfonic acid, amino, azido and alkynyl;
Q is absent or is composed of one or more of the following substituted or unsubstituted groups: -NH-, -O-, -CH₂-, hydroxyl, carbonyl, amido, sulfonyl, sulfonylurea group, carbamoyl, oximido, -NH-S(=O)₂-NH-C(=O)O-, -C(=O)NH-, -NHC(=O)- or C₂₋₆ alkynylene, and when being substituted, each of the groups is substituted by substituent(s) independently selected from hydrogen, halogen, hydroxyl, -CN, -C₁₋₆ alkyl, -C₁₋₆ haloalkyl, -OC₁₋₆ alkyl, and -OC₁₋₆ haloalkyl;
Z₁ is absent or is selected from substituted or unsubstituted phenyl, substituted or unsubstituted 5- to 6-membered heteroaryl, amido, substituted or unsubstituted -CH₂- and substituted or unsubstituted C₂₋₆ alkynylene, and when being substituted, each of the phenyl, the 5- to 6-membered heteroaryl, the -CH₂- and the C₂₋₆ alkynylene is substituted by substituent(s) independently selected from hydrogen, halogen, hydroxyl, -CN, -C₁₋₆ alkyl, -C₁₋₆ haloalkyl, -OC₁₋₆ alkyl and -OC₁₋₆ haloalkyl;
W₁ is absent or is one or more groups selected from substituted or unsubstituted C₁₋₁₀ alkylene, -(CH₂CH₂O)ₚ- and -(OCH₂CH₂)ₚ-, wherein p is an integer from 1 to 20, and when being substituted, the C₁₋₁₀ alkylene is substituted by substituent(s) selected from hydrogen, halogen, hydroxyl, -CN, -C₁₋₆ alkyl, -C₁₋₆ haloalkyl, -OC₁₋₆ alkyl and -OC₁₋₆ haloalkyl; and
J₁ is selected from -COOH, -NH₂, substituted -NH₂, 3- to 10-membered nitrogen-containing heterocycle, substituted 3- to 10-membered nitrogen-containing heterocycle, alkynyl, 8- to 16-membered alkynyl group-containing cyclic group, substituted 8- to 16-membered alkynyl group-containing cyclic group, azido, tetrazinyl, hydroxylamine, aldehyde, keto, sulfonylurea group, isocyanate, thioisocyanate, maleimide and hydroxyl, and the term "substituted" means substitution by substituent(s) independently selected from hydrogen, halogen, hydroxyl, -CN, -C₁₋₆ alkyl, -C₁₋₆ haloalkyl, -OC₁₋₆ alkyl and -OC₁₋₆ haloalkyl.

In some embodiments, Ring A is selected from substituted or unsubstituted C₆₋₁₀ aromatic ring, substituted or unsubstituted 5- to 12-membered heteroaromatic ring and substituted or unsubstituted 5- to 12-membered heterocycle, and when being substituted, each of the C₆₋₁₀ aromatic ring, the 5- to 12-membered heteroaromatic ring and the 5- to 12-membered heterocycle is substituted by substituent(s) independently selected from carboxyl, polyethylene glycol, amino acid, phosphoric acid, sulfonic acid, amino, azido and alkynyl.

In some embodiments, X is a leaving group, such as Cl, Br, I, OMs, OTs, OTf or 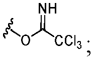
Y is absent or carbonyl;
Ring A is selected from substituted or unsubstituted C₆₋₁₀ aromatic ring, substituted or unsubstituted 5- to 12-membered heteroaromatic ring and substituted or unsubstituted 5- to 12-membered heterocycle, and when being substituted, each of the C₆₋₁₀ aromatic ring, the 5- to 12-membered heteroaromatic ring and the 5- to 12-membered heterocycle is substituted by substituent(s) independently selected from hydrogen, halogen, hydroxyl, -CN, -C₁₋₆ alkyl, -C₁₋₆ haloalkyl, -OC₁₋₆ alkyl, and -OC₁₋₆ haloalkyl;
Q is absent or is substituted or unsubstituted -C(=O)NH-, and when being substituted, the - C(=O)NH- is substituted by substituent(s) selected from hydrogen, halogen, hydroxyl, -CN, -C₁₋₆ alkyl, -C₁₋₆ haloalkyl, -OC₁₋₆ alkyl, and -OC₁₋₆ haloalkyl;
Z₁ is absent or is substituted or unsubstituted -CH₂- or substituted or unsubstituted C₂₋₆ alkynylene, and when being substituted, the -CH₂- or the C₂₋₆ alkynylene is substituted by substituent(s) independently selected from hydrogen, halogen, hydroxyl, -CN, -C₁₋₆ alkyl, -C₁₋₆ haloalkyl, -OC₁₋₆ alkyl and -OC₁₋₆ haloalkyl;
W₁ is absent or is one or more groups selected from substituted or unsubstituted C₁₋₁₀ alkylene, -(CH₂CH₂O)ₚ- and -(OCH₂CH₂)ₚ-, wherein p is an integer from 1 to 10, and when being substituted, the C₁₋₁₀ alkylene is substituted by substituent(s) selected from hydrogen, halogen, hydroxyl, -CN, -C₁₋₆ alkyl, -C₁₋₆ haloalkyl, -OC₁₋₆ alkyl and -OC₁₋₆ haloalkyl; and
J₁ is selected from -COOH, -NH₂, 3- to 10-membered nitrogen-containing heterocycle, sulfonylurea group and hydroxyl.

In some embodiments, X is a leaving group, such as F, Cl, Br, I, OMs, OTs, OTf, p-nitrophenol ester, fluorophenol ester, C₁₋₆ alkyl sulfonyl or 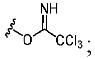
Y is absent or is selected from C₁₋₆ alkylene, sulfonyl, and carbonyl;
Ring A is selected from substituted or unsubstituted C₆₋₁₀ aromatic ring, 5- to 12-membered heteroaromatic ring or 5- to 12-membered heterocycle, and the substituent is selected from carboxyl, polyethylene glycol, amino acid, phosphoric acid, sulfonic acid, amino, azido and alkynyl;
Q is absent or is a fragment composed of one or more of the following groups: -NH-, -O-, - CH₂-, hydroxyl, carbonyl, amido, sulfonyl, sulfonylurea group, carbamoyl, oximido, -NH-S(=O)₂-NH-C(=O)O-, -C(=O)NH-, -NHC(=O)- or C₂₋₆ alkynylene; preferably, Z₂ is absent or is a fragment composed of one or more of the following groups: -NH-, -CH₂-, carbonyl or C₂₋₆ alkynylene;
Z₁ is absent or is selected from phenyl, 5- to 6-membered heteroaryl, amido, -CH₂- or C₂₋₆ alkynylene;
W₁ is absent or is one or more groups selected from C₁₋₁₀ alkylene, -(CH₂CH₂O)ₚ-, and - (OCH₂CH₂)ₚ-; p is an integer from 1 to 20;
J₁ is selected from -COOH, -NH₂, 3- to 10-membered nitrogen-containing heterocycle, alkynyl, 8- to 16-membered alkynyl group-containing cyclic group, azido, tetrazinyl, hydroxylamine, aldehyde, keto, sulfonylurea group, isocyanate, thioisocyanate, maleimide or hydroxyl;
p is an integer from 1 to 10.

In some embodiments, X is selected from one or more of Cl, Br, I, OMs, OTs, and OTf.

In some embodiments, Y is absent or is C₁₋₆ alkylene.

In some embodiments, Q is absent or is -C(=O)-NH- or - NH-C(=O)-.

In some embodiments, Z₁ is absent or is C₂₋₆ alkynylene.

In some embodiments, W₁ is absent or is one or more selected from C₁₋₁₀ alkylene and - (CH₂CH₂O)ₚ-.

In some embodiments, W₁ is C₁₋₁₀ alkylene, preferably C₁₋₆ alkylene, further preferably C₁₋₃ alkylene.

In some embodiments, J₁ is -COOH, alkynyl, 8- to 16-membered alkynyl group-containing cyclic group, azido, tetrazinyl, hydroxylamine, aldehyde, keto, isocyanate, thioisocyanate or maleimide.

In some embodiments, Ring A is selected from substituted or unsubstituted 5- to 12-membered nitrogen-containing heteroaromatic ring or 5- to 12-membered nitrogen-containing heterocycle, and when being substituted, each of the 5- to 12-membered nitrogen-containing heteroaromatic ring and the 5- to 12-membered nitrogen-containing heterocycle is substituted by substituent(s) independently selected from carboxyl, polyethylene glycol, amino acid, phosphoric acid, sulfonic acid, amino, azido and alkynyl; preferably, Ring A is selected from 5- to 12-membered nitrogen-containing heteroaromatic ring or 5- to 12-membered nitrogen-containing heterocycle that is unsubstituted or is substituted by oxo or -COOH.

In some embodiments, p is 1, 2, 3, 4, 5, 6, 7, 8, 9, or 10.

In some embodiments, X is selected from Cl, Br, I, OMs, OTs, OTf, C₁₋₆ alkyl sulfonyl or 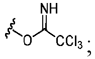
Y is absent;
Ring A is selected from 5- to 12-membered nitrogen-containing heteroaromatic ring or 5- to 12-membered nitrogen-containing heterocycle which is unsubstituted or is substituted by oxo or - COOH.
Q is absent or is -C(=O)-NH-.
Z₁ is absent or is -CH₂-;
W₁ is absent or is one or more selected from C₁₋₁₀ alkylene and -(CH₂CH₂O)ₚ-;
J₁ is -COOH;
p is an integer from 1 to 10.

In some embodiments, formula I is selected from the group consisting of:

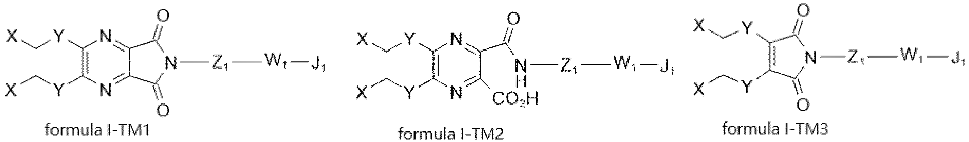

In some embodiments, the compound of formula I has a structure selected from the group consisting of: 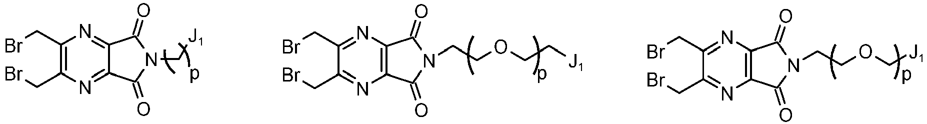 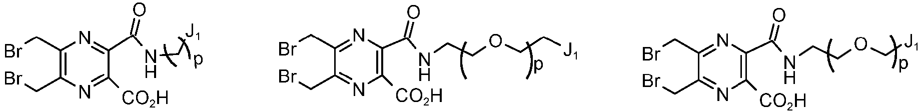 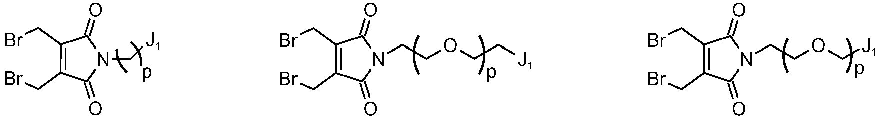 wherein p is an integer from 1 to 10 and J₁ is -COOH or -NH₂.

In some embodiments, the compound of formula I has a structure selected from the group consisting of:

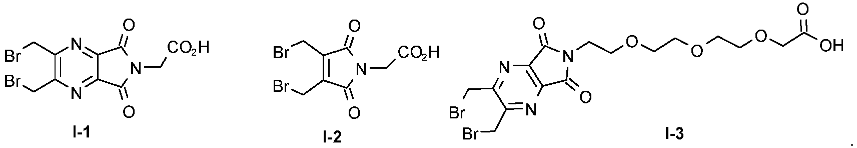

In a second aspect, the present disclosure provides a compound or a pharmaceutically acceptable salt, ester, stereoisomer, tautomer, polymorph, solvate, N-oxide or isotope labeled compound thereof, wherein the compound has a structure of formula II: 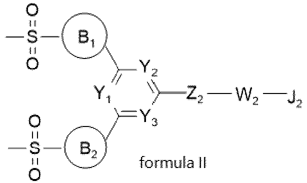
wherein each of B₁ and B₂ is independently a single bond or a substituted or unsubstituted 5-to 12-membered nitrogen-containing heteroaromatic ring, and when being substituted, the 5- to 12-membered nitrogen-containing heteroaromatic ring is substituted by substituent(s) composed of one or more of the following groups: hydrogen, halogen, hydroxyl, -CN, substituted or unsubstituted C₁₋₁₀ alkylene, -C₁₋₆ haloalkyl, -OC₁₋₆ alkyl, -OC₁₋₆ haloalkyl, carboxyl, substituted or unsubstituted amido, substituted or unsubstituted carbamoyl, substituted or unsubstituted polyethylene glycol, alkynyl and azido, and when being substituted, each of the C₁₋₁₀ alkylene, the amido, the carbamoyl and the polyethylene glycol is substituted by substituent(s) independently selected from hydrogen, halogen, hydroxyl, -CN, -C₁₋₆ alkyl, -C₁₋₆ haloalkyl, -OC₁₋₆ alkyl and - OC₁₋₆ haloalkyl;
Y₁, Y₂ and Y₃ are each independently selected from C(R) and N;
Z₂ is absent or is a group composed of one or more of the following substituted or unsubstituted functional groups: -NH-, -O-, -CH₂-, hydroxyl, carbonyl, amido, sulfonyl, sulfonylurea group, carbamoyl, oximido, -NH-S(=O)₂-NH-C(=O)O-, -C(=O)NH-, -NHC(=O)- or C₂₋₆ alkynylene, and when being substituted, each of the functional groups is substituted by substituent(s) independently selected from hydrogen, halogen, hydroxyl, -CN, -C₁₋₆ alkyl, -C₁₋₆ haloalkyl, -OC₁₋₆ alkyl, and -OC₁₋₆ haloalkyl;
preferably, Z₂ is absent or is a group composed of one or more of the following substituted or unsubstituted functional groups: -NH-, -CH₂-, carbonyl or C₂₋₆ alkynylene, and when being substituted, each of the functional groups is substituted by substituent(s) independently selected from hydrogen, halogen, hydroxyl, -CN, -C₁₋₆ alkyl, -C₁₋₆ haloalkyl, -OC₁₋₆ alkyl, and -OC₁₋₆ haloalkyl;
W₂ is absent or is one or more groups selected from substituted or unsubstituted C₁₋₁₀ alkylene, -(CH₂CH₂O)ₚ- and -(OCH₂CH₂)ₚ-, and when being substituted, the C₁₋₁₀ alkylene is substituted by substituent(s) selected from hydrogen, halogen, hydroxyl, -CN, -C₁₋₆ alkyl, -C₁₋₆ haloalkyl, -OC₁₋₆ alkyl and -OC₁₋₆ haloalkyl; p is an integer from 1 to 10;
J₂ is selected from -COOH, -N(R)(R'), substituted or unsubstituted 3- to 10-membered nitrogen-containing heterocycle, sulfonylurea group, alkynyl, substituted or unsubstituted 8- to 16-membered alkynyl group-containing cyclic group, azido, tetrazinyl, substituted or unsubstituted hydroxylamine, aldehyde, keto, isocyanate, thioisocyanate, maleimide and hydroxyl, and when being substituted, each of the 3- to 10-membered nitrogen-containing heterocycle, the 8- to 16-membered alkynyl group-containing cyclic group and the hydroxylamine is substituted by substituent(s) independently selected from hydrogen, halogen, hydroxyl, -CN, -C₁₋₆ alkyl, -C₁₋₆ haloalkyl, -OC₁₋₆ alkyl and -OC₁₋₆ haloalkyl;
each of R and R' is independently selected from hydrogen, halogen, hydroxyl, -CN, -C₁₋₆ alkyl, -C₁₋₆ haloalkyl, -OC₁₋₆ alkyl, and -OC₁₋₆ haloalkyl.

In some embodiments, each of B₁ and B₂ is independently a single bond or a 5- to 12-membered nitrogen-containing heteroaromatic ring; the 5- to 12-membered nitrogen-containing heteroaromatic ring is optionally substituted by fragment(s) composed of one or more of the following functional groups: hydrogen, C₁₋₁₀ alkylene, carboxyl, amido, carbamoyl, polyethylene glycol, alkynyl, and azido;
Y₁, Y₂ and Y₃ are each independently selected from CH and N at each occurrence;
Z₂ is absent or is a fragment composed of one or more of the following groups: -NH-, -O-, - CH₂-, hydroxyl, carbonyl, amido, sulfonyl, sulfonylurea group, carbamoyl, oximido, -NH-S(=O)₂-NH-C(=O)O-, -C(=O)NH-, -NHC(=O)- or C₂₋₆ alkynylene; preferably, Z₂ is absent or is a fragment composed of one or more of the following groups: -NH-, -CH₂-, carbonyl or C₂₋₆ alkynylene;
W₂ is absent or is one or more groups selected from C₁₋₁₀ alkylene, -(CH₂CH₂O)ₚ- or - (OCH₂CH₂)ₚ-;
J₂ is selected from -COOH, -NH₂, 3- to 10-membered nitrogen-containing heterocycle, sulfonylurea group, alkynyl, 8- to 16-membered alkynyl group-containing cyclic group, azido, tetrazinyl, hydroxylamine, aldehyde, keto, isocyanate, thioisocyanate, maleimide or hydroxyl;
p is an integer from 1 to 10.

In some embodiments, each of B₁ and B₂ is independently a single bond or a substituted or unsubstituted 5- to 12-membered nitrogen-containing heteroaromatic ring, and when being substituted, the 5- to 12-membered nitrogen-containing heteroaromatic ring is substituted by substituent(s) composed of one or more of the following groups: hydrogen, substituted or unsubstituted C₁₋₁₀ alkylene, carboxyl, substituted or unsubstituted amido, substituted or unsubstituted carbamoyl, substituted or unsubstituted polyethylene glycol, alkynyl and azido, and when being substituted, each of the C₁₋₁₀ alkylene, the amido, the carbamoyl and the polyethylene glycol is substituted by substituent(s) independently selected from hydrogen, halogen, hydroxyl, - CN, -C₁₋₆ alkyl, -C₁₋₆ haloalkyl, -OC₁₋₆ alkyl and -OC₁₋₆ haloalkyl.

In some embodiments, each of B₁ and B₂ is independently a single bond or a substituted or unsubstituted 5- to 12-membered nitrogen-containing heteroaromatic ring; and when being substituted, the 5- to 6-membered nitrogen-containing heteroaromatic ring is substituted by substituent(s) selected from the group consisting of: hydrogen, halogen, hydroxyl, -CN, C₁₋₆ alkyl, -C₁₋₆ haloalkyl, -OC₁₋₆ alkyl, or -OC₁₋₆ haloalkyl.

In some embodiments, each of B₁ and B₂ is independently a single bond or a 5- to 12-membered nitrogen-containing heteroaromatic ring; the 5- to 12-membered nitrogen-containing heteroaromatic ring is optionally substituted by fragment(s) composed of one or more of the following functional groups: hydrogen, C₁₋₁₀ alkylene, carboxyl, amido, carbamoyl, polyethylene glycol, alkynyl, and azido;
Y₁, Y₂ and Y₃ are each independently selected from CH and N at each occurrence;
Z₂ is absent or is a fragment composed of one or more of the following groups: -NH-, -O-, - CH₂-, hydroxyl, carbonyl, amido, sulfonyl, sulfonylurea group, carbamoyl, oximido, -NH-S(=O)₂-NH-C(=O)O-, -C(=O)NH-, -NHC(=O)- or C₂₋₆ alkynylene; preferably, Z₂ is absent or is a fragment composed of one or more of the following groups: -NH-, -CH₂-, carbonyl or C₂₋₆ alkynylene;
W₂ is absent or is selected from one or more of C₁₋₁₀ alkylene, -(CH₂CH₂O)ₚ- or - (OCH₂CH₂)ₚ-;
J₂ is selected from -COOH, -NH₂, 3- to 10-membered nitrogen-containing heterocycle, sulfonylurea group, alkynyl, 8- to 16-membered alkynyl group-containing cyclic group, azido, tetrazinyl, hydroxylamine, aldehyde, keto, isocyanate, thioisocyanate, maleimide or hydroxyl;
p is an integer from 1 to 10.

In some embodiments, each of B₁ and B₂ is independently a single bond or a substituted or unsubstituted 5- to 6-membered nitrogen-containing heteroaromatic ring, and when being substituted, the 5- to 6-membered nitrogen-containing heteroaromatic ring is substituted by substituent(s) selected from the group consisting of: hydrogen, halogen, hydroxyl, -CN, substituted or unsubstituted C₁₋₁₀ alkylene, -C₁₋₆ haloalkyl, -OC₁₋₆ alkyl, -OC₁₋₆ haloalkyl, carboxyl, substituted or unsubstituted amido, substituted or unsubstituted carbamoyl, substituted or unsubstituted polyethylene glycol, alkynyl and azido, and when being substituted, each of the C₁₋₁₀ alkylene, the amido, the carbamoyl and the polyethylene glycol is substituted by substituent(s) independently selected from hydrogen, halogen, hydroxyl, -CN, -C₁₋₆ alkyl, -C₁₋₆ haloalkyl, -OC₁₋₆ alkyl and -OC₁₋₆ haloalkyl; preferably, each of B₁ and B₂ is independently a single bond or a substituted or unsubstituted pyrimidine ring, and when being substituted, the pyrimidine ring is substituted by substituent(s) selected from hydrogen, halogen, hydroxyl, -CN, -C₁₋₆ alkyl, -C₁₋₆ haloalkyl, -OC₁₋₆ alkyl and -OC₁₋₆ haloalkyl;
Y₁, Y₂ and Y₃ are each independently selected from CH and N;
Z₂ is absent or is a group composed of one or more of the following substituted or unsubstituted functional groups: -NH-, -CH₂-, carbonyl, -C(=O)NH-, -NHC(=O)- or C₂₋₆ alkynylene, and when being substituted, each of the functional groups is substituted by substituent(s) independently selected from hydrogen, halogen, hydroxyl, -CN, -C₁₋₆ alkyl, -C₁₋₆ haloalkyl, -OC₁₋₆ alkyl, and -OC₁₋₆ haloalkyl;
W₂ is absent or is one or more groups selected from substituted or unsubstituted C₁₋₁₀ alkylene, -(CH₂CH₂O)ₚ- and -(OCH₂CH₂)ₚ-, and when being substituted, the C₁₋₁₀ alkylene is substituted by substituent(s) selected from hydrogen, halogen, hydroxyl, -CN, -C₁₋₆ alkyl, -C₁₋₆ haloalkyl, -OC₁₋₆ alkyl and -OC₁₋₆ haloalkyl;
J₂ is selected from -COOH, -NH₂, 3- to 10-membered nitrogen-containing heterocycle, sulfonylurea group and hydroxyl; and
p is an integer from 1 to 10.

In some embodiments, each of B₁ and B₂ is independently a single bond or a substituted or unsubstituted 5- to 6-membered nitrogen-containing heteroaromatic ring and the substituent is independently selected from the group consisting of: hydrogen, halogen, hydroxyl, -CN, -C₁₋₆ alkyl, -C₁₋₆ haloalkyl, -OC₁₋₆ alkyl and -OC₁₋₆ haloalkyl; preferably, each of B₁ and B₂ is independently a single bond or a substituted or unsubstituted pyrimidine ring, and the substituent is independently selected from the group consisting of: hydrogen, halogen, hydroxyl, -CN, -C₁₋₆ alkyl, -C₁₋₆ haloalkyl, -OC₁₋₆ alkyl or -OC₁₋₆ haloalkyl.
Y₁, Y₂ and Y₃ are each independently selected from CH and N;
Z₂ is absent or is composed of one or more of the following substituted or unsubstituted groups: -NH-, -CH₂-, carbonyl, -C(=O)NH-, -NHC(=O)- or C₂₋₆ alkynylene, and the substituent is independently selected from hydrogen, halogen, hydroxyl, -CN, -C₁₋₆ alkyl, -C₁₋₆ haloalkyl, -OC₁₋₆ alkyl, or -OC₁₋₆ haloalkyl;
W₂ is absent or is one or more groups selected from substituted or unsubstituted C₁₋₁₀ alkylene, -(CH₂CH₂O)ₚ- or -(OCH₂CH₂)ₚ-, and the substituent is independently selected from hydrogen, halogen, hydroxyl, -CN, -C₁₋₆ alkyl, -C₁₋₆ haloalkyl, -OC₁₋₆ alkyl or -OC₁₋₆ haloalkyl;
J₂ is selected from -COOH, -NH₂, 3- to 10-membered nitrogen-containing heterocycle, sulfonylurea group or hydroxyl; and
p is an integer from 1 to 10.

In some embodiments, each of B₁ and B₂ is independently a substituted or unsubstituted 5- to 6-membered nitrogen-containing heteroaromatic ring; when being substituted, the 5- to 6-membered nitrogen-containing heteroaromatic ring is substituted by substituent(s) composed of one or more of the following groups: hydrogen, C₁₋₁₀ alkylene, carboxyl, amido, carbamoyl, polyethylene glycol, alkynyl, and azido;
Y₁, Y₂ and Y₃ are each independently selected from CH and N;
Z₂ is absent or is a group composed of one or more of the following functional groups: -NH-, -CH₂-, carbonyl and C₂₋₆ alkynylene;
W₂ is absent or is one or more groups selected from C₁₋₁₀ alkylene, -(CH₂CH₂O)ₚ- and - (OCH₂CH₂)ₚ-;
J₂ is selected from -COOH, -NH₂, 3- to 10-membered nitrogen-containing heterocycle, sulfonylurea group and hydroxyl; and
p is an integer from 1 to 10.

In some embodiments, each of B₁ and B₂ is independently selected from substituted or unsubstituted pyridinyl and pyrimidyl; when being substituted, each of the pyridinyl and the pyrimidyl is substituted by substituent(s) composed of one or more of the following groups: hydrogen, C₁₋₁₀ alkylene, carboxyl, amido, carbamoyl, polyethylene glycol, alkynyl, and azido;
Y₁, Y₂ and Y₃ are each independently selected from CH and N;
Z₂ is absent or is a group composed of one or two of the following functional groups: -NH-and carbonyl;
W₂ is absent or is one or more groups selected from C₁₋₁₀ alkylene, -(CH₂CH₂O)ₚ- and - (OCH₂CH₂)ₚ-;
J₂ is -COOH or -NH₂; and
p is an integer from 1 to 10.

In some embodiments, each of B₁ and B₂ is independently a single bond or a 5- to 6-membered nitrogen-containing heteroaromatic ring; preferably, each of B₁ and B₂ is independently selected from a single bond, oxazole ring, thiazole ring, or pyrimidine ring.

In some embodiments, B₁ is independently a single bond or a 5- to 6-membered nitrogen-containing heteroaromatic ring at each occurrence; preferably, B₁ is independently a single bond or a 5- to 6-membered nitrogen-containing heteroaromatic ring at each occurrence; further preferably, B₁ is independently selected from a single bond, oxazole ring, thiazole ring, or pyrimidine ring at each occurrence.

In some embodiments, Y₁, Y₂ and Y₃ are all N; or Y₁ is CH, Y₂ and Y₃ are both N; or Y₁ is N, Y₂ and Y₃ are both CH; or Y₁, Y₂ and Y₃ are all CH.

In some embodiments, Z₂ is absent or is a group composed of one or more of the following substituted or unsubstituted functional groups: -NH-, -CH₂-, carbonyl or C₂₋₆ alkynylene, and when being substituted, each of the functional groups is substituted by substituent(s) selected from hydrogen, halogen, hydroxyl, -CN, -C₁₋₆ alkyl, -C₁₋₆ haloalkyl, -OC₁₋₆ alkyl, or -OC₁₋₆ haloalkyl.

In some embodiments, Z₂ is absent or is a group composed of one or two of the following functional groups: -NH- and carbonyl.

In some embodiments, Z₂ is absent or is amido group (-C(=O)NH-).

In some embodiments, Z₂ is absent or is carbamoyl.

In some embodiments, Z₂ is absent or is C₂₋₆ alkynylene.

In some embodiments, W₂ is absent or is C₁₋₁₀ alkylene.

In some embodiments, J₂ is -COOH or -NH₂.

In some embodiments, J₂ is -COOH.

In some embodiments, B₁ is independently a single bond or a pyrimidine ring at each occurrence;
Y₁, Y₂ and Y₃ are all CH;
Z₂ is absent or is -C(=O)NH-;
W₂ is absent or is selected from one or more of C₁₋₁₀ alkylene and -(CH₂CH₂O)ₚ-;
J₂ is -COOH;
p is an integer from 1 to 10.

In some embodiments, each of B₁ and B₂ is independently a single bond or a pyrimidine ring;
Y₁, Y₂ and Y₃ are all CH;
Z₂ is absent or is -C(=O)NH-.
W₂ is absent or is selected from one or more of C₁₋₁₀ alkylene and -(CH₂CH₂O)ₚ-;
J₂ is -COOH;
p is an integer from 1 to 10.

In some embodiments, each of B₁ and B₂ is independently a substituted or unsubstituted 5- to 12-membered nitrogen-containing heteroaromatic ring; when being substituted, the 5- to 12-membered nitrogen-containing heteroaromatic ring is substituted by substituent(s) composed of one or more of the following groups: hydrogen, C₁₋₁₀ alkylene, carboxyl, amido, carbamoyl, polyethylene glycol, alkynyl, and azido;
preferably, each of B₁ and B₂ is independently a substituted or unsubstituted 5- to 6-membered nitrogen-containing heteroaromatic ring; when being substituted, the 5- to 6-membered nitrogen-containing heteroaromatic ring is substituted by substituent(s) composed of one or more of the following groups: hydrogen, C₁₋₁₀ alkylene, carboxyl, amido, carbamoyl, polyethylene glycol, alkynyl, and azido;
preferably, each of B₁ and B₂ is independently selected from substituted or unsubstituted pyridinyl and pyrimidyl; when being substituted, the pyridinyl or the pyrimidyl is substituted by substituent(s) composed of one or more of the following groups: hydrogen, C₁₋₁₀ alkylene, carboxyl, amido, carbamoyl, polyethylene glycol, alkynyl, and azido;
preferably, each of B₁ and B₂ is independently pyridinyl or pyrimidyl;
   and/or
Z₂ is absent or is a group composed of one or more of the following functional groups: -NH-, -CH₂- and carbonyl;
preferably, Z₂ is absent or is a group composed of one or more of the following functional groups: -NH- or carbonyl;
preferably, Z₂ is absent or is -C(=O)NH-;
   and/or
J₂ is -COOH or -NH₂;
preferably, J₂ is -COOH;
   and/or
p is an integer from 3 to 8.

In some embodiments, each of B₁ and B₂ is independently a substituted or unsubstituted 5- to 6-membered nitrogen-containing heteroaromatic ring; when being substituted, the 5- to 6-membered nitrogen-containing heteroaromatic ring is substituted by substituent(s) composed of one or more of the following groups: hydrogen, C₁₋₁₀ alkylene, carboxyl, amido, carbamoyl, polyethylene glycol, alkynyl, and azido;
Y₁, Y₂ and Y₃ are each independently selected from CH and N;
Z₂ is absent or is a fragment composed of one or more of the following functional groups: - NH-, -CH₂-, carbonyl or C₂₋₆ alkynylene;
W₂ is absent or is selected from one or more of C₁₋₁₀ alkylene, -(CH₂CH₂O)ₚ- or - (OCH₂CH₂)ₚ-;
J₂ is selected from -COOH, -NH₂, 3- to 10-membered nitrogen-containing heterocycle, sulfonylurea group or hydroxyl; and
p is an integer from 1 to 10;
preferably,
each of B₁ and B₂ is independently selected from substituted or unsubstituted pyridinyl and pyrimidyl; when being substituted, the pyridinyl or the pyrimidyl is substituted by substituent(s) composed of one or more of the following groups: hydrogen, C₁₋₁₀ alkylene, carboxyl, amido, carbamoyl, polyethylene glycol, alkynyl, and azido;
Y₁, Y₂ and Y₃ are each independently selected from CH and N;
Z₂ is absent or is a group composed of one or more of the following functional groups: -NH-or carbonyl;
J₂ is -COOH or -NH₂; and
p is an integer from 1 to 10.

In some embodiments, the compound of formula II has a structure selected from the group consisting of: 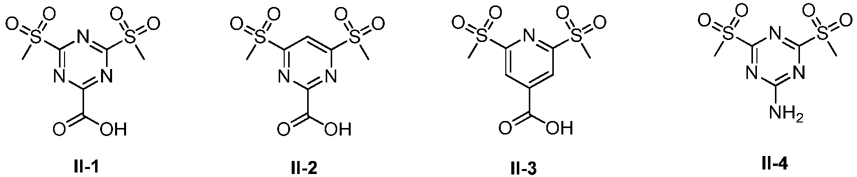 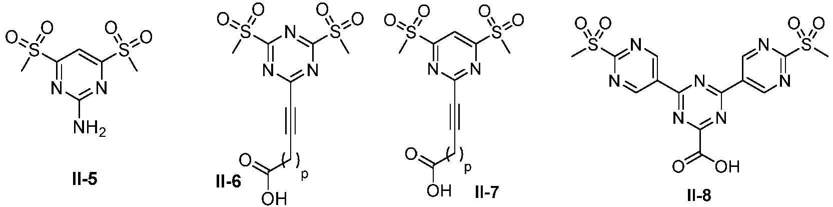 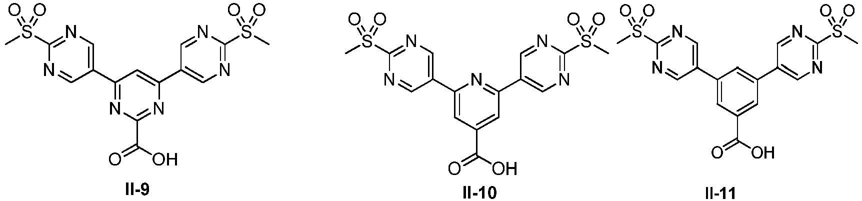 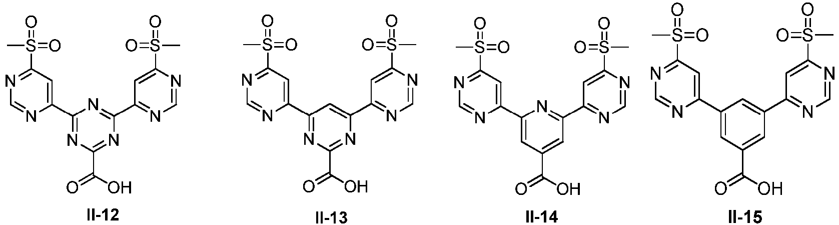 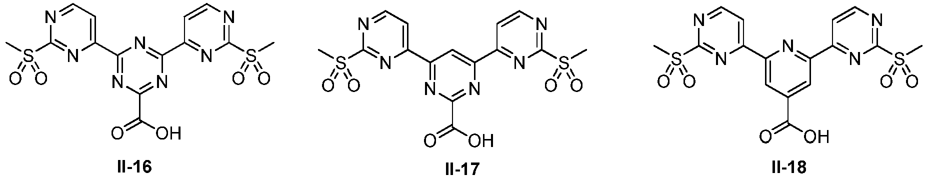 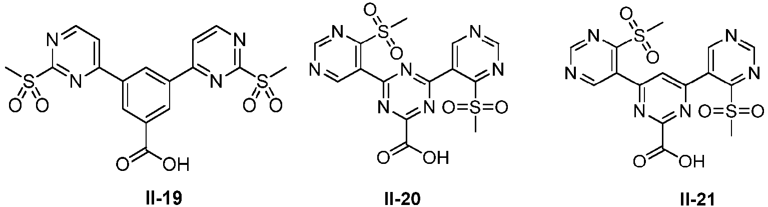 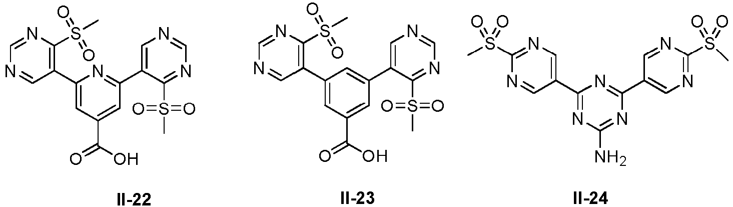 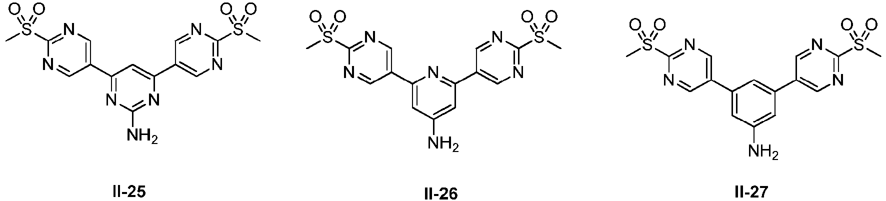 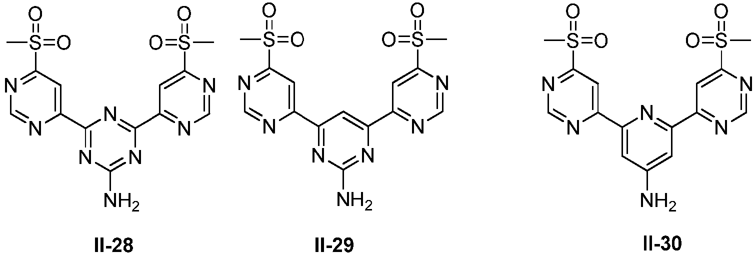 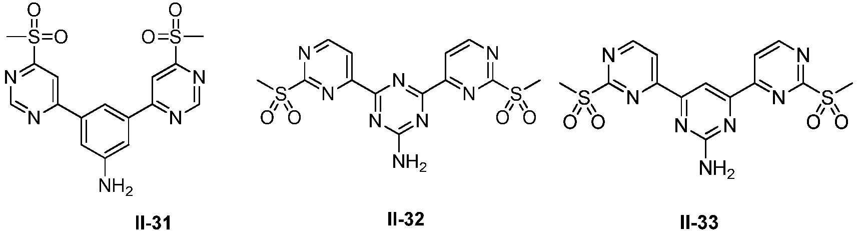 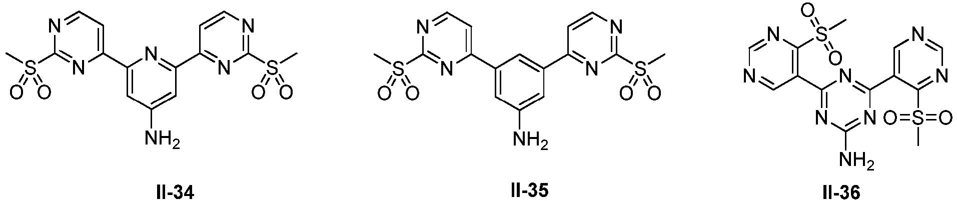 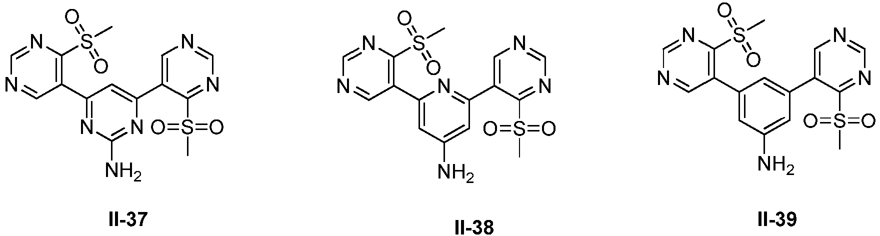 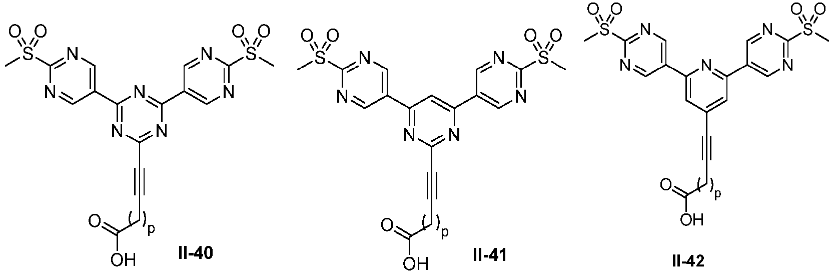 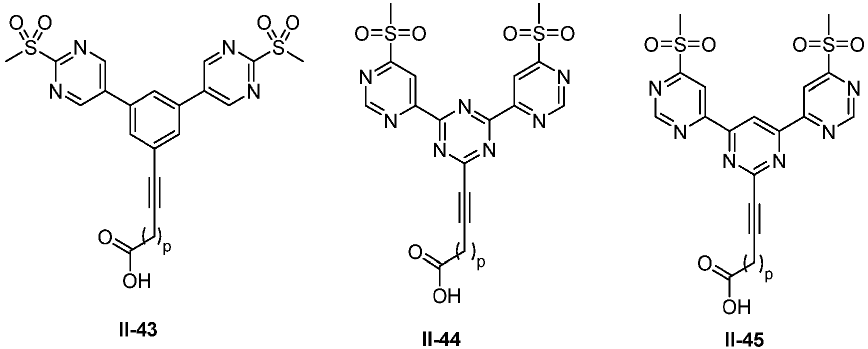 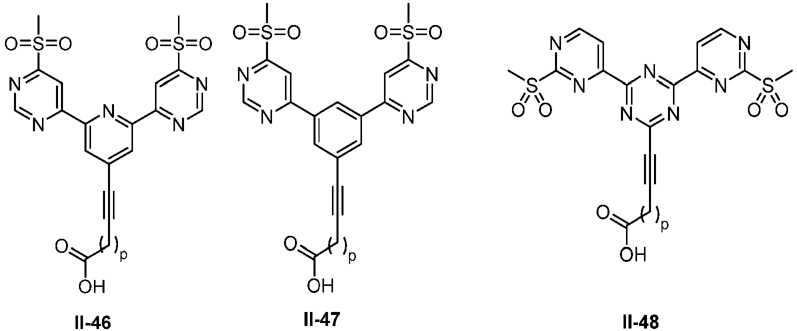 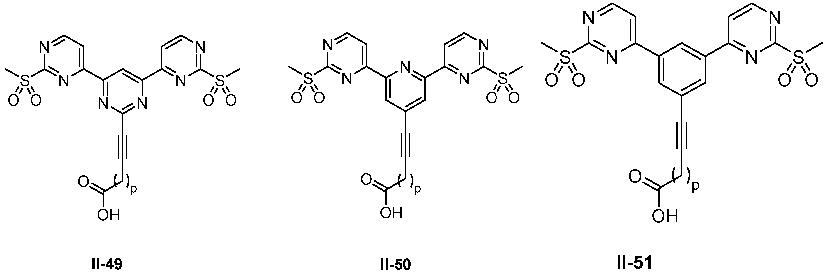 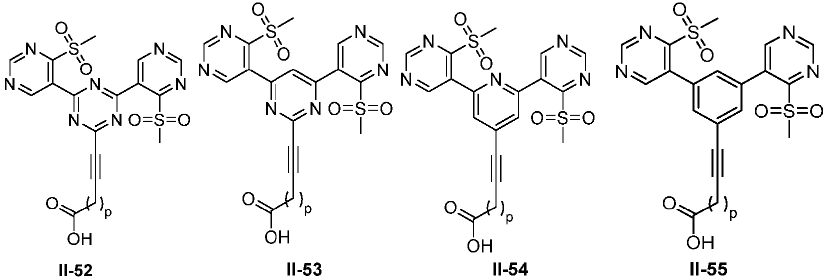 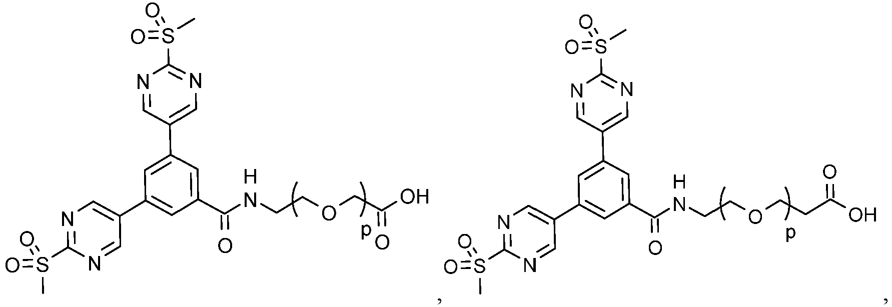 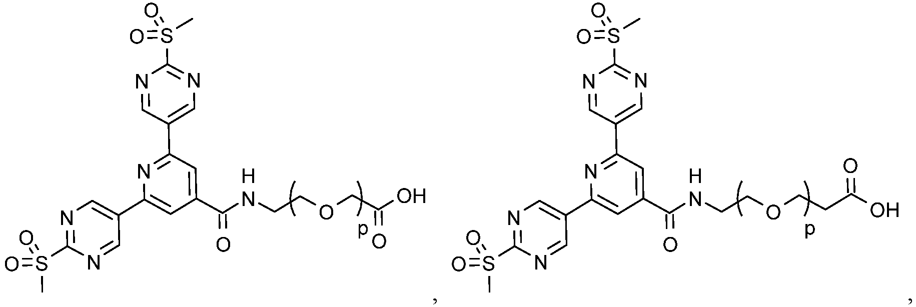 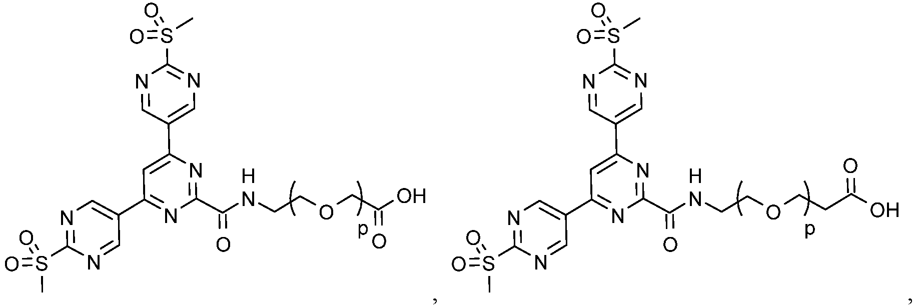 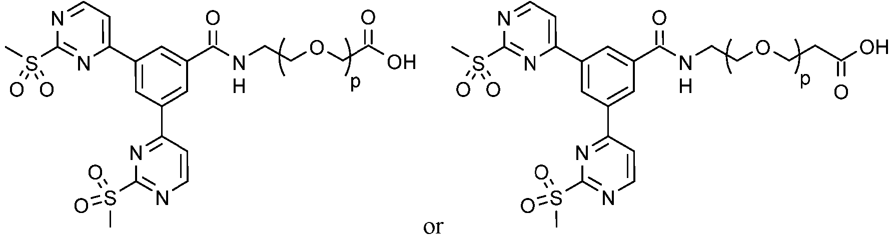 p is an integer from 1 to 10.

In some embodiments, p is an integer from 1 to 8; further preferably, p is an integer from 1 to 5.

In some embodiments, the compound of formula II has a structure selected from the group consisting of:

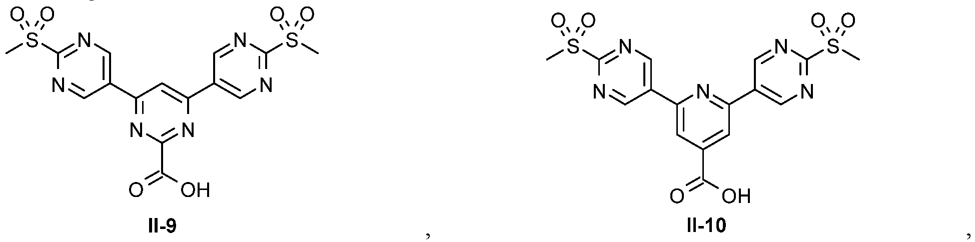

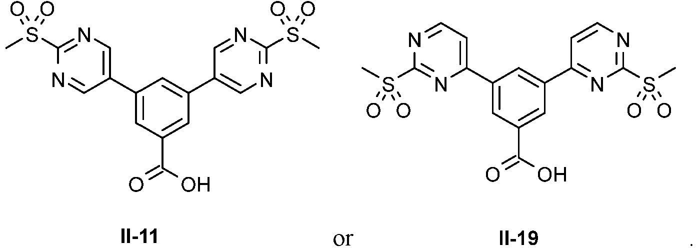

In some embodiments, the compound of formula II has a structure selected from the group consisting of: 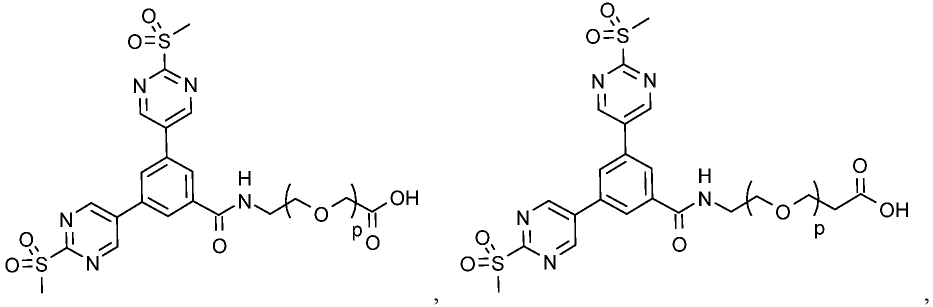 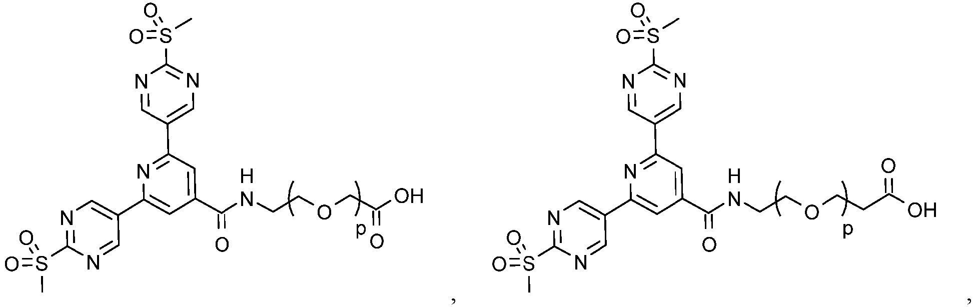 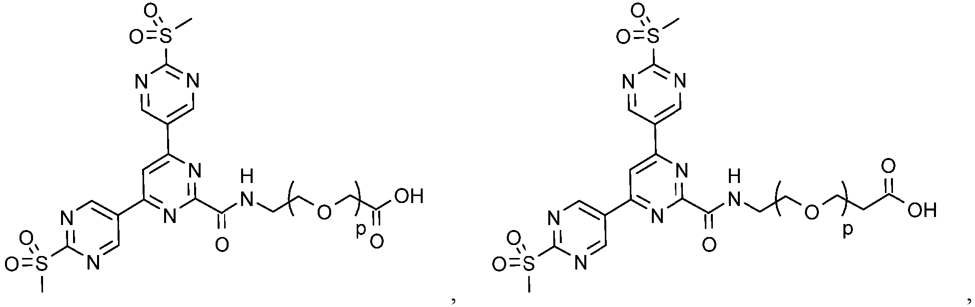 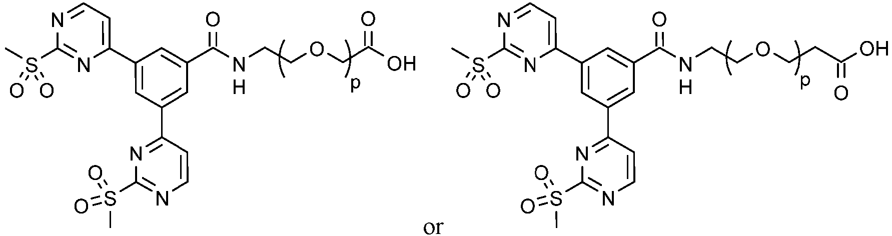 p is an integer from 1 to 10.

In some embodiments, the compound of formula II has a structure selected from the group consisting of:

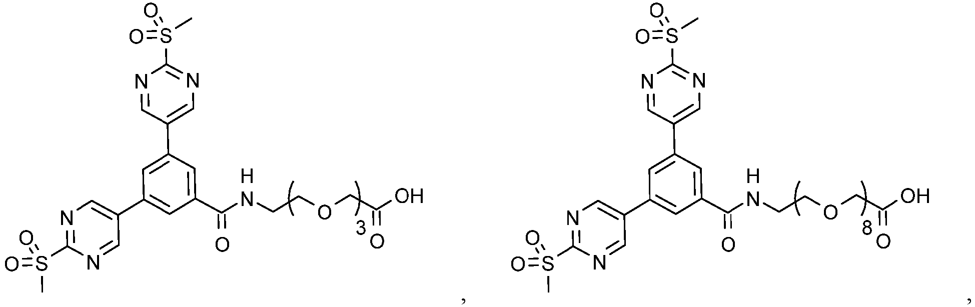

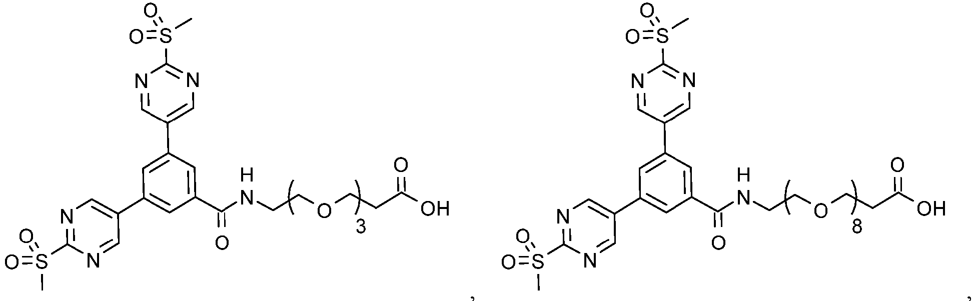

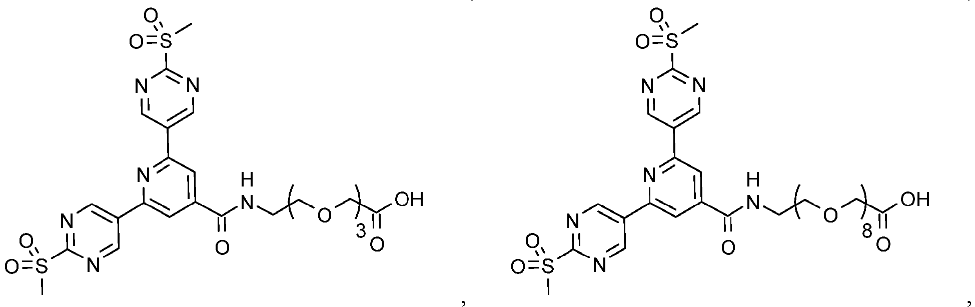

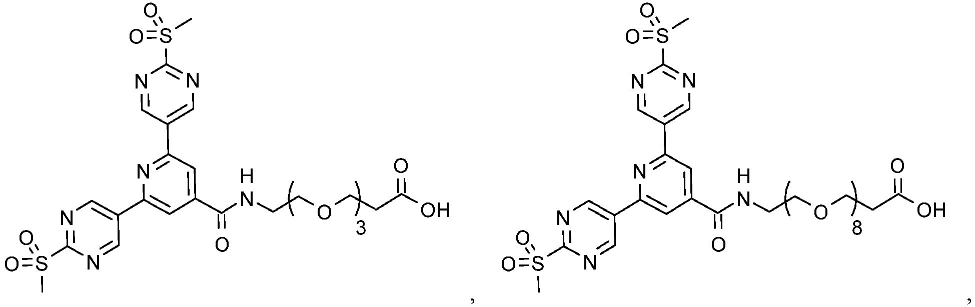

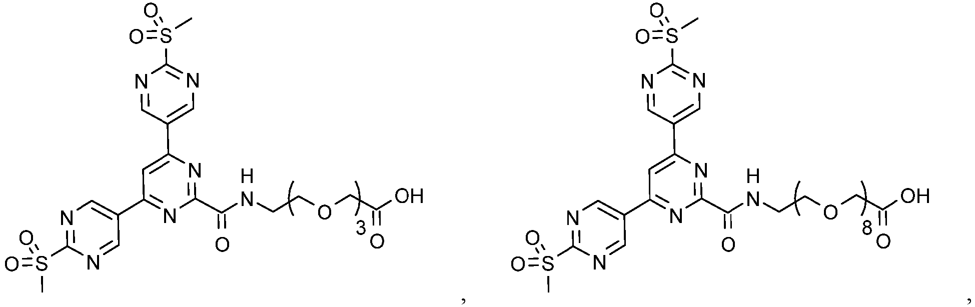

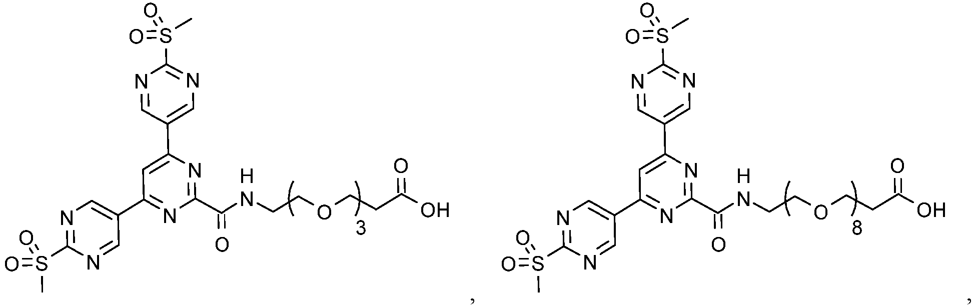

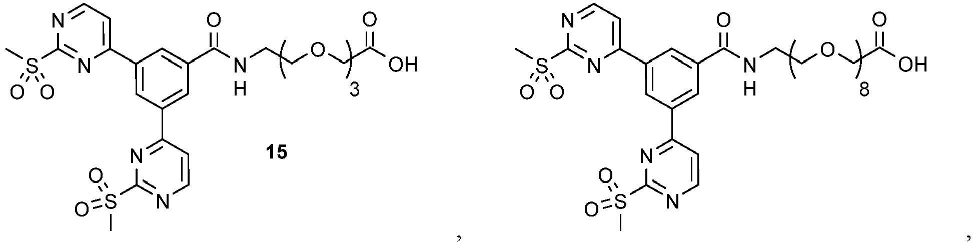

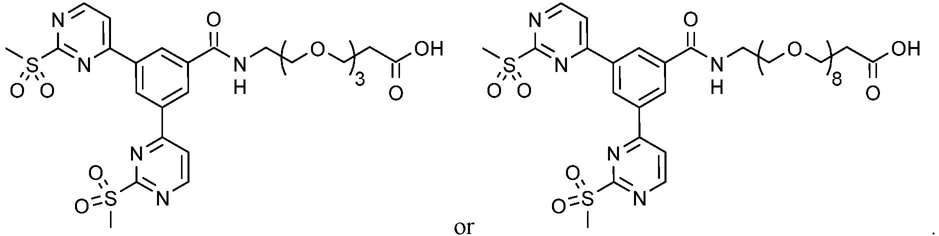

In some embodiments, the compound of formula II has a structure selected from the group consisting of:

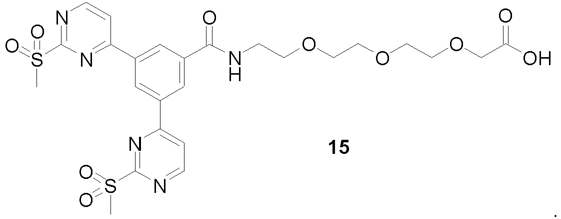

In some embodiments, the present disclosure provides a compound or a pharmaceutically acceptable salt, ester, stereoisomer, tautomer, polymorph, solvate, N-oxide or isotope labeled compound thereof, wherein the compound has a structure of formula IIA: 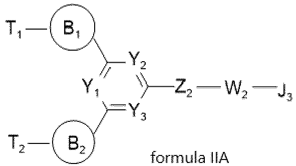
wherein T₁ and T₂ are leaving groups; T₁ and T₂ may be identical or different;
J₃ is selected from -COOR₃, -NH₂, 3- to 10-membered nitrogen-containing heterocycle, sulfonylurea group, alkynyl, 8- to 16-membered alkynyl group-containing cyclic group, azido, tetrazinyl, hydroxylamine, aldehyde, keto, isocyanate, thioisocyanate, maleimide or hydroxyl; R₃ is selected from H, C₁₋₆ alkyl, C₆₋₁₀ aryl, 3- to 8-membered heterocycle and 5- to 10-membered heterocycle; each of the C₁₋₆ alkyl, the C₆₋₁₀ aryl, the 3- to 8-membered heterocycle, and the 5- to 10-membered heterocycle is optionally substituted by one or more substituents selected from hydroxyl, halogen and C₁₋₆ alkoxy;
B₁, B₂, Y₁, Y₂, Y₃, Z₂ and W₂ are as defined in any of aspects described above.

In some embodiments, T₁ and T₂ are each independently selected from halogen, OMs, OTs, OTf, nitro, and the following groups optionally substituted by one or more R₄: alkyl thioether, aryl thioether, heteroaryl thioether, alkyl sulfinyl, aryl sulfinyl, heteroaryl sulfinyl, alkyl sulfonyl, aryl sulfonyl, and heteroaryl sulfonyl; wherein R₄ is independently selected from H (hydrogen), D (deuterium), halogen, CN, nitro, C₁₋₆ alkyl, C₁₋₆ haloalkyl, C₁₋₆ alkoxy, 6- to 10-membered aryl and 5- to 12-membered heteroaryl;
preferably, T₁ and T₂ are each independently selected from the following groups optionally substituted by one or more R₄: alkyl thioether, aryl thioether, heteroaryl thioether, alkyl sulfonyl, aryl sulfonyl, and heteroaryl sulfonyl; wherein R₄ is independently selected from H (hydrogen), D (deuterium), halogen, CN, nitro, C₁₋₆ alkyl, C₁₋₆ haloalkyl, C₁₋₆ alkoxy, 6- to 10-membered aryl and 5- to 12-membered heteroaryl;
preferably, T₁ and T₂ are each independently selected from alkyl thioether and alkyl sulfonyl which are optionally substituted by one or more R₄; R₄ is independently selected from H (hydrogen), D (deuterium), halogen, CN, nitro, C₁₋₆ alkyl, C₁₋₆ haloalkyl, and C₁₋₆ alkoxy;
preferably, T₁ and T₂ are each independently selected from C₁₋₆ alkyl thioether or C₁₋₆ alkyl sulfonyl which are optionally substituted by one or more R₄; R₄ is independently selected from H (hydrogen), D (deuterium), halogen, CN, nitro, C₁₋₆ alkyl, C₁₋₆ haloalkyl, and C₁₋₆ alkoxy;
preferably, T₁ and T₂ are each independently C₁₋₆ alkyl thioether or C₁₋₆ alkyl sulfonyl;
preferably, T₁ and T₂ are each independently -S(O)₂-CH₃ or -S-CH₃.

In some embodiments, J₃ is -COOR₃ or -NH₂; R₃ is selected from H, D, C₁₋₆ alkyl, C₆₋₁₀ aryl, 3- to 8-membered heterocycle and 5- to 10-membered heterocycle; each of the C₁₋₆ alkyl, the C₆₋₁₀ aryl, the 3- to 8-membered heterocycle, and the 5- to 10-membered heterocycle is optionally substituted by one or more substituents selected from hydroxyl, halogen and C₁₋₆ alkoxy;
preferably, J₃ is -COOR₃ or -NH₂; R₃ is selected from H, D and C₁₋₆ alkyl; the C₁₋₆ alkyl is optionally substituted by one or more substituents selected from hydroxyl, halogen and C₁₋₆ alkoxy;
preferably, J₃ is selected from -COOC₁₋₆ alkyl, -COOH, and -NH₂; the C₁₋₆ alkyl is optionally substituted by one or more substituents selected from hydroxyl, halogen and C₁₋₆ alkoxy;
preferably, J₃ is selected from -COOCH₃,-COOH, and -NH₂.

In some embodiments, the compound of formula IIA has a structure selected from the group consisting of:

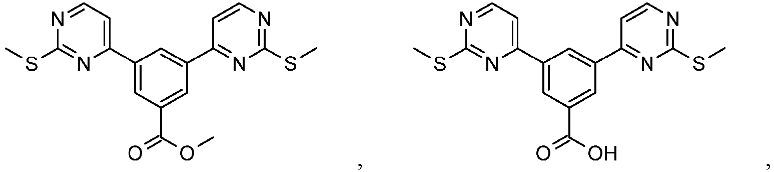

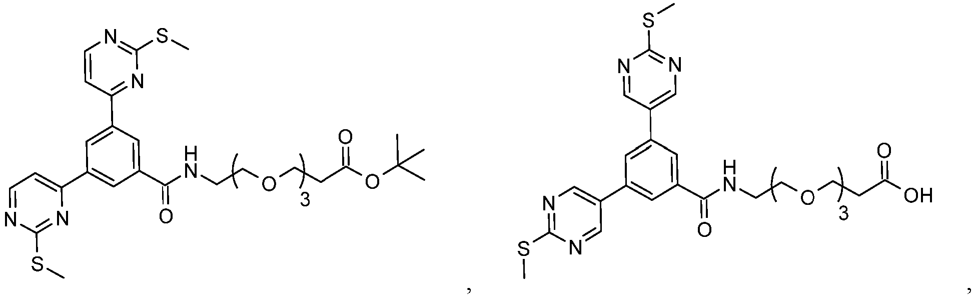

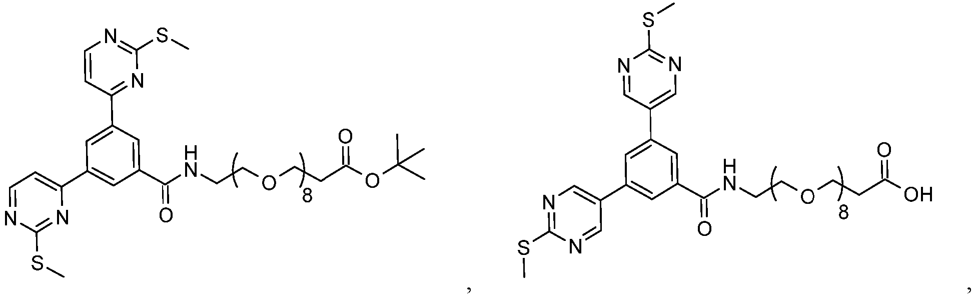

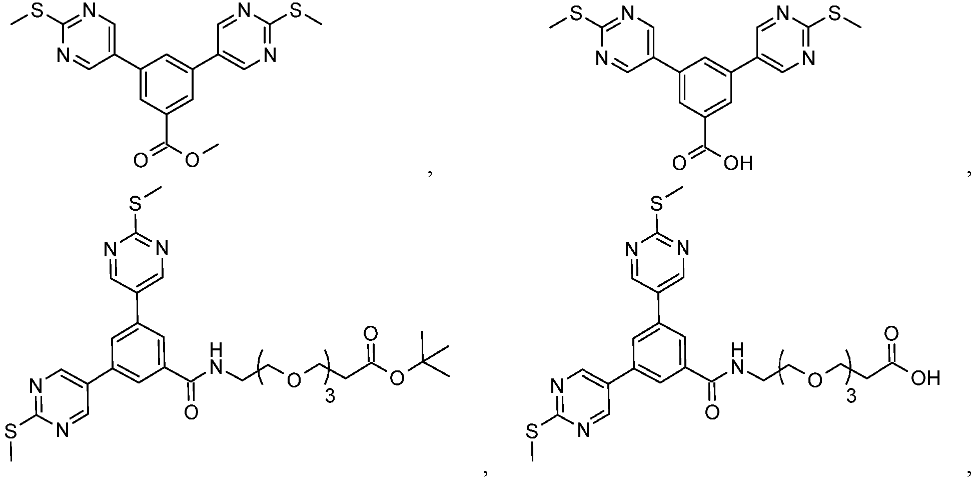

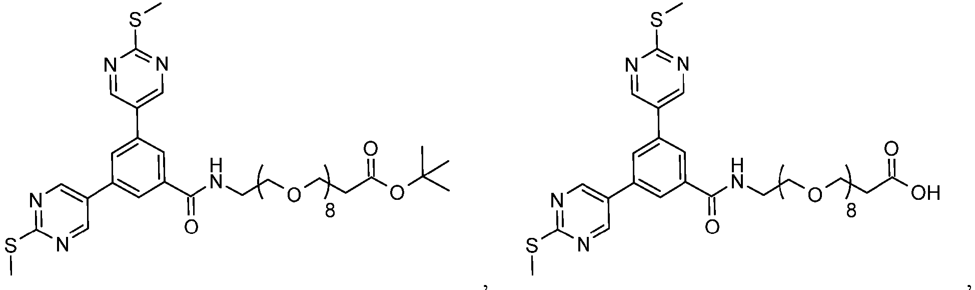

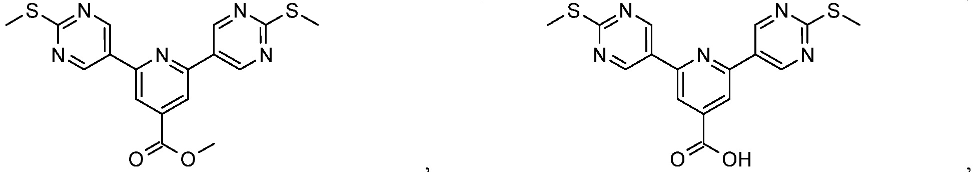

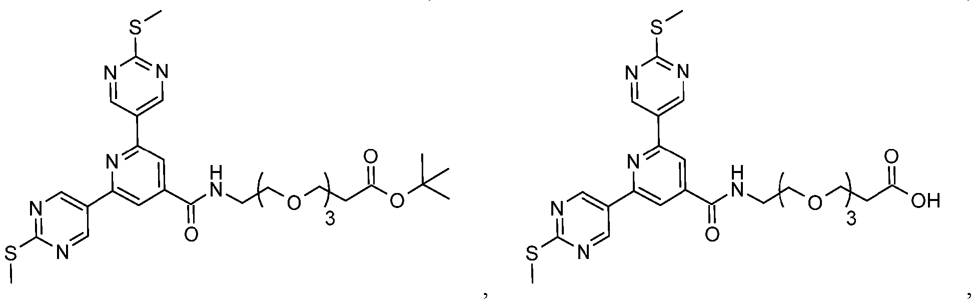

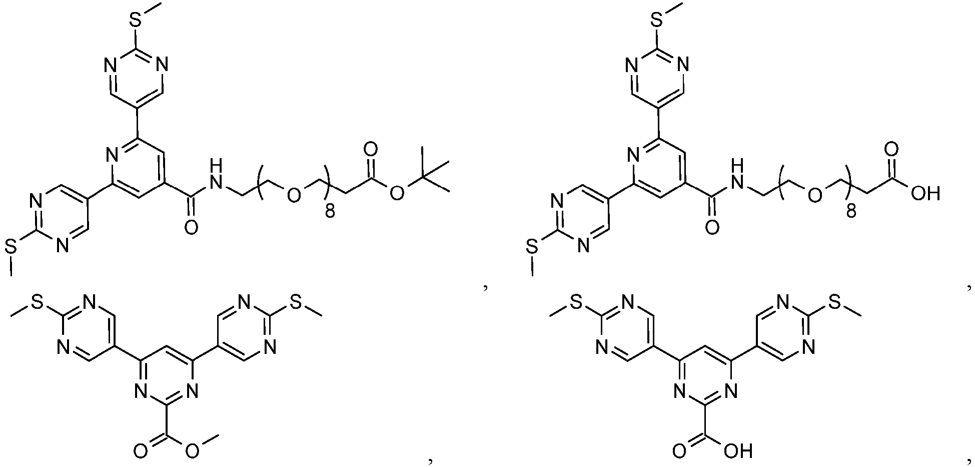

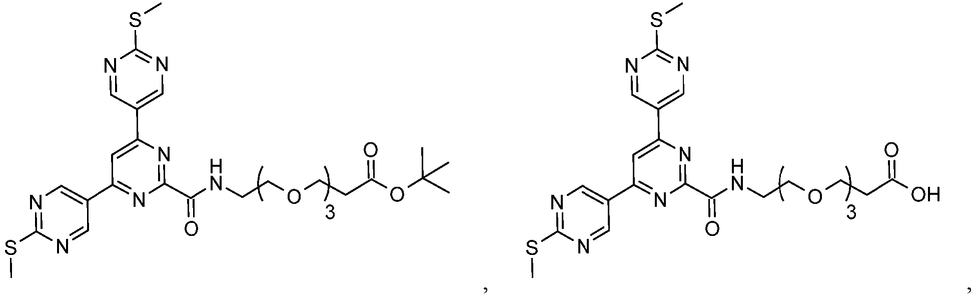

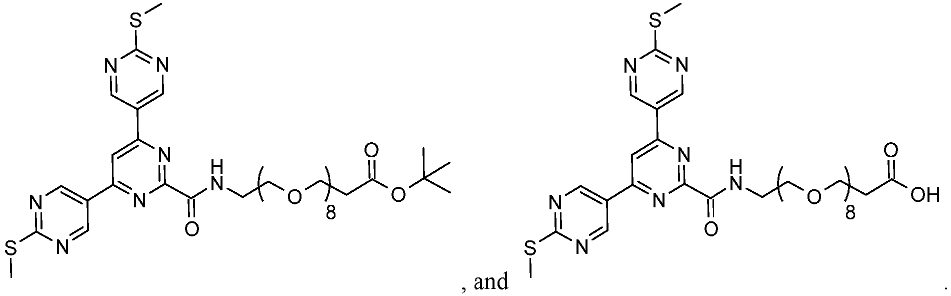

In some embodiments, the present disclosure provides a compound or a pharmaceutically acceptable salt, ester, stereoisomer, tautomer, polymorph, solvate, N-oxide or isotope labeled compound thereof, the compound is selected from the group consisting of:
(1) 3,5-bis(2-(methylthio)pyrimidin-4-yl)benzoic acid;
(2) 3,5-bis(2-(methylsulfonyl)pyrimidin-4-yl)benzoic acid;
(3) tert-butyl 1-(3,5-bis(2-(methylsulfonyl)pyrimidin-4-yl)phenyl)-1-oxo-5,8,11-trioxa-2-azatridecan-13-oleate;
(4) 3,5-bis(2-(methylthio)pyrimidin-5-yl)benzoic acid;
(5) 3,5-bis(2-(methylsulfonyl)pyrimidin-5-yl)benzoic acid;
(6) 1-(3,5-bis(2-(methylsulfonyl)pyrimidin-5-yl)phenyl)-1-oxo-5,8,11-trioxa-2-azatridecan-13-oic acid;
(7) 1-(3,5-bis(2-(methylthio)pyrimidin-5-yl)phenyl)-1-oxo-5,8,11-trioxa-2-azatetradecan-14-oic acid;
(8) 1-(3,5-bis(2-(methylsulfonyl)pyrimidin-5-yl)phenyl)-1-oxo-5,8,11-trioxa-2-azatetradecan-14-oic acid;
(9) 1-(3,5-bis(2-(methylthio)pyrimidin-5-yl)phenyl)-1-oxo-5,8,11,14,17,20,23,26-octaoxa-2-azanonacosan-29-oic acid;
(10) 1-(3,5-bis(2-(methylsulfonyl)pyrimidin-5-yl)phenyl)-1-oxo-5,8,11,14,17,20,23,26-octaoxa-2-azanonacosan-29-oic acid;
(11) 2,6-bis(2-(methylthio)pyrimidin-5-yl)isonicotinic acid;
(12) 1-(2,6-bis(2-(methylthio)pyrimidin-5-yl)pyridin-4-yl)-1-oxo-5,8,11-trioxa-2-azatetradecan-14-oic acid;
(13) 1-(2,6-bis(2-(methylsulfonyl)pyrimidin-5-yl)pyridin-4-yl)-1-oxo-5,8,11-trioxa-2-azatetradecan-14-oic acid;
(14) 1-(2,6-bis(2-(methylthio)pyrimidin-5-yl)pyridin-4-yl)-1-oxo-5,8,11,14,17,20,23,26-octaoxa-2-azanonacosanoic-29-acid;
(15) 1-(2,6-bis(2-(methylsulfonyl)pyrimidin-5-yl)pyridin-4-yl)-1-oxo-5,8,11,14,17,20,23,26-octaoxa-2-azanonacosan-29-oic acid;
(16) 2,2"-bis(methylthio)-[5,4':6',5"-tripyrimidine]-2'-carboxylic acid;
(17) 2,2"-bis(methylsulfonyl)-[5,4':6',5"-tripyrimidine]-2'-carboxylic acid;
(18) 1-(2,2"-bis(methylthio)-[5,4':6',5"-tripyrimidin]-2'-yl)-1-oxo-5,8,11-trioxa-2-azatetradecan-14-oic acid; or
(19) 1-(2,2"-bis(methylsulfonyl)-[5,4':6',5"-tripyrimidin]-2'-yl)-1-oxo-5,8,11-trioxa-2-azatetradecan-14-oic acid.

In a third aspect, the present disclosure provides a compound or a pharmaceutically acceptable salt, ester, stereoisomer, tautomer, polymorph, solvate, N-oxide or isotope labeled compound thereof, wherein the compound has a structure of formula III: 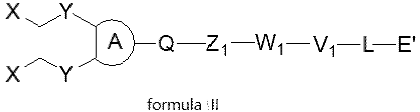 wherein
V₁ is a group formed when J₁ in the compound of formula I in the first aspect of the present disclosure is linked to L; preferably, V₁ is selected from -C(=O)-, -N(R₁)-, -O-, 3- to 10-membered nitrogen-containing heterocycle and sulfonylurea group, wherein R₁ is H, C₁₋₆ alkyl or C₂₋₆ alkoxyalkyl; further preferably, V₁ is -C(O)- or -N(R₁)-, wherein R₁ is H, C₁₋₆ alkyl or C₂₋₆ alkoxyalkyl;
L is a linker that links V₁ and E';
E' is selected from H, -NHCH₂-Lg, -COOH, 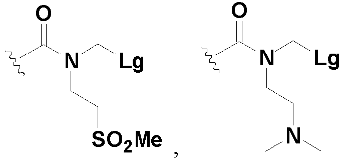 and 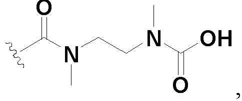 wherein Lg represents a leaving group, such as Cl, Br, I, OMs, OTs, OTf or 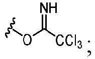
X, Y, A, Q, Z₁ and W₁ are as defined in any of the first aspect described above.

In some embodiments, L is selected from one or more of the following groups: C₁₋₆ alkyl, - N(R₆)-, carbonyl, -O-, Val, Cit, Phe, Lys, D-Val, Leu, Gly, Phe, Ala, Asn, D-Val-Leu-Lys, Ala-Ala, Ala-Lys, Ala-Lys(Ac), Ala-Pro, Gly-Glu, Gly-Gly, Phe-Lys, Phe-Lys(Ac), Val-Ala, Val-Lys, Val-Lys(Ac), Val-Cit, Ala-Ala-Ala, Ala-Ala-Asn, Leu-Ala-Glu, D-Leu-Ala-Glu, Gly-Gly-Arg, Gly-Glu-Gly, Gly-Gly-Gly, Gly-Ser-Lys, Glu-Val-Ala, Glu-Val-Cit, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly, 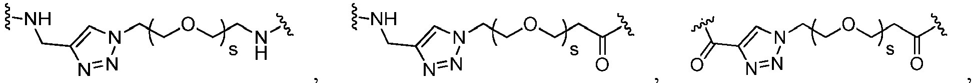 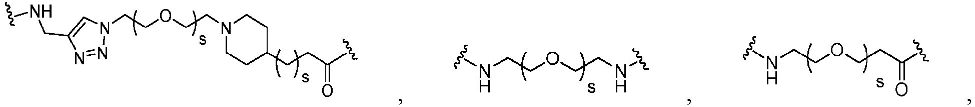 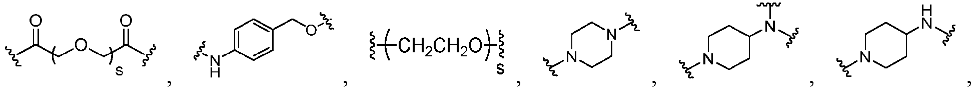 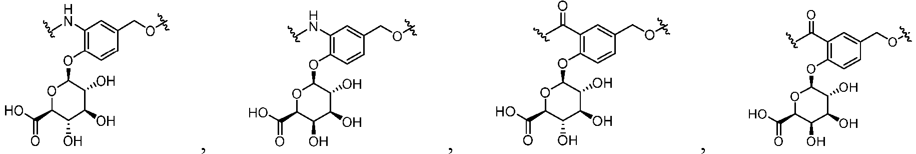 and 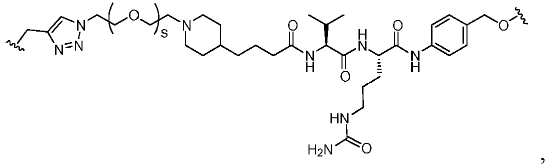 wherein R₆ is H, C₁₋₆ alkyl or C₂₋₆ alkoxyalkyl, and s is an integer from 1 to 10.

In some embodiments, L is selected from one or more of the following groups: Val, Cit, Gly, Phe, Ala, Val-Cit, Val-Ala, Gly-Gly-Phe-Gly,

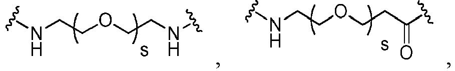

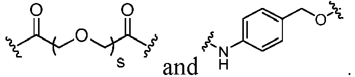

In some embodiments, L is a structure composed of one or more of the followings: 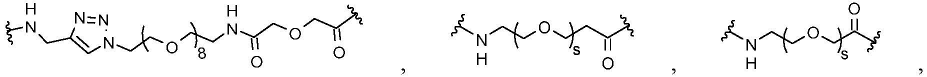 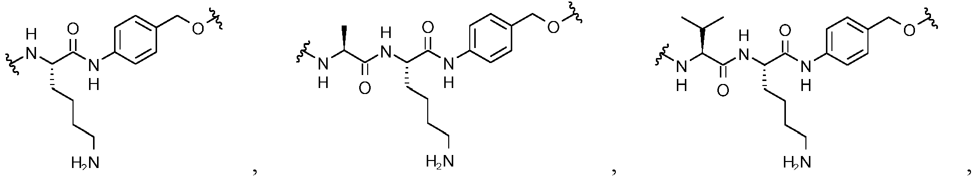 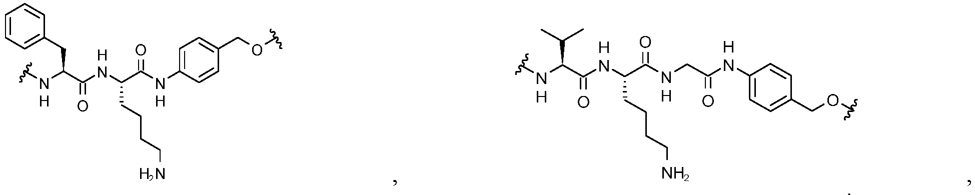 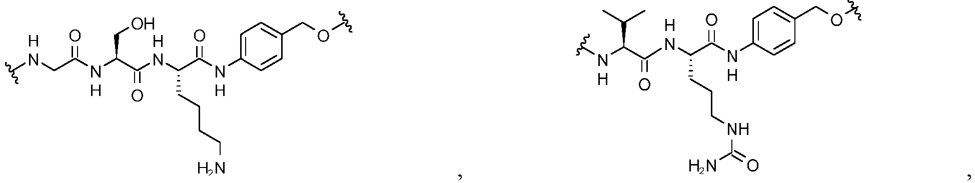 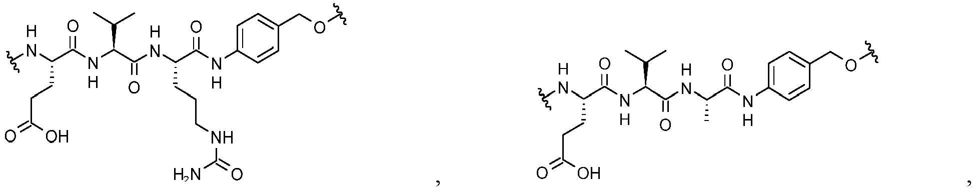 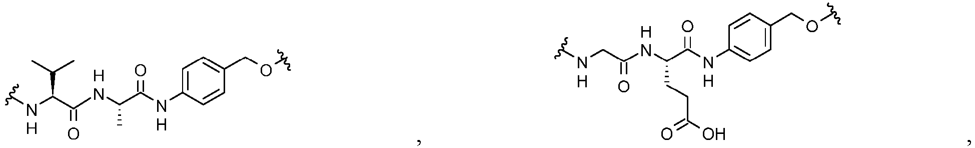 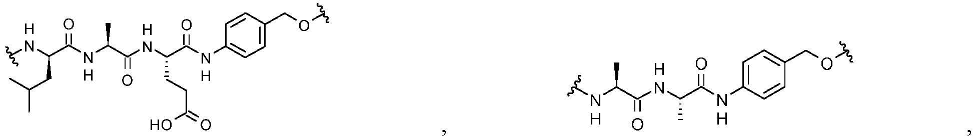 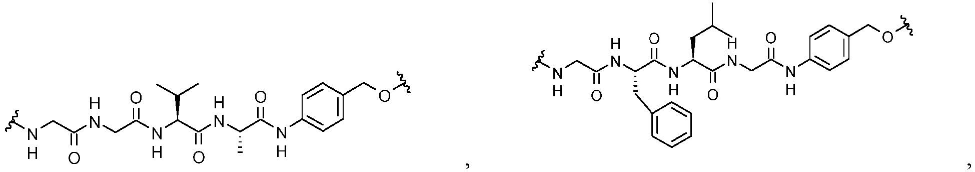 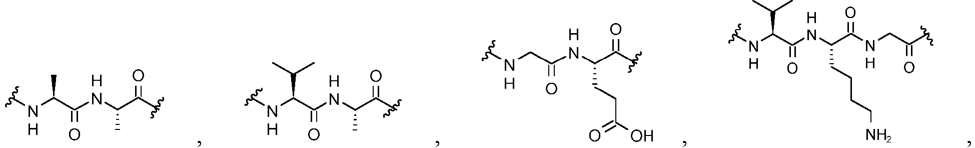 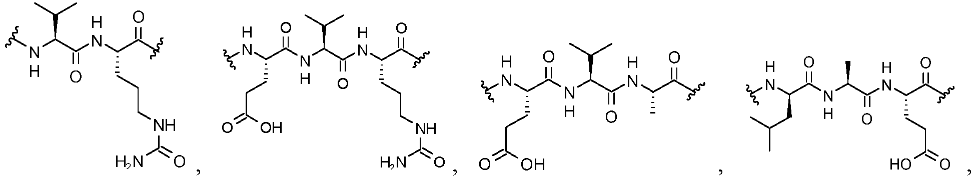 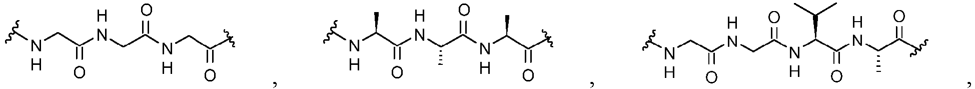 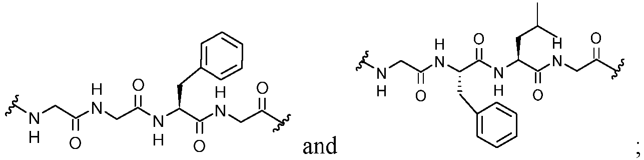 s is an integer from 1 to 10;

In some embodiments, L is selected from: 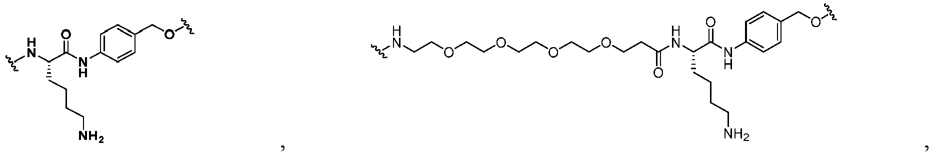 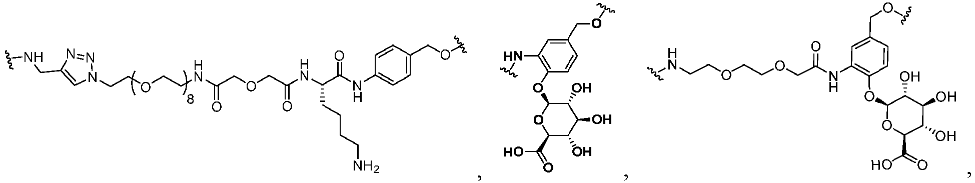 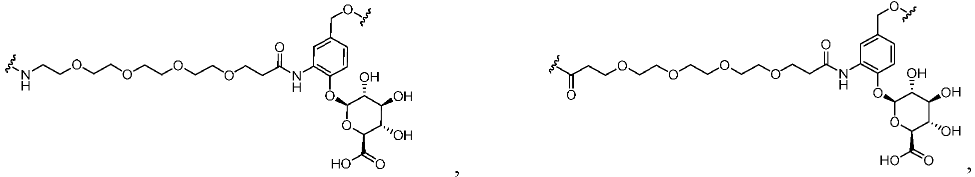 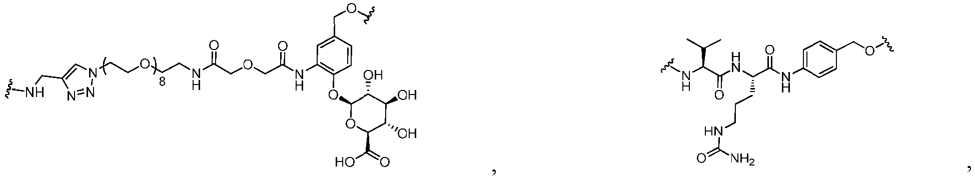 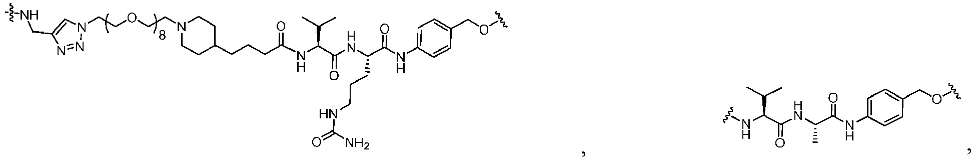 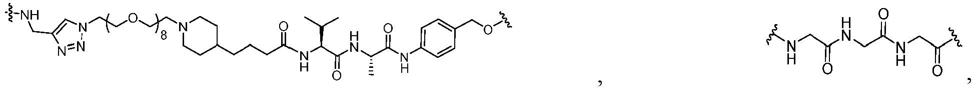 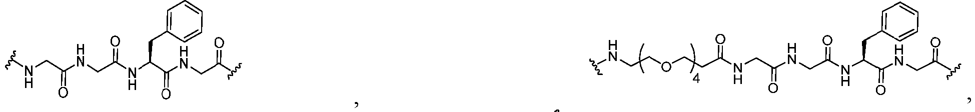 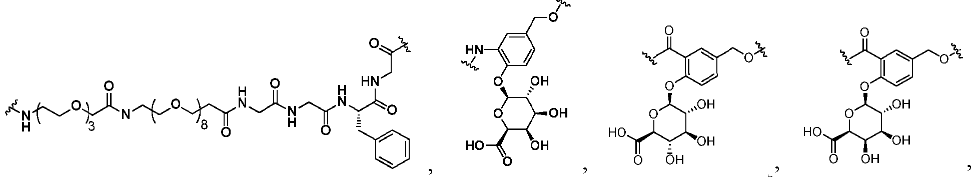 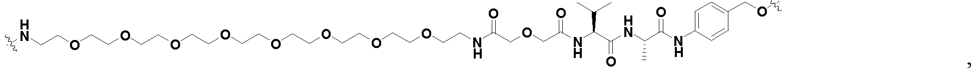 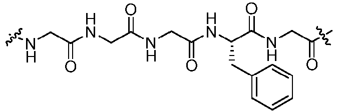 and

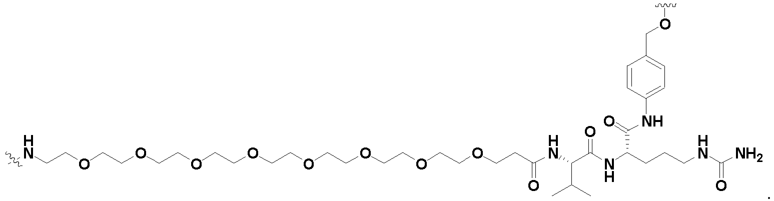

In some embodiments, L is selected from the following structures: 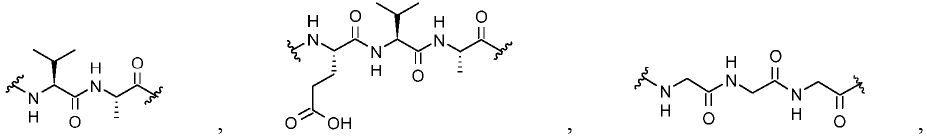 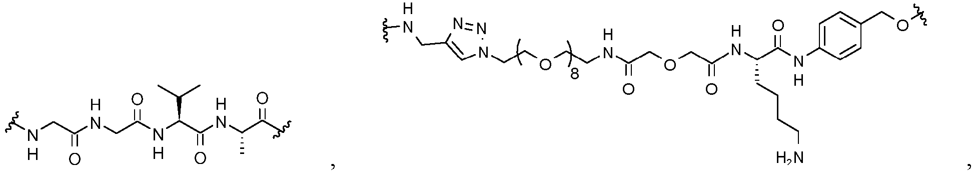 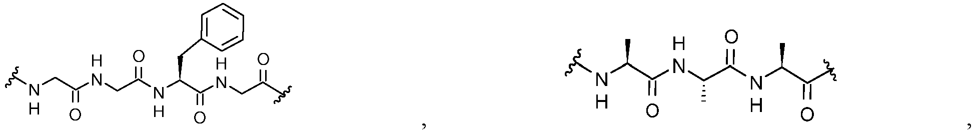 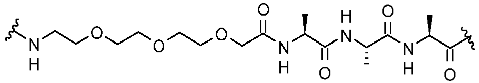 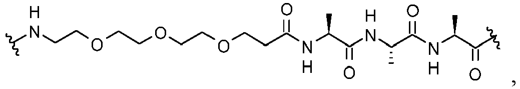 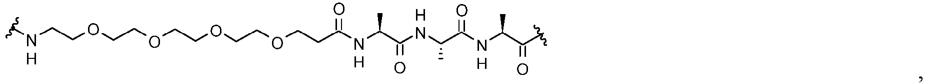 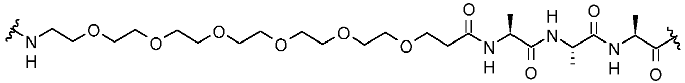 and

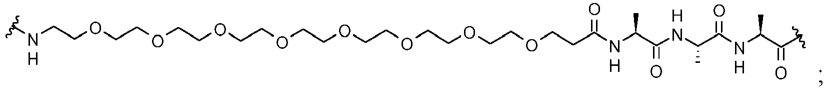

In some embodiments, L is selected from: 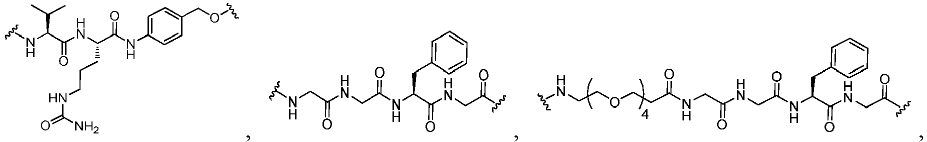 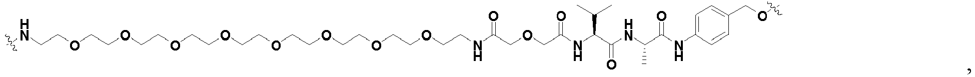 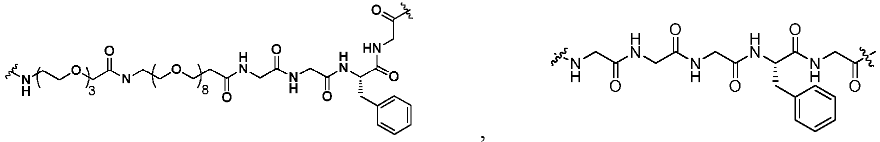 and

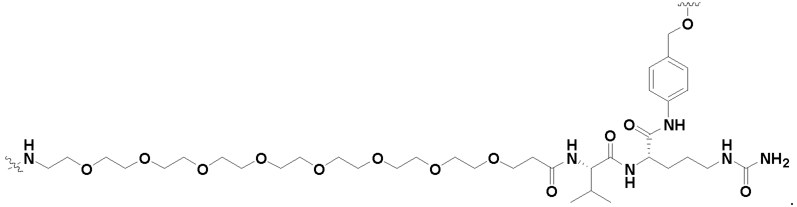

In a fourth aspect, the present disclosure provides a compound or a pharmaceutically acceptable salt, ester, stereoisomer, tautomer, polymorph, solvate, N-oxide or isotope labeled compound thereof, wherein the compound has a structure of formula IV: 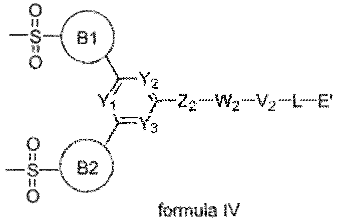 wherein
V₂ is a group formed when J₂ in the compound of formula II in the second aspect of the present disclosure is linked to L; preferably, V₂ is selected from -C(=O)-, -N(R₂)-, -O-, 3- to 10-membered nitrogen-containing heterocycle and sulfonylurea group, wherein R₂ is H, C₁₋₆ alkyl or C₂₋₆ alkoxyalkyl; further preferably, V₂ is -C(O)- or -N(R₂)-, wherein R₂ is H, C₁₋₆ alkyl or C₂₋₆ alkoxyalkyl;
B₁, B₂, Y₁, Y₂, Y₃, Z₂ and W₂ are as defined in any of the second aspect described above.
L and E' are as defined in any of the third aspect described above.

In a fifth aspect, the present disclosure provides a compound or a pharmaceutically acceptable salt, ester, stereoisomer, tautomer, polymorph, solvate, N-oxide or isotope labeled compound thereof, wherein the compound has a structure of formula V: 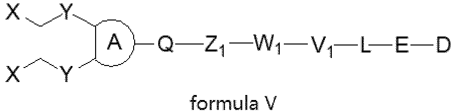 wherein
E is selected from a single bond, -NH-CH₂-, 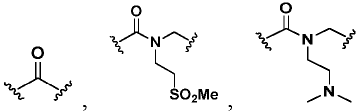 and 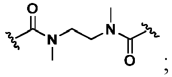
D is a fragment of a bioactive molecule (e.g., a cytotoxic drug);
X, Y, A, Q, Z₁ and W₁ are as defined in any of the first aspect described above;
V₁ and L are as defined in any of the third aspect described above.

In some embodiments, the bioactive molecule is selected from the group consisting of: a tubulin inhibitor, a DNA intercalator, a DNA topoisomerase inhibitor, and an RNA polymerase inhibitor.

In some embodiments, the bioactive molecule is selected from the group consisting of: a tubulin inhibitor such as an auristatin compound and a maytansinoid compound; a DNA intercalator, such as pyrrole benzodiazepine (PBD); a DNA topoisomerase inhibitor, for example, a topoisomerase I inhibitor (such as camptothecin, hydroxycamptothecin, 9-amino-camptothecin, SN-38, irinotecan, Topotecan, Belotecan, Rubitecan) or a topoisomerase II inhibitor (such as adriamycin, doxorubicin, PNU-159682, duocarmycin, daunorubicin, mitoxantrone, podophyllotoxin or etoposide); a RNA polymerase inhibitor including α-amanitin, etc.; and pharmaceutically acceptable salts, esters and analogues thereof.

In some embodiments, the bioactive molecule is selected from: a topoisomerase I inhibitor (such as camptothecin, hydroxycamptothecin, 9-amino-camptothecin, SN-38, irinotecan, Topotecan, Belotecan, Rubitecan), MMAE and a MMAE derivative.

In some embodiments, the bioactive molecule is selected from: MMAE and a MMAE derivative.

In some embodiments, the D is selected from:

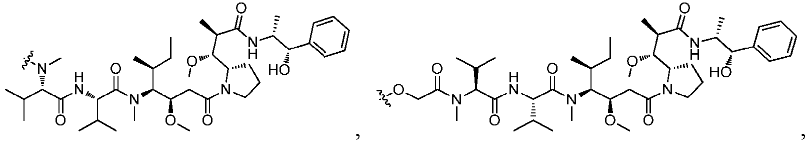

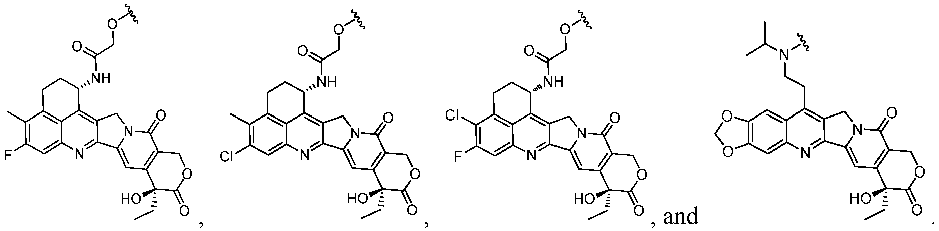

In some embodiments, the compound of formula V is selected from:

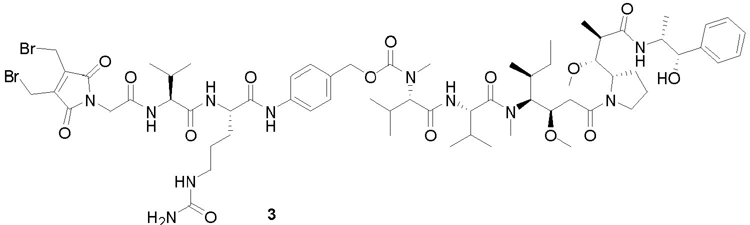

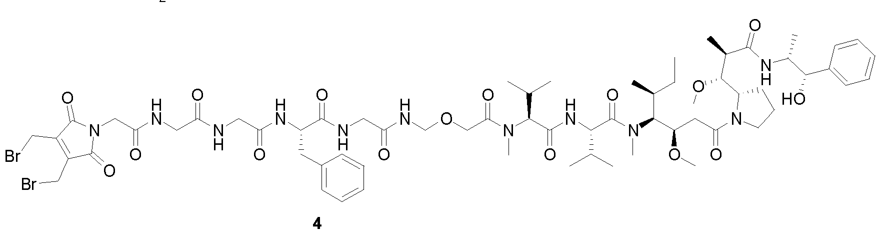

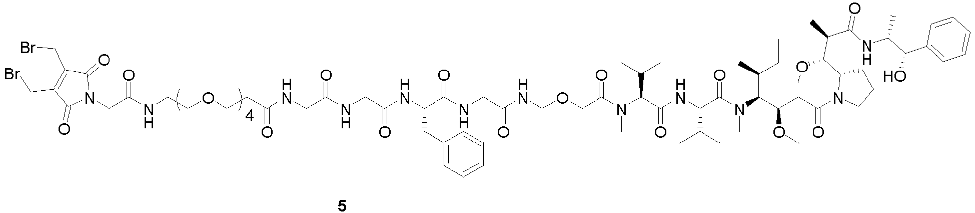

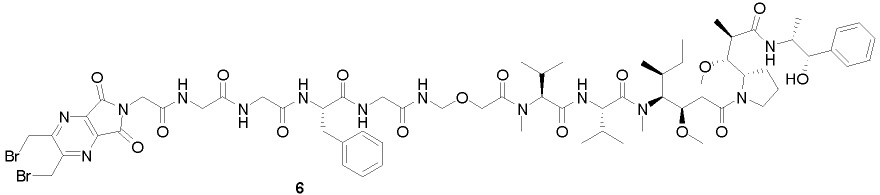

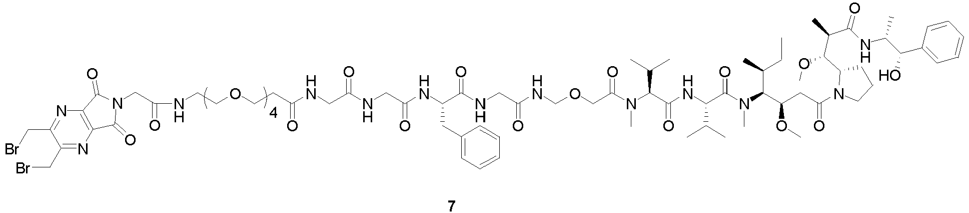

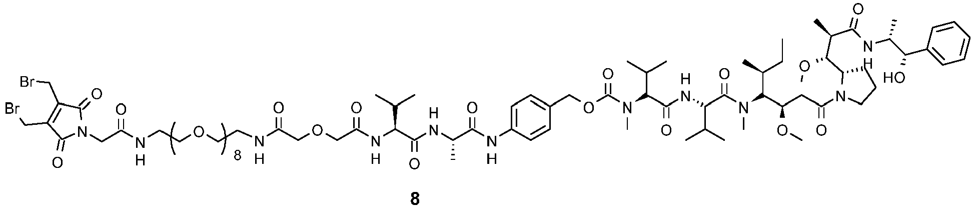

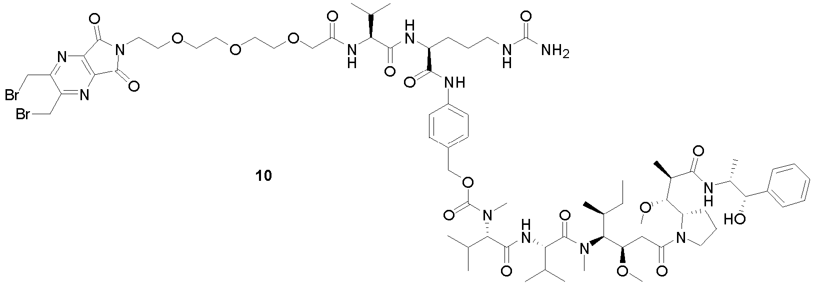

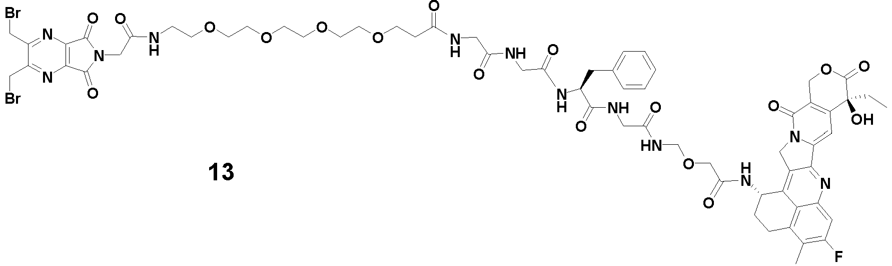

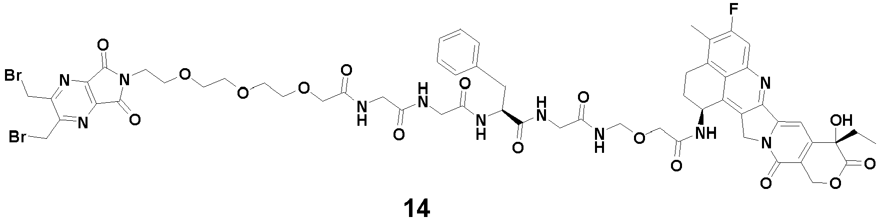

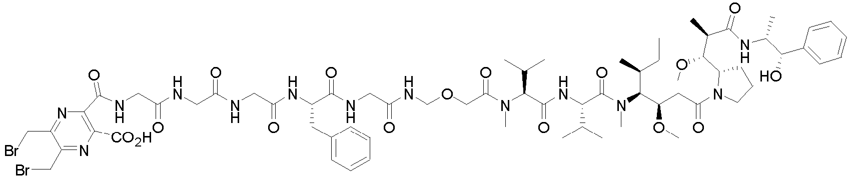

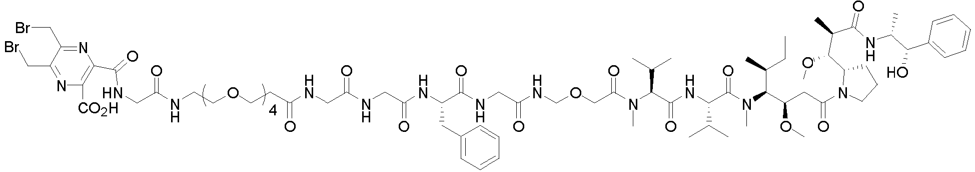

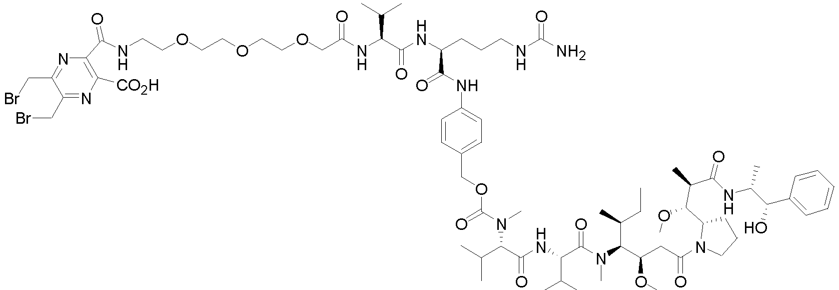

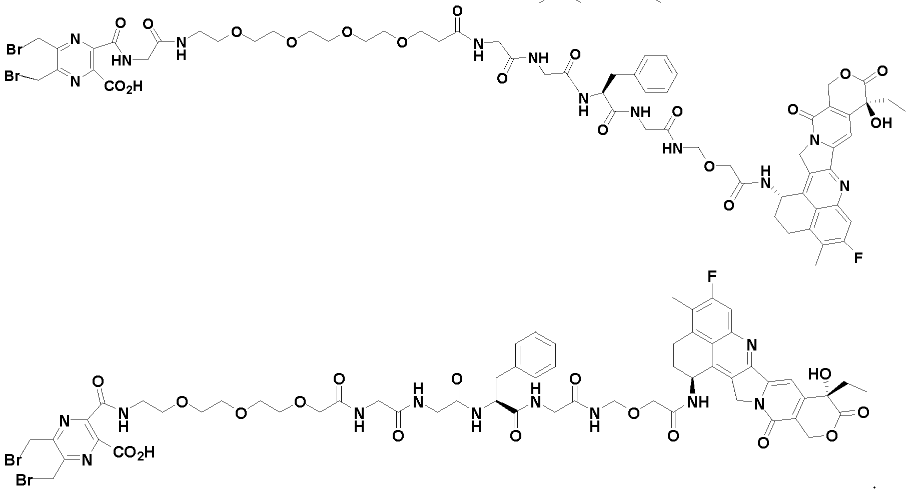

In a sixth aspect, the present disclosure provides a compound or a pharmaceutically acceptable salt, ester, stereoisomer, tautomer, polymorph, solvate, N-oxide or isotope labeled compound thereof, wherein the compound has a structure of formula VI: 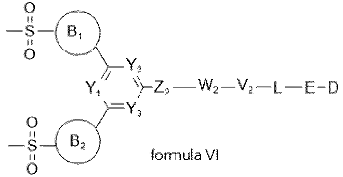
B₁, B₂, Y₁, Y₂, Y₃, Z₂ and W₂ are as defined in any item of the second aspect described above;
L is as defined in any item of the third aspect described above;
V₂ is as defined in any item of the fourth aspect described above;
E and D are as defined in any item of the fifth aspect described above.

In some embodiments, the compound of formula VI is selected from:

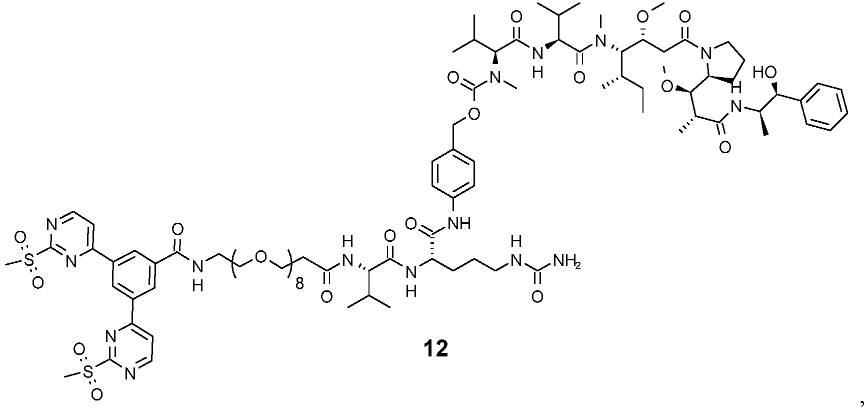

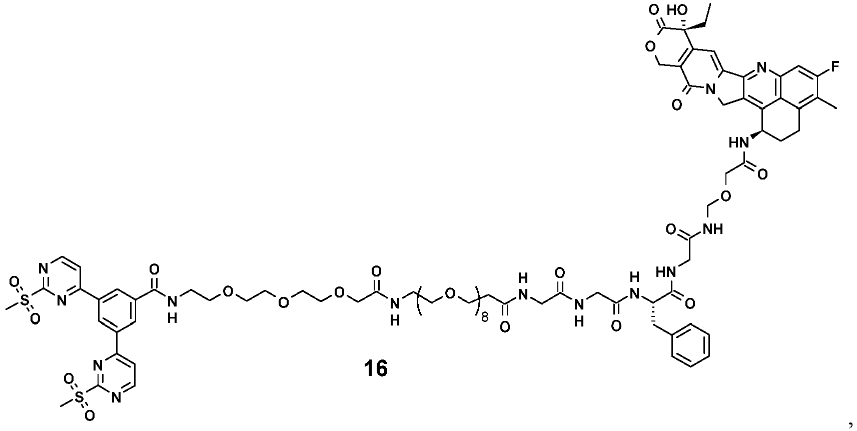

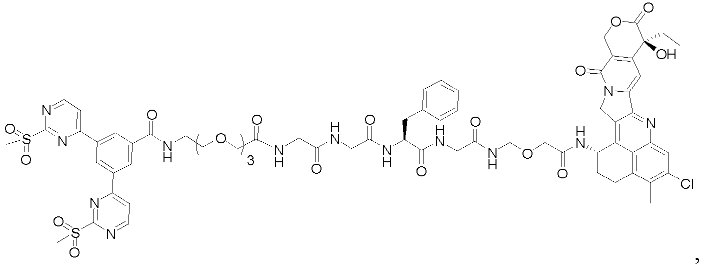

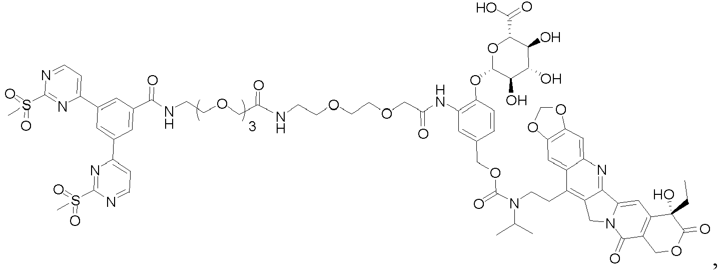

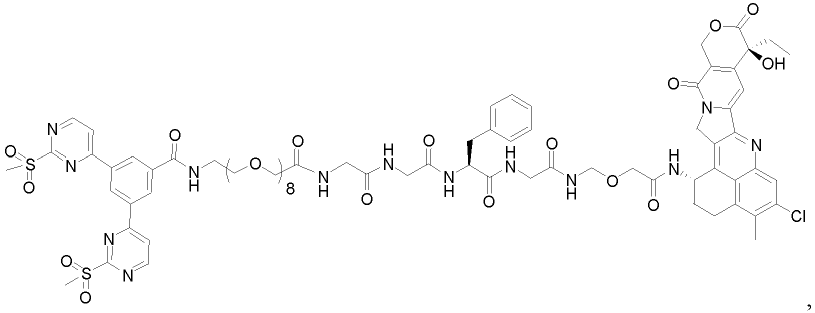

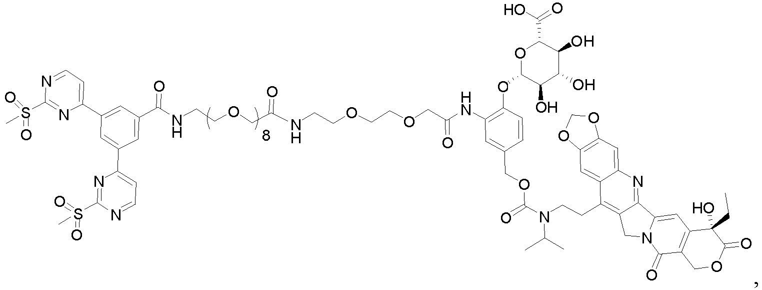

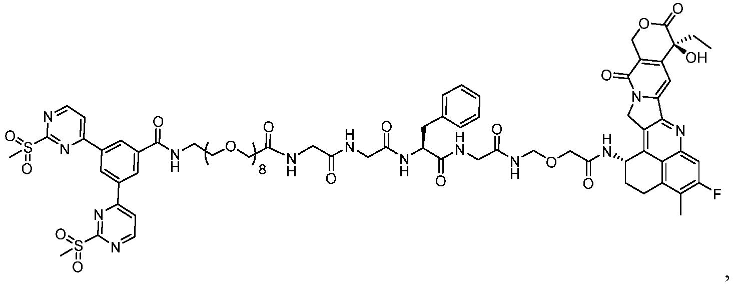

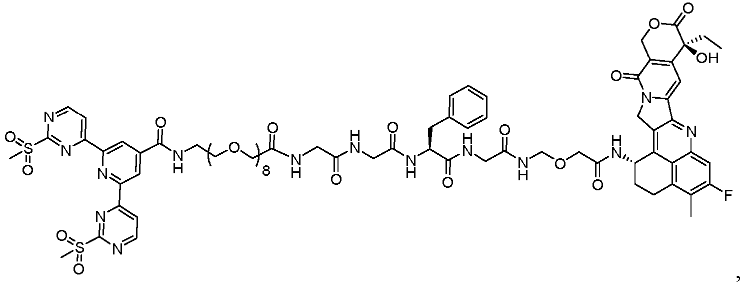

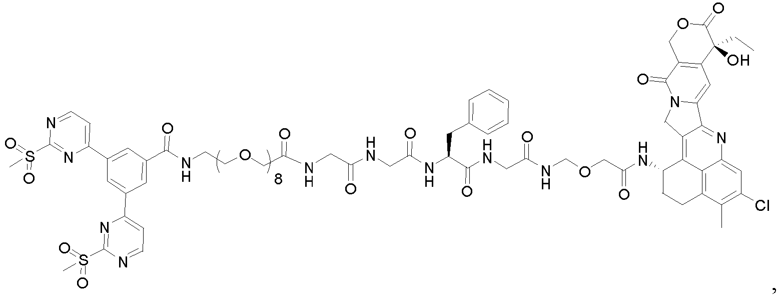

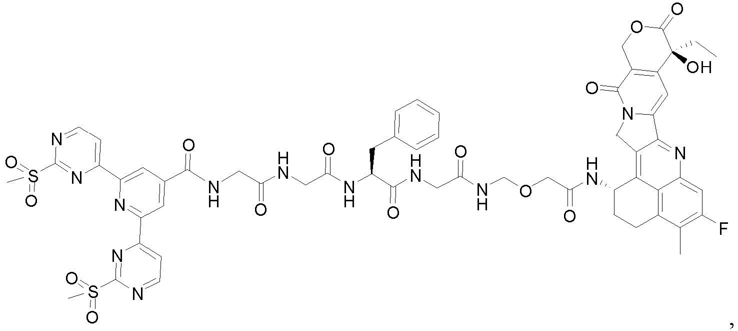

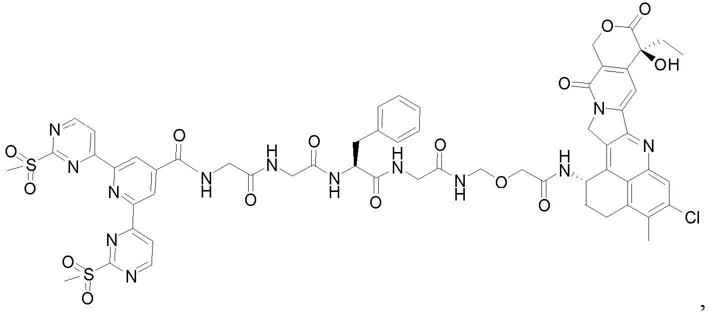

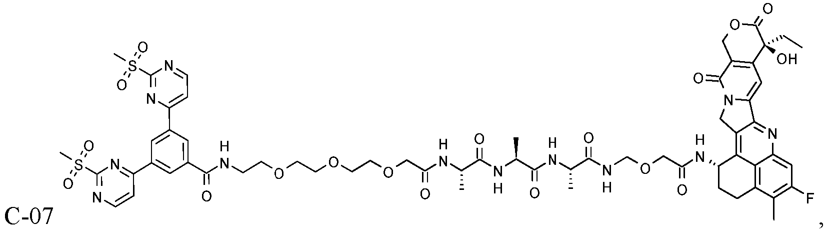

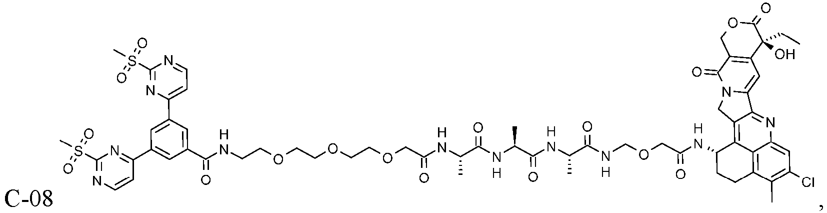

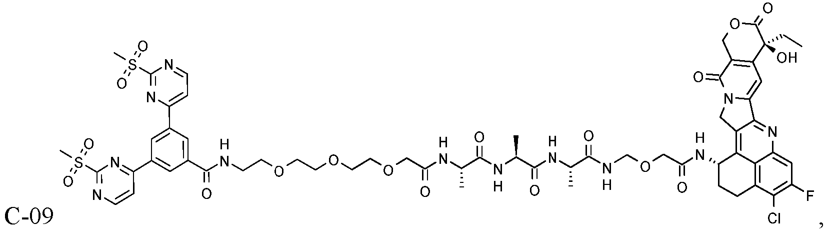

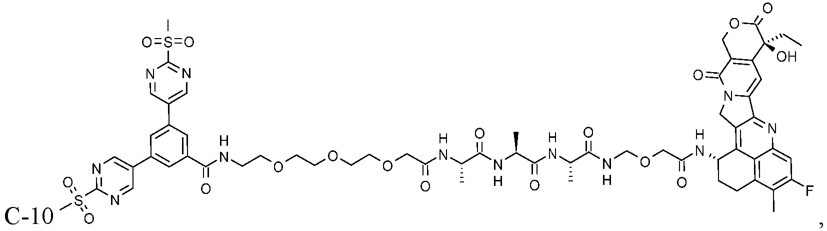

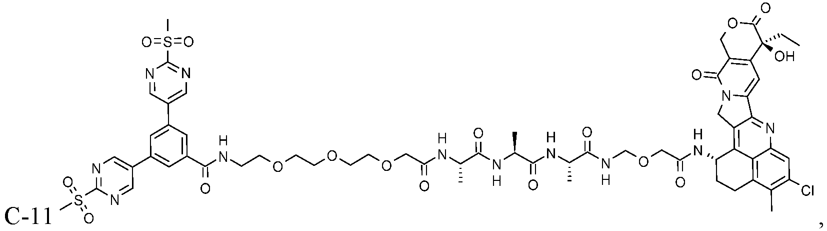

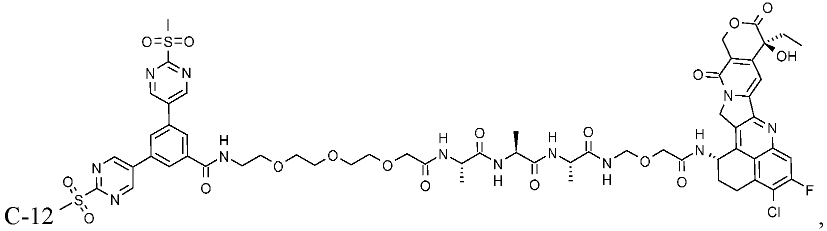

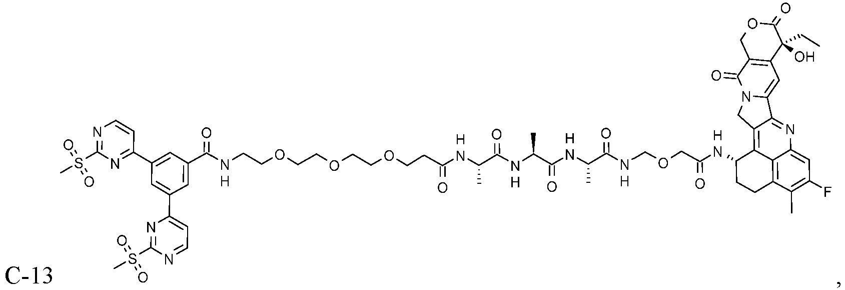

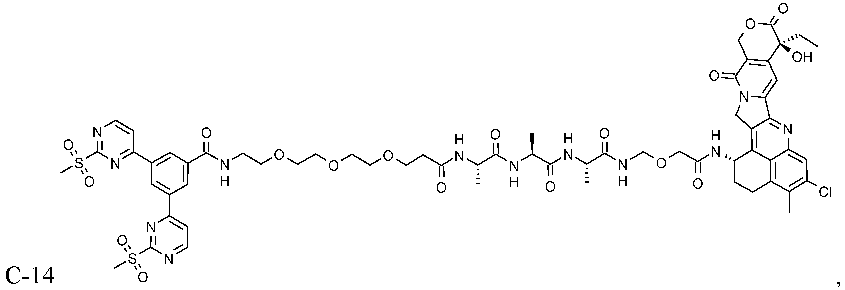

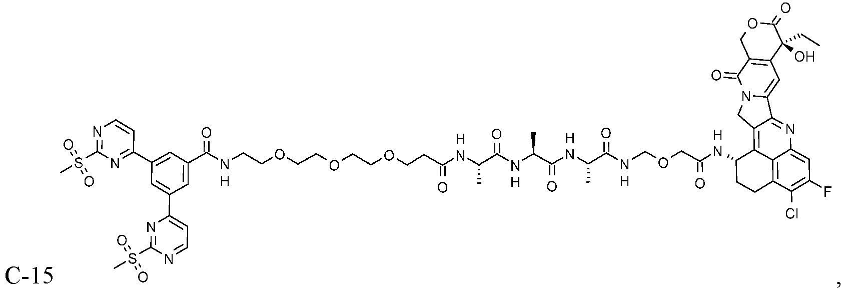

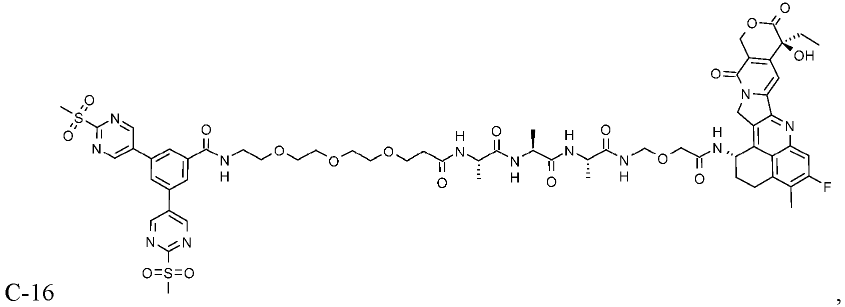

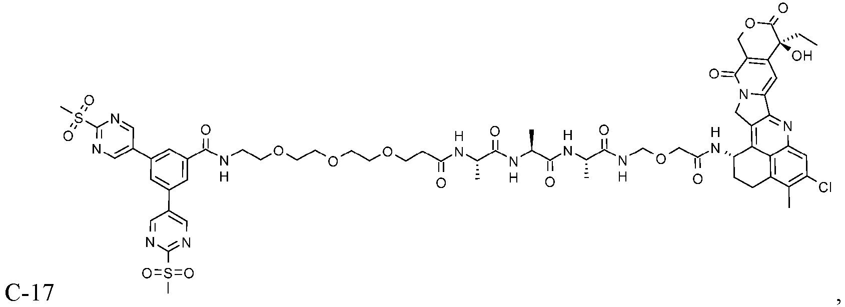

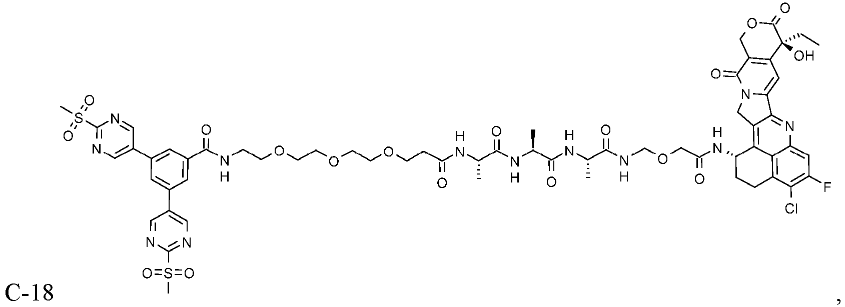

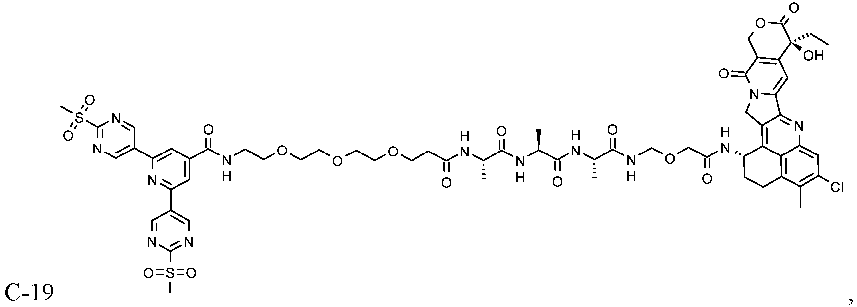

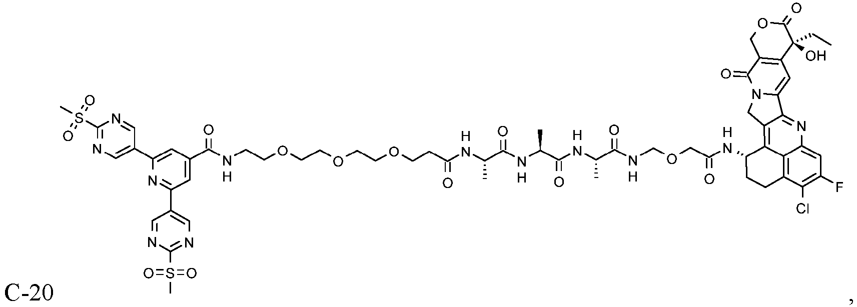

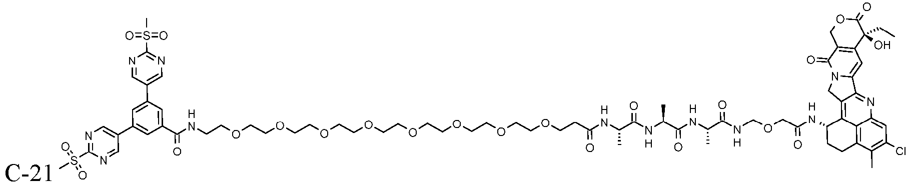

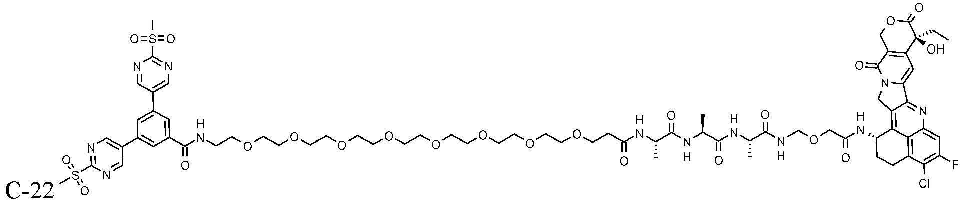

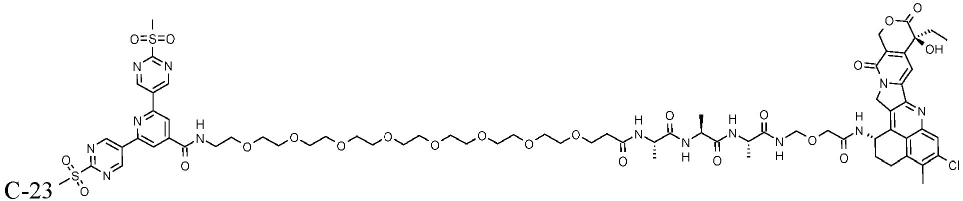

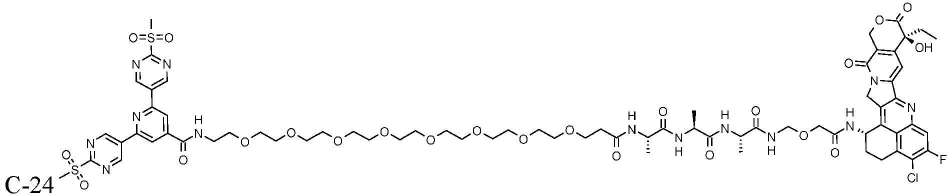

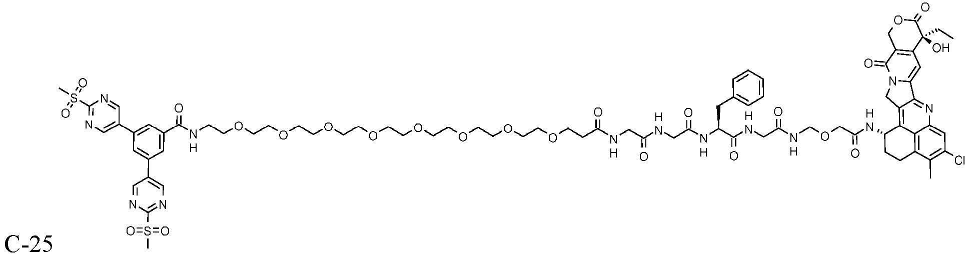

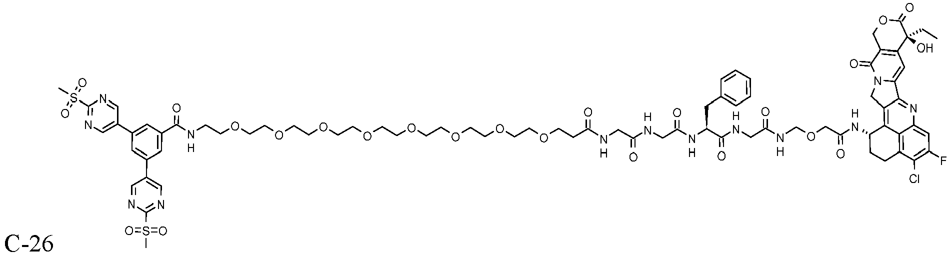

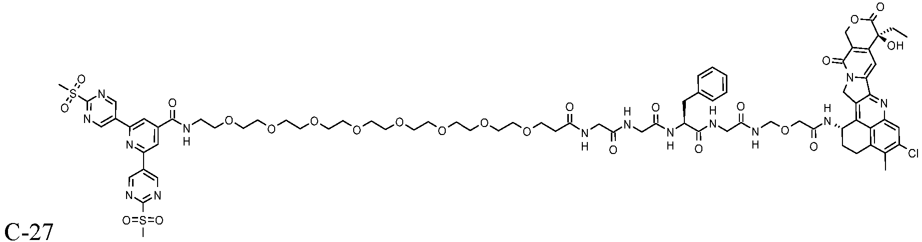

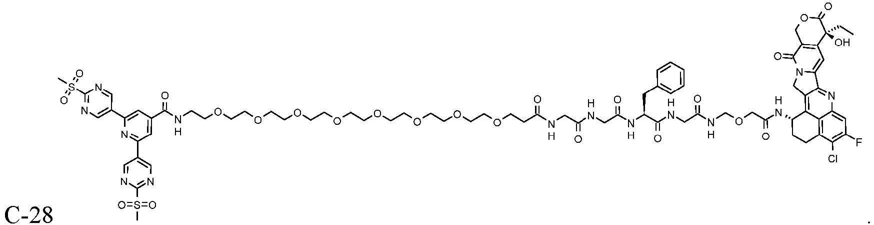

In a seventh aspect, the present disclosure provides a bioactive conjugate, having a structure of formula VII: 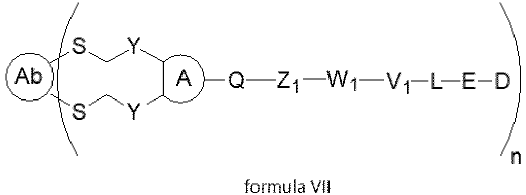
wherein Ab is a targeting moiety (such as a small molecular ligand, a protein (e.g., an antibody), a polypeptide, a non-protein reagent (e.g., sugar, RNA or DNA)); n is an integer or a decimal from 1 to 10;
V₁ is -C(O)- or -N(R₁)-, wherein R₁ is H, C₁₋₆ alkyl or C₂₋₆ alkoxyalkyl;
L is a linker that links V₁ and E;
E is a structural fragment that links L and D;
D is a fragment of a bioactive molecule (e.g., a cytotoxic drug); 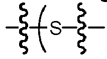 in the conjugate indicates, when the targeting moiety is an antibody, a specific linkage from a sulfhydryl in the antibody to the rest of the conjugate;
the remaining groups are as defined in any item of any aspect described above.

Another aspect of the present disclosure provides a bioactive conjugate, having a structure of formula VIII: 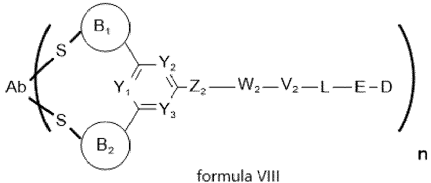
wherein Ab is a targeting moiety (such as a small molecular ligand, a protein (e.g., an antibody), a polypeptide, a non-protein reagent (e.g., sugar, RNA or DNA)); n is an integer or a decimal from 1 to 10;
V₂ is -C(O)- or -N(R₂)-, wherein R₂ is H, C₁₋₆ alkyl or C₂₋₆ alkoxyalkyl;
L is a linker that links V₂ and E;
E is a structural fragment that links L and D;
D is a fragment of a bioactive molecule (e.g., a cytotoxic drug); 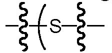 in the conjugate indicates, when the targeting moiety is the antibody, a specific linkage from a sulfhydryl in the antibody to the rest of the conjugate;
the remaining groups are as defined in any item of any aspect described above.

In some embodiments, Ab is selected from the group consisting of: an epidermal growth factor, Trop-2, CD37, HER2, CD70, EGFRvIII, Mesothelin, Folate receoptor1, Mucin 1, CD138, CD20, CD19, CD30, SLTRK6, Nectin 4, Tissue factor, Mucin16, Endothelin receoptor, STEAP1, SLC39A6, Guanylyl cyclase C, PSMA, CCD79b, CD22, Sodium phosphate cotransporter 2B, GPNMB, Trophoblast glycoprotein, AGS-16, EGFR, CD33, CD66e, CD74, CD56, PD-L1, TACSTD2, DR5, E16, STEAP1, 0772P, MPF, Napi3b, Sema 5b, PSCA hlg, ETBR, MSG783, STEAP2, TrpM4, CRIPTO, CD21, CD79b, FcRH2, NCA, MDP, IL20Rα, Brevican, EphB2R, ASLG659, PSCA, GEDA, BAFF-R, CD22, CD79a, CXCR5, HLA-DOB, P2X5, CD72, LY64, FcRH1, IRTA2, TENB2, integrin α5β6,α4β7, FGF2, FGFR2, Her3, CD70, CA6, DLL3, DLL4, P-cadherin, EpCAM, pCAD, CD223, LYPD3, LY6E, EFNA4, ROR1, SLITRK6, 5T4, ENPP3, SLC39A6, Claudin18.2, BMPR1B, E16, STEAP1, Tyro7, 0772P, MPF, Napi3b, Sema 5b, PSCA hlg, ETBR, MSG783, STEAP2, TrpM4, CRIPTO, CD21, CD79b, FcRH2, NCA, MDP, IL20Rα, Brevican, EphB2R, ASLG659, PSCA, GEDA, CD22, CD79a, CXCR5, HLA-DOB, P2X5, CD72, LY64, FcRH1, IRTA2, c-Met, ApoE, CD1lc, CD40, CD45(PTPRC), CD49D(ITGA4), CD80, CSF1R, CTSD, GZMB, Ly86, MS4A7, PIK3AP1, PIK3CD, CCR5, IFNG, IL10RA1, IL-6, ACTA2, COL7A1, LOX, LRRC15, MCPT8, MMP10, NOG, SERPINEl, STAT1, TGFBR1, CTSS, PGF, VEGFA, C1QA, C1QB, ANGPTL4, EGLN, ANGPTL4, EGLN3, BNIP3, AIF1, CCL5, CXCL10, CXCL11, IFI6, PLOD2, KISS1R, STC2, DDIT4, PFKFB3, PGK1, PDK1, AKR1C1, AKR1C2, CADM1, CDH11, COL6A3, CTGF, HMOX1, KRT33A, LUM, WNT5A, IGFBP3, MMP14, CDCP1, PDGFRA, TCF4, TGF, TGFB1, TGFB2, CDl lb, ADGRE1, EMR2, TNFRSF21, UPK1B, TNFSF9, MMP16, MFI2, IGF-1R, RNF43, NaPi2b, BCMA, B7H3 and TENB2;
preferably, Ab is selected from an anti-Her2 antibody (e.g., Trastuzumab), an anti-Trop2 antibody (e.g., Sacituzumab), an anti-ROR1 antibody (e.g., 19F6_Hu35V1), or an anti-B7H3 antibody (e.g., 2#8890).

In some embodiments, L is selected from one or more of the following groups: C₁₋₆ alkyl, - N(R₆)-, carbonyl, -O-, Val, Cit, Phe, Lys, D-Val, Leu, Gly, Phe, Ala, Asn, Ala-Lys, Ala-Lys(Ac), Ala-Pro, Gly-Glu, Gly-Gly, Phe-Lys, Phe-Lys(Ac), Val-Ala, Val-Lys, Val-Lys(Ac), Val-Cit, Ala-Ala-Ala, Ala-Ala-Asn, Leu-Ala-Glu, D-Leu-Ala-Glu, Gly-Gly-Arg, Gly-Glu-Gly, Gly-Gly-Gly, Gly-Ser-Lys, Glu-Val-Ala, Glu-Val-Cit, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly, 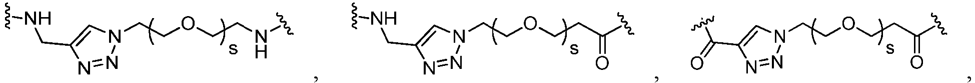 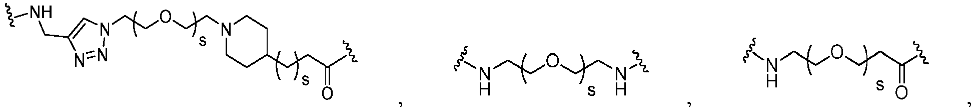 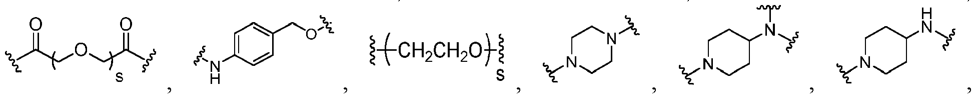 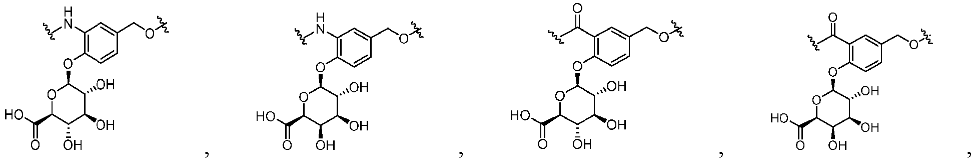 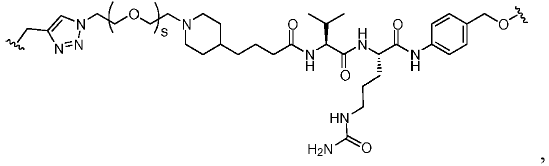 wherein R₆ is H, C₁₋₆ alkyl or C₂₋₆ alkoxyalkyl, and s is an integer from 1 to 10;
preferably, L is a structure composed of one or more of: 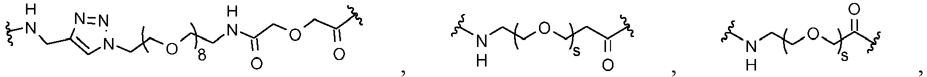 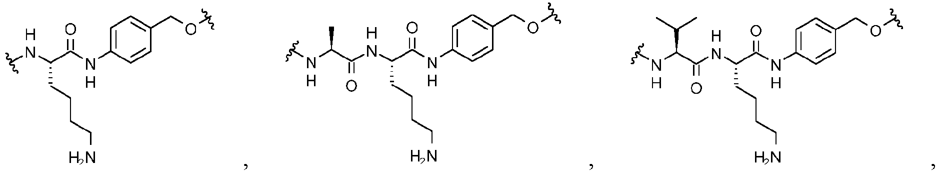 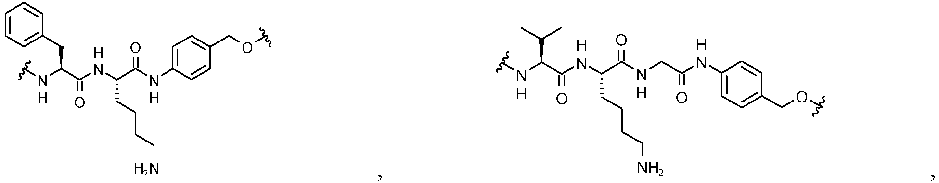 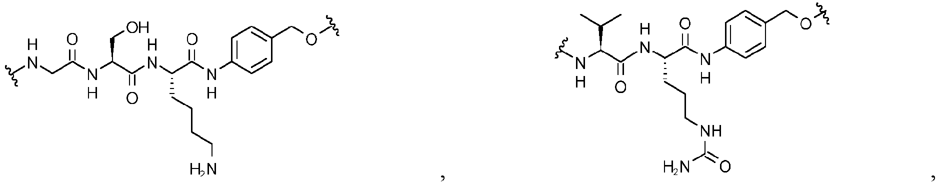 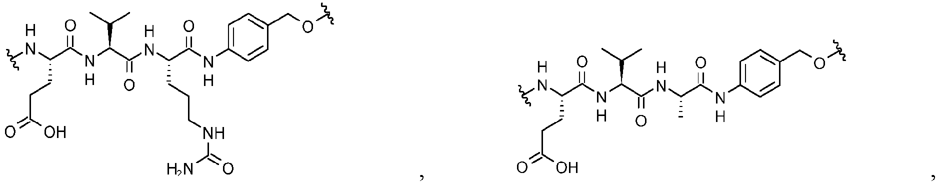 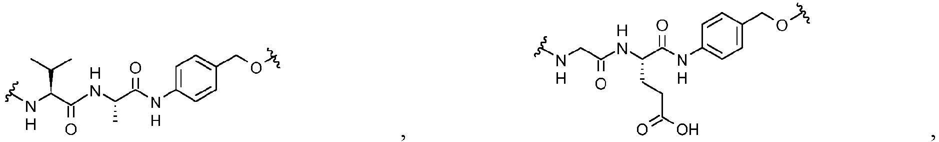 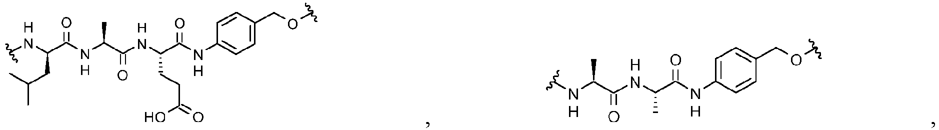 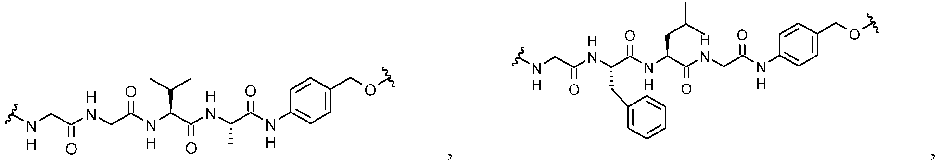 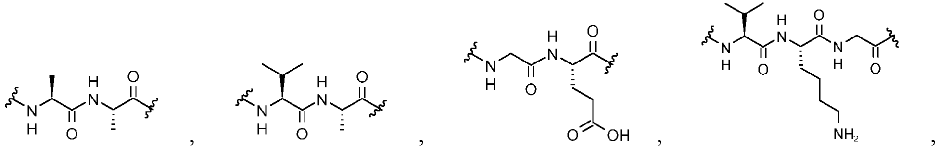 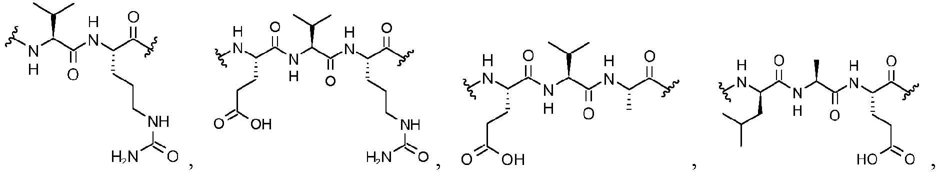 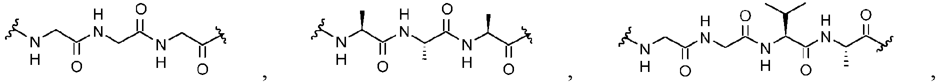 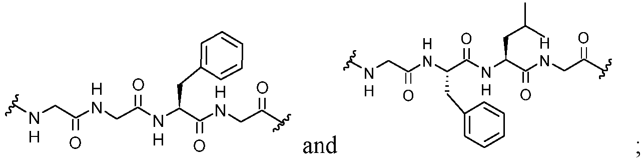 s is an integer from 1 to 10;
preferably, L is selected from the following structures: 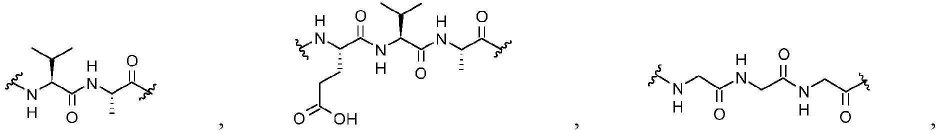 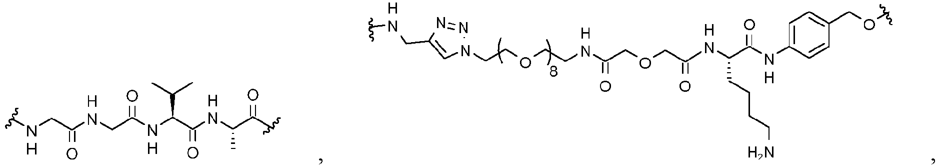 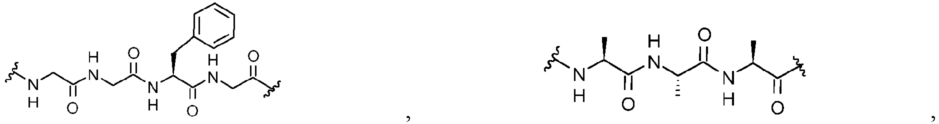 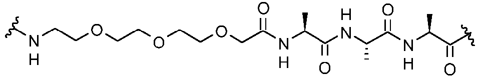 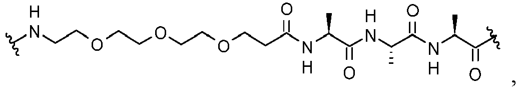 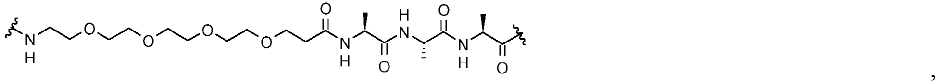 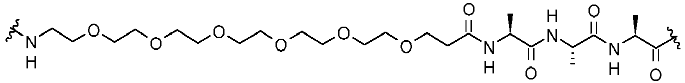 and 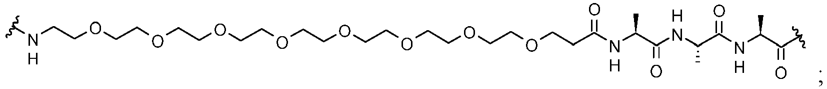
preferably, L is selected from the following structures:

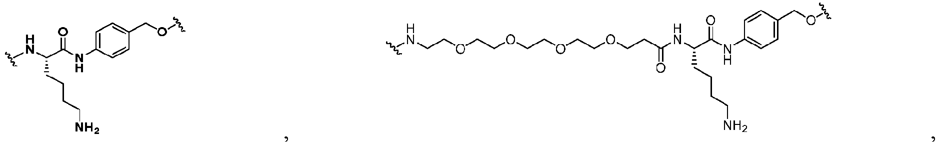

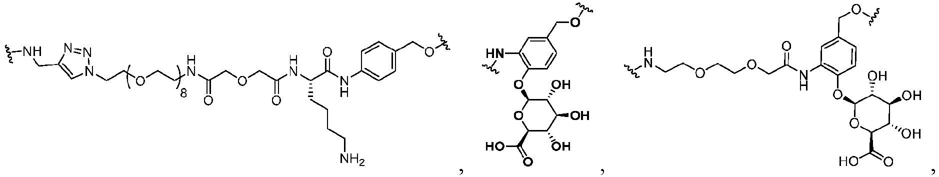

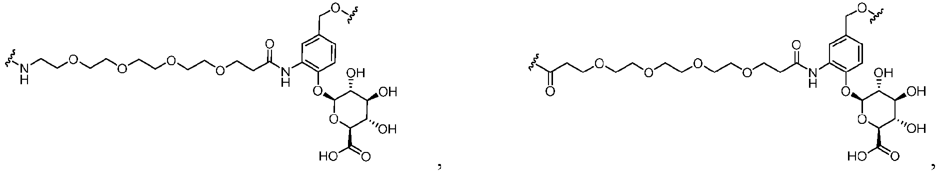

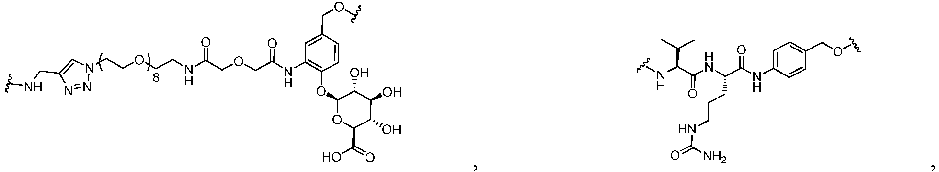

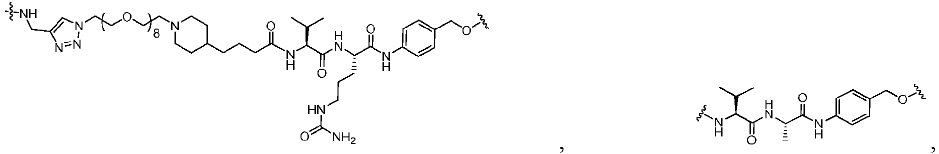

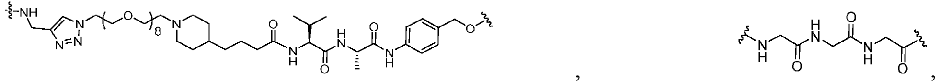

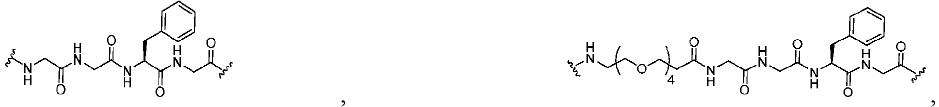

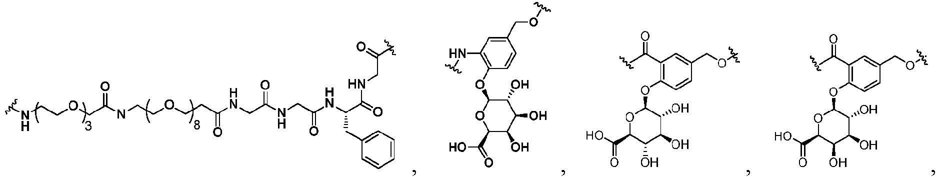

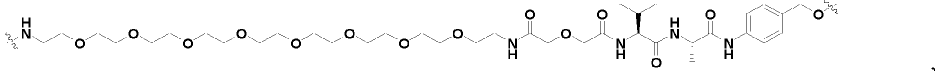

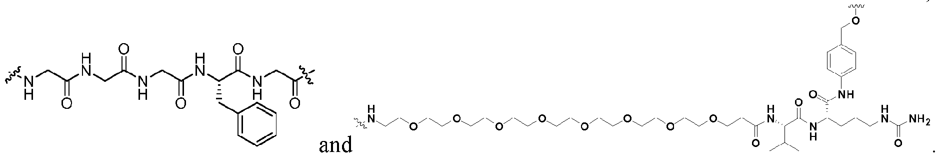

In some embodiments, E is a single bond, -NH-CH₂-, 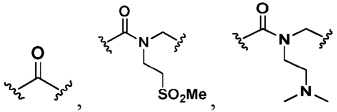 or

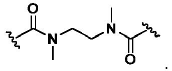

In some embodiments, E is -NH-CH₂-.

In some embodiments, the bioactive molecule is selected from the group consisting of: a tubulin inhibitor, a DNA intercalator, a DNA topoisomerase inhibitor, and an RNA polymerase inhibitor.

In some embodiments, the bioactive molecule is selected from the group consisting of: a tubulin inhibitor such as an auristatin compound and a maytansinoid compound; a DNA intercalator, such as pyrrole benzodiazepine (PBD); a DNA topoisomerase inhibitor, for example, a topoisomerase I inhibitor (such as camptothecin, hydroxycamptothecin, 9-amino-camptothecin, SN-38, irinotecan, Topotecan, Belotecan, Rubitecan) or a topoisomerase II inhibitor (such as adriamycin, doxorubicin, PNU-159682, duocarmycin, daunorubicin, mitoxantrone, podophyllotoxin or etoposide); an RNA polymerase inhibitor including α-amanitin, etc.; and pharmaceutically acceptable salts, esters and analogues thereof.

In some emboditions, the bioactive molecule is selected from the group consisting of: a topoisomerase I inhibitor (such as camptothecin, hydroxycamptothecin, 9-amino-camptothecin, SN-38, Irinotecan, Topotecan, Belotecan, Rubitecan), MMAE and a MMAE derivative.

In some embodiments, the bioactive molecule is selected from the group consisting of: MMAE and a MMAE derivative.

In some embodiments, the D is selected from the group consisting of:

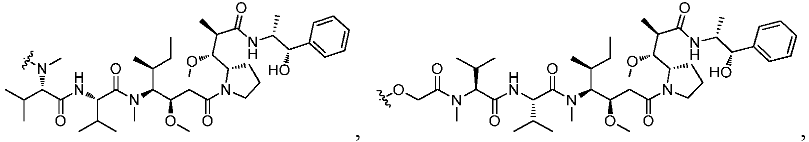

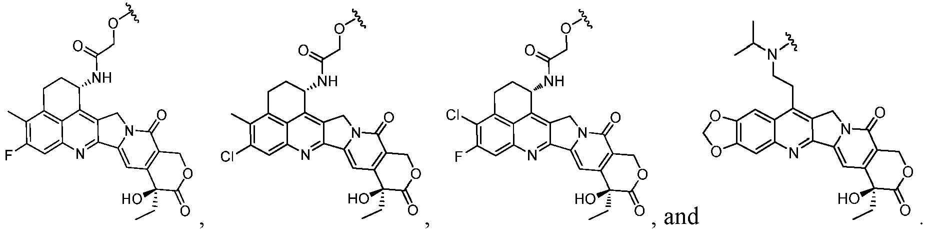

In some embodiments, n ranges from 1 to 8; further preferably, n ranges from 3 to 5.

In some embodiments, the bioactive conjugate has a structure selected from the following group, wherein Ab is selected from an anti-Her2 antibody (e.g., Trastuzumab), an anti-Trop2 antibody (e.g., Sacituzumab), or an anti-ROR1 antibody (e.g., 19F6_Hu35V1), and n₁ ranges from 1 to 8, further preferably from 3 to 5:

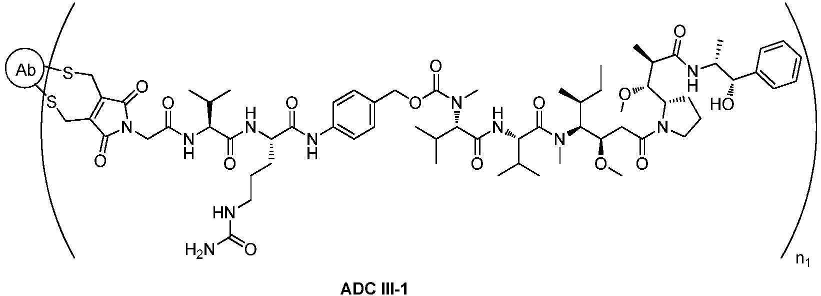

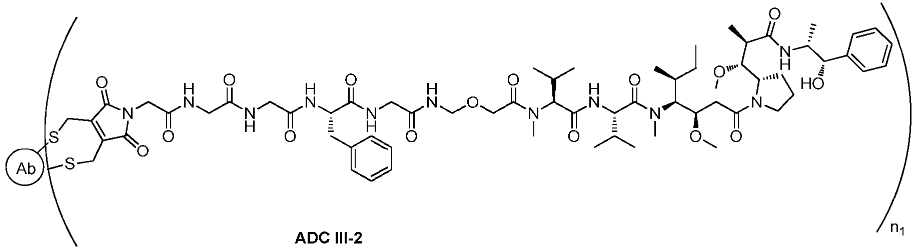

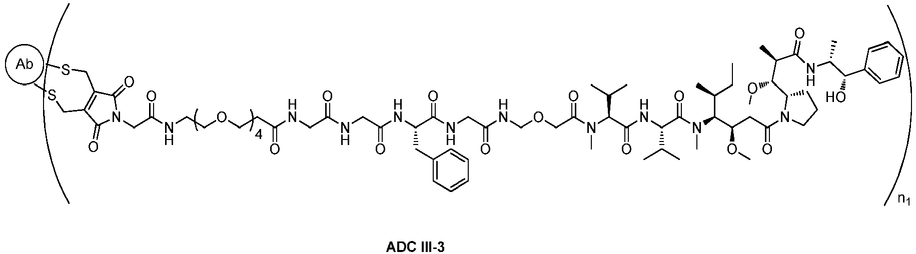

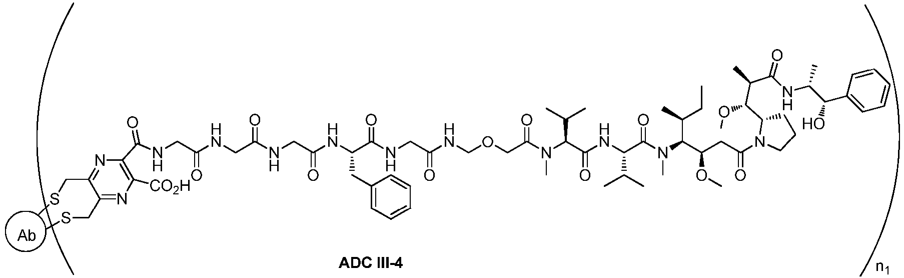

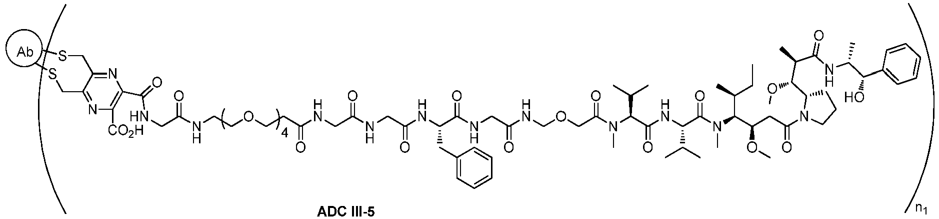

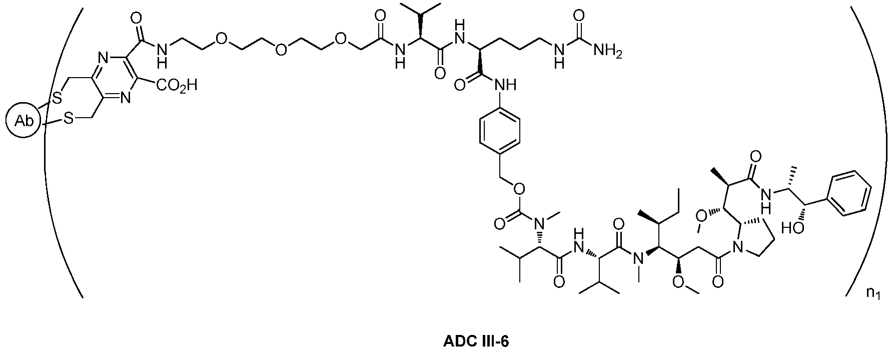

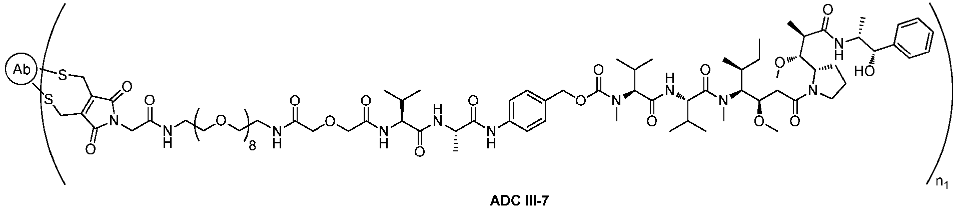

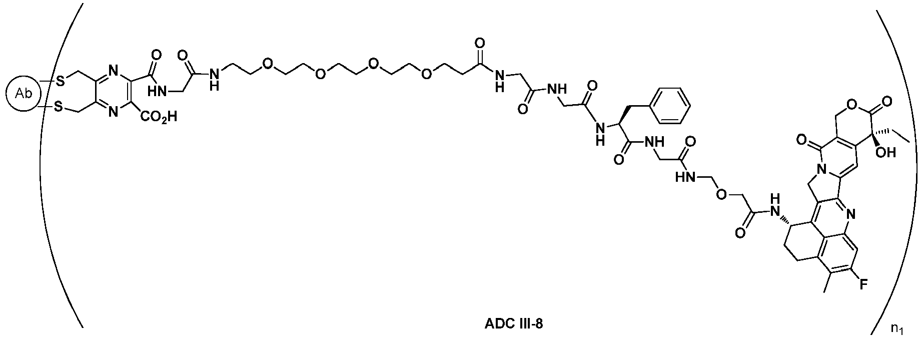

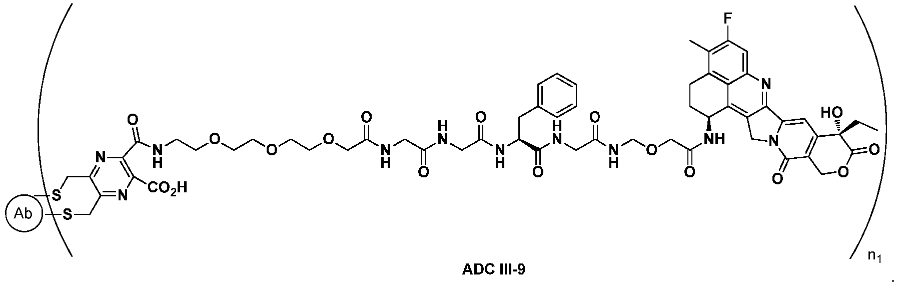

In some embodiments, structure of the bioactive conjugate is as below, wherein Ab is selected from an anti-Her2 antibody (e.g., Trastuzumab), an anti-Trop2 antibody (e.g., Sacituzumab), an anti-ROR1 antibody (e.g., 19F6_Hu35V1) or anti-B7H3 antibody (e.g., 2#8890), and n₁ ranges from 1 to 8, further preferably from 3 to 5; x ranges from 1 to 10, further preferably from 3 to 5:

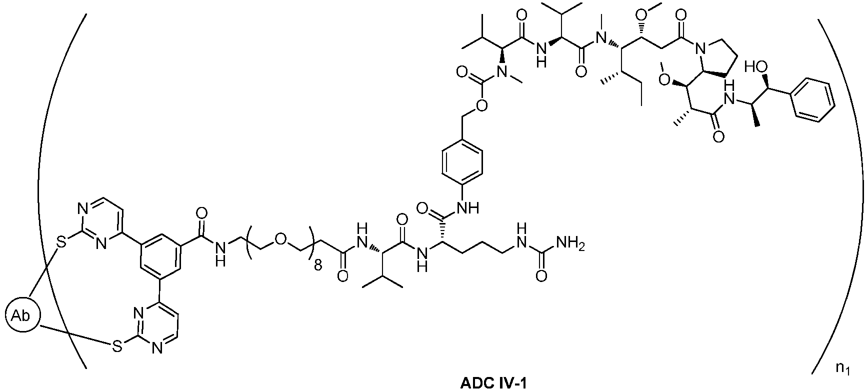

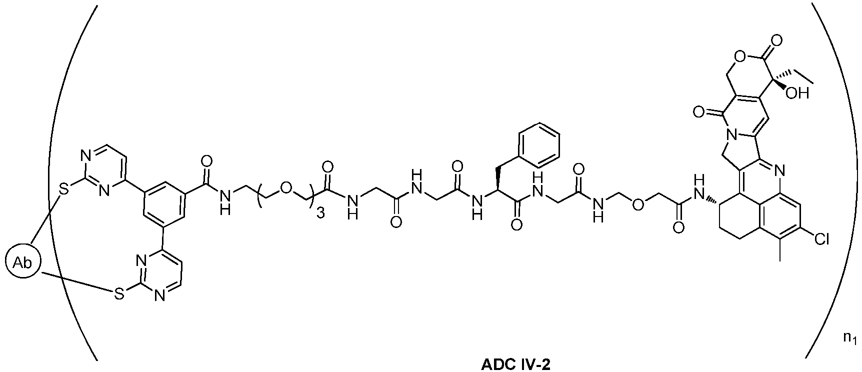

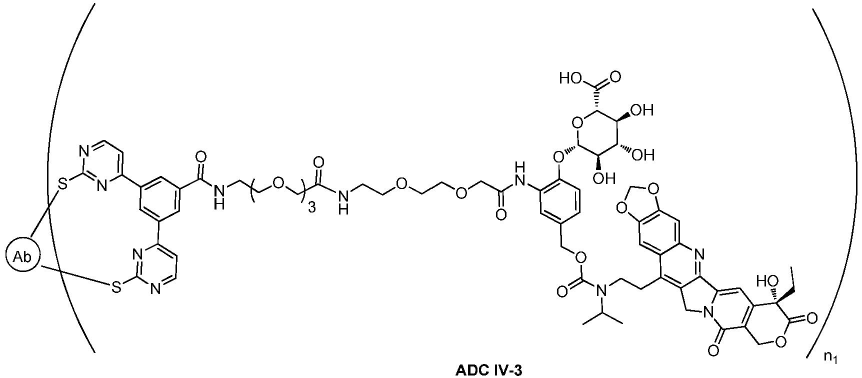

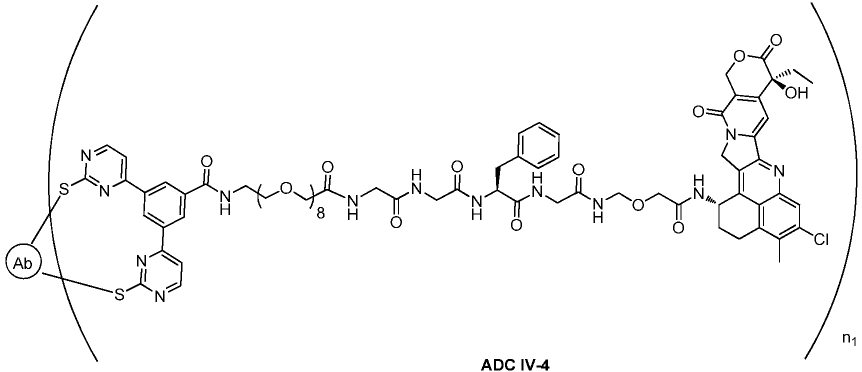

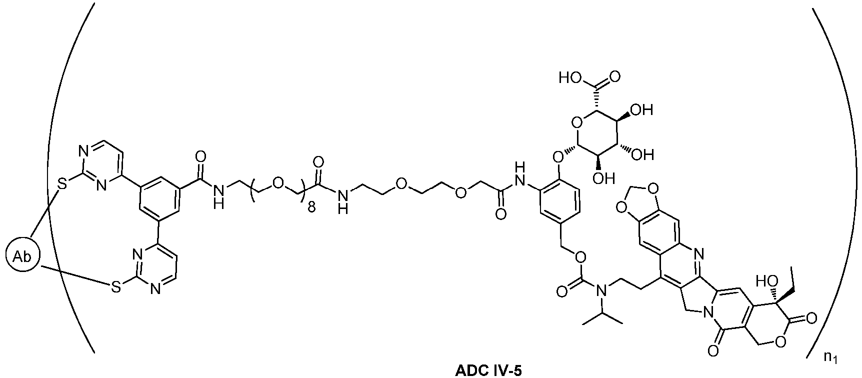

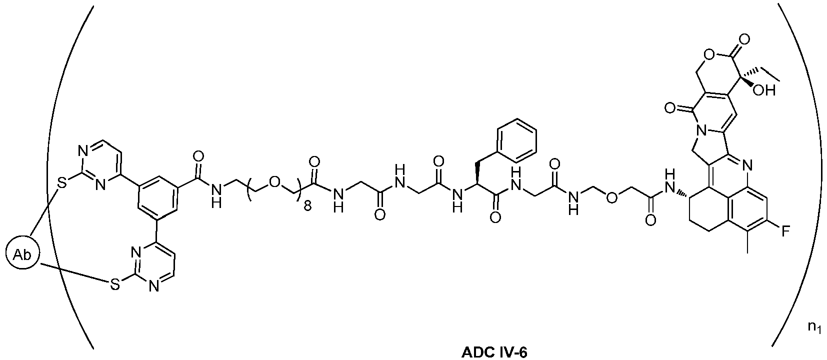

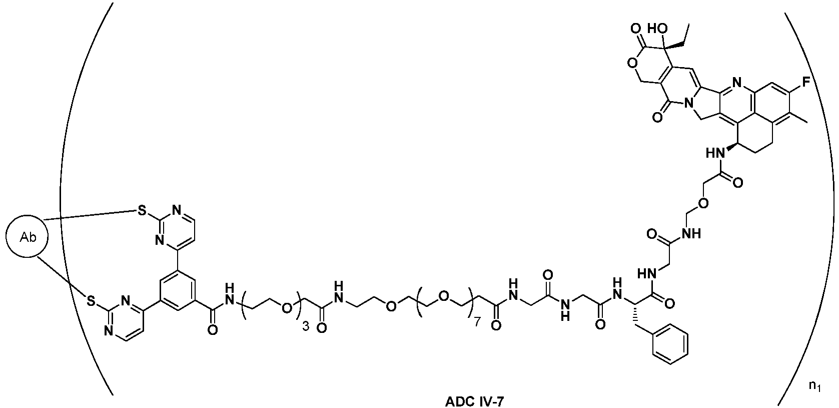

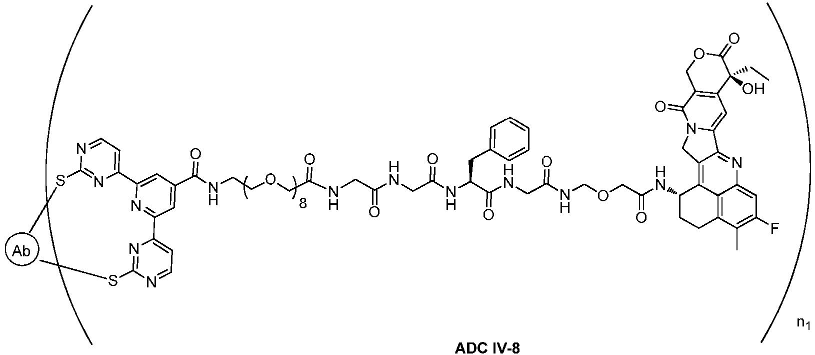

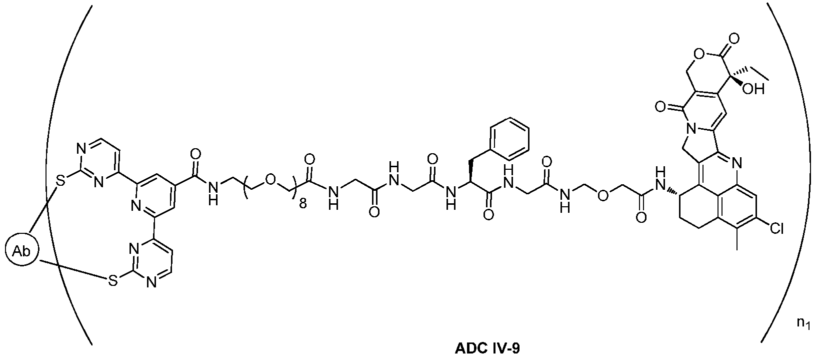

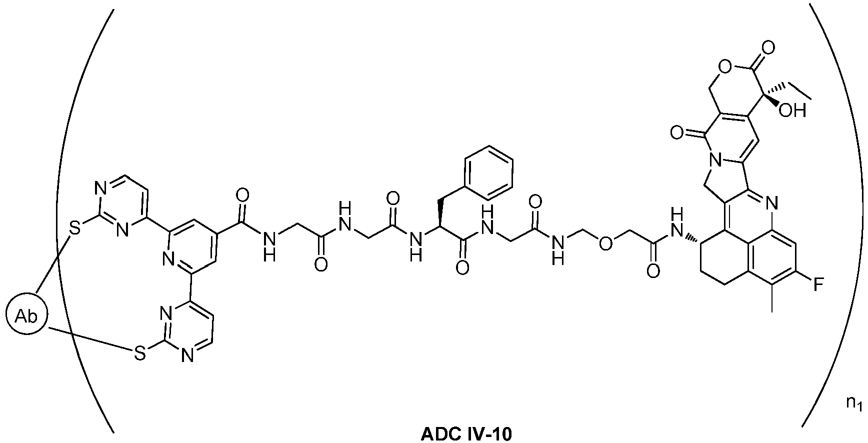

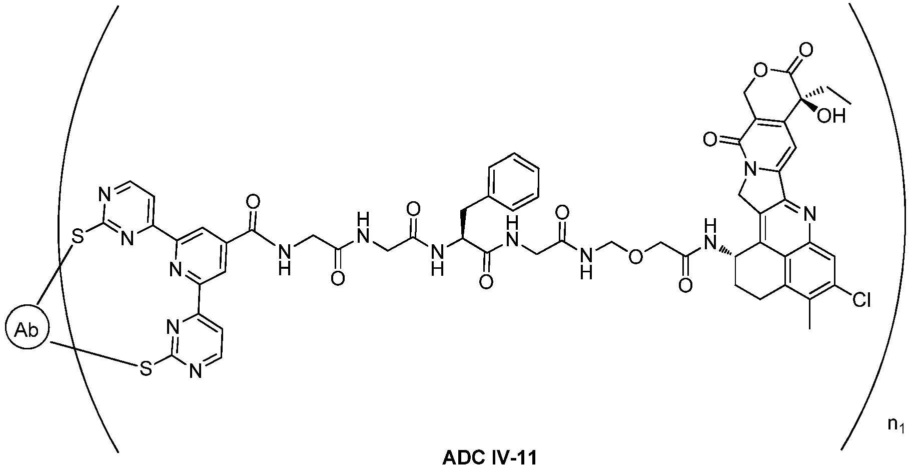

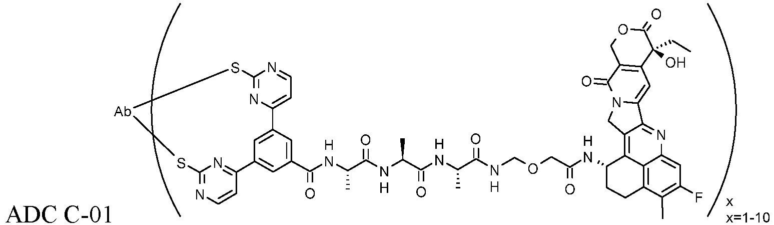

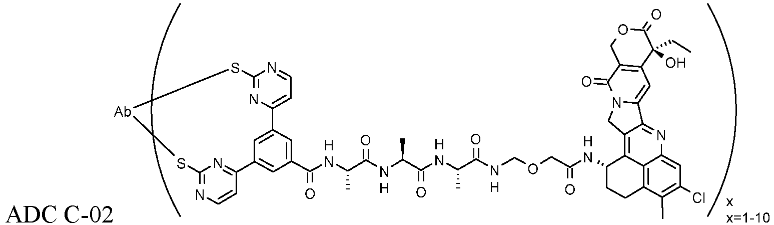

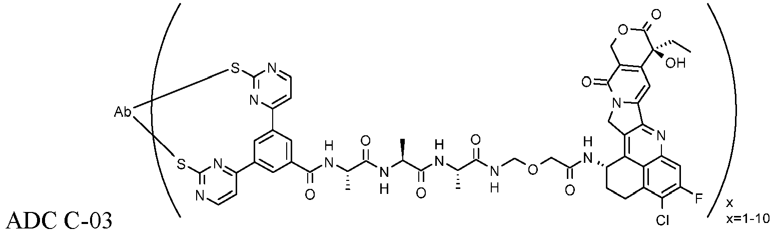

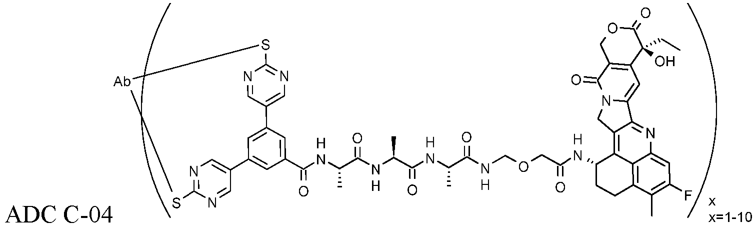

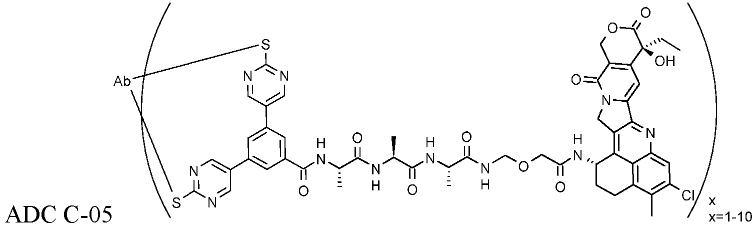

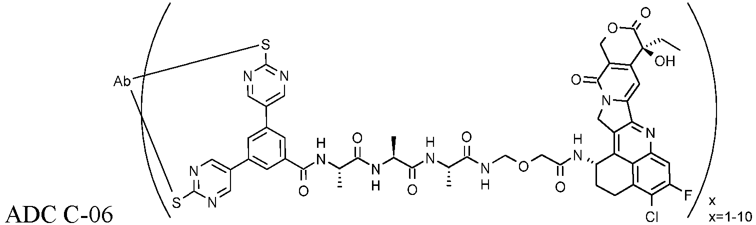

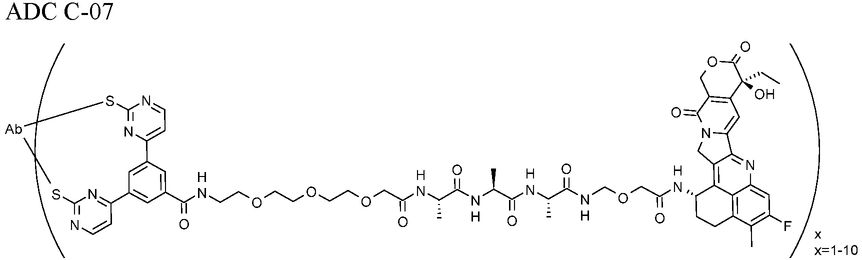

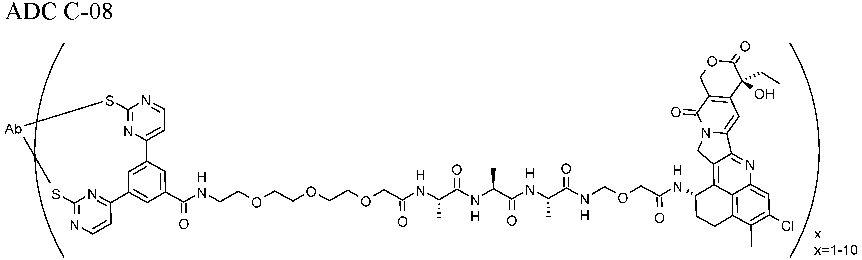

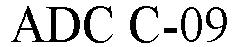

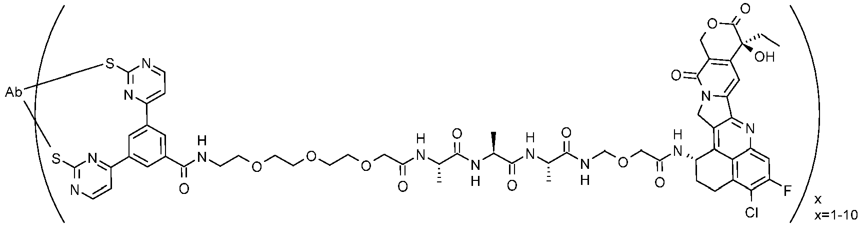

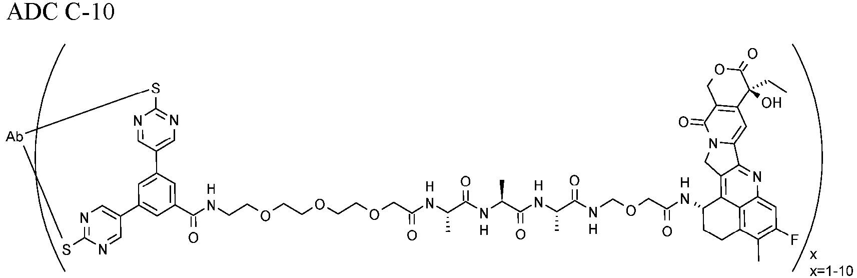

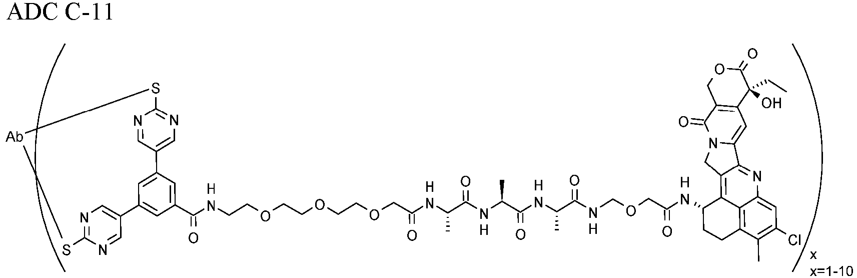

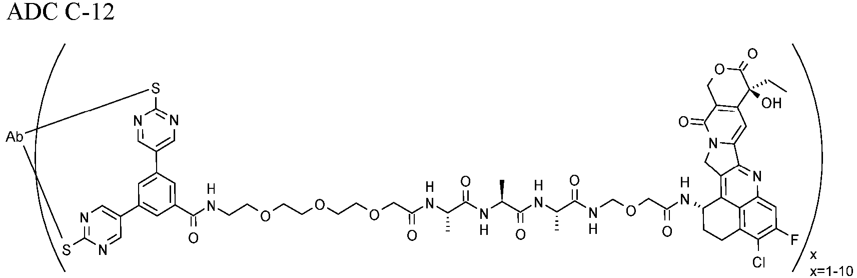

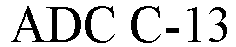

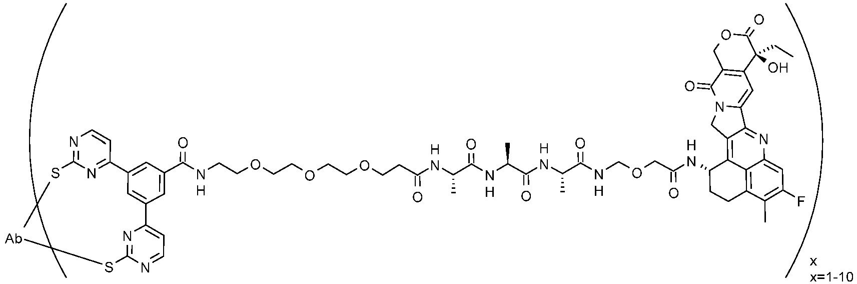

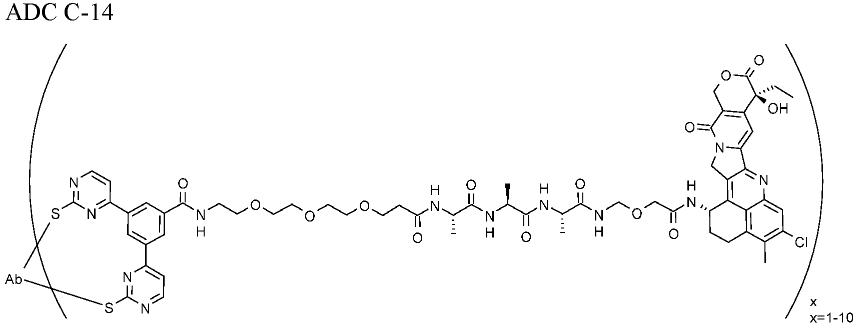

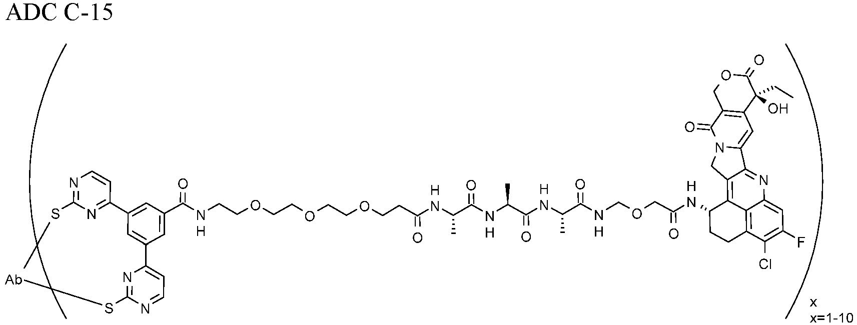

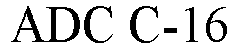

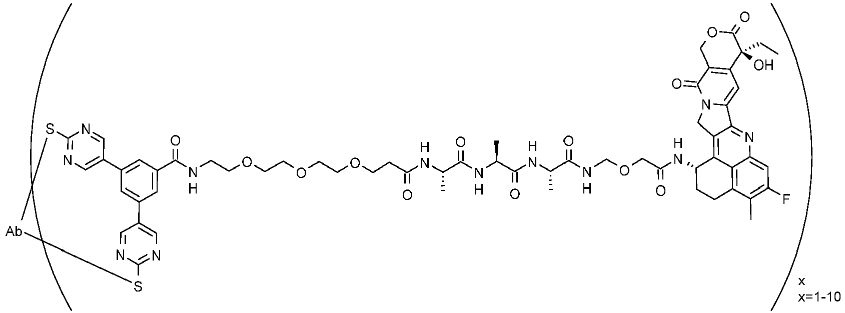

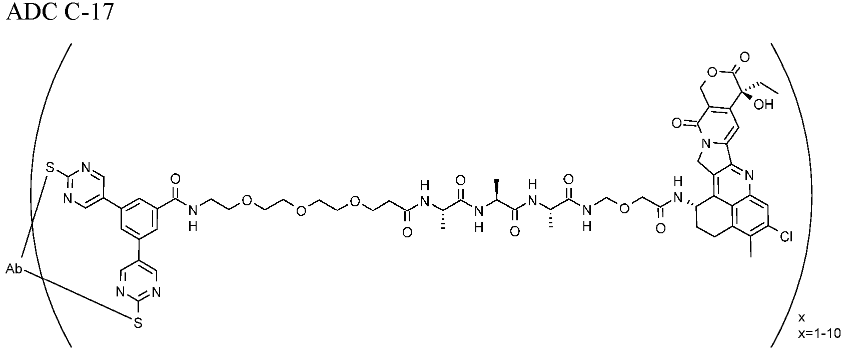

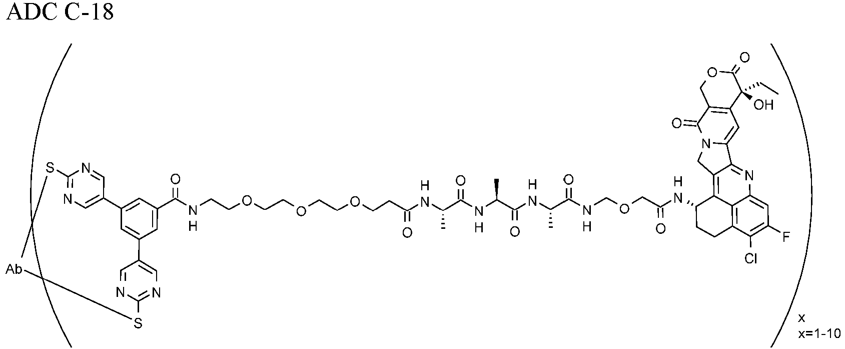

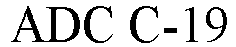

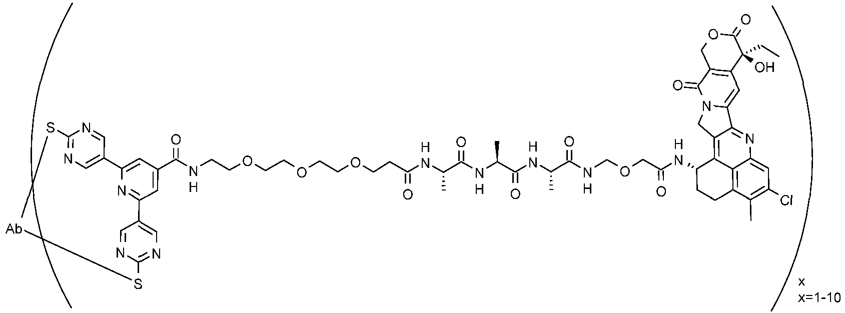

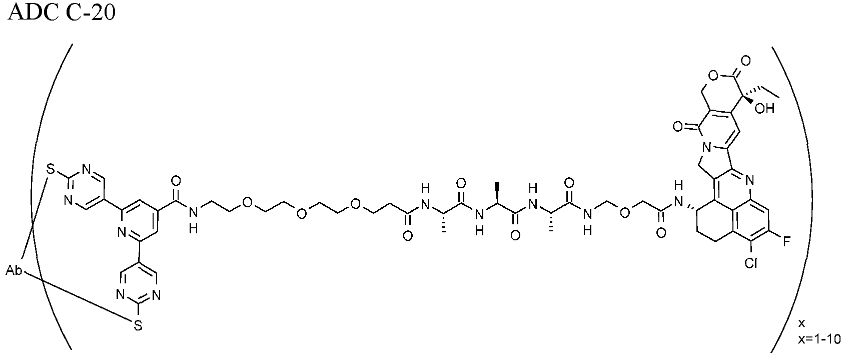

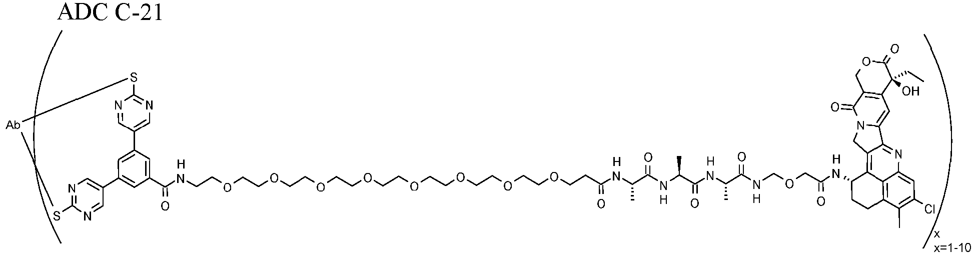

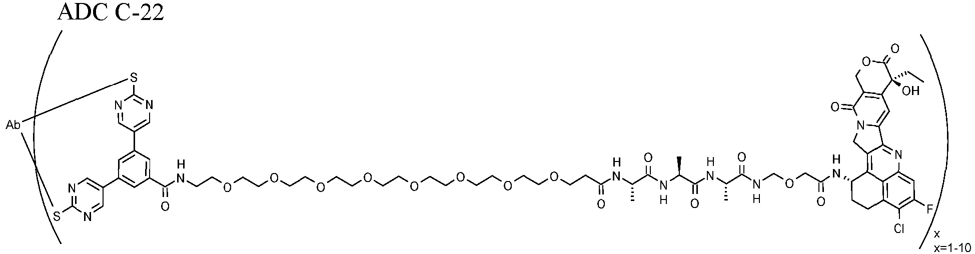

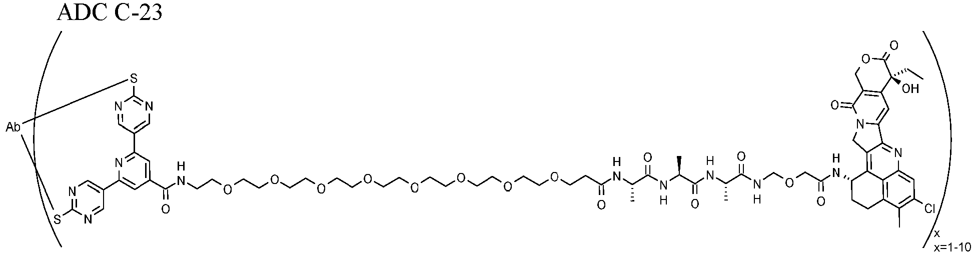

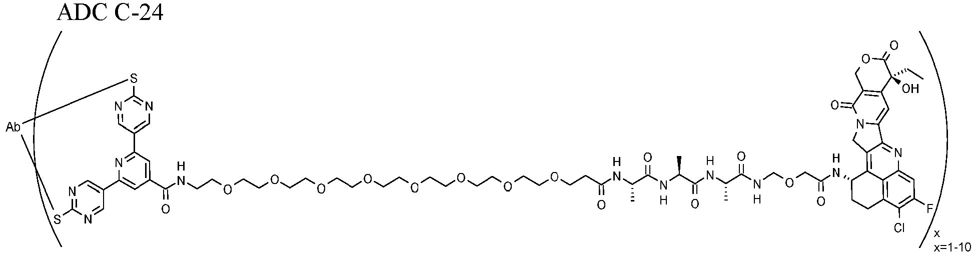

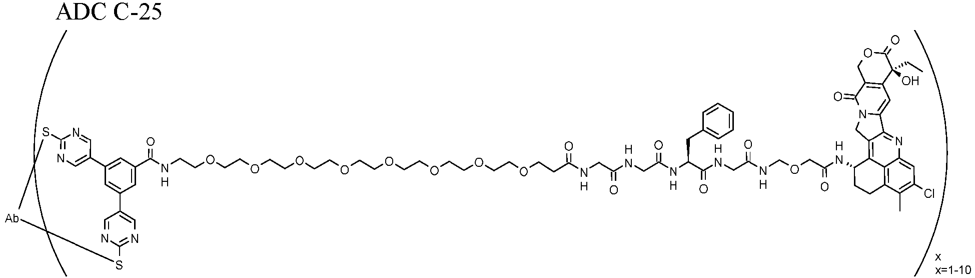

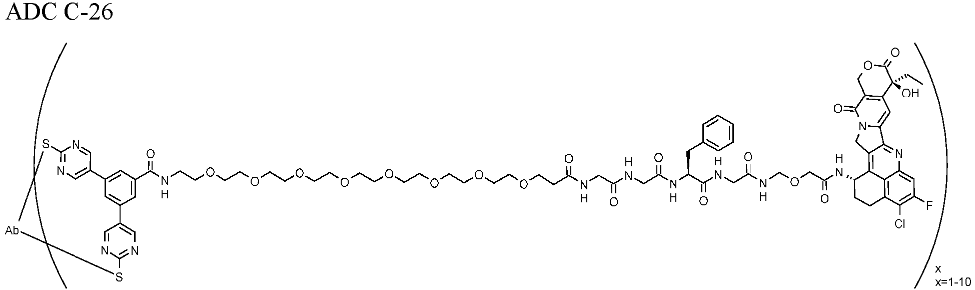

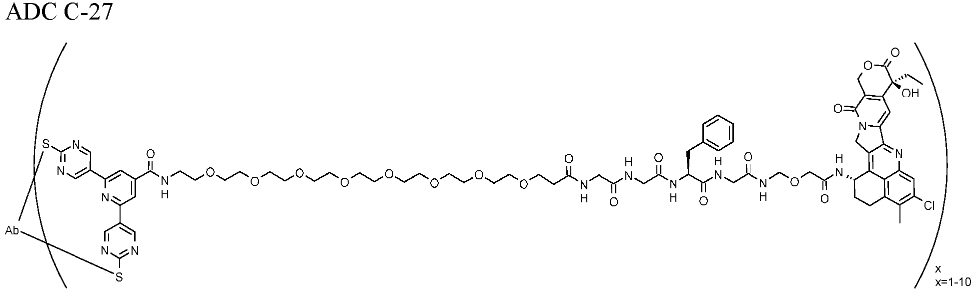

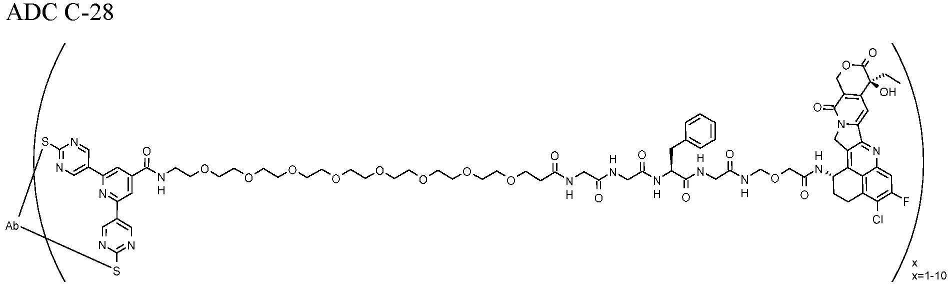

In some embodiments, the anti-Her2 antibody described in any of the above items is Trastuzumab; an antibody containing a heavy chain variable region having heavy chain complementary determining regions of Trastuzumab and a light chain variable region having light chain complementary determining regions of Trastuzumab; or an antibody containing a heavy chain variable region of Trastuzumab and a light chain variable region of Trastuzumab;
any of the aforementioned anti-Trop2 antibody is Sacituzumab; an antibody containing a heavy chain variable region having heavy chain complementary determining regions of Sacituzumab and a light chain variable region having light chain complementary determining regions of Sacituzumab; or an antibody containing a heavy chain variable region of Sacituzumab and a light chain variable region of Sacituzumab; and/or
any of the aforementioned anti-ROR1 antibody is:
   i) an antibody containing a heavy chain variable region having CDR-H1 as set forth in SEQ ID NO: 3, CDR-H2 as set forth in SEQ ID NO: 4 and CDR-H3 as set forth in SEQ ID NO: 5, and a light chain variable region having CDR-L1 as set forth in SEQ ID NO: 6, CDR-L2 as set forth in SEQ ID NO: 7 and CDR-L3 as set forth in SEQ ID NO: 8, defined according to the Chothia numbering system;
   ii) an antibody containing a heavy chain variable region having CDR-H1 as set forth in SEQ ID NO: 9, CDR-H2 as set forth in SEQ ID NO: 10 and CDR-H3 as set forth in SEQ ID NO: 5, and a light chain variable region having CDR-L1 as set forth in SEQ ID NO: 6, CDR-L2 as set forth in SEQ ID NO: 7 and CDR-L3 as set forth in SEQ ID NO: 8, defined according to the AbM numbering system;
   iii) an antibody containing a heavy chain variable region having CDR-H1 as set forth in SEQ ID NO: 11, CDR-H2 as set forth in SEQ ID NO: 12 and CDR-H3 as set forth in SEQ ID NO: 5, and a light chain variable region having CDR-L1 as set forth in SEQ ID NO: 6, CDR-L2 as set forth in SEQ ID NO: 7 and CDR-L3 as set forth in SEQ ID NO: 8, defined according to the Kabat numbering system;
   iv) an antibody containing a heavy chain variable region having CDR-H1 as set forth in SEQ ID NO: 13, CDR-H2 as set forth in SEQ ID NO: 14 and CDR-H3 as set forth in SEQ ID NO: 15, and a light chain variable region having CDR-L1 as set forth in SEQ ID NO: 16, CDR-L2 as set forth in SEQ ID NO: 17 and CDR-L3 as set forth in SEQ ID NO: 8, defined according to the IMGT numbering system;
   v) an antibody containing a heavy chain variable region as set forth in SEQ ID NO: 1 and a light chain variable region as set forth in SEQ ID NO: 2; or
   vi) 19F6_Hu35V1, containing a heavy chain variable region as set forth in SEQ ID NO: 1, a light chain variable region as set forth in SEQ ID NO: 2, a heavy chain constant region as set forth in SEQ ID NO: 18 and a light chain constant region as set forth in SEQ ID NO: 19.

In some embodiments, n₁ ranges from 1 to 6, for example from 3 to 5.

In some embodiments, x ranges from 1 to 6, for example from 3 to 5.

### Definitions

Unless otherwise defined below, all technical and scientific terms used herein are intended to have the same meaning as those generally understood by those skilled in the art. References to technology as used herein are intended to refer to technologies commonly understood in the art, including variations or substitutions of equivalent technologies that are obvious to those skilled in the art. Although the following terms are believed to be well understood by those skilled in the art, the following definitions are set forth to better explain the present disclosure.

As used herein, the term "alkylene" refers to a saturated divalent hydrocarbyl group, preferably a saturated divalent hydrocarbyl group with 1, 2, 3, 4, 5 or 6 carbon atoms, such as methylene, ethylidene, propylidene or butylene.

As used herein, the term "alkyl" is defined as a linear or branched saturated aliphatic hydrocarbyl group. In some embodiments, the alkyl has 1 to 12, for example 1 to 6 carbon atoms. For example, as used herein, the term "C₁₋₆ alkyl" refers to a linear or branched aliphatic hydrocarbyl group (such as methyl, ethyl, n-propyl, isopropyl, n-butyl, isobutyl, sec-butyl, tert-butyl, n-amyl, isoamyl, neopentyl, or n-hexyl) with 1 to 6 carbon atoms, which is optionally substituted by one or more (e.g., 1 to 3) suitable substituents (e.g., halogen) (in this case, the group is called "haloalkyl") (such as, CH₂F, CHF₂, CF₃, CCl₃, C₂F₅, C₂Cl₅, CH₂CF₃, CH₂Cl or - CH₂CH₂CF₃). The term "C₁₋₄ alkyl" refers to a linear or branched aliphatic hydrocarbyl group with 1 to 4 carbon atoms (such as methyl, ethyl, n-propyl, isopropyl, n-butyl, isobutyl, sec-butyl, or tert-butyl).

As used herein, the term "alkoxy" is defined as -O-alkyl, and the alkyl is as defined above. For example, as used herein, the term "C₁₋₆ alkoxy" refers to -O-C₁₋₆ alkyl.

As used herein, the term "alkoxyalkyl" is defined as an alkoxy-substituted alkyl and the alkyl is as defined above. For example, as used herein, the term "C₂₋₆ alkoxyalkyl" refers to an alkoxy-substituted alkyl group with 2 to 6 carbon atoms.

As used herein, the term "alkynylene" refers to a divalent hydrocarbyl group containing at least one carbon-carbon triple bond, preferably having 1, 2, 3, 4, 5 or 6 carbon atoms, such as ethynylene, propynylene, or butynylene.

As used herein, the term "heterocyclic group", "heterocycle" refers to a saturated or partially unsaturated (i.e., having one or more double and/or triple bonds within the ring) cyclic group, wherein at least one of the ring-forming atoms is a heteroatom selected from N, O and S and the remaining ring-forming atoms are C. For example, the "5- to 12-membered heterocycle (heterocyclic group)" is a saturated or partially unsaturated heterocyclic group with 4 to 11 (such as 2, 3, 4, 5, 6, 7, 8 or 9) ring-forming carbon atoms and one or more (such as 1, 2, 3 or 4) heteroatoms independently selected from N, O and S. The "5- to 12-membered nitrogen-containing heterocycle (heterocyclic group)" refers to a heterocycle (heterocyclic group) wherein at least one of the ring-forming atoms is N. Examples of the heterocyclic group include but are not limited to: oxiranyl, azetidinyl, azetidinyl, oxetanyl, tetrahydrofuryl, dioxolinyl, pyrrolidinyl, pyrrolidone, imidazolidyl, pyrazolidyl, pyrrolidinyl, tetrahydropyranyl, piperidyl, morpholinyl, dithianyl, thiomorpholinyl, piperazinyl or trithianyl. The heterocyclic group may optionally be substituted by one or more (such as 1, 2, 3 or 4) suitable substituents, and may optionally form a fused ring structure with one or more aromatic rings and heteroaromatic rings.

As used herein, the term "aromatic ring" or "aryl" refers to a monocyclic or polycyclic aromatic ring system having, such as, 5, 6, 8, 9, 10, 11, 12, 13 or 14 ring forming carbon atoms, in particular 1 or 2 or 3 or 4 or 5 or 6 or 9 or 10 carbon atoms.

As used herein, the term "aromatic heterocycle" or "heteroaryl" means a monocyclic or polycyclic aromatic ring system having, such as, 5, 6, 8, 9, 10, 11, 12, 13 or 14 ring atoms, in particular 1 or 2 or 3 or 4 or 5 or 6 or 9 or 10 carbon atoms, and which contains at least one heteroatom that may be identical or different (the heteroatom is, such as, N, O or S), and, additionally, in each case may be benzo-fused.

As used herein, the term "halogen" includes F, Cl, Br, or I.

As used herein, the term "sulfonylurea group" refers to -SO₂-NH-(C=O)-NH₂ or -NH-(C=O)-NH-SO₂H.

The term "substituted" means that one or more (such as, 1, 2, 3 or 4) hydrogens on a specified atom are substituted by groups selected from the indicated groups, provided that the normal valence of the specified atom is not exceeded in the current situation and the substitution forms a stable compound. Combinations of substituents and/or variables are permitted only if such combinations form stable compounds.

If a substituent is described as "optionally substituted with ......", the substituent may be (1) unsubstituted or (2) substituted. If carbon atom of the substituent is described as being optionally substituted by one or more of the substituents in the list, one or more hydrogens (to the extent of any hydrogen present) on the carbon atom may be individually and/or together substituted by an optional substituent(s) independently selected. If nitrogen of the substituent is described as being optionally substituted by one or more of the substituents in the list, then one or more hydrogens (to the extent of any hydrogen present) on the nitrogen may each be substituted by an optional substituent(s) independently selected.

If a substituent is described as "independently selected from" a group of groups, then each substituent is selected independently of the other. Thus, each substituent may be identical to or different from another (other) substituent.

As used herein, the term "one or more" means one or more than one under reasonable conditions, such as 2, 3, 4, 5 or 10.

Unless otherwise specified, the connecting position on the substituent may come from any suitable position on the substituent.

When a bond of a substituent is displayed as passing through a bond connecting two atoms of a ring, such a substituent may be bonded to any of the ring-forming atoms of the substitutable ring.

The present disclosure further includes all pharmaceutically acceptable isotope labeled compounds that are identical to the compounds of the present disclosure except that one or more atoms are substituted by atoms having the same atomic number but with an atomic mass or mass number different from the atomic mass or mass number of atoms prevailing in nature. Examples of isotopes suitable for inclusion in compounds of the present disclosure include (but are not limited to) isotopes of hydrogen (such as deuterium (²H), tritium (³H)); isotopes of C (such as ¹¹C, ¹³C and ¹⁴C); isotopes of Cl (such as ³⁶Cl); isotopes of F (such as ¹⁸F); isotopes of I (such as ¹²³I and ¹²⁵I); isotopes of N (such as ¹³N and ¹⁵N); isotopes of O (such as ¹⁵O, ¹⁷O and ¹⁸O); isotopes of P (such as ³²P); and isotopes of S (such as ³⁵S).

The term "stereoisomer" refers to an isomer formed due to at least one asymmetric center. In compounds with one or more (such as 1, 2, 3 or 4) asymmetric centers, racemic mixtures, single enantiomers, diastereoisomer mixtures and individual diastereoisomers may be generated. Specific individual molecules may also exist in the form of (cis/trans) geometric isomers. Similarly, the compounds of the present disclosure may exist in mixtures of two or more structurally different forms in rapid equilibrium (commonly referred to as tautomers). Representative examples of tautomers include keto-enol tautomers, phenol-ketone tautomers, nitroso-oxime tautomers, and imine-enamine tautomers. It should be understood that the scope of the present disclosure covers all such isomers at any ratio (such as, 60%, 65%, 70%, 75%, 80%, 85%, 90%, 95%, 96%, 97%, 98% and 99%) or mixtures thereof. A solid line ( 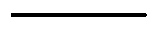 ), a solid wedge ( 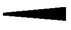 ) or a dotted wedge ( 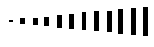 ) may be used herein to depict the carbon-carbon bond of the compounds of the present disclosure. The use of solid lines to describe the bond to an asymmetric carbon atom indicates that all possible stereoisomers (such as specific enantiomers and racemic mixtures ) at the carbon atom are included. The use of solid and dotted wedges to depict the bond to an asymmetric carbon atom indicates the presence of the stereoisomer as shown. When present in racemic mixtures, solid and dotted wedges are used to define relative stereochemistry rather than absolute stereochemistry. Unless otherwise specified, the compounds of the present disclosure are intended to exist in the form of stereoisomers (including cis- and trans-isomers, optical isomers (such as R- and S-enantiomers), diastereoisomers, geometric isomers, rotamers, conformational isomers, atropisomer and mixtures thereof). The compounds of the present disclosure may exhibit more than one type of isomerism and consist of mixtures thereof, such as racemic mixtures and diastereoisomer pairs.

The present disclosure covers all possible crystalline forms or polymorphs of the compounds of the present disclosure, which may be a single polycrystalline form or mixtures of more than one polymorph at any ratio.

It should also be understood that certain compounds of the present disclosure may exist in free form for therapeutic use or, where appropriate, in the form of pharmaceutically acceptable derivatives thereof. In the present disclosure, the pharmaceutically acceptable derivatives include, but are not limited to, pharmaceutically acceptable salts, esters, solvates, N-oxides, metabolites, or prodrugs capable of directly or indirectly providing the compounds of the present disclosure or metabolites or residues thereof after administration to patients in need. Therefore, when the "compound of the present disclosure" is referred to herein, it is also intended to cover the various derivative forms of the compounds described above.

The pharmaceutically acceptable salts of the compounds of the present disclosure include acid addition salts and base addition salts thereof.

Suitable acid addition salts are formed from acids that form pharmaceutically acceptable salts. Examples include aspartates, benzoates, bicarbonates/carbonates, glucoheptonates, gluconates, nitrates, orotates, palmitates, and the like.

Suitable base addition salts are formed from bases that form pharmaceutically acceptable salts. Examples include aluminum salts, arginine salts, choline salts, magnesium salts, and the like.

For an overview of suitable salts, see Stahl and Wermuth "Handbook of Pharmaceutical Salts: Properties, Selection, and Use" (Wiley-VCH, 2002). Methods for preparing pharmaceutically acceptable salts for the compounds of the present disclosure are known to those skilled in the art.

As used herein, the term "ester" refers to an ester derived from the compound of each general formula in the present disclosure, including a physiologically hydrolyzed ester (a compound of the present disclosure that can be hydrolyzed under physiological conditions to release a free acid or alcohol form). The compounds of the present disclosure may be esters, per se.

The compounds of the present disclosure may exist in the form of solvates (preferably hydrates), wherein the compounds of the present disclosure contain polar solvents that are structural elements of the lattice of the compounds, in particular, for example, water, methanol or ethanol. The amounts of polar solvents, especially water, can be present in stoichiometric or non-stoichiometric ratios.

Those skilled in the art will understand that since available lone pair electrons are required to oxidize nitrogen into oxides, not all nitrogen-containing heterocycles are capable of forming N-oxides. Those skilled in the art will identify nitrogen-containing heterocycles capable of forming N-oxides. Those skilled in the art will also recognize that tertiary amines can form N-oxides. The synthesis methods for preparation of N-oxides from heterocycles and tertiary amines are well known to those skilled in the art and include the oxidation of heterocycles and tertiary amines with peroxoic acids such as peracetic acid and m-chloroperoxybenzoic acid (MCPBA), hydrogen peroxide, alkyl hydrogen peroxide such as tert-butyl hydrogen peroxide, sodium perborate and dioxirane such as dimethyldioxirane. These methods for the preparation of N-oxides have been extensively described and summarized in the literature, see, e.g., T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

The scope of the present disclosure also includes metabolites of the compounds of the present disclosure, which are substances formed in the body when the compounds of the present disclosure are administered. Such products may be produced by, for example, oxidation, reduction, hydrolysis, amidation, deamidation, esterification, enzymatic hydrolysis of the compounds being administered. Therefore, the present disclosure includes metabolites of the compounds of the present disclosure, including compounds obtained by means of bringing the compounds of the present disclosure into contact with mammals for a time sufficient to produce their metabolites.

The present disclosure further includes, within its scope, prodrugs of the compounds of the present disclosure, which are certain derivatives of the compounds of the present disclosure that may themselves have minor pharmacological activity or no pharmacological activity and which, when administered to or on the body, can be converted into the compounds of the present disclosure with the desired activity by, for example, hydrolytic cleavage. Usually, such prodrugs will be functional group derivatives of the compounds, which are readily converted in vivo into the desired therapeutic active compounds. For other information about the use of the prodrugs, see "Pro-drugs as Novel Delivery Systems", Vol. 14, ACS Symposium Series (T. Higuchi and V. Stella). The prodrugs of the present disclosure may be prepared, for example, by substituting appropriate functional groups present in compounds of the present disclosure with certain parts known to those skilled in the art as "pro-moiety" (e.g., as described in "Design of Prodrugs", H. Bundgaard (Elsevier, 1985)).

The present disclosure also covers compounds of the present disclosure containing protective groups. In any process of preparing the compounds of the present disclosure, protecting sensitive or reactive groups on any of the molecules related may be necessary and/or desirable, thereby forming a form of chemical protection for the compounds of the present disclosure. This may be achieved by conventional protective groups, such as those described in T.W. Greene & P.G.M. Wuts, Protective Groups in Organic Synthesis, John Wiley & Sons, 1991, which are incorporated herein by reference. Using methods known in the art, the protective groups may be removed at an appropriate subsequent stage.

### Preparation method

In another aspect, the present disclosure provides a preparation method for the compound of formula I, wherein the method comprises the following steps:
when Y is absent, the compounds of formula I-TM1 and formula I-TM2 in the present disclosure may be synthesized by the following synthesis route: 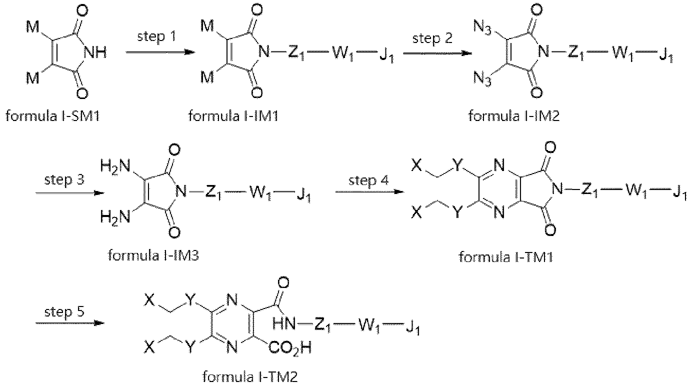 wherein
X, Z₁, W₁ and J₁ are as defined for the general formulas above;
M is a leaving group for substitution reaction, including but not limited to halogen, trifluoromethane sulfonate, and p-toluene sulfonate, preferably halogen.
Step 1
subject compounds of formula I-SM1 and M-Z₁-W₁-J₁ to a substitution reaction, obtaining a compound of formula I-IM1;
in some embodiments, the reaction is carried out under basic condition;
in some embodiments, the reaction is carried out at a suitable temperature and the temperature is 20 °C, 25 °C, 50 °C, 60 °C and 100 °C, preferably 20 °C;
in some embodiments, the reaction is carried out in a suitable solvent and the solvent includes but is not limited to acetone, tetrahydrofuran, dichloromethane, N, N-dimethylformamide, N-methylpyrrolidone and dimethyl sulfoxide, preferably acetone.
Step 2
subject the compound of formula I-IM1 and sodium azide to a substitution reaction, obtaining a compound of formula I-IM2;
in some embodiments, the reaction is carried out at a suitable temperature and the temperature is 20 °C, 25 °C, 50 °C, 60 °C and 100 °C, preferably 20 °C;
in some embodiments, the reaction is carried out in a suitable solvent and the solvent includes but is not limited to acetone, tetrahydrofuran, dichloromethane, N,N-dimethylformamide, N-methylpyrrolidone and dimethyl sulfoxide, preferably acetone.
Step 3
subject the compound of formula I-IM2 to a reduction reaction, obtaining a compound of formula I-IM3;
in some embodiments, this step is carried out in the presence of a suitable reducing agent and the reducing agent may be selected from a palladium catalyst, a platinum catalyst and a rhodium catalyst, preferably a palladium catalyst;
in some embodiments, this step is carried out at a suitable temperature and the temperature is 20°C, 25 °C, 50°C, 60 °C and 100 °C, preferably 20 °C;
in some embodiments, this step is carried out in a suitable organic solvent and the organic solvent may be selected from tetrahydrofuran, dichloromethane, N,N-dimethylformamide, N-methylpyrrolidone, dimethyl sulfoxide, n-heptane, n-hexane and ethyl acetate, preferably ethyl acetate.
Step 4
subject the compound of formula I-IM3 and 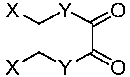 to a dehydration and ring-closure reaction, obtaining a compound of formula I-TM1;
in some embodiments, this step is carried out at a suitable temperature and the temperature is 20°C, 25 °C, 50°C, 60 °C and 100 °C, preferably 20 °C;
in some embodiments, this step is carried out in a suitable organic solvent and the organic solvent may be selected from acetonitrile, ethanol, tetrahydrofuran, dichloromethane, N,N-dimethylformamide, dimethyl sulfoxide, n-heptane, n-hexane and ethyl acetate, preferably acetonitrile.
Step 5
subject the compound of formula I-TM1 to a hydrolysis reaction, obtaining a compound of formula I-TM2;
in some embodiments, the reaction is carried out under basic condition, and the basic condition includes, but is not limited to, PB buffer with pH 7.0, 7.4, or 8.0;
in some embodiments, the reaction is carried out at a suitable temperature and the temperature is 20 °C, 25 °C, 50 °C, 60 °C and 100 °C, preferably 20 °C;
in some embodiments, the reaction is carried out in a suitable solvent and the solvent includes but is not limited to acetonitrile, tetrahydrofuran, dichloromethane, N,N-dimethylformamide and dimethyl sulfoxide, preferably acetonitrile.

When Y is absent, the compound of formula I-TM3 in the present disclosure may be synthesized via the following synthesis route: 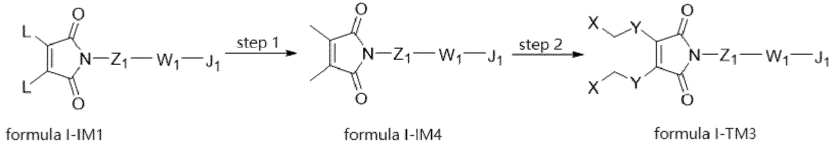 wherein,
X, Z₁, W₁ and J₁ are as defined for the general formula above;
L is a leaving group for substitution reaction, including but not limited to halogen, trifluoromethane sulfonate and p-toluene sulfonate, preferably halogen and OTf.
Step 1
subject the compound of formula I-IM1 to a coupling reaction, obtaining a compound of formula I-IM4;
in some embodiments, reagents for the coupling reaction include, but are not limited to, methylboric acid and trimethylboroxine, preferably trimethylboroxine;
in some embodiments, the coupling reaction is carried out under basic condition and the base includes but is not limited to triethylamine, DIPEA, NMM, sodium tert-butanol, potassium acetate, sodium acetate, cesium fluoride, potassium fluoride, potassium carbonate, sodium carbonate, sodium bicarbonate, cesium carbonate, potassium phosphate and potassium dihydrogen phosphate, preferably cesium fluoride.

In some embodiments, the coupling reaction is carried out in the presence of a catalyst and the catalyst includes, but is not limited to, tetrakis-triphenylphosphine palladium, palladium acetate, Pd₂(dba)₃, Pd(PPh₃)₂Cl₂, Pd(PPh₃)₂Cl₂/dichloromethane complex, Pd(dppf)Cl₂ and Pd(Amphos)Cl₂, preferably tetrakis-triphenylphosphine palladium.

In some embodiments, the coupling reaction is carried out at a temperature of 0-200°C, preferably at a temperature of 50-150 °C.

In some embodiments, the reaction is carried out in a suitable solvent and the solvent includes, but is not limited to, 1,4-dioxane, water, toluene, tetrahydrofuran, N,N-dimethylformamide, N-methylpyrrolidone, dimethyl sulfoxide and any combination thereof, preferably 1,4-dioxane.
Step 2
subject the compound of formula I-IM4 to a halogenation reaction, obtaining a compound of formula I-TM3;
in some embodiments, reagents for the halogenation reaction include, but are not limited to, bromine, N-iodosuccinimide, N-bromosuccinimide and N-chloro-succinimide, preferably N-bromosuccinimide;
in some embodiments, the halogenation reaction is carried out in the presence of a catalyst, and the catalyst is benzoyl peroxide.

In some embodiments, the halogenation reaction is carried out at a temperature of 0-200°C, preferably at a temperature of 50-150°C.

In some embodiments, the reaction is carried out in a suitable solvent and the solvent includes, but is not limited to, halogenated hydrocarbons (such as carbon tetrachloride, dichloromethane, chloroform and 1,2-dichloroethane), methanol, ethanol, DMF, acetonitrile, ethers (such as GDE (glycol dimethyl ether), tetrahydrofuran and dioxane), aromatics (such as toluene, benzene and xylene), water and any combination thereof, preferably carbon tetrachloride.

When Z₂ and W₂ are absent, the compound of formula II-TM1 in the present disclosure may be synthesized via the following synthesis route: 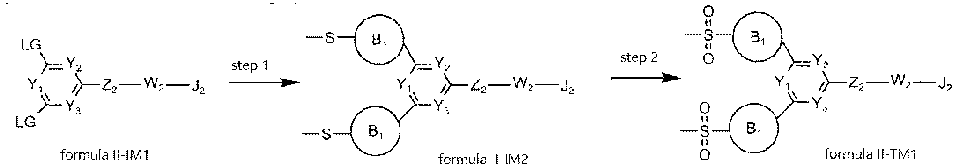 wherein
Y₁, Y₂, Y₃, B₁, B₂, Z₂, W₂ and J₂ are as defined for the general formula above;
LG is a leaving group for coupling reaction, including but not limited to halogen and trifluoromethane sulfonate, preferably halogen.
Step 1
subject the compound of formula II-IM1 to a coupling reaction, obtaining a compound of formula II-IM2;
in some embodiments, the reagent for the coupling reaction is 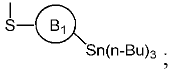
in some embodiments, the coupling reaction is carried out in the presence of a catalyst and the catalyst includes, but is not limited to, tetrakis-triphenylphosphine palladium, palladium acetate, Pd₂(dba)₃, Pd(PPh₃)₂Cl₂, Pd(PPh₃)₂Cl₂/dichloromethane complex, Pd(dppf)Cl₂ and Pd(Amphos)Cl₂, preferably tetrakis-triphenylphosphine palladium.

In some embodiments, the coupling reaction is carried out at a temperature of 0-200 °C, preferably at a temperature of 50-150 °C.

In some embodiments, the reaction is carried out in a suitable solvent and the solvent includes, but is not limited to, 1,4-dioxane, water, toluene, tetrahydrofuran, N,N-dimethylformamide, N-methylpyrrolidone, dimethyl sulfoxide and any combination thereof, preferably 1,4-dioxane.
Step 2
subject the compound of formula II-IM2 to an oxidation reaction, obtaining a compound of formula II-TM1;
in some embodiments, the oxidation reaction is carried out in the presence of an oxidant, and the catalyst is m-chloroperoxy benzoic acid.

In some embodiments, the oxidation reaction is carried out at a temperature of 0-120°C, preferably at a temperature of 50-80°C.

In some embodiments, the reaction is carried out in a suitable solvent and the solvent includes, but is not limited to, halogenated hydrocarbons (such as carbon tetrachloride, dichloromethane, chloroform and 1, 2-dichloroethane), methanol, ethanol, DMF, acetonitrile, ethers (such as GDE (glycol dimethyl ether), tetrahydrofuran and dioxane), aromatics (such as toluene, benzene and xylene), water and any combination thereof, preferably methanol.

In addition, the compounds of the present disclosure may also be prepared by a variety of methods known to those skilled in the field of organic synthesis. The compounds of the present disclosure may be synthesized using the methods described below as well as synthesis methods known in the field of synthetic organic chemistry or variations thereof known to a person skilled in the art. Preferred methods include, but are not limited to, those described above. The reaction may be carried out in solvents or mixtures of solvents suitable for the reagents and materials used and suitable for the conversion. Those skilled in the field of organic synthesis should understand that the functional groups present on the molecule should be consistent with the provided conversion. This will sometimes require a determination to modify the order of the synthesis steps or to select another particular method route relative to one method route to obtain the desired compound of the present disclosure.

It should also be recognized that another major consideration in the design of any synthesis route in the art is the correct selection of protective groups for the protection of reactive functional groups present in the compounds of the present disclosure. An authoritative description of various alternatives was introduced to those skilled in the art by Greene, et. al. (Protective Groups in Organic Synthesis, 4th Ed., Wiley-Interscience (2006)).

Substituents of compounds in the above routes are as defined in the present disclosure unless otherwise stated. Those skilled in the art will understand that one or more of the steps in the above routes may be omitted depending on a desired product structure. Those skilled in the art may also adjust the order of reaction steps as appropriate.

In some embodiments, the present disclosure further provides use of the compound for the preparation of a payload-linker compound;
preferably, the payload-linker compound is selected from the compounds described above or pharmaceutically acceptable salts, esters, stereoisomers, tautomers, polymorphs, solvates, N-oxides or isotope labeled compounds thereof;

The payload-linker compound is prepared from the compound described above by the following steps: 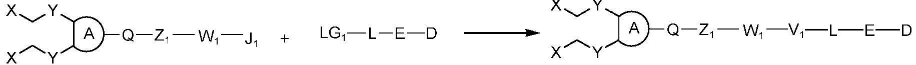 wherein X, Y, A, Q, Z₁, J₁, W₁, V₁, L, E and D are as defined above; LG₁ is selected from groups that condensate with J₁, and preferably, LG₁ is -COOH or -NH(R₁), wherein R₁ is H, C₁₋₆ alkyl or C₂₋₆ alkoxyalkyl.

In some embodiments, the present disclosure further provides use of the compound for the preparation of a payload-linker compound;
preferably, the payload-linker is selected from the compounds described above or the pharmaceutically acceptable salts, esters, stereoisomers, tautomers, polymorphs, solvates, N-oxides or isotope labeled compounds thereof;
preferably, the payload-linker compound is prepared from the compound described above by the following steps: 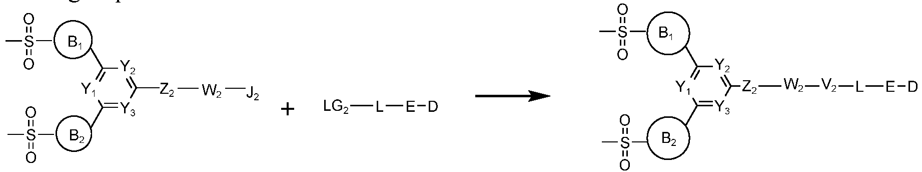
wherein B₁, B₂, Y₁, Y₂, Y₃, Z₂, J₂, W₂, V₂, L, E and D are as defined above; LG₂ is selected from groups that condensate with J₂, and preferably, LG₂ is -COOH or -NH(R₂), wherein R₂ is H, C₁₋₆ alkyl or C₂₋₆ alkoxyalkyl.

In some embodiments, the present disclosure further provides use of the compound for the preparation of a bioactive conjugate;
preferably, the bioactive conjugate is selected from the bioactive conjugates described above;
preferably, the bioactive conjugate is prepared from the compound described above by steps 1a and 1b below:
   step 1a: 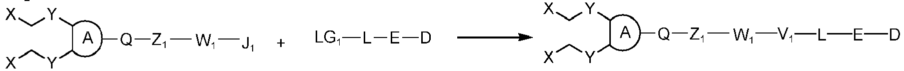
   step 1b: 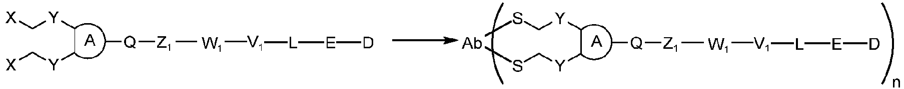
   wherein X, Y, A, Q, Z₁, J₁, W₁, V₁, L, E, D, Ab and n are as defined above; LG₁ is selected from groups that condensate with J₁, and preferably, LG₁ is -COOH or -NH(R₁), wherein R₁ is H, C₁₋₆ alkyl or C₂₋₆ alkoxyalkyl.

In some embodiments, the present disclosure further provides use of the compound for the preparation of a bioactive conjugate;
the bioactive conjugate is selected from the bioactive conjugates described above;
preferably, the bioactive conjugate is prepared from the compound described above by steps 2a and 2b below:
   step 2a: 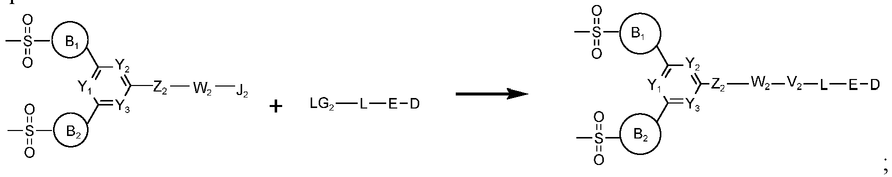
   step 2b: 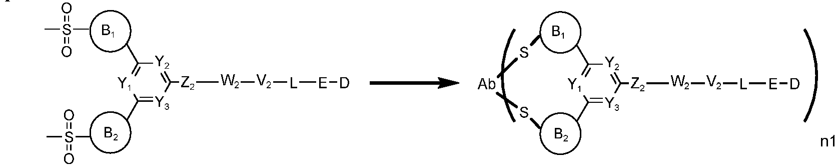
   wherein B₁, B₂, Y₁, Y₂, Y₃, Z₂, J₂, W₂, V₂, L, E, D, Ab and n1 are as defined above; LG₂ is selected from groups that condensate with J₂, and preferably, LG₂ is -COOH or -NH(R₂), wherein R₂ is H, C₁₋₆ alkyl or C₂₋₆ alkoxyalkyl.

In some embodiments, the present disclosure further provides a method for preparing a compound, and the method includes the following steps: 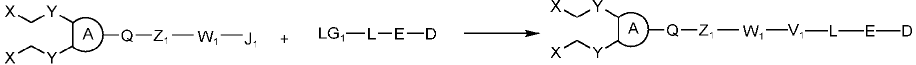 wherein X, Y, A, Q, Z₁, J₁, W₁, V₁, L, E and D are as defined above; LG₁ is selected from groups that condensate with J₁, and preferably, LG₁ is -COOH or -NH(R₁), wherein R₁ is H, C₁₋₆ alkyl or C₂₋₆ alkoxyalkyl.

In some embodiments, the present disclosure further provides a method for preparing a compound, and the method includes the following steps: 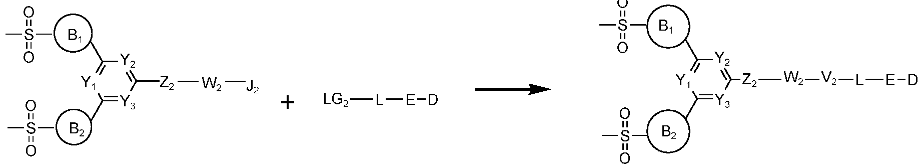 wherein B₁, B₂, Y₁, Y₂, Y₃, Z₂, J₂, W₂, V₂, L, E and D are as defined above; LG₂ is selected from groups that condensate with J₂, and preferably, LG₂ is -COOH or -NH(R₂), wherein R₂ is H, C₁₋₆ alkyl or C₂₋₆ alkoxyalkyl.

In some embodiments, the present disclosure further provides a method for preparing an antibody-drug conjugate, and the method includes the following steps:
step 1a: 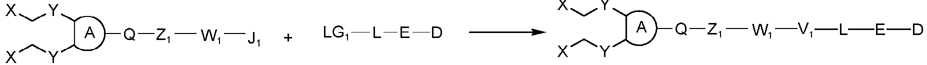
step 1b: 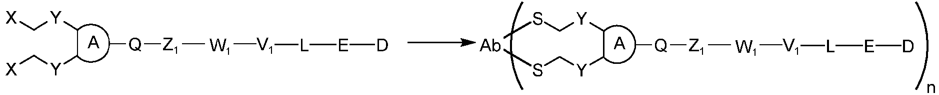
wherein X, Y, A, Q, Z₁, J₁, W₁, V₁, L, E, D, Ab and n are as defined above; LG₁ is selected from groups that condensate with J₁, and preferably, LG₁ is -COOH or -NH(R₁), wherein R₁ is H, C₁₋₆ alkyl or C₂₋₆ alkoxyalkyl.

In some embodiments, the present disclosure further provides a method for preparing an antibody-drug conjugate, and the method includes the following steps:
step 2a: 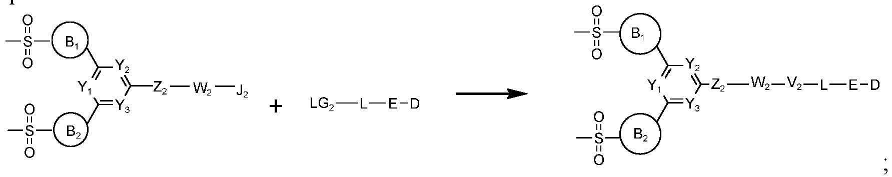
step 2b: 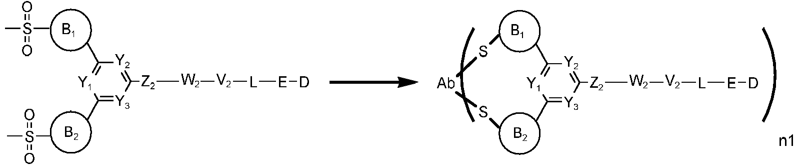
wherein B₁, B₂, Y₁, Y₂, Y₃, Z₂, J₂, W₂, V₂, L, E, D, Ab and n₁ are as defined above; LG₂ is selected from groups that condensate with J₂, and preferably, LG₂ is -COOH or -NH(R₂), wherein R₂ is H, C₁₋₆ alkyl or C₂₋₆ alkoxyalkyl.

### Pharmaceutical composition

The present disclosure further provides a pharmaceutical composition comprising the bioactive conjugate of the present disclosure and one or more pharmaceutically acceptable carriers. In some embodiments, the drug-to-antibody ratio (DAR) of the pharmaceutical composition is 1.0-6.0, such as 1, 2, 3, 4, 5 or 6, and such as 1.0-1.5, 1.0-2.0, 1.0-2.5, 1.0-3.0, 1.0-3.5, 1.0-4.0, 1.0-4.5, 1.0-5.0, 1.0-5.5, 1.0-6.0, 1.5-2.0, 1.5-2.5, 1.5-3.0, 1.5-3.5, 1.5-4.0, 1.5-4.5, 1.5-5.0, 1.5-5.5, 1.5-6.0, 2.0-2.5, 2.0-3.0, 2.0-3.5, 2.0-4.0, 2.0-4.5, 2.0-5.0, 2.0-5.5, 2.0-6.0, 2.5-3.0, 2.5-3.5, 2.5-4.0, 2.5-4.5, 2.5-5.0, 2.5-5.5, 2.5-6.0, 3.0-3.5, 3.0-4.0, 3.0-4.5, 3.0-5.0, 3.0-5.5, 3.0-6.0, 3.5-4.0, 3.5-4.5, 3.5-5.0, 3.5-5.5, 3.5-6.0, 4.0-4.5, 4.0-5.0, 4.0-5.5, 4.0-6.0, 4.5-5.0, 4.5-5.5, 4.5-6.0, 5.0-5.5, 5.0-6.0 or 5.5-6.0.

The pharmaceutic adjuvants used herein refer to excipients and additives used in the production and formulation of drugs, and refer to substances, other than active ingredients, that have been reasonably assessed for safety and are included in pharmaceutical preparations.

The pharmaceutical composition may be administered in any form, provided that, it achieves prevention, alleviation, or cure of symptoms in human or animal patients. For example, the pharmaceutical composition may be prepared into various suitable dosage forms according to the route of administration.

The present disclosure further provides a kit product, containing the bioactive conjugate of the present disclosure, or the pharmaceutical composition of the present disclosure, and an optional drug specification.

### Treatment methods and use

In another aspect, the present disclosure provides use of the bioactive conjugate in the preparation of a medicament for the prevention or treatment of a tumor disease.

In another aspect, the present disclosure provides the bioactive conjugate for the prevention or treatment of a tumor disease.

In another aspect, the present disclosure provides a method for the prevention or treatment of a tumor disease, including administration of an effective amount of the bioactive conjugate, or a pharmaceutical composition comprising the bioactive conjugate, to a subject in need.

In an embodiment of the present disclosure, the tumor disease is a solid tumor or a hematological malignant tumor, and is, for example, selected from colon cancer, stomach cancer, breast cancer, lung cancer (such as non-small cell lung cancer, specifically lung adenocarcinoma), and lymphoma.

As used herein, the term "effective amount" refers to the amount of a conjugate given to relieve to some extent one or more of the symptoms of a disease being treated.

Unless otherwise stated, as used herein, the term "treatment" means reversing, alleviating the progression of the applied disease or illness state or of one or more of the symptoms of such disease or illness state.

The term "individual" or "subject" as used herein includes human or non-human animals. Examples of human individuals include human individuals (called patients) who have a disease (such as the disease described herein) or normal individuals. The term "non-human animals" in the present disclosure includes all vertebrates, such as non-mammals (such as birds, amphibians and reptiles) and mammals, such as non-human primates, domestic animals and/or domesticated animals (such as sheep, dogs, cats, cows and pigs).

### Specific Mode for Carrying Out the Invention

The present disclosure will be described in detail below with reference to Examples and Test Examples. However, these examples are not intended to limit the scope of the present disclosure and may be modified without departing from the scope of the present disclosure.

### 1. Antibody Preparation

### 1.1 Preparation of the anti-ROR1 antibody 19F6_Hu35V1 of the present disclosure

In the early stage, the murine antibody 19F6 was obtained by immunizing Balb/c, C57Bl/6, NZB and A/J mice and screening hybridomas. After humanization, the humanized antibody 19F6_Hu35V1, wherein VH was as set forth in SEQ ID NO:1; VL was as set forth in SEQ ID NO:2; CH was the heavy chain constant region of human IgG1 (SEQ ID NO:18); and CL was the human kappa light chain constant region (SEQ ID NO: 19), was obtained. The coding DNA sequence of the above humanized antibody was synthesized and subjected to codon optimization, and then was cloned into the pcDNA3.4 plasmid. The pcDNA3.4 plasmids corresponding to the heavy chain and light chain of the humanized antibody were simultaneously transfected into Expi293F cells, and the expressed antibody in the supernatant was purified by Protein A to obtain corresponding antibody.

### Sequences information table-1 for 19F6 Hu35V1:

| Antib ody | Numbering system | SEQ ID NO: | | | | | | | | | |
|---|---|---|---|---|---|---|---|---|---|---|---|
| | | CDR H1 | CDR H2 | CDR H3 | CDR L1 | CDR L2 | CDR L3 | VH | VL | CH | CL |
| 19F6_ Hu35 V1 | Chothia | 3 | 4 | 5 | 6 | 7 | 8 | 1 | 2 | 18 | 19 |
| | AbM | 9 | 10 | 5 | 6 | 7 | 8 | | | | |
| | Kabat | 11 | 12 | 5 | 6 | 7 | 8 | | | | |
| | IMGT | 13 | 14 | 15 | 16 | 17 | 8 | | | | |

### Sequences information table-2 for 19F6_Hu35V1:

| SEQ ID NO: | Description | Sequence information |
|---|---|---|
| 1 | 19F6_Hu35V1 VH | QVQLQESGPGLVKPSQTLSLTCTVSGYSITSDYAWNW IRQFPGKGLEWIGYISYSGSIRYNPSLKSRVTISRDTSK NQFSLKLSSVTAADTAVYYCARGEITYYFDYWGQGT LVTVSS |
| 2 | 19F6_Hu35V1 VL | ETTLTQSPAFMSATPGDKVTISCKASTSIDDDMNWYQ QKPGEPPKLIISEGNTLRPGIPPRFSSSGYGTDFTFTINK IKSEDAATYYCLQSNDLPLTFGQGTKLEIK |
| 3 | 19F6_Hu35V1 Chothia CDR-H1 | GYSITSDY |
| 4 | 19F6_Hu35V1 Chothia CDR-H2 | SYSGS |
| 5 | 19F6_Hu35V1 Chothia/AbM/Ka bat CDR-H3 | GEITYYFDY |
| 6 | 19F6_Hu35V1 AbM/Kabat/Chot hia CDR-L1 | KASTSIDDDMN |
| 7 | 19F6_Hu35V1 AbM/Kabat/Chot hia CDR-L2 | EGNTLRP |
| 8 | 19F6_Hu35V1 AbM/Kabat/Chot hia /IMGT CDR-L3 | LQSNDLPLT |
| 9 | 19F6_Hu35V1 Abm CDR-H1 | GYSITSDYAWN |
| 10 | 19F6_Hu35V1 Abm CDR-H2 | YISYSGSIR |
| 11 | 19F6 Hu35V1 Kabat CDR-H1 | SDYAWN |
| 12 | 19F6_Hu35V1 Kabat CDR-H2 | YISYSGSIRYNPSLKS |
| 13 | 19F6_Hu35V1 IMGT CDR-H1 | GYSITSDYA |
| 14 | 19F6_Hu35V1 IMGT CDR-H2 | ISYSGSI |
| 15 | 19F6_Hu35V1 IMGT CDR-H3 | ARGEITYYFDY |
| 16 | 19F6_Hu35V1 IMGT CDR-L1 | TSIDDD |
| 17 | 19F6_Hu35V1 IMGT CDR-L2 | EGN |
| 18 | CH of 19F6_Hu35V1 | |
| 19 | CL of 19F6_Hu35V1 | |

### 1.2 Preparation of B7-H3 antibody 2#8890 of the present disclosure

Human B7-H3-4Ig-His protein was used to immunize fully-human antibody transgenic mice, and serum titers were monitored by ELISA and FACS. The best mice were selected according to the titer results, spleen cells were fused, screened and subcloned, and different monoclones were tested for binding affinity to human\monkey proteins and cells to obtain the preferred clone 20G11G6. The antibody sequence was modified by PTM site removal, PI reduction, and ADCC removal from the CH, and finally the fully human antibody 2#8890 was obtained. The codon optimization and gene synthesis for the antibody was done by Nanjing GenScript, and the gene was constructed into the pTT5 plasmid. The heavy chain and light chain plasmids were transfected into CHO-S cells simultaneously, and the expressed antibody in the supernatant was purified by Protein A to obtain the corresponding antibody protein 2#8890. The amino acid sequences of the heavy and light chains in 2#8890 are shown in SEQ ID NO: 20 and SEQ ID NO: 21, respectively.

### Sequences information table of 2# 8890:

| SEQ ID NO: | Sequence Name | Sequence Information |
|---|---|---|
| 20 | Amino acid sequence of heavy chain of antibody 2#8890 | QVQLQESGPGLVQPSGTLSLTCAVSGGSDSSTNWWT WVRQSPGQGLEWIGEISPIGFTSYSPSLRSRVTISVDKS KTQFSLKLSSVTAADTAVYYCARDRLYFAFWGQGTL VTVSSASTKGPSVFPLAPSSKSTSGGTAALGCLVKDYF PEPVTVSWNSGALTSGVHTFPAVLQSSGLYSLSSVVT VPSSSLGTQTYICNVNHKPSNTKVDKKVEPKSCDKTH TCPPCPAPEAAGAPSVFLFPPKPKDTLMISRTPEVTCV VVDVSHEDPEVKFNWYVDGVEVHNAKTKPREEQYN STYRVVSVLTVLHQDWLNGKEYKCKVSNKALPAPIE KTISKAKGQPREPQVYTLPPSRDELTKNQVSLTCLVK GFYPSDIAVEWESNGQPENNYKTTPPVLDSDGSFFLYS KLTVDKSRWQQGNVFSCSVMHEALHNHYTQKSLSLS PGK |
| 21 | Amino acid sequence of light chain of antibody 2#8890 | EIVMTQSPATLSVSPGERATLSCRASQSVSSNLAWYQ QKPGQAPRLLIYGASTRATGIPDRFSGSGSGTEFTLTIS SLESEDFAVYYCQQYNNWPITFGQGTRLEIKRTVAAP SVFIFPPSDEQLKSGTASVVCLLNNFYPREAKVQWKV DNALQSGNSQESVTEQDSKDSTYSLSSTLTLSKADYE KHKVYACEVTHQGLSSPVTKSFNRGEC |

### 2. Synthesis of payload-linker

The abbreviations as used herein have the following meanings:

| Abbreviatio | Meaning | Abbreviatio | Meaning |
|---|---|---|---|
| ns | | ns | |
| NBS | N-bromosuccinimide | TFA | trifluoroacetic acid |
| HOBt | 1-Hydroxybenzotriazole | DIPEA | N, N-Diisopropylethylamine |
| DIC | N,N-diisopropylcarbodiimide | HATU | O-(7-azabenzotriazole-1-yl)-N,N,N',N'-tetramethyluronium hexafluorophosphate |
| EEDQ | 2-ethoxy-1-ethoxycarbonyl-1,2-dihydroquinoline | DIAD | Diisopropyl azodicarboxylate |
| IPA | Isopropyl alcohol | EDCI | 1-(3-Dimethylaminopropyl)-3-ethylcarbodiimide hydrochloride |
| Pd/C | palladium on carbon | DMTMM | 4-(4,6-Dimethoxytriazin-2-yl)-4-methylmorpholine hydrochloride |
| NaIO₄ | Sodium periodate | RuCl₃.H₂O | Ruthenium trichloride hydrate |
| XPhos Pd G3 | Palladium (2-dicyclohexylphosphino-2',4',6'-tri-isopropyl-1,1'-biphenyl(2'-amino-1,1'-biphenyl-2-yl) methanesulfonate (II) | LiOH.H₂O | Lithium hydroxide hydrate |
| K₃PO₄ | potassium phosphate | m-CPBA | M-chloroperoxybenzoic acid |
| THF | tetrahydrofuran | DMSO | Dimethyl sulfoxide |
| DCM | dichloromethane | MeOH | methanol |
| HPLC | High performance liquid chromatography | LC-MS | Liquid chromatography-mass spectrometry |
| TCEP | Tris(2-carboxyethyl)phosphine | Na₂HPO₄ | Disodium hydrogen phosphate |
| EDTA | Ethylenediaminetetraacetic acid | PB | Phosphate buffer |
| DAR | Drug-to-antibody ratio | DMF | N,N-Dimethylformamide |

The structures of the compounds described in the following examples are determined by nuclear magnetic resonance (¹H NMR) or mass spectrometry (MS).

The ¹H NMR instrument used was Bruker 400 MHz nuclear magnetic resonance instrument; deuterated chloroform (CDCl₃); the internal standard substance was tetramethylsilane (TMS).

The abbreviations in the NMR spectra used in the examples are shown below.

s: singlet, d: doublet, t: triplet, q: quartet, m: multiplet, br: broad, J: Coupling constant, Hz: Hertz, CDCl₃: deuterated chloroform. δ values are expressed in ppm.

The mass spectrometry (MS) instrument used was Agilent (ESI) mass spectrometer, model: Agilent 6120B.

### Example 1 2-(2,3-bis(bromomethyl)-5,7-dioxo-5,7-dihydro-6H-pyrrolo[3,4-b]pyrazin-6-yl) acetic acid (I-1)

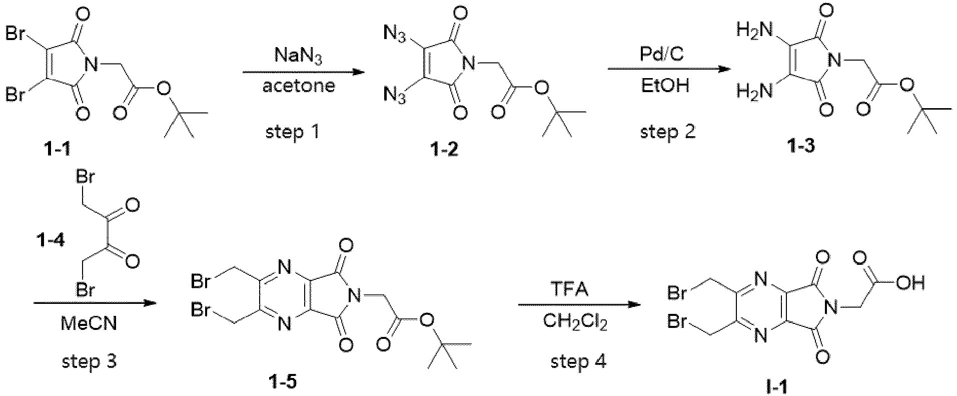

### Step 1: Synthesis of tert-butyl 2-(3,4-diazido-2,5-dioxo-2,5-dihydro-1H-pyrrol-1-yl)acetate (1-2)

Compound 1-1 (0.40 g, 1.08 mmol, the synthesis method refers to patent WO2019057964) and sodium azide (141.00 mg, 2.17 mmol) was dissolved in acetone (10 mL), and the resulting solution was reacted for 8 h at 25 °C. The reaction was monitored by HPLC-MS. Water was added to the reaction solution and extraction with ethyl acetate was carried out. The organic phases were combined, dried with anhydrous sodium sulfate, and filtered. The filtrate was concentrated under reduced pressure to obtain 0.28 g of crude title compound, which was directly used in the next reaction without purification.

The structural characterization data were as follows:
ESI-MS (m/z):311.0[M+18]⁺.

### Step 2: Synthesis of tert-butyl 2-(3,4-diamino-2,5-dioxo-2,5-dihydro-1H-pyrrol-1-yl)acetate (1-3)

Compound 1-2 (0.15 g, 0.51 mmol) and 10% palladium on carbon (15.00 mg) were dissolved in ethanol (20 mL), hydrogen replacement was carried out three times, and the reaction was carried out for 3 h under hydrogen atmosphere at 25 °C. The reaction was monitored by HPLC-MS. The reaction solution was filtered with diatomaceous earth, and the filtrate was concentrated under reduced pressure to obtain 101.00 mg of crude title compound, which was directly used in the next reaction without purification.

The structural characterization data were as follows:
ESI-MS (m/z):185.9[M-56]⁺.

### Step 3: Synthesis of tert-butyl 2-(2,3-bis(bromomethyl)-5,7-dioxo-5,7-dihydro-6H-pyrrol o[3,4-b]pyrazin-6-yl)acetate (1-5)

Compound 1-3 (50.00 mg, 0.21 mmol) and compound 1-4 (54.00 mg, 0.21 mmol) were dissolved in acetonitrile (5 mL), and the resulting solution was reacted for 3 h at 25 °C. The reaction was monitored by HPLC-MS. The reaction solution was purified directly by a flash silica gel column (petroleum ether: ethyl acetate=5:1) to obtain 40.00 mg of the title compound.

The structural characterization data were as follows:
ESI-MS (m/z):466.8[M+18]⁺.
¹H NMR (400 MHz, CDCl₃): δ 4.88 (s, 4H), 4.47 (s, 2H), 1.48 (s, 9H).

### Step 4: Synthesis of 2-(2,3-bis(bromomethyl)-5,7-dioxo-5,7-dihydro-6H-pyrrolo[3,4-b]p yrazin-6-yl)acetic acid (I-1)

Compound 1-5 (20.00 mg, 0.05 mmol) was dissolved in dichloromethane (5 mL) and trifluoroacetic acid (1 mL), and the resulting solution was reacted for 5 h at 25 °C. The reaction was monitored by HPLC-MS. The reaction solution was directly concentrated under reduced pressure to obtain 15.00 mg of crude title compound, which was directly used in the next reaction without purification.

The structural characterization data were as follows:
ESI-MS (m/z):410.8[M+18]⁺.

### Example 2 2-(3,4-bis(bromomethyl)-2,5-dioxo-2,5-dihydro-1H-pyrrol-1-yl)acetic acid (I-2)

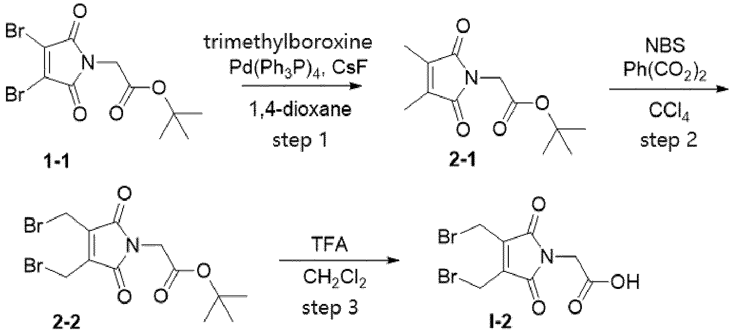

### Step 1: Synthesis of tert-butyl 2-(3,4-dimethyl-2,5-dioxo-2,5-dihydro-1H-pyrrol-1-yl)acetate (2-1)

Under the protection of nitrogen, compound 1-1 (0.20 g, 0.54 mmol), trimethylboroxine (0.34 g, 2.71 mmol), cesium fluoride (0.41 g, 2.71 mmol) and tetrakis-triphenylphosphine palladium (63.00 mg, 0.05 mmol) were dissolved in 1,4-dioxane (20 mL), and the resulting solution was reacted for 3 h at 110 °C. The reaction was monitored by HPLC-MS. The reaction solution was purified directly by a flash silica gel column (petroleum ether: ethyl acetate=5:1) to obtain 80.00 mg of the title compound.

The structural characterization data were as follows:
ESI-MS (m/z):184.0[M+H-56]⁺.

### Step 2: Synthesis of tert-butyl 2-(3,4-bis(bromomethyl)-2,5-dioxo-2,5-dihydro-1H-pyrrol -1-yl)acetate (2-2)

Under the protection of nitrogen, compound 2-1 (72.00 mg, 0.30 mmol), NBS (118.00 mg, 0.66 mmol) and dibenzoyl peroxide (7.00 mg, 0.03 mmol) were dissolved in carbon tetrachloride (8 mL), and the resulting solution was reacted for 12 h at 85 °C. The reaction was monitored by HPLC-MS. The reaction solution was purified directly by a flash silica gel column (petroleum ether: ethyl acetate=10:1) to obtain 65.00 mg of the title compound.

The structural characterization data were as follows:
ESI-MS (m/z):414.8[M+18]⁺.
¹H-NMR (400 MHz, CDCl₃): δ 4.28 (s, 4H), 4.20 (s, 2H), 1.45 (s, 9H).

### Step 3: Synthesis of 2-(3,4-bis(bromomethyl)-2,5-dioxo-2,5-dihydro-1H-pyrrol-1-yl)acetic acid (I-2)

Compound 2-2 (60.00 mg, 0.15 mmol) was dissolved in dichloromethane (10 mL) and trifluoroacetic acid (2 mL), and the resulting solution was reacted for 5 h at 25 °C. The reaction was monitored by HPLC-MS. The reaction solution was directly concentrated under reduced pressure to obtain 50.00 mg of crude title compound, which was directly used in the next reaction without purification.

The structural characterization data were as follows:
ESI-MS (m/z):358.9[M+18]⁺.

### Example 3 4-((S)-2-((S)-2-(2-(3,4-bis(bromomethyl)-2,5-dioxo-2,5-dihydro-1H-pyrrol-1-y l)acetamido)-3-methylbutanamido)-5-ureidopentanamido)benzyl ((S)-1-((3R,4S,5S)-1-((S)-2-((1R,2R)-3-((1S),2R)-1-hydroxy-1-phenylpropan-2-yl)amino)-1-methoxy-2-methyl-3-oxopropyl) pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate (payload-linker 3)

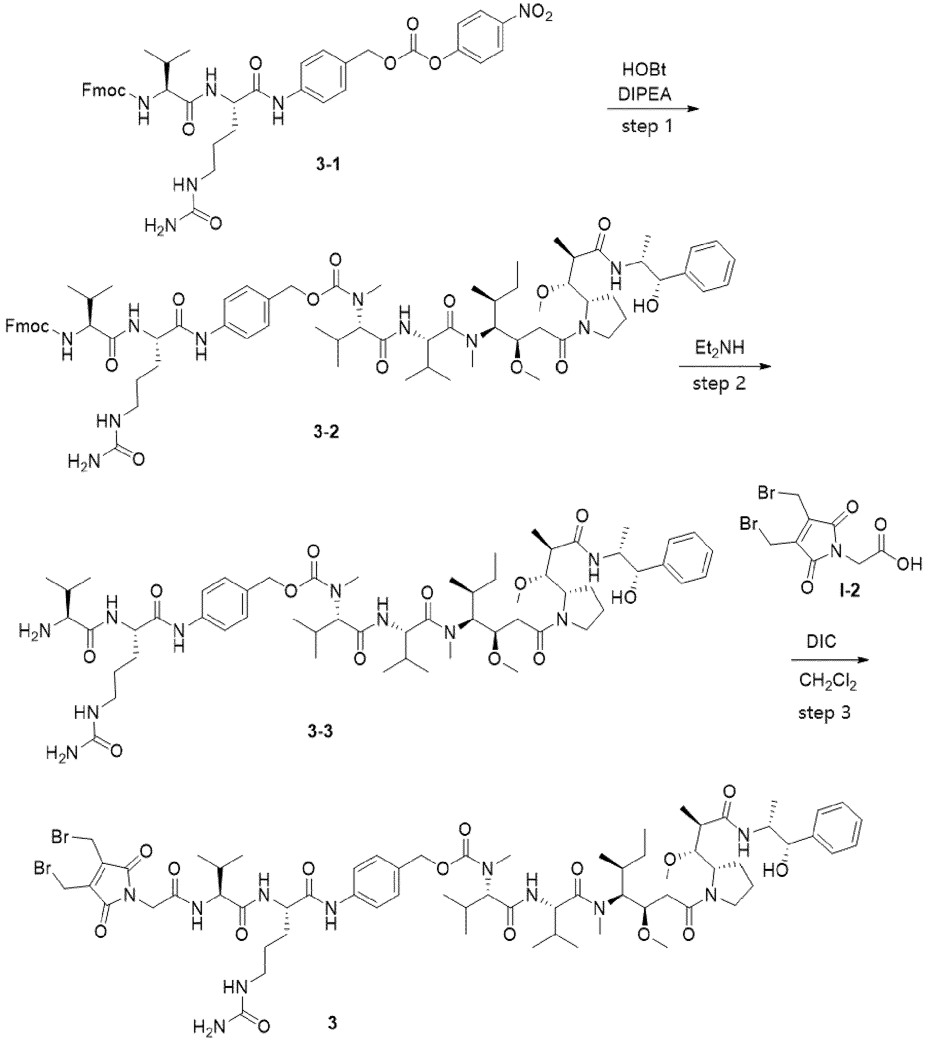

### Step 1: Synthesis of 4-((S)-2-((S)-2-(((9H-fluoren-9-yl)methoxy)carbonyl)amino)-3-meth ylbutanamido)-5-ureidopentanamido)benzyl ((S)-1-((S)-1-((3R,4S,5S)-1-((S)-2-((1R,2R)-3-((1S, 2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-met hyl-1-oxobutan-2-yl)(methyl)carbamate (3-2)

(S)-N-((3R,4S,5S)-1-((S)-2-((1R,2R)-3-((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptyl-4-yl)-N,3-dimethyl-2-((S)-3-methyl-2-(methylamino)butyramide (30.00 mg, 0.04 mmol) and compound 3-1 (38.50 mg, 0.05 mmol) were dissolved in DMF (3 mL) at 25 °C, followed by addition of HOBt (8.46 mg, 0.06 mmol) and DIPEA (10.80 mg, 0.08 mmol), and the resulting solution was reacted for 2 h at 25 °C. The reaction was monitored by HPLC-MS. The reaction solution was directly used in the next reaction without treatment.

The structural characterization data were as follows:
ESI-MS (m/z): 1345.2 [M+H]⁺.

### Step 2: Synthesis of 4-((S)-2-((S)-2-amino-3-methylbutanamido)-5-ureidopentanamido)b enzyl((S)-1-((S)-1-((3R,4S,5S)-1-((S)-2-((1R,2R)-3-((1S,2R)-1-hydroxy-1-phenylpropan-2-yl)a mino)-1-methoxy-2-methyl-3-oxopropyl)pyrrolidin-1-yl)-3-methoxy-5-methyl-1-oxoheptyl-4-yl) (methyl)amino)-3-methyl-1-oxobutan-2-yl)amino)-3-methyl-1-oxobutan-2-yl(methyl)carbamate (3-3)

Diethylamine (0.30 mL) was added to the reaction solution of compound 3-2 at 25 °C, and the resulting solution was reacted for 2 h at 25 °C. The reaction was monitored by HPLC-MS. The reaction solution was concentrated under reduced pressure, the concentrate was directly purified by preparative high performance liquid chromatography, and the preparation was lyophilized to obtain 36.30 mg of formate of the title compound.
Chromatographic column: SunFire Prep C18 OBD 5µm 19x150 mm

### Mobile phase A: acetonitrile Mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 18.00 | 90 | 10 | 28 |

The structural characterization data were as follows:
ESI-MS (m/z): 1123.2 [M+H]⁺.

### Step 3: Synthesis of 4-((S)-2-((S)-2-(2-(3,4-bis(bromomethyl)-2,5-dioxo-2,5-dihydro-1H-pyrrol-1-yl)acetamido)-3-methylbutanamido)-5-ureidopentamido)benzyl ((S)-1-((3R,4S,5S)-1-((S)-2-((1R,2R)-3-((1S),2R)-1-hydroxy-1-phenylpropan-2-yl)amino)-1-methoxy-2-methyl-3-oxo propyl)pyrrolidin-1-yl)-3-methoxy-5-methyl-1-oxoheptan-4-yl)(methyl)amino)-3-methyl-1-oxob utan-2-yl)amino)-3-methyl-1-oxobutan-2-yl)(methyl)carbamate (3)

Compound I-2 (12.75 mg, 0.04 mmol) was dissolved in dichloromethane (3 mL), then DIC (2.36 mg, 0.02 mmol) and Compound 3-3 (21.00 mg, 0.02 mmol) were added, and the resulting solution was reacted for 1 h at 25 °C. The reaction was monitored by HPLC-MS. The reaction solution was concentrated under reduced pressure, the concentrate was directly purified by preparative high performance liquid chromatography, and the preparation was lyophilized to obtain 14.30 mg of the title compound.
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 17.00 | 90 | 10 | 28 |

The structural characterization data were as follows:
ESI-MS (m/z):1445.9[M+H]⁺.

### Example 4 (S)-2-((2S,13S)-13-benzyl-22-(3,4-bis(bromomethyl)-2,5-dioxo-2,5-dihydro-1 H-pyrrol-1-yl)-2-isopropyl-3-methyl-4,9,12,15,18,21-hexaoxo-6-oxa-3,8,11,14,17,20-hexaazado cosanamido)-N-((3R,4S,5S)-1-((S)-2-((1R,2R)-3-(((1S,2R)-1-hydroxy-1-phenylpropan-2-yl)ami no)-1-methoxy-2-methyl-3-oxopropan)pyrrolidin-1-yl)-3-methoxy-5-methyl-1-oxoheptan-4-yl)-N,3-dimethyl butyramide (payload-linker 4)

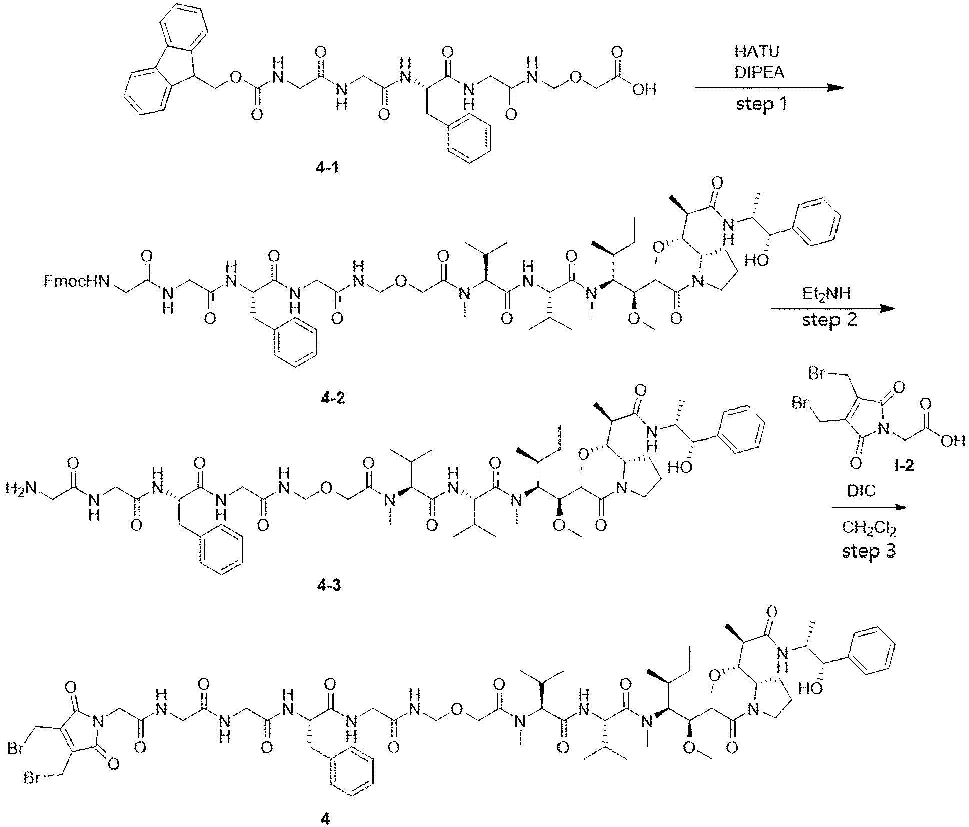

### Step 1: Synthesis of (9H-fluoren-9-yl)methyl((3R,4S,7S,10S,21S)-21-benzyl-4-((S)-sec-b utyl)-3-(2-((S)-2-((1R,2R)-3-(((1S,2R)-1-hydroxy-1-phenylpropan-2-yl)amino)-1-methoxy-2-me thyl-3-oxopropyl)pyrrolidin-1-yl)-2-oxoethyl)-7,10-diisopropyl-5,11-dimethyl-6,9,12,17,20,23,2 6-heptaoxo-2,14-dioxa-5,8,11,16,19,22,25-heptaazaheptacosan-27-yl)carbamate (4-2)

Compound 4-1 (50.00 mg, 0.08 mmol) and (S)-N-((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)-N,3-dimethyl-2-((S)-3-methyl-2-(methylamino)butyrylamido)butyramide (55.60 mg, 0.08 mmol) were weighed and dissolved in DMF (1 mL), then HATU (32.37 mg, 85.18 µmol) and DIPEA (20.02 mg, 154.88 µmol) were added, and the resulting solution was reacted for 1 h at room temperature. The reaction was monitored by HPLC-MS. The reaction solution was directly purified by preparative high performance liquid chromatography to obtain 35.00 mg of the title compound.
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 40 | 60 | 28 |
| 2.00 | 40 | 60 | 28 |
| 18.00 | 90 | 10 | 28 |

The structural characterization data were as follows:
ESI-MS (m/z):1345.1 [M+H]⁺.

### Step 2: Synthesis of (S)-2-((2S,13S)-19-amino-13-benzyl-2-isopropyl-3-methyl-4,9,12,1 5,18-pentaoxo-6-oxa-3,8,11,14,17-pentaazanonadecanamido)-N-((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidi n-1-yl)-3-methoxy-5-methyl-1-oxoheptan-4-yl)-N,3-dimethylbutanamide (4-3)

Compound 4-2 (20.00 mg, 0.02 mmol) was dissolved in dichloromethane (2 mL), followed by addition of diethylamine (1 mL), and the resulting solution was reacted for 1 h at room temperature. The reaction was monitored by HPLC-MS. The reaction solution was concentrated under reduced pressure, the concentrate was directly purified by preparative high performance liquid chromatography, and the preparation was lyophilized to obtain 15.00 mg of formate of the title compound.
Chromatographic column: SunFire Prep C18 OBD 19 mm×150 mm×5.0µm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 10 | 90 | 28 |
| 2.00 | 10 | 90 | 28 |
| 18.00 | 90 | 10 | 28 |

The structural characterization data were as follows:
ESI-MS (m/z):1123.1[M+H]⁺.

### Step 3: Synthesis of (S)-2-((2S,13S)-13-benzyl-22-(3,4-bis(bromomethyl)-2,5-dioxo-2,5-dihydro-1H-pyrrol-1-yl)-2-isopropyl-3-methyl-4,9,12,15,18,21-hexaoxo-6-oxa-3,8,11,14,17,20-h exaazadocosanamido)-N-((3R,4S,5S)-1-((S)-2-((1R,2R)-3-(((1S,2R)-1-hydroxy-1-phenylpropan-2-yl)amino)-1-methoxy-2-methyl-3-oxopropan)pyrrolidin-1-yl)-3-methoxy-5-methyl-1-oxohepta n-4-yl)-N,3-dimethylbutanamide (4)

Compound I-2 (4.00 mg, 12.0 µmol) was dissolved in dichloromethane (3 mL), then DIC (1.12 mg, 8.00 µmol) and Compound 4-3 (6.58 mg, 6.00 µmol) were added, and the resulting solution was reacted for 1 h at 25 °C. The reaction was monitored by HPLC-MS. The reaction solution was concentrated under reduced pressure, the concentrate was directly purified by preparative high performance liquid chromatography, and the preparation was lyophilized to obtain 4.77 mg of the title compound.
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 35 | 65 | 28 |
| 18.00 | 90 | 10 | 28 |

The structural characterization data were as follows:
ESI-MS (m/z):1445.9[M+H]⁺.

### Example 5 N-(((3R,4S,7S,10S,21S)-21-benzyl-4-((S)-sec-butyl)-3-(2-((S)-2-((1R,2R)-3-((((1S,2R)-1-hydroxy-1-phenylpropan-2-yl)amino)-1-methoxy-2-methyl-3-oxopropyl)pyrrolidin-1-yl)-2-oxoethyl)-7,10-diisopropyl-5,11-dimethyl-6,9,12,17,20,23,26-heptaoxo-2,14-dioxa-5,8,1 1,16,19,22,25-heptaazaheptacosan-27-yl)-1-(2-(3,4-bis(bromomethyl)-2,5-dioxa-2-,5-dihydro-1 H-pyrrol-1-yl)acetamido)-3,6,9,12-tetraoxapentadecane-pentadecanamide (payload-linker 5)

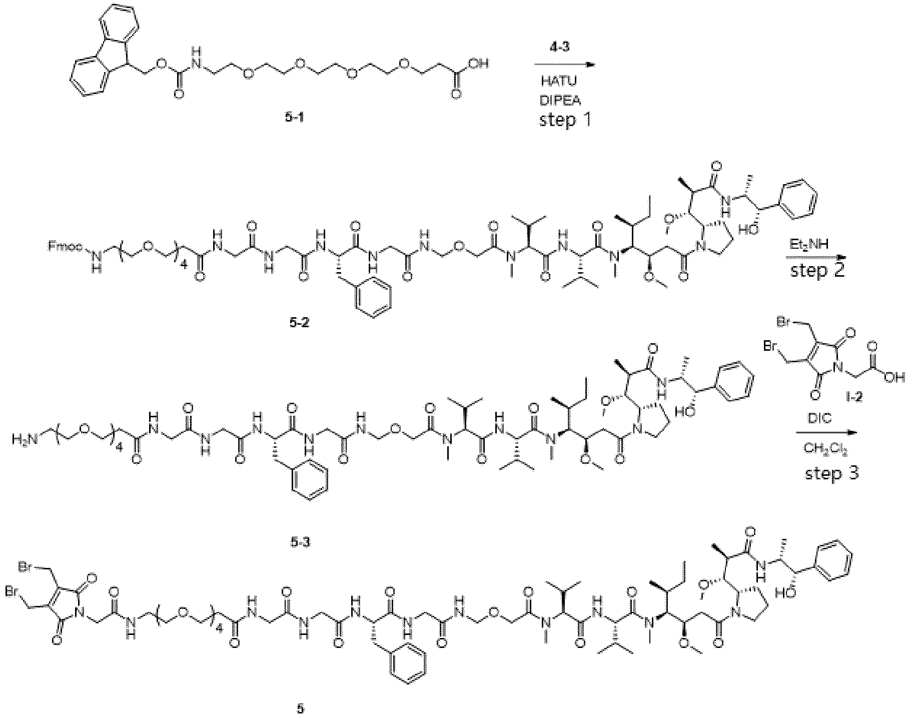

### Step 1: Synthesis of (9H-fluoren-9-yl)methyl((3R,4S,7S,10S,21S)-21-benzyl-4-((S)-sec-b utyl)-3-(2-((S)-2-(((1R,2R)-3-(((1S,2R)-1-hydroxy-1-phenylpropan-2-yl)amino)-1-methoxy-2-m ethyl-3-oxopropyl)pyrrolidin-1-yl)-2-oxoethyl)-7,10-diisopropyl-5,11-dimethyl-6,9,12,17,20,23, 26,29-octaoxo-2,14,32,35,38,41-hexaoxa-5,8,11,16,19,22,25,28-octaazatritetracontan-43-yl)carb amate (5-2)

Compound 5-1 (5.21 mg, 11.00 µmol) and 4-3 (10.00 mg, 9.00 µmol) were weighed and dissolved in DMF(1 mL), followed by addition of HATU (4.06 mg, 11.00 µmol) and DIPEA (2.30 mg, 18.00 µmol), and the resulting solution was reacted for 1 h at room temperature. The reaction was monitored by HPLC-MS. The reaction solution was directly purified by preparative high performance liquid chromatography to obtain 9.00 mg of the title compound.
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 20 | 80 | 28 |
| 16.00 | 90 | 10 | 28 |

The structural characterization data were as follows:
ESI-MS (m/z):1592.8[M+H]⁺.

### Step 2: Synthesis of 1-amino-N-(((3R,4S,7S,10S,21S)-21-benzyl-4-((S)-sec-butyl)-3-(2-((S)-2- ((1R,2R)-3-(((((S,2R)-1-hydroxy-1-phenylpropanyl-2-yl)amino)-1-methoxy-2-methyl-3-oxopropyl)pyrrolidin-1-yl)-2-oxoethyl)-7,10-diisopropyl-5,11-dimethyl-6,9,12,17,20,23,26-hept aoxo-2,14-dioxa-5,8,11,16,19,22,25-heptaazaheptacosan-27-yl)-3,6,9,12-tetraoxapentadecan-15-amide (5-3)

Compound 5-2 (10.00 mg, 6.00 µmol) was dissolved in dichloromethane (2 mL), then diethylamine (1 mL) was added, and the resulting solution was reacted for 1 h at room temperature. The reaction was monitored by HPLC-MS. The reaction solution was concentrated under reduced pressure, the concentrate was directly purified by preparative high performance liquid chromatography, and the preparation was lyophilized to obtain 7.00 mg of formate of the title compound.
Chromatographic column: SunFire Prep C18 OBD 5µm 19x150 mm

### Mobile phase A: acetonitrile; Mobile phase B: water (0.05% formic acid

| Time [min] | Mobile phase A [%] | Mobile phase B [%] | Flow rate [mL/min] |
|---|---|---|---|
| 0.00 | 10 | 90 | 28 |
| 20.00 | 90 | 10 | 28 |

The structural characterization data were as follows:
ESI-MS (m/z):1370.9[M+H]⁺.

### Step 3: Synthesis of N-(((3R,4S,7S,10S,21S)-21-benzyl-4-((S)-sec-butyl)-3-(2-((S)-2-((1R,2R)-3-((((1S,2R)-1-hydroxy-1-phenylpropan-2-yl)amino)-1-methoxy-2-methyl-3-oxopropyl)pyrrolidin-1-yl)-2-oxoethyl)-7,10-diisopropyl-5,11-dimethyl-6,9,12,17,20,23,26-heptaoxo-2,14-dioxa-5,8,11,16,19,22,25-heptaazaheptacosan-27-yl)-1-(2-(3,4-bis(bromomethyl)-2,5-dioxa-2-,5-dihydro-1H-pyrrol-1-yl)acetamido)-3,6,9,12-tetraoxapentadecane-pentadecanamide (5)

Compound I-2 (4.00 mg, 12.00 µmol) was dissolved in dichloromethane (2 mL), then DIC (1.12 mg, 8.00 µmol) and Compound 5-3 (8.04 mg, 6.00 µmol) were added, and the resulting solution was reacted for 1 h at 25 °C. The reaction was monitored by HPLC-MS. The reaction solution was concentrated under reduced pressure, the concentrate was directly purified by preparative high performance liquid chromatography, and the preparation was lyophilized to obtain 4.68 mg of the title compound.
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 35 | 65 | 28 |
| 18.00 | 90 | 10 | 28 |

The structural characterization data were as follows:
ESI-MS (m/z):1693.7[M+H]⁺.

### Example 6 (S)-2-((2S,13S)-13-benzyl-22-(2,3-bis(bromomethyl)-5,7-dioxo-5,7-dihydro-6 H-pyrrolo[3,4-b]pyrazin-6-yl)-2-isopropyl-3-methyl-4,9,12,15,18,21-hexaoxo-6-oxa-3,8,11,14,1 7,20-hexaazadocosanamide)-N-(((3R,4S,5S)-1-((S)-2-((1R,2R)-3-(((1S,2R)-1-hydroxy-1-phenyl propan-2-yl)amino))-1-methoxy-2-methyl-3-oxopropyl)pyrrolidin-1-yl)-3-methoxy-5-methyl-1-oxoheptan-4-yl)-N,3-dimethylbutanamide (payload-linker 6)

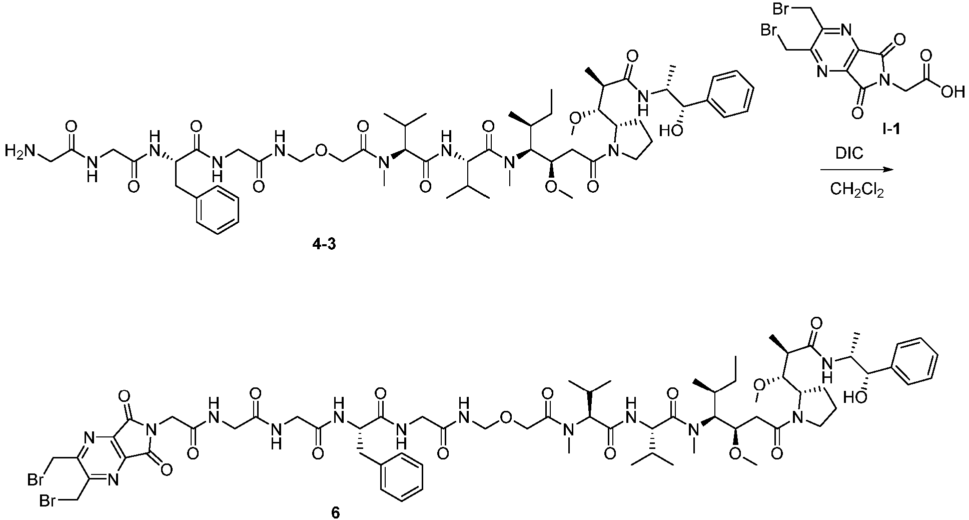

Compound I-1 (6.03 mg, 15.00 µmol) was dissolved in dichloromethane (2 mL), then DIC (1.45 mg, 12.00 µmol) and Compound 4-3(8.62 mg, 8.00 µmol) were added, and the resulting solution was reacted for 1 h at 25 °C. The reaction was monitored by HPLC-MS. The reaction solution was concentrated under reduced pressure, the concentrate was directly purified by preparative high performance liquid chromatography, and the preparation was lyophilized to obtain 4.21 mg of the title compound.
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 18.00 | 90 | 10 | 28 |

The structural characterization data were as follows:
ESI-MS (m/z):1498.5[M+H]⁺.

### Example 7 N-(((3R,4S,7S,10S,21S)-21-benzyl-4-((S)-sec-butyl)-3-(2-((S)-2-((1R,2R)-3-((((1S,2R)-1-hydroxy-1-phenylpropan-2-yl)amino)-1-methoxy-2-methyl-3-oxopropyl)pyrrolidin-1-yl)-2-oxoethyl)-7,10-diisopropyl-5,11-dimethyl-6,9,12,17,20,23,26-heptaoxo-2,14-dioxa-5,8,1 1,16,19,22,25-heptaazaheptacosan-27-yl)-1-(2-(2,3-bis(bromomethyl)-5,7-dioxo-5,7-dihydro-6H -pyrrolo[3,4-b]pyrazin-6-yl)acetamido)-3,6,9,12-tetraoxapentadecan-15-amide (payload-linker 7)

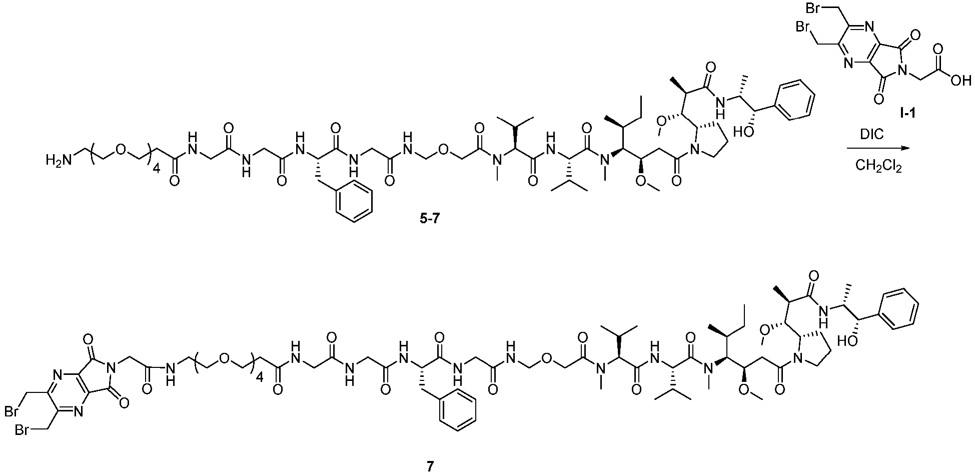

Compound I-1 (12.06 mg, 0.03 mmol) was dissolved in dichloromethane (4 mL), then DIC (3.00 mg, 0.02 mmol) and Compound 5-3 (20.00 mg, 15.00 µmol) were added, and the resulting solution was reacted for 1 h at 25 °C. The reaction was monitored by HPLC-MS. The reaction solution was concentrated under reduced pressure, the concentrate was directly purified by preparative high performance liquid chromatography, and the preparation was lyophilized to obtain 12.00 mg of the title compound.
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 18.00 | 90 | 10 | 28 |

The structural characterization data were as follows:
ESI-MS (m/z):1745.8[M+H]⁺.

### Example 8 4-((2S,55)-41-(3,4-bis(bromomethyl)-2,5-dioxo-2,5-dihydro-1H-pyrrol-1-yl)-5 -isopropyl-2-methyl-4,7,11,40-tetraoxo-9,15,18,21,24,27,30,33,36-nonaoxa-3,6,12,39-tetraazahe ntetracontanamido)benzyl ((S)-1-(((S)-1-(((3R,4S,5S)-1-((S)-2-((1R,2R)-3-(((1S,2R)-1-hydroxy -1-phenylpropan-2-yl)amino)-1-methoxy-2-methyl-3-oxopropan)pyrrolidin-1-yl)-3-methoxy-5-methyl-1-oxoheptan-4-yl)(methyl)amino)-3-methyl-1-oxobutan-2-yl)amino)-3-methyl-1-oxobuta n-2-yl)(methyl)carbamate (payload-linker 8)

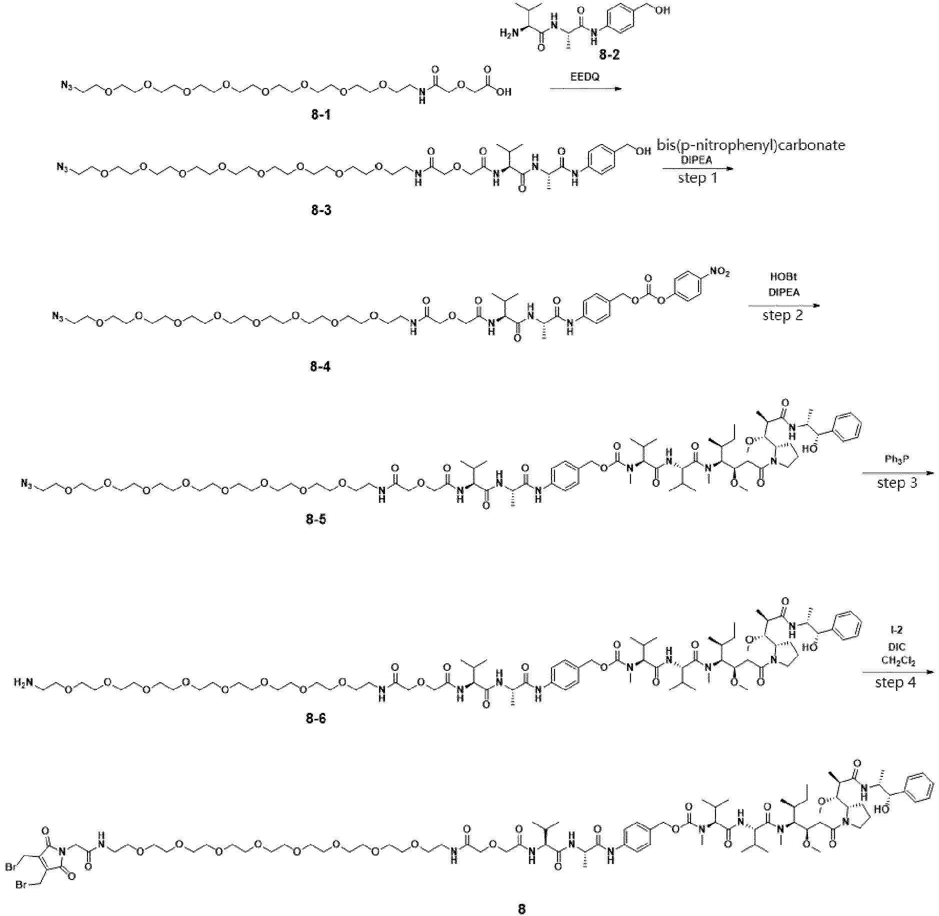

### Step 1: Synthesis of (S)-2-(32-azido-5-oxo-3,9,12,15,18,21,24,27,30-nonaoxa-6-azadotri acontanamido)-N-((S)-1-((4-(hydroxymethyl)phenyl)amino)-1-oxopropan-2-yl)-3-methylbutana mide (8-3)

Compound 8-2 (3.60 g, 12.27 mmol) was dissolved in dichloromethane (30 mL), followed by addition of compound 8-1 (7.49 g, 13.50 mmol) and EEDQ (6.07 g, 24.54 mmol), and the resulting solution was reacted for 4 h at 25 °C. The reaction was concentrated under reduced pressure, and the concentrate was separated on a reversed-phase C18 column (70% acetonitrile/0.1% formic acid aqueous solution) to obtain the title compound 8-3 (7.80 g).

The structural characterization data were as follows:
ESI-MS (m/z):830.4[M+H]⁺.

### Step 2: Synthesis of 4-((2S,5S)-38-azido-5-isopropyl-2-methyl-4,7,11-trioxo-9,15,18,21, 24,27,30,33,36-nonaoxa-3,6,12-triazaoctatriacontanamido)benzyl(4-nitrophenyl)carbonate (8-4)

Compound 8-3 (2.50 g, 3.01 mmol) was dissolved in dichloromethane (20 mL), followed by addition of bis(p-nitrophenyl)carbonate (3.66 g, 12.05 mmol) and DIPEA (1.56 g, 12.05 mmol), and the resulting solution was reacted for 4 h at 25 °C. The reaction solution was directly purified by silica gel column (ethyl acetate-dichloromethane: methanol=84:16) to obtain the title compound 8-4 (2.07 g).

The structural characterization data were as follows:
ESI-MS (m/z):995.4[M+H]⁺.

### Step 3: Synthesis of 4-((2S,5S)-38-azido-5-isopropyl-2-methyl-4,7,11-trioxo-9,15,18,21, 24,27,30,33,36-nonaoxa-3,6,12-triazaoctatriacontanamido)benzyl ((S)-1-(((S)-1-(((3R,4S,5S)-1-((S)-2-((1R,2R)-3)-(((1S,2R)-1-hydroxy-1-phenylpropan-2-yl)amino)-1-methoxy-2-methyl-3-ox opropyl)pyrrolidin-1-yl)-3-methoxy-5-methyl-1-oxoheptan-4-yl)(methyl)amino)-3-methyl-1-oxo butan-2-yl)amino)-3-methyl-1-oxobutan-2-yl)(methyl)carbamate (8-5)

(S)-N-((3R,4S,5S)-1-((S)-2-((1R,2R)-3-((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)- N,3-dimethyl-2-((S)-3-methyl-2-(methylamino)butyramide (100 mg, 139.28 µmol), compound 8-4 (180.17 mg, 181.06 µmol) and HOBt (56.46 mg, 417.84 µmol) were dissolved in DMF (3 mL), then DIPEA (54.00 mg, 417.84 µmol) was added, and the resulting solution was reacted for 6 h at 25 °C. The reaction was concentrated under reduced pressure, and the concentrate was separated on a reversed-phase C18 column (55% acetonitrile/0.1% formic acid aqueous solution) to obtain the title compound 8-5 (60.00 mg).

The structural characterization data were as follows:
ESI-MS (m/z):1573.9[M+H]⁺.

### Step 4: Synthesis of 4-((2S,5S)-38-amino-5-isopropyl-2-methyl-4,7,11-trioxo-9,15,18,21, 24,27,30,33,36-nonaoxa-3,6,12-triazaoctatriacontanamido)benzyl ((S)-1-(((S)-1-(((3R,4S,5S)-1-((S)-2-((1R,2R)-3)-(((1S,2R)-1-hydroxy-1-phenylpropan-2-yl)amino)-1-methoxy-2-methyl-3-ox opropyl)pyrrolidin-1-yl)-3-methoxy-5-methyl-1-oxoheptan-4-yl)(methyl)amino)-3-methyl-1-oxo butan-2-yl)amino)-3-methyl-1-oxobutan-2-yl)(methyl)carbamate (8-6)

Compound 8-5 (0.60 g, 381.22 µmol) was dissolved in THF (10 mL), triphenylphosphine (232.02 mg, 762.44 µmol), and water (10 mL) were added in sequence. The resulting solution was reacted for 6 h at 50 °C. Water was added and the reaction solution was extracted with dichloromethane, dried with anhydrous sodium sulfate, and filtered. The filtrate was concentrated under reduced pressure, and purified by silica gel column (dichloromethane: methanol=84:16) to obtain the title compound 8-6 (50.00 mg).

The structural characterization data were as follows:
ESI-MS (m/z):1548.9[M+H]⁺.

### Step 5: Synthesis of 4-((2S,55)-41-(3,4-bis(bromomethyl)-2,5-dioxo-2,5-dihydro-1H-pyr rol-1-yl)-5-isopropyl-2-methyl-4,7,11,40-tetraoxo-9,15,18,21,24,27,30,33,36-nonaoxa-3,6,12,39-tetraazahentetracontanamido)phenyl ((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropan)pyrrolidin-1-yl)-3-m ethoxy-5-methyl-1-oxoheptan-4-yl)(methyl)amino)-3-methyl-1-oxobutan-2-yl)amino)-3-methyl-1-oxobutan-2-yl)(methyl)carbamate (8)

Compound I-2 (10.00 mg, 0.03 mmol) was dissolved in dichloromethane (4 mL), then DIC (3.70 mg, 0.03 mmol) and Compound 8-6(23.37 mg, 15.00 µmol) were added, and the resulting solution was reacted for 1 h at 25 °C. The reaction was monitored by HPLC-MS. The reaction solution was concentrated under reduced pressure, the concentrate was directly purified by preparative high performance liquid chromatography, and the preparation was lyophilized to obtain the title compound 8 (12.88 mg).
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 35 | 65 | 28 |
| 2.00 | 35 | 65 | 28 |
| 18.00 | 95 | 5 | 28 |

The structural characterization data were as follows:
ESI-MS (m/z):1871.8[M+H]⁺.

### Example 9 2-(2-(2-(2-(2,3-bis(bromomethyl)-5,7-dioxo-5,7-dihydro-6H-pyrrolo[3,4-b]pyr azin-6-yl)ethoxy)ethoxy)acetic acid (I-3)

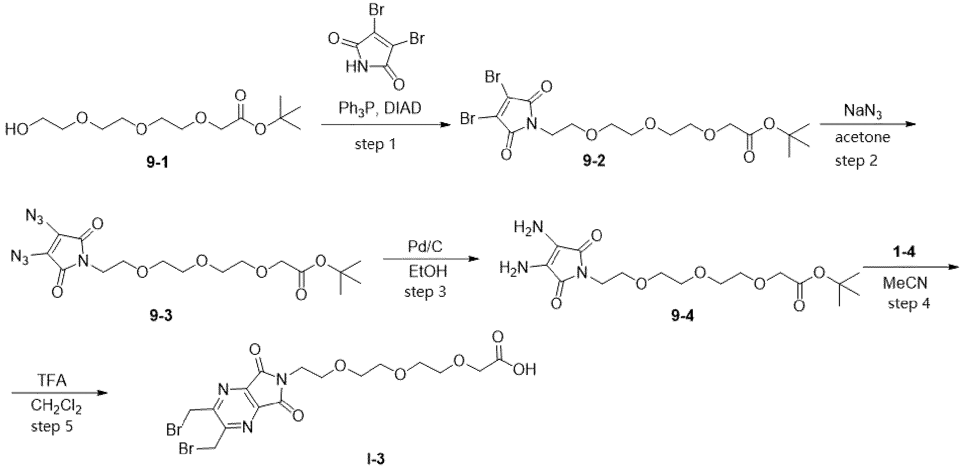

### Step 1: Synthesis of tert-butyl 2-(2-(2-(2-(3,4-dibromo-2,5-dioxo-2,5-dihydro-1H-pyrrol-1-yl)ethoxy)ethoxy)acetate (9-2)

Compound 9-1 (523.00 mg, 1.98 mmol) and triphenylphosphine (518.97 mg, 1.98 mmol) were dissolved in tetrahydrofuran (8 mL) at 0 °C, and **DIAD** (400.11 mg, 1.98 mmol, 389.59 µL) was added and stirred for 5 min. 3,4-dibromopyrrol-2,5-dione (504.30 mg, 1.98 mmol) was added to the resulting solution to have a reaction for 3 h at 0 °C, and saturated aqueous ammonium chloride was added. The reaction solution was extracted with ethyl acetate, dried with anhydrous sodium sulfate, filtered, and the filtrate was concentrated under reduced pressure. The concentrate was chromatographed on silica gel (petroleum ether: ethyl acetate=1:1) to obtain the title compound 9-2 (0.90 g).

The structural characterization data were as follows:
ESI-MS (m/z):519.0[M+18]⁺.

### Step 2: Synthesis of tert-butyl 2-(2-(2-(2-(3,4-diazido-2,5-dioxo-2,5-dihydro-1H-pyrrol-1-yl)ethoxy)ethoxy)acetate (9-3)

Compound 9-2 (0.20 g, 399.07 µmol) was dissolved in acetone (8 mL), then sodium azide (51.89 mg, 798.14 µmol) was added, and the reaction solution was reacted for 8 h at 25 °C. Water was added and the reaction solution was extracted with ethyl acetate, dried with anhydrous sodium sulfate, and filtered. The filtrate was concentrated under reduced pressure to obtain crude title compound 9-3 (160.00 mg).

The structural characterization data were as follows:
ESI-MS (m/z):443.1[M+18]⁺.

### Step 3: Synthesis of tert-butyl 2-(2-(2-(2-(3,4-diamino-2,5-dioxo-2,5-dihydro-1H-pyrrol-1-yl)ethoxy)ethoxy)acetate (9-4)

Compound 9-3 (170.00 mg, 399.63 µmol) and 10% palladium on carbon (85.00 mg) were dissolved in ethanol (34 mL), and the resulting solution was reacted for 8 h at 25 °C in hydrogen atmosphere. The reaction solution was filtered, and the filtrate was drained under reduced pressure to obtain a crude product of the title compound 9-4 (140.00 mg).

The structural characterization data were as follows:
ESI-MS (m/z):391.3[M+18]⁺.

### Step 4: Synthesis of 2-(2-(2-(2-(2,3-bis(bromomethyl)-5,7-dioxo-5,7-dihydro-6H-pyrrolo [3,4-b]pyrazin-6-yl)ethoxy)ethoxy)acetic acid (I-3)

Compound 9-4 (70.00 mg, 187.47 µmol) and compound 1-4 (45.72 mg, 187.47 µmol) were dissolved in acetonitrile (5 mL), and the resulting solution was reacted for 2 h at 25 °C. The reaction solvent was drained under reduced pressure and the concentrate was separated by silica gel column chromatography (petroleum ether: ethyl acetate=1:1) to obtain the title compound I-3 (26.00 mg).

The structural characterization data were as follows:
ESI-MS (m/z):391.3[M+18]⁺.

### Example 10 4-((2S,5S)-17-(2,3-bis(bromomethyl)-5,7-dioxo-5,7-dihydro-6H-pyrrolo[3,4-b]pyrazin-6-yl)-5-isopropyl-4,7-dioxo-2-(3-ureidopropyl)-9,12,15-trioxa-3,6-diazahetpadecanam ido)benzyl ((S)-1-(((S)-1-(((3R,4S,5S)-1-((S)-2-((1R,2R)-3-(((1S,2R)-1-hydroxy-1-phenylpropa n-2-yl)amino)-1-methoxy-2-methyl-3-oxopropyl)pyrrolidin-1-yl)-3-methoxy-5-methyl-1-oxohep tan-4-yl)(methyl)amino)-3-methyl-1-oxobutan-2-yl)amino)-3-methyl-1-oxobutan-2-yl)(methyl)c arbamate (payload-linker 10)

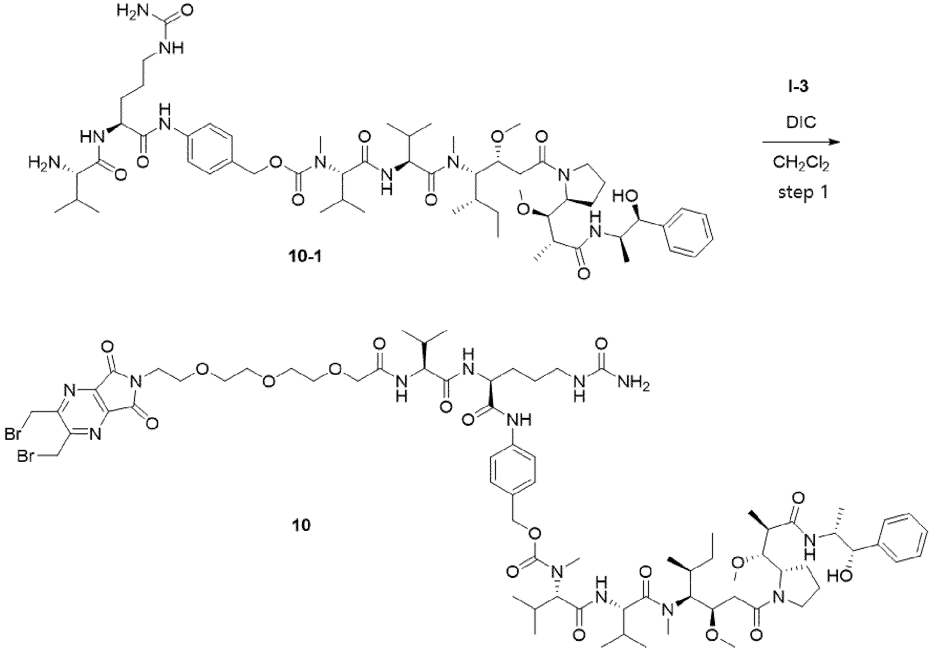

Compound I-3 (13.47 mg, 25.65 µmol) was dissolved in dichloromethane (4 mL), followed by addition of DIC (2.43 mg, 19.24 µmol, 2.98 µL). After stirring for 20 min, formate of compound 10-1 (15.00 mg, 12.83 µmol) was added, and the reaction solution was reacted for 2 h at 20 °C. The solvent was drained under reduced pressure, the concentrate was directly purified by preparative high performance liquid chromatography, and the preparation was lyophilized to obtain the title compound 10 (9.00 mg).
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 35 | 65 | 28 |
| 2.00 | 35 | 65 | 28 |
| 16.00 | 90 | 10 | 28 |

The structural characterization data were as follows:
ESI-MS (m/z):1630.4[M+H]⁺.

### Example 11 3,5-bis(2-(methylsulfonyl)pyrimidin-4-yl)benzoic acid (II-19)

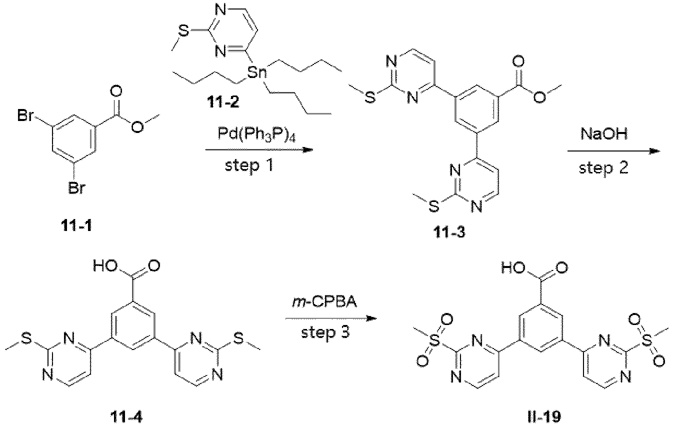

### Step 1: Synthesis of methyl 3,5-bis(2-(methylthio)pyrimidin-4-yl)benzoate(11-3)

methyl 3,5-dibromobenzoate (1.00 g, 3.40 mmol), 4-tributylstannyl-2-thiomethylpyrimidine (3.11 g, 7.48 mmol) and tetrakis(triphenylphosphine)palladium (393.00 mg, 0.034 mmol) were dissolved in 1,4-dioxane (10 mL). After nitrogen replacement, the reaction was carried out at 110 °C microwave for 6 h. The reaction solution was concentrated under reduced pressure, and the concentrate was purified by silica gel column (petroleum ether: ethyl acetate=5:1) to obtain compound 11-3 (398.00 mg).

The structural characterization data were as follows:
ESI-MS (m/z):385.0[M+H]⁺.

### Step 2: Synthesis of 3,5-bis(2-(methylthio)pyrimidin-4-yl)benzoic acid (11-4)

Compound 11-3 (398.00 mg, 1.04 mmol) was dissolved in methanol (5 mL), tetrahydrofuran (5 mL) and water (1 mL), then sodium hydroxide (166.00 mg, 4.14 mmol) was added, and the reaction solution was reacted for 1 h with stirring. The reaction was neutralized by dropping 3 N hydrochloric acid aqueous solution. The reaction solution was concentrated under reduced pressure, stirred with water and filtered. The solid was washed with water, and dried under vacuum to obtain the title compound 11-4 (0.38 g).

The structural characterization data were as follows:
ESI-MS (m/z):371.1[M+18]⁺.

### Step 3: Synthesis of 3,5-bis(2-(methylsulfonyl)pyrimidin-4-yl)benzoic acid (II-19)

Compound 11-4 (0.38 g, 1.03 mmol) was dissolved in methanol (20 mL), M-chloroperoxybenzoic acid (1.25 g, 80%, 6.15 mmol) was added with stirring, and the reaction solution was heated to 60 °C to react for 4 h. The solvent was blown dried with nitrogen. The solid was dissolved in dichloromethane and then directly purified by silica gel column (dichloromethane: methanol=10:1) to obtain the title compound II-19 (0.25 g).

The structural characterization data were as follows:
ESI-MS (m/z):452.0[M+18]⁺.

### Example 12 4-((31S,34S)-1-(3,5-bis(2-(methylsulfonyl)pyrimidin-4-yl)phenyl)-31-isoprop yl-1,29,32-trioxo-34-(3-ureidopropyl) )-5,8,11,14,17,20,23,26-octaoxa-2,30,33-triazapentatriaco ntan-35-amido)benzyl ((S)-1-(((S)-1-(((3R,4S,5S)-1-((S)-2-((1R,2R)-3-(((1S,2R)-1-hydroxy-1-p henylpropan-2-yl)amino)-1-methoxy-2-methyl-3-oxopropyl)pyrrolidin-1-yl)-3-methoxy-5-methy 1-1-oxoheptan-4-yl)(methyl)amino)-3-methyl-1-oxobutan-2-yl)amino)-3-methyl-1-oxobutan-2-y l)(methyl)carbamate (payload-linker 12)

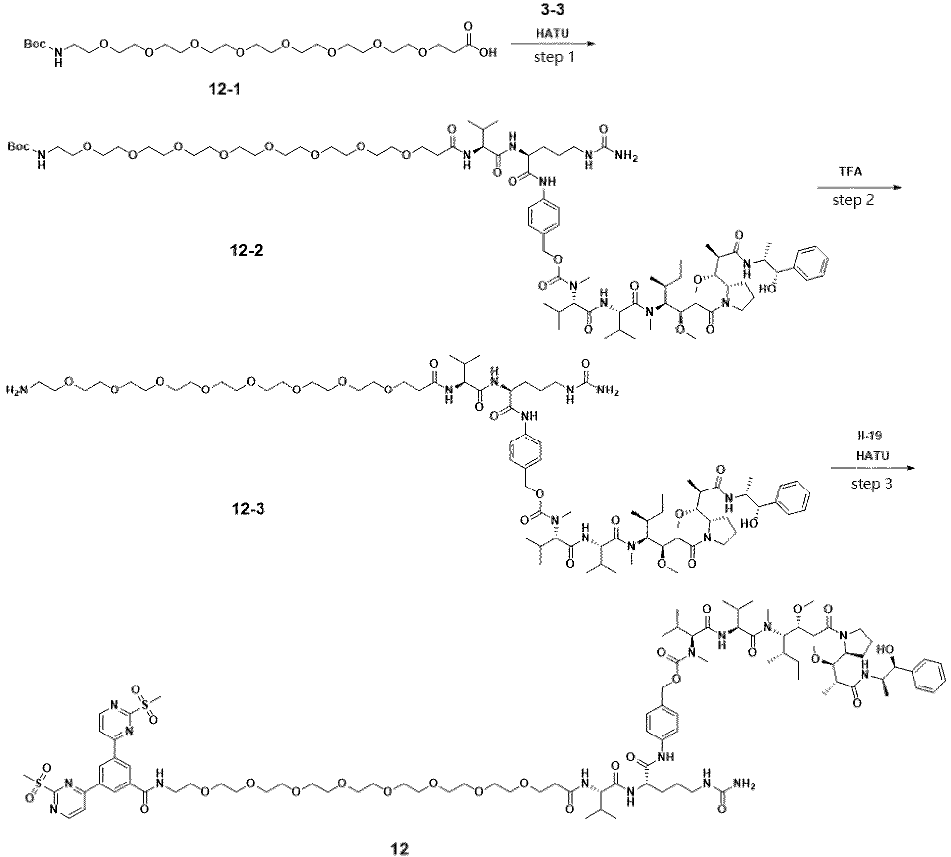

### Step 1: Synthesis of 4-((34S,37S)-34-isopropyl-2,2-dimethyl-4,32,35-trioxo-37-(3-ureido propyl)-3,8,11,14,17,20,23,26,29-nonaoxa-5,33,36-triazaoctatriacontan-38-amido)benzyl ((S)-1-(((S)-1-((((3R,4S,5S)-1-((S)-)-2-((1R,2R)-3-(((1S,2R)-1-hydroxy-1-phenylpropan-2-yl)amino)-1 -methoxy-2-methyl-3-oxopropyl)pyrrolidin-1-yl)-3-methoxy-5-methyl-1-oxoheptan-4-yl)(methy l)amino)-3-methyl-1-oxobutan-2-yl)amino)-3-methyl-1-oxobutan-2-yl)(methyl)carbamate (12-2)

Formate of compound 3-3 (200.00 mg, 171.02 µmol), HATU (91.04 mg, 239.43 µmol) and compound 12-1 (120.42 mg, 222.33 µmol) were dissolved in DMF (6 mL), then DIPEA (66.31 mg, 513.06 µmol) was added, and the resulting solution was reacted for 0.5 h at room temperature. The solvent was drained under reduced pressure, the concentrate was directly purified by preparative high performance liquid chromatography, and the preparation was lyophilized to obtain the title compound 12-2 (210.00 mg).
Chromatographic column: SunFire Prep C18 OBD 5µm 19x150 mm

### Mobile phase A: acetonitrile Mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 18.00 | 90 | 10 | 28 |

The structural characterization data were as follows:
ESI-MS (m/z):824.0[(M+H)/2] ⁺.

### Step 2: Synthesis of 4-((29S,32S)-1-amino-29-isopropyl-27,30-dioxo-32-(3-ureidopropyl) -3,6,9,12,15,18,21,24-octaoxa-28,31-diazatricosan-33-amido)benzyl ((S)-1-(((S)-1-(((3R,4S,5S) -1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-o xobutan-2-yl)amino)-3-methyl-1-oxobutan-2-yl)(methyl)carbamate (12-3)

Compound 12-2 (140.00 mg, 85.00 µmol) was dissolved in trifluoroacetic acid (0.5 mL) and dichloromethane (5 mL), and the resulting solution was reacted for 2 h at 0 °C. The solvent was drained under reduced pressure to obtain 73.00 mg crude formate of title compound 12-3.

The structural characterization data were as follows:
ESI-MS (m/z):774.1[(M+H)/2]⁺.

### Step 3: Synthesis of 4-((31S,34S)-1-(3,5-bis(2-(methylsulfonyl)pyrimidin-4-yl)phenyl)-3 1-isopropyl-1,29,32-trioxo-34-(3-ureidopropyl))-5,8,11,14,17,20,23,26-octaoxa-2,30,33-triazape ntatriacontan-35-amido)benzyl ((S)-1-(((S)-1-(((3R,4S,SS)-1-((S)-2-((1R,2R)-3-(((1S,2R)-1-hyd roxy-1-phenylpropan-2-yl)amino)-1-methoxy-2-methyl-3-oxopropyl)pyrrolidin-1-yl)-3-methoxy -5-methyl-1-oxoheptan-4-yl)(methyl)amino)-3-methyl-1-oxobutan-2-yl)amino)-3-methyl-1-oxob utan-2-yl)(methyl)carbamate (12)

Compound II-19 (5.45 mg, 12.56 µmol), HATU (14.32 mg, 37.67 µmol) and formate of compound 12-3 (20.00 mg, 12.56 µmol) were dissolved in DMF (2 mL), then DIPEA (8.11 mg, 62.78 µmol, 11.18 µL) was added, and the resulting solution was reacted for 2 h at room temperature. The solvent was drained under reduced pressure, the concentrate was directly purified by preparative high performance liquid chromatography, and the preparation was lyophilized to obtain the title compound 12 (6.50 mg).
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 40 | 60 | 28 |
| 2.00 | 40 | 60 | 28 |
| 18.00 | 80 | 20 | 28 |

The structural characterization data were as follows:
ESI-MS (m/z):982.5[(M+H)/2]⁺.

### Example 13 N-((S)-10-benzyl-1-((1S,9S)-9-ethyl-5-fluoro-9-hydroxy-4-methyl-10,13-diox o-1,2,3,9,10,12,13,15-octahydrobenzo[de]pyrano[3',4':6,7]indolizino[1,2-b]quinolin-1-yl)amino) -1,6,9,12,15-pentaoxo-3-oxa-5,8,11,14-tetraazahexadecan-16-yl)-1-(2-(2,3-bis(bromomethyl)-5, 7-dioxo-5H-pyrrolo[3,4-b]pyrazin-6(7H)-yl)acetamido)-3,6,9,12-tetraoxapentadecanamide (payl oad-linker 13)

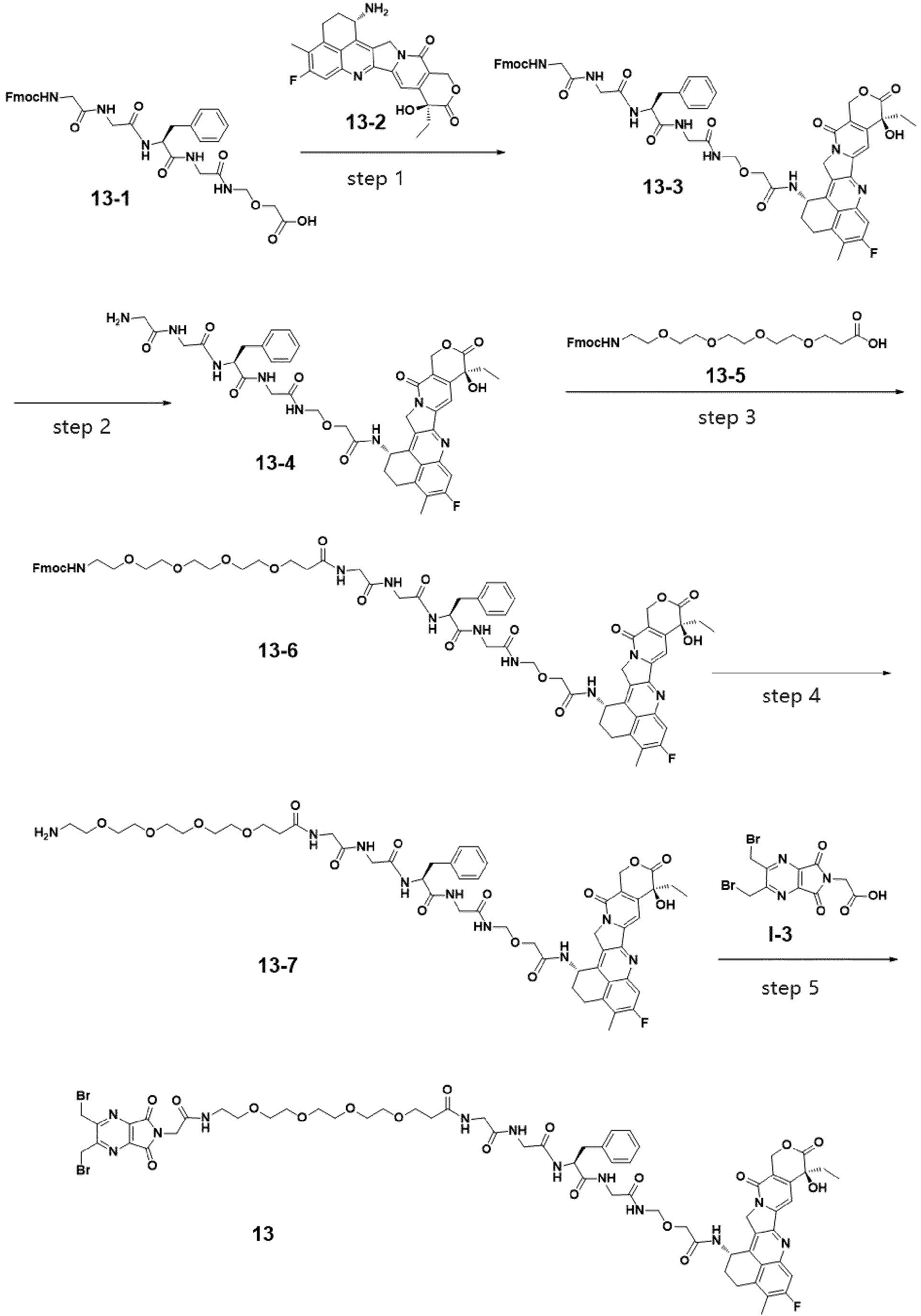

### Step 1: Synthesis of (9H-fluoro-9-yl)methyl ((S)-10-benzyl-1-((1S,9S)-9-ethyl-5-fluoro-9-hydroxy-4-methyl-10,13-dioxo-1,2,3,9,10,12,13,15-octahydrobenzo[de]pyrano[3',4':6,7]indoli zino[1,2-b]quinolin-1-yl)amino)-1,6,9,12,15-pentaoxo-3-oxa-5,8,11,14-tetraazahexadecan-16-y l))carbamate (13-3)

Compound 13-1 (1.00 g, 1.55 mmol) was dissolved in DMF (5 mL), then HATU (647.40 mg, 1.70 mmol), methanesulfonate of compound exatecan 13-2 (1.00 g, 1.55 mmol) and DIPEA (400.34 mg, 3.10 mmol) were added in sequence. The reaction solution was reacted for 2 h at 25 °C. The reaction solvent was drained under reduced pressure, and the concentrate was directly prepared by HPLC and lyophilized to obtain the title compound 13-3 (1.05 g).
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 18.00 | 90 | 10 | 28 |

The structural characterization data were as follows:
ESI-MS (m/z):1063.4[M+H]⁺.

### Step 2: Synthesis of (S)-2-(2-aminoacetamido)acetamido)-N-(2-((2-(((1S,9S)-9-ethyl-5-fl uoro-9-hydroxy-4-methyl-10,13-dioxo-1,2,3,9,10,12,13,15-octahydrobenzo[de]pyrano[3',4':6,7]i ndolizino[1,2-b]quinolin-1-yl)amino)-2-oxoethoxy)methyl)-2-oxo-3-phenylpropionamide (13-4)

Compound 13-3 (1.05 g, 987.69 µmol) was dissolved in dichloromethane (100 mL), then diethylamine (20 mL) was added, and the resulting solution was reacted for 1 h at 25 °C. The reaction solvent was drained under reduced pressure, and the concentrate was directly prepared by HPLC and lyophilized to obtain the formate of compound 13-4 (285.00 mg).
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 18.00 | 90 | 10 | 28 |

The structural characterization data were as follows:
ESI-MS (m/z):841.2[M+H]⁺.

### Step 3: Synthesis of (9H-fluoren-9-yl)methyl ((S)-10-benzyl-1-((1S,9S)-9-ethyl-5-fluoro -9-hydroxy-4-methyl-10,13-dioxo-1,2,3,9,10,12,15-octahydrobenzo[de]pyrano[3',4':6,7]indolizin o[1,2-b]quinolin-1-yl)amino)-1,6,9,9,15,18-hexaoxo-3,21,24,27,30-pentaoxa-5,8,11,14,17-penta azatricarboxylate (13-6)

Formate of compound 13-4 (83.00 mg, 98.71 µmol), HATU (45.04 mg, 118.45 µmol) and compound 13-5 (57.75 mg, 118.45 µmol) were dissolved in DMF (2 mL), then DIPEA (25.51 mg, 197.42 µmol, 35.14 µL) was added, and the resulting solution was reacted for 0.5 h at room temperature. The reaction solvent was drained under reduced pressure, and the concentrate was directly prepared by HPLC and lyophilized to obtain the title compound 13-6 (30.00 mg).
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 16.00 | 90 | 10 | 28 |

The structural characterization data were as follows:
ESI-MS (m/z):1310.5[M+H]⁺.

### Step 4: Synthesis of 1-amino-N-((S)-10-benzyl-1-((1S,9S)-9-ethyl-5-fluoro-9-hydroxy-4-methyl-10,13-dioxo-1,2,3,9,10,12,13,15-octahydrobenzo[de]pyrano[3',4':6,7]indolizino[1,2-b]qu inolin-1-yl)amino-1,6,9,12,15-pentaoxo-3-oxa-5,8,11,14-tetraazahexadecan-16-yl)-3,6,9,12-tetra oxa-15-decanamide (13-7)

Compound 13-6 (30.00 mg, 22.89 µmol) was dissolved in diethylamine (1 mL) and DMF (2 mL), and the resulting solution was reacted for 1 h at room temperature. The reaction solvent was drained under reduced pressure, and the concentrate was directly prepared by HPLC and lyophilized to obtain formate of title compound 13-7 (16.00 mg).
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 5 | 95 | 28 |
| 18.00 | 90 | 10 | 28 |

The structural characterization data were as follows:
ESI-MS (m/z):1088.4[M+H]⁺.

### Step 5: Synthesis of N-((S)-10-benzyl-1-((1S,9S)-9-ethyl-5-fluoro-9-hydroxy-4-methyl-1 0,13-dioxo-1,2,3,9,10,12,13,15-octahydrobenzo[de]pyrano[3',4':6,7]indolizino[1,2-b]quinolin-1-yl)amino)-1,6,9,12,15-pentaoxo-3-oxa-5,8,11,14-tetraazahexadecan-16-yl)-1-(2-(2,3-bis(bromo methyl)-5,7-dioxo-5H-pyrrolo[3,4-b]pyrazin-6(7H)-yl)acetamide)-3,6,9,12-tetraoxapentadecana mide (13)

Formate of compound 13-7 (16.00 mg, 14.70 µmol), Compound I-3 (11.56 mg, 29.41 µmol) and DIC (2.60 mg, 20.59 µmol, 3.19 µL) were dissolved in dichloromethane (4 mL), and the resulting solution was reacted for 1 h at 20 °C. The reaction solvent was drained under reduced pressure, and the concentrate was directly prepared by HPLC and lyophilized to obtain the title compound 13 (5.33 mg).
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 3.00 | 30 | 70 | 28 |
| 18.00 | 95 | 5 | 28 |

The structural characterization data were as follows:
ESI-MS (m/z):1463.3 [M+H]⁺.

### Example 14 (S)-2-(17-(2,3-bis(bromomethyl)-5,7-dioxo-5H-pyrrolo[3,4-b]pyrazin-6(7H)-yl)-4,7-dioxo-9,12,15-trioxa-3,6-diazaheptanamido)-N-(2-((2-((1S,9S)-9-ethyl-5-fluoro-9-hydro xy-4-methyl-10,13-dioxo)-1,2,3,9,10,12,13,15-octahydrobenzo[de]pyrano[3',4':6,7]indolizino[1, 2-b]quinolin-1-yl)amino)-2-oxoethoxy(methyl)amino)-2-oxoethyl)-3-phenylpropionamide (payl oad-linker 14)

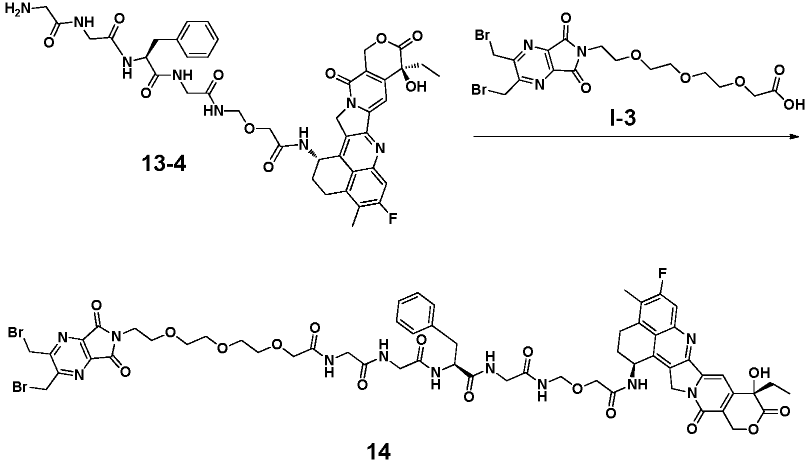

Compound I-3 (40.26 mg, 76.67 µmol) was dissolved in dichloromethane (5 mL), then DIC (9.68 mg, 76.67 µmol) was added, after stirring for 20 min, formate of compound 13-4 (34.00 mg, 38.34 µmol) was added, and the resulting solution was reacted for 2 h at 20 °C. The reaction solvent was drained under reduced pressure, and the concentrate was directly prepared by HPLC and lyophilized to obtain the title compound 14 (5.33 mg).
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 18.00 | 90 | 10 | 28 |

The structural characterization data were as follows:
ESI-MS (m/z):1348.3[M+H]⁺.

### Example 15 1-(3,5-bis(2-(methylsulfonyl)pyrimidin-4-yl)phenyl)-1-oxo-5,8,11-trioxa-2-az atridecan-13-oleic acid (15)

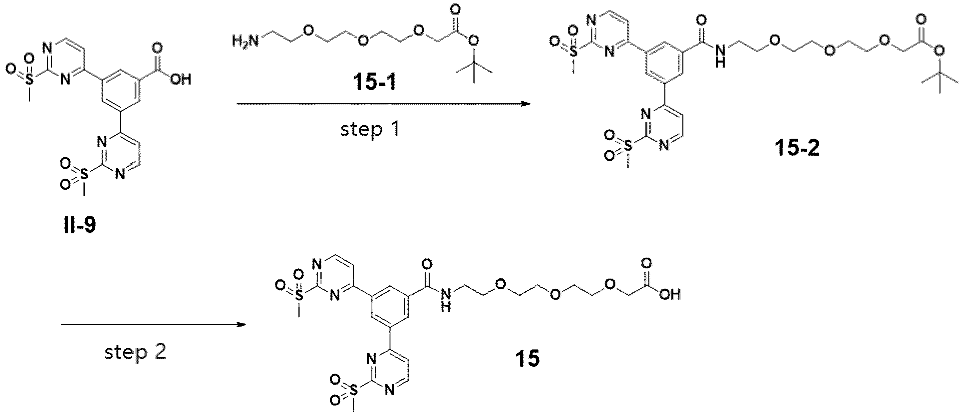

### Step 1: Synthesis of tert-butyl 1-(3,5-bis(2-(methylsulfonyl)pyrimidin-4-yl)phenyl)-1-ox o-5,8,11-trioxa-2-azatridecan-13-oleate (15-2)

Compound II-9 (50.00 mg, 115.09 µmol) was dissolved in DMF (2 mL), then HATU (65.60 mg, 172.53 µmol) was added, after stirring compound 15-1 (36.37 mg, 138.11 µmol) and DIPEA (44.62 mg, 345.27 µmol, 61.46 µL) were added, and the resulting solution was reacted for 2 h at 25 °C. The reaction solvent was drained under reduced pressure, and the concentrate was directly prepared by HPLC and lyophilized to obtain the title compound 15-2 (25.00 mg).
Chromatographic column: SunFire Prep C18 OBD 5µm 19x150 mm

### Mobile phase A: acetonitrile Mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 18.00 | 90 | 10 | 28 |

The structural characterization data were as follows:
ESI-MS (m/z):697.2[M+18]⁺.

### Step 2: Synthesis of 1-(3,5-bis(2-(methylsulfonyl)pyrimidin-4-yl)phenyl)-1-oxo-5,8,11-tr ioxa-2-azatridecan-13-oleic acid (15)

Compound 15-2 (20.00 mg, 29.42 µmol) was dissolved in trifluoroacetic acid (1 mL) and dichloromethane (5 mL), and the resulting solution was reacted for 2 h at 25 °C. The reaction solvent was drained under reduced pressure, and the concentrated yield crude title compound 15 (15.00 mg), which was directly used in the next reaction without purification.

The structural characterization data were as follows:
ESI-MS (m/z):641.1[M+18]⁺.

### Example 16 N-((S)-10-benzyl-1-((1R,9R)-9-ethyl-5-fluoro-9-hydroxy-4-methyl-10,13-dio xo-1,2,3,9,10,12,13,15-octahydrobenzo[de]pyrano[3',4':6,7]indolizino[1,2-b]quinolin-1-yl)amin o)-1,6,9,12,15,18,46-heptaoxo-3,21,24,27,30,33,36,39,42,48,51,54-dodecaoxa-5,8,11,14,17,45-h exaazahexapentan-56-yl)-3,5-bis(2-(methylsulfonyl)pyrimidin-4-yl)benzamide (payload-linker 16)

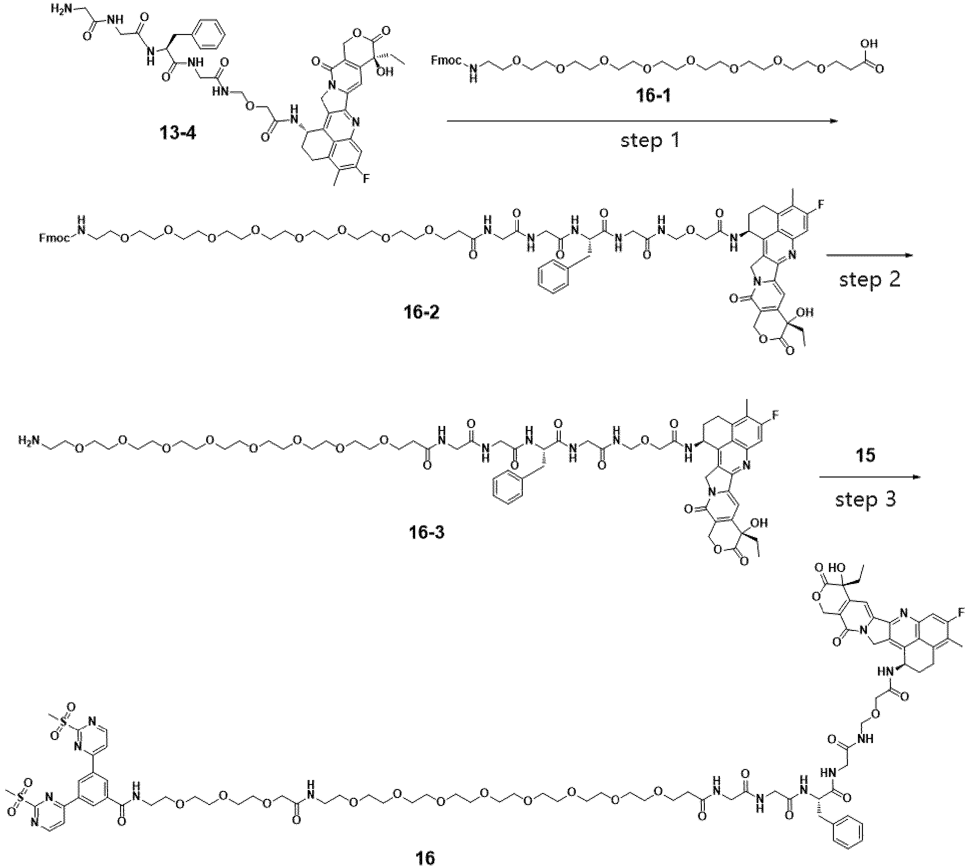

### Step 1: Synthesis of (9H-fluoro-9-yl)methyl((S)-10-benzyl-1-((1S,9S)-9-ethyl-5-fluoro-9-hydroxy-4-methyl-10,13-dioxo-1,2,3,9,10,12,13,15-octahydrobenzo[de]pyrano[3',4':6,7]indolizi no[1,2-b]quinolin-1-yl)amino)-1,6,9,12,15,18-hexaoxo-3,21,24,27,30,33,36,39,42-nonaoxa-5,8,1 1,14,17-pentaazatetradecan-44-yl)carbamate (16-2)

Methanesulfonate of compound 13-4 (132.00 mg, 148.84 µmol) was dissolved in DMF (5 mL), then HATU (90.55 mg, 238.14 µmol), compound 16-1 (148.19 mg, 223.26 µmol) and DIPEA (96.18 mg, 744.19 µmol, 132.48 µL) were added, and the resulting solution was reacted for 2 h at 25 °C. The reaction solvent was drained under reduced pressure, and the concentrate was directly prepared by HPLC and lyophilized to obtain the title compound 16-2 (110.00 mg).
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 20 | 80 | 28 |
| 3.00 | 20 | 80 | 28 |
| 17.00 | 90 | 10 | 28 |

The structural characterization data were as follows:
ESI-MS (m/z):1486.5[M+1]⁺.

### Step 2: Synthesis of 1-amino-N-((S)-10-benzyl-1-((1S,9S)-9-ethyl-5-fluoro-9-hydroxy-4-methyl-10,13-dioxo-1,2,3,9,10,12,13,15-octahydrobenzo[de]pyrano[3',4':6,7]indolizino[1,2-b]qu inolin-1-yl)amino-1,6,9,12,15-pentaoxo-3-oxa-5,8,11,14-tetraazahexadecan-16-yl)-3,6,9,12,15,1 8,21,2-octaoxaheptan-27-amide (16-3)

Compound 16-2 (110.00 mg, 74.00 µmol, FR) was dissolved in diethylamine (1 mL) and dichloromethane (5 mL), and the resulting solution was reacted for 2 h at 25 °C. The reaction solvent was drained under reduced pressure, and the concentrate was directly prepared by HPLC and lyophilized to obtain formate of title compound 16-3 (40.00 mg).
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 10 | 90 | 28 |
| 2.00 | 10 | 90 | 28 |
| 18.00 | 90 | 10 | 28 |

The structural characterization data were as follows:
ESI-MS (m/z):1264.6[M+1]⁺.

### Step 3: Synthesis of N-((S)-10-benzyl-1-((1R,9R)-9-ethyl-5-fluoro-9-hydroxy-4-methyl-1 0,13-dioxo-1,2,3,9,10,12,13,15-octahydrobenzo[de]pyrano[3',4':6,7]indolizino[1,2-b]quinolin-1-yl)amino)-1,6,9,12,15,18,46-heptaoxo-3,21,24,27,30,33,36,39,42,48,51,54-dodecaoxa-5,8,11,14, 17,45-hexaazahexapentan-56-yl)-3,5-bis(2-(methylsulfonyl)pyrimidin-4-yl)benzamide (16)

Compound 15 (7.00 mg, 11.22 µmol) was dissolved in DMF (3 mL), then HATU (7.76 mg, 20.41 µmol), formate of Compound 16-3 (13.37 mg, 10.20 µmol) and DIPEA (3.96 mg, 30.61 µmol, 5.45 µL) were added, and the resulting solution was reacted for 2 h at 25 °C. The reaction solvent was drained under reduced pressure, and the concentrate was directly prepared by HPLC and lyophilized to obtain the title compound 16 (7.00 mg).
Chromatographic column: SunFire Prep C18 OBD 5µm 19x150 mm

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 15 | 85 | 28 |
| 2.00 | 15 | 85 | 28 |
| 18.00 | 80 | 20 | 28 |

The structural characterization data were as follows:
ESI-MS (m/z):1869.7[M+1]⁺.

### Example 17 N-((7S,10S,13S)-1-(((18S,9S)-9-ethyl-5-fluoro-9-hydroxy-4-methyl-10,13-dio xo-2,3,9,10,13,15-hexahydro-1H,12H-benzo[de]pyrano[3',4':6,7]indolizino[1,2-b]quinolin-1-yl)a mino)-7,10,13-trimethyl-1,6,9,12,15-pentaoxo-3,17,20,23-tetraoxa-5,8,11,14-tetraazapentan-25-yl)-3,5-bis(2-(methylsulfonyl)pyrimidin-5-yl)benzamide(C-10)

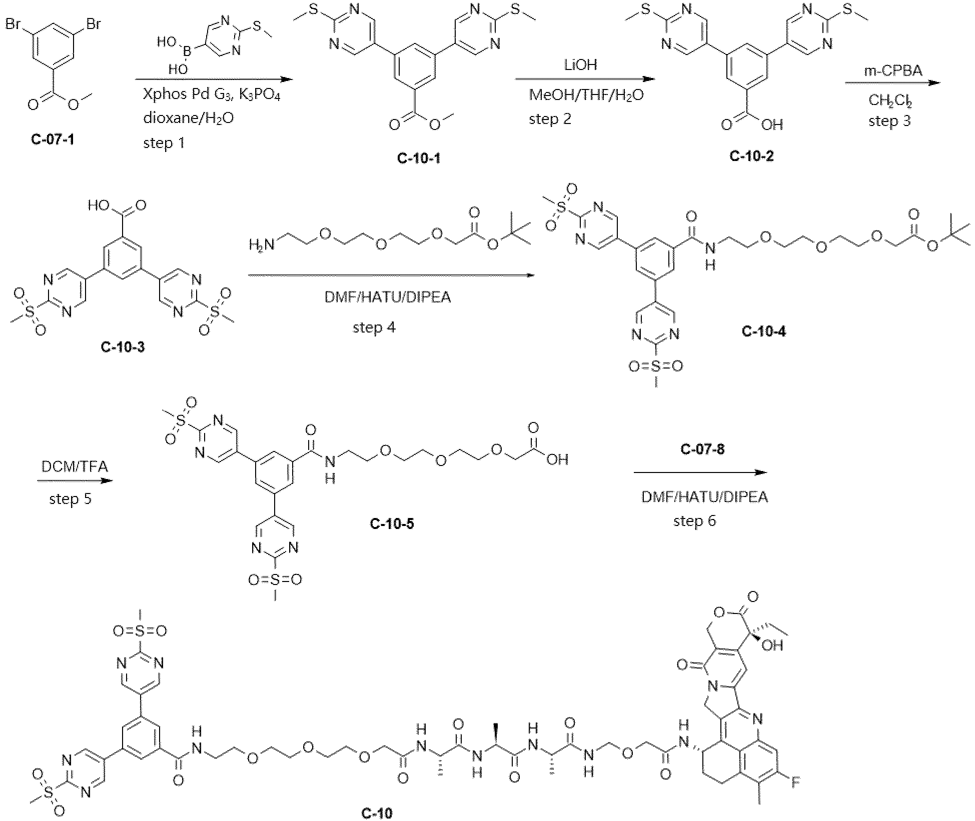

### Step 1:

Raw materials methyl 3,5- dibromobenzoate (720 mg,2.45 mmol), 2-methylthiopyrimidin-5-boric acid (874 mg,5.14 mmol), XPhosPd G3 (207 mg, 245 µmol) and K₃PO₄ (1.56 g, 7.35 mmol) were added to dioxane (12 mL) and H₂O (4 mL). The reaction system was stirred and reacted at 90 °C under nitrogen atmosphere for 3 h. The reaction was monitored by LC-MS and the reaction solution was filtered with diatomaceous earth. The filtrate was extracted with water and ethyl acetate, concentrated to obtain a crude product. The crude product was purified by column chromatography (EA/PE=0-25%) to obtain 710 mg of methyl 3,5-bis(2-(methylthio)pyrimidin-5-yl)benzoate.

The structural characterization data were as follows:
ESI-MS (m/z):385.1 [M+H]⁺.

### Step 2:

Compound methyl 3,5-bis(2-(methylthio)pyrimidin-5-yl)benzoate (650 mg, 1.69 mol) and lithium hydroxide (121 mg, 5.07 mmol) were dissolved in THF (2 mL), MeOH (2 mL) and water (2 mL). The reaction solution was reacted for 2 h at 25 °C. The reaction was monitored by LC-MS. The pH of the system was adjusted to about 2 with 1N HCl. A large amount of solid was precipitated, filtered, and the filter cake was collected, and dried to obtain 560 mg of 3,5-bis(2-(methylthio)pyrimidin-5-yl)benzoic acid.

The structural characterization data were as follows:
ESI-MS (m/z):371.1 [M+H]⁺.

### Step 3:

Compound 3,5-bis(2-(methylthio)pyrimidin-5-yl)benzoic acid (450.80 mg, 1.22 mmol) was dissolved in DCM(10 mL), then m-CPBA (2.46 g, 12.1 mmol, purity of 85% ) was added to the reaction system. The reaction was carried out for 12 h at 25 °C and monitored by LC-MS. The solvent was blown dry with nitrogen flow to obtain a crude product. The crude product was purified by preparative high performance liquid chromatography and then lyophilized to obtain 153 mg of 3,5-bis(2-(methylsulfonyl) pyrimidin-5-yl)benzoic acid.

The structural characterization data were as follows:
ESI-MS (m/z):435.0 [M+H]⁺.

The preparation method was as follows:
Chromatographic column: Phenomenex Luna C18 200*40mm*10um.
Mobile phase A: acetonitrile; Mobile phase B: water (0.05% hydrochloric acid)

Mobile phase :[water(HCl)-ACN]; B%: 13%-43%,10min).

| Time [min] | Mobile phase A [%] | Mobile phase B [%] | Flow rate [mL/min] |
|---|---|---|---|
| 0.00 | 13 | 87 | 25 |
| 10.00 | 43 | 57 | 25 |
| 18.00 | 90 | 10 | 25 |

### Step 4:

Compound 3,5-bis(2-(methylsulfonyl)pyrimidin-5-yl)benzoic acid (140 mg, 322.25 µmol), tert-butyl 2-[2-[2-(2-aminoethoxy)ethoxy]ethoxy acetate (84.86 mg, 322.25 µmol), HATU (183.80 mg, 483.37 µmol) and DIPEA (124.94 mg, 966.75 µmol) were added to DMF (4 mL), and the resulting solution was reacted for 2 h at 25 °C. The reaction was monitored by LC-MS. The reaction solution was purified by preparative high performance liquid chromatography and then lyophilized to obtain 51 mg of tert-butyl 1-(3,5-bis(2-(methylsulfonyl)pyrimidin-5-yl)phenyl)-1-oxo-5,8,11-trioxa-2-azatetradecan-14-oate.

The structural characterization data were as follows:
ESI-MS (m/z):694.2 [M+H]⁺.

The preparation method was as follows:
Chromatographic column: Waters XBridge Prep C18OBD (5 µm*19mm*150 mm)

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 30 | 70 | 24 |
| 2.00 | 30 | 70 | 24 |
| 18.00 | 90 | 10 | 24 |

### Step 5:

Compound tert-butyl 1-(3,5-bis(2-(methylsulfonyl)pyrimidin-5-yl)phenyl)-1-oxo-5,8,11-trioxa-2-azatridecan-13-oate (50 mg, 73.56 µmol) was added to DCM (2 mL) and TFA (1 mL), and the resulting solution was reacted for 1 h at 25 °C. The reaction was monitored by LC-MS. The reaction system was concentrated to dryness to obtain 45 mg of compound 1-(3,5-bis(2-(methylsulfonyl)pyrimidin-5-yl)phenyl)-1-oxo-5,8,11-trioxa-2-azatetradecan-14-oic acid.

The structural characterization data were as follows:
ESI-MS (m/z):637.2 [M+H]⁺.

### Step 6:

Compound 1-(3,5-bis(2-(methylsulfonyl)pyrimidin-5-yl)phenyl)-1-oxo-5,8,11-trioxa-2-azatridecan-13-oic acid (31.91 mg, 51.17 µmol), (2S)-2-amino-N-((2S)-1-(((2S)-1-(((2- (((9S)-5-fluoro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-2-oxoethoxy)methyl)amino)-1-oxopropan-2-yl)amino)-1-oxopropan-2-yl)propionamide (C-07-8, 40 mg, 51.17 µmol), HATU (29.18 mg, 76.75 µmol), and DIPEA (19.84 mg, 153.50 µmol) were added to DMF (3 mL), and the resulting solution was reacted for 2 h at 25 °C. The reaction was monitored by LC-MS. The reaction solution was purified by preparative high performance liquid chromatography and then lyophilized to obtain 13 mg of N-((7S,10S,135)-1-(((1S,95)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-7,10,13-trimethyl-1,6,9,12,15-pentaoxo-3,17,20,23-tetraoxa-5,8,11,14-tetraazapentan-25-yl)-3,5-bis(2-(methylsulfonyl)pyrimidin-5-yl)benzamide.

The structural characterization data were as follows:
ESI-MS (m/z):1342.5 [M+H]⁺.

The preparation method was as follows:
Chromatographic column: Waters XBridge Prep C18OBD (5 µm*19mm*150 mm)

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 20 | 80 | 24 |
| 2.00 | 20 | 80 | 24 |
| 18.00 | 90 | 10 | 24 |

### Example 18 N-((7S,10S,13S)-1-(((1S,9S)-9-ethyl-5-chloro-9-hydroxy-4-methyl-10,13-dio xo-2,3,9,10,13,15-hexahydro-1H,12H-benzo[de]pyrano[3',4':6,7]indolizino[1,2-b]quinolin-1-yl)a mino)-7,10,13-trimethyl-1,6,9,12,15 -pentaoxo-3,17,20,23-tetraoxa-5,8,11,14-tetraazapentan-25-yl)-3,5-bis(2-(methylsulfonyl)pyrimidin-5-yl)benzamide (C-17)

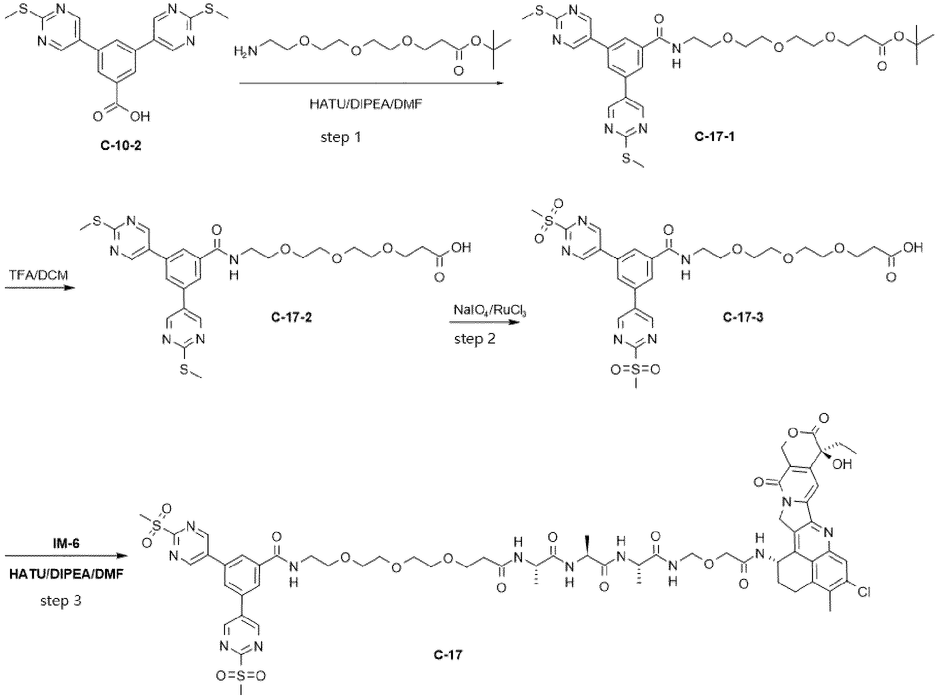

### Step 1:

3,5-bis(2-(methylthio)pyrimidin-5-yl)benzoic acid (3.00 g, 8.10 mmol) and tert-butyl 3-[2-[2-(2-aminoethoxy)ethoxy]ethoxy]-propionate (2.25 g, 8.10 mmol) were added to DMF (3 mL), followed by addition of HOBt (3.28 g, 24.3 mmol), EDCI (4.66 g, 24.3 mmol) and DIPEA (4.19 g, 32.4 mmol, 5.64 mL), and the resulting solution was heated to 60 °C to react for 2 h. Water (50 mL) was added to the reaction solution, and extraction with ethyl acetate (30 mL x 3) was carried out. The organic phases were combined and dried with anhydrous sodium sulfate, filtered and concentrated to obtain a crude product tert-butyl 1-(3,5-bis(2-(methylthio)pyrimidin-5-yl)phenyl)-1-oxo-5,8,11-trioxa-2-azatetradecan-14-oate (3.8 g, 4.75 mmol), which was directly used in the next reaction without purification.

### Step 2:

Tert-butyl 1-(3,5-bis(2-(methylthio)pyrimidin-5-yl)phenyl)-1-oxo-5,8,11-trioxa-2-azatetradecan-14-oate (3.40 g, 5.40 mmol) was dissolved in dichloromethane (30 mL), then trifluoroacetic acid (10.8 g, 94.2 mmol, 7 mL) was added, and the reaction system was stirred for 2 h at 25 °C. The reaction solution was directly concentrated, purified by preparative high performance liquid chromatography and then lyophilized to obtain 1-(3,5-bis(2-(methylthio)pyrimidin-5-yl)phenyl)-1-oxo-5,8,11-trioxa-2-azatetradecan-14-oic acid (2.09 g, 3.64 mmol).

The structural characterization data were as follows:
ESI-MS (m/z):574.2 [M+H]⁺.

The preparation method was as follows:
Chromatographic column: Phenomenex luna C18 (250 mm*70mm*10 µm)

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 30 | 70 | 30 |
| 22.00 | 60 | 40 | 30 |

### Step 3:

1-(3,5-bis(2-(methylthio)pyrimidin-5-yl)phenyl)-1-oxo-5,8,11-trioxa-2-azatetradecan-14-oic acid (56 mg, 97.61 µmol) was added to acetonitrile (6 mL) and water (3 mL), and then sodium periodate (208.79 mg, 976.15 µmol) and ruthenium trichloride hydrate (8.10 mg, 39.05 µmol) were added to the reaction system. The reaction solution was stirred and reacted for 30 min at 25 °C. The reaction was monitored by LC-MS. Water and ethyl acetate were added for an extraction, and the reaction solution was concentrated to obtain 1-(3,5-bis(2-(methylsulfonyl)pyrimidin-5-yl)phenyl)-1-oxo-5,8,11-trioxa-2-azatetradecan-14-oic acid (60 mg).

The structural characterization data were as follows:
ESI-MS (m/z):638.2 [M+H]⁺.

### Step 4:

(2S)-2-amino-N-((2S)-1-(((2S)-1-(((2-(((9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-2-oxoethoxy)methyl)amino)-1-oxopropan-2-yl)amino)-1-oxopropan-2-yl)propionamide (IM-6, 20 mg, 25.06 µmol), 1-(3,5-bis(2-(methylsulfonyl)pyrimidin-5-yl)phenyl)-1-oxo-5,8,11-trioxa-2-azatetradecan-14-oic acid (16mg, 25.06 µmol), HATU (19.05 mg, 50.11 µmol) and DIPEA (16.19 mg, 125.28 µmol) were added to DMF (3 mL) in sequence, and the reaction system was reacted for 1 h at 25 °C. The reaction solution was directly purified by preparative high performance liquid chromatography and then lyophilized to obtain N-((7S,10S,13S)-1-(((1S,9S)-9-ethyl-5-chloro-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-7,10,13-trimethyl-1,6,9,12,15-pentaoxo-3,17,20,23-tetraoxa-5,8,11,14-tetraazapentan-25-yl)-3,5-bis(2-(methylsulfonyl)pyrimidin-5-yl)benzamide (16 mg).

The structural characterization data were as follows:
ESI-MS (m/z):1371.4 [M+H]⁺.

The preparation method was as follows:
Chromatographic column: Waters XBridge Prep C18OBD (5 µm*19mm*150 mm)

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 20 | 80 | 30 |
| 2.00 | 20 | 80 | 30 |
| 18.00 | 80 | 20 | 30 |

### Example 19 N-((7S,10S,13S)-1-(((1S,9S)-5-chloro-9-ethyl-9-hydroxy-4-methyl-10,13-dio xo-2,3,9,10,13,15-hexahydro-1H,12H-benzo[de]pyrano[3',4':6,7]indolizino[1,2-b]quinolin-1-yl)a mino)-7,1,13-trimethyl-1,6,9,12,15-pentaoxo-3,18,21,24,27,30,33,36,39-nonyloxa-5,8,11,14-tetr aazabut-41-yl)-3,5-bis(2-(methylsulfonyl)pyrimidin-5-yl)benzamide (C-21)

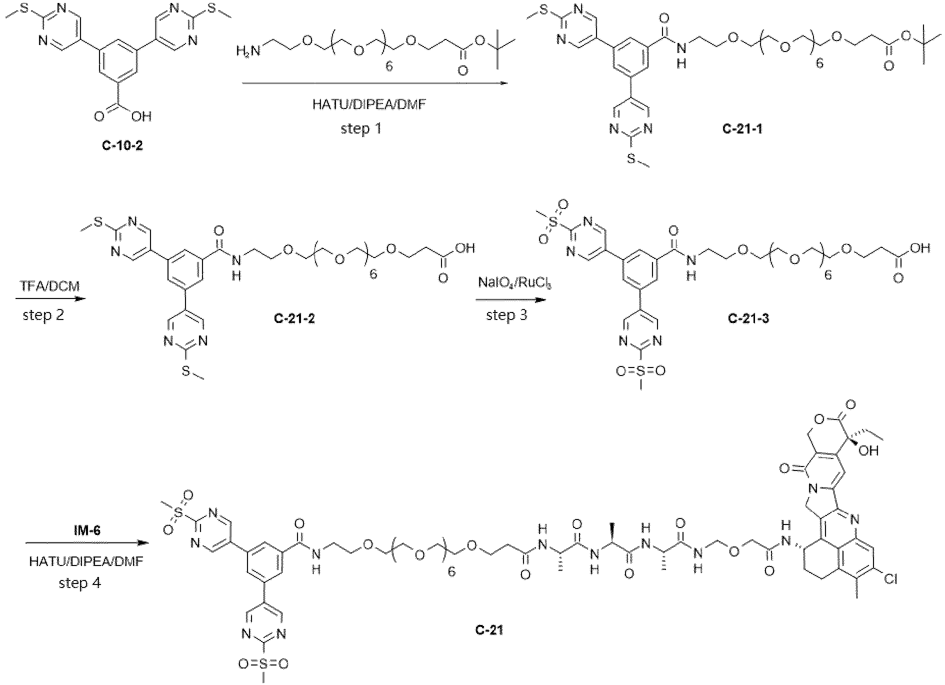

### Step 1:

3,5-bis(2-(methylthio)pyrimidin-5-yl)benzoic acid (3.00 g, 8.10 mmol) and tert-butyl 1-amino-3,6,9,12,15,18,21,24-octaoxaheptacosan-27-oate (4.03 g, 8.10 mmol) were added to DMF (40 mL), followed by addition of HOBt (3.28 g, 24.3 mmol), EDCI (4.66 g, 24.3 mmol) and DIPEA (4.19 g, 32.4 mmol, 5.64 mL) to the reaction system. The reaction system was stirred for 2 h at 60 °C. Water (100 mL) and ethyl acetate (60 mL x 3) were added to the reaction solution for an extraction. The organic phases were combined, dried with anhydrous sodium sulfate, filtered and concentrated to obtain tert-butyl 1-(3,5-bis(2-(methylthio)pyrimidin-5-yl)phenyl)-1-oxo-5,8,11,14,17,20,23,26-octaoxa-2-azanonan-29-oate (4.20 g, 4.14 mmol),which was directly used in the next reaction without purification.

### Step 2:

Tert-butyl 1-(3,5-bis(2-(methylthio)pyrimidin-5-yl)phenyl)-1-oxo-5,8,11,14,17,20,23,26-octaoxa-2-azanonacosan-29-oate (3.60 g, 4.24 mmol) was dissolved in dichloromethane (30 mL), and then TFA (15.3 g, 134 mmol, 10 mL) was added to the resulting solution. The reaction system was stirred for 6 h at 25 °C. Water (60 mL) and ethyl acetate (40 mL x 3) were added to the reaction solution for an extraction. The organic phases were combined and dried with anhydrous sodium sulfate, filtered and concentrated to obtain a crude product. The crude product was purified by preparative high performance liquid chromatography and then lyophilized to obtain 1-(3,5-bis(2-(methylthio)pyrimidin-5-yl)phenyl)-1-oxo-5,8,11,14,17,20,23,26-octaoxa-2-azanonacosan-29-oic acid (2.93 g, 3.63 mmol).

The structural characterization data were as follows:
ESI-MS (m/z):794.3 [M+H]⁺.

The preparation method was as follows:
Chromatographic column: Phenomenex luna C18 (250 mm*70mm*10 µm)

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 30 | 70 | 30 |
| 15.00 | 60 | 40 | 30 |

### Step 3: 1-(3,5-bis(2-(methylthio)pyrimidin-5-yl)phenyl)-1-oxo-5,8,11,14,17,20,23,26-octaoxa-2-azanonacosan-29-oic acid (148 mg, 0.186 mmol) was added to acetonitrile (15 mL) and water (7.5 mL), followed by addition of sodium periodate (398.71 mg,1.86 mmol) and ruthenium trichloride hydrate (15.47 mg, 74.56 µmol) to the reaction system, and the reaction solution was stirred at 25 °C to react for 30 min. The reaction system was extracted with water and ethyl acetate, concentrated to obtain 1-(3,5-bis(2-(methylsulfonyl)pyrimidin-5-yl)phenyl)-1-oxo-5,8,11,14,17,20,23,26-octaoxa-2-azanonacosan-29-oic acid (155 mg).

The structural characterization data were as follows:
ESI-MS (m/z):858.3 [M+H]⁺.

### Step 4:

(2S)-2-amino-N-((2S)-1-(((2S)-1-(((2-(((9S)-5-chloro-9-ethyl-9-hydroxy-4-methyl-10,13-di oxo-2,3,9,10,13,15-hexahydro-1H,12H-benzo[de]pyrano[3',4':6,7]indolizino[1,2-b]quinolin-1-yl) amino)-2-oxoethoxy)methyl)amino)-1-oxopropan-2-yl)amino)-1-oxopropan-2-yl)propionamide (IM-6, 27.91 mg, 34.97 µmol), 1-(3,5-bis(2-(methylsulfonyl)pyrimidin-5-yl)phenyl)-1-oxo-5, 8,11,14,17,20,23,26-octaoxa-2-azanonacosan-29-oic acid (30 mg, 34.97 µmol), HATU (26.5 9 mg, 69.93 µmol) and DIPEA (22.60 mg, 174.84 µmol) were added to DMF (3 mL), and the reaction system was reacted for 1 h at 25 °C. The reaction solution was purified by high performance liquid chromatography and then lyophilized to obtain N-((7S,10S,1 3S)-1-(((1S,9S)-5-chloro-9-ethyl-9-hydroxy-4-methyl-10,13-dioxo-2,3,9,10,13,15-hexahydro-1 H,12H-benzo[de]pyrano[3',4':6,7]indolizino[1,2-b]quinolin-1-yl)amino)-7,1,13-trimethyl-1,6,9,1 2,15-pentaoxo-3,18,21,24,27,30,33,36,39-nonaoxa-5,8,11,14-tetraazabut-41-yl)-3,5-bis(2-(methy lsulfonyl)pyrimidin-5-yl)benzamide (15 mg).

The structural characterization data were as follows:
ESI-MS (m/z):1591.7 [M+H]⁺.

The preparation method was as follows:
Chromatographic column: Waters XBridge Prep C18OBD (5 µm*19mm*150 mm)

### Mobile phase A: acetonitrile; Mobile phase B: water (0.05% formic acid)

| Time [min] | Mobile phase A [%] | Mobile phase B [%] | Flow rate[mL/min] |
|---|---|---|---|
| 0.00 | 20 | 80 | 30 |
| 2.00 | 20 | 80 | 30 |
| 18.00 | 90 | 10 | 30 |

### Example 20 N-((7S,10S,13S)-1-(((1S,9S)-5-chloro-9-ethyl-9-hydroxy-4-methyl-10,13-dio xo-2,3,9,10,13,15-hexahydro-1H,12H-benzo[de]pyrano[3',4':6,7]indolizino[1,2-b]quinolin-1-yl)a mino)-7,10,13-trimethyl-1,6,9,12,15-pentaoxo-3,18,21,24-tetraoxa-5,8,11,14-tetraazahexan-26-y l)-2,6-bis(2-(methylsulfonyl)pyrimidin-5-yl)isonicotinamides (C-19)

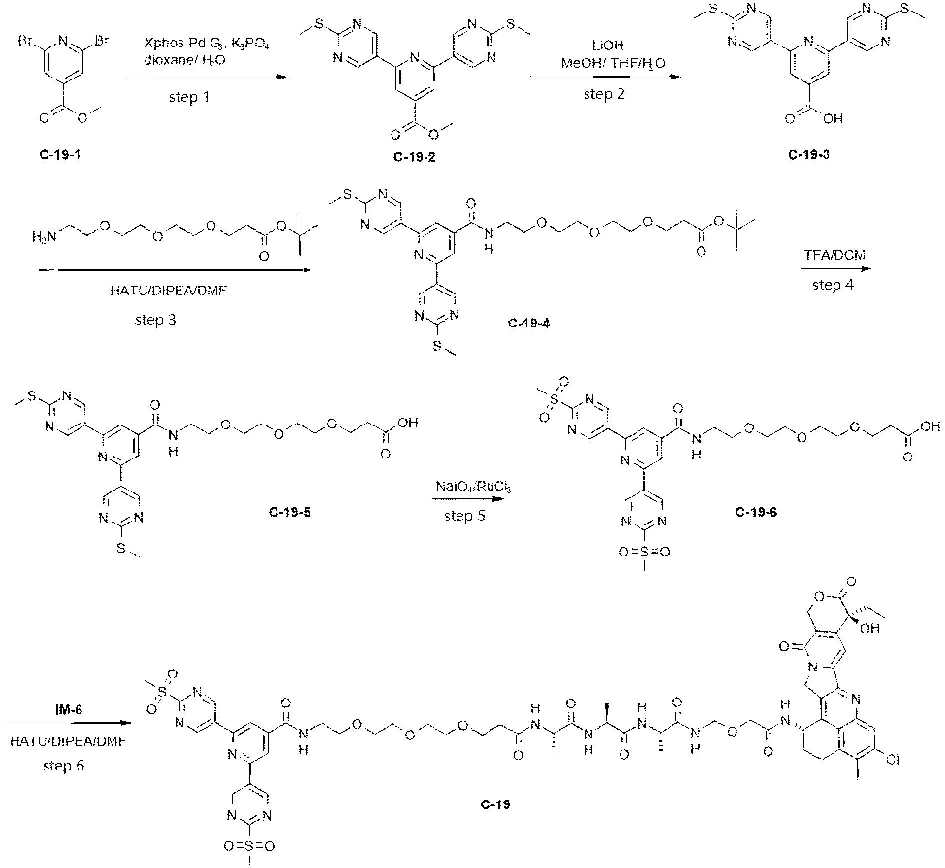

### Step 1:

Methyl 2,6-dibromoisonicotinate (5.00 g, 16.9 mmol), (2-(methylthio)pyrimidin-5-yl)boric acid (6.34 g, 37.3 mmol), XPhos Pd G3 (1.44 g, 1.70 mmol) and potassium phosphate (10.80 g, 50.9 mmol) were added to 1,4-dioxane (51.0 mL) and water (17.0 mL). The reaction system was replaced three times with nitrogen and then reacted at 100 °C for 5 h. After the reaction system was cooled to room temperature, water (50.0 mL) was added into the reaction solution, filtered, and the filtrate was concentrated to obtain a crude product. The crude product was pulped by petroleum ether, and filtered again. The filter cake was vacuum dried to obtain methyl 2,6-bis(2-(methylthio)pyrimidin-5-yl)isonicotinate (5.65 g).

### Step 2:

Methyl 2,6-bis(2-(methylthio)pyrimidin-5-yl) isonicotinate (5.26 g, 13.7 mmol) was dissolved in THF (30.0 mL), MeOH (30.0 mL) and water (30.0 mL), followed by addition of LiOH•H₂O (1.72 g, 40.9 mmol), and the resulting solution was stirred for 2 h at 25 °C. The pH of the reaction solution was adjusted to 3 with 1 N hydrochloric acid aqueous solution. The solid was precipitated, filtered, and the filter cake was dried by vacuum to obtain 2,6-bis(2-(methylthio)pyrimidin-5-yl)isonicotinic acid (4.20 g).

The structural characterization data were as follows:
ESI-MS (m/z):372.1 [M+H]⁺.

### Step 3:

2,6-bis(2-(methylthio)pyrimidin-5-yl)isonicotinic acid (1.50 g, 4.04 mmol) and tert-butyl 3-(2-(2-aminoethoxy)ethoxyethoxyethyl)propionate (1.12 g, 4.04 mmol) were dissolved in DMF (20.0 mL), followed by addition of HOBt (1.64 g, 12.1 mmol), EDCI (2.32 g, 12.1 mmol) and DIPEA(2.09 g, 16.2 mmol), and the resulting solution was heated to 60 °C and stirred for 2 h. After the reaction system was cooled to room temperature, water (10.0 mL) and ethyl acetate (20.0 mL) were added to the reaction solution. Aqueous phase was extracted twice with ethyl acetate (25.0 mL * 2). The organic phases were combined and dried with anhydrous sodium sulfate, filtered and the filtrate was concentrated to obtain crude product tert-butyl 1-(2,6-bis(2-(methylthio)pyrimidin-5-yl)pyridin-4-yl)-1-oxo-5,8,11-trioxa-2-azatetradecan-14-oate (2.50 g), which was directly used in the next reaction without purification.

### Step 4:

Tert-butyl 1-(2,6-bis(2-(methylthio)pyrimidin-5-yl)pyridin-4-yl)-1-oxo-5,8,11-trioxa-2-azatetradecan-14-oate (2.50 g, 3.96 mmol) was dissolved in dichloromethane (3.00 mL), then TFA (4.61 g, 40.4 mmol) was added, and the reaction system was stirred for 12 h at 25 °C. The reaction solution was directly concentrated, purified by preparative high performance liquid chromatography and then lyophilized to obtain 1-(2,6-bis(2-(methylthio)pyrimidin-5-yl)pyridin-4-yl)-1-oxo-5,8,11-trioxa-2-azatetradecan-14-oic acid (1.20 g).

The structural characterization data were as follows:
ESI-MS (m/z):575.2 [M+H]⁺.

The preparation method was as follows:
Chromatographic column: Phenomenex luna C18 (150 mm*25mm*10 µm)

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 36 | 64 | 30 |
| 15.00 | 66 | 34 | 30 |

### Step 5:

1-(2,6-bis(2-(methylthio)pyrimidin-5-yl)pyridin-4-yl)-1-oxo-5,8,11-trioxa-2-azatetradecan-14-oic acid (1.10 g, 1.91 mmol) was dissolved in a mixed solvent of acetonitrile (30 mL) and water (15 mL), then ruthenium trichloride hydrate (39.70 mg, 0.19 mmol) and sodium periodate (4.09 g, 19.14 mmol) were added, and the reaction system was reacted at 25 °C for 1 hour. Extraction with water (50 mL) and ethyl acetate (80 mL) was carried out. The organic phase was concentrated to obtain a crude product, which was purified by column chromatography (MeOH/DCM=10-20%), and then concentrated to obtain 1-(2,6-bis(2-(methylsulfonyl)pyrimidin-5-yl)pyridin-4-yl)-1-oxo-5,8,11-trioxa-2-azatetradecan-14-oic acid (130 mg).

The structural characterization data were as follows:
ESI-MS (m/z):639.2 [M+H]⁺.

### Step 6:

(2S)-2-amino-N-((2S)-1-(((2S)-1-(((2-(((9S)-5-chloro-9-ethyl-9-hydroxy-4-methyl-10,13-di oxo-2,3,9,10,13,15-hexahydro-1H,12H-benzo[de]pyrano[3',4':6,7]indolizino[1,2-b]quinolin-1-yl) amino)-2-oxoethoxy)methyl)amino)-1-oxopropan-2-yl)amino)-1-oxopropan-2-yl)propionamide (IM-6, 20.0 mg, 0.025 mmol) and 1-(2,6-bis(2-(methylsulfonyl)pyrimidin-5-yl)pyridin-4-yl)-1-oxo-5,8,11-trioxa-2-azatetradecan-14-oic acid (16.0 mg, 0.025 mmol) were dissolved in DMF (1 mL) with stirring, followed by addition of HATU (19.0 mg, 0.050 mmol) and DIPEA (12.9 mg, 0.100 mmol), and the resulting solution was reacted for 2 h at room t emperature. The reaction solution was directly purified by preparative high performance li quid chromatography and then lyophilized to obtain N-((7S,10S,13S)-1-(((1S,9S)-5-chloro-9 -ethyl-9-hydroxy-4-methyl-10,13-dioxo-2,3,9,10,13,15-hexahydro-1H,12H-benzo[de]pyrano[3', 4':6,7]indolizino[1,2-b]quinolin-1-yl)amino)-7,10,13-trimethyl-1,6,9,12,15-pentaoxo-3,18,21,24-tetraoxa-5,8,11,14-tetraazahexan-26-yl)-2,6-bis(2-(methylsulfonyl)pyrimidin-5-yl)isonicotinami de (20.4 mg).

The structural characterization data were as follows:
ESI-MS (m/z):1372.4 [M+H]⁺.
Chromatographic column: Waters XBridge Prep C18OBD (5 µm*19mm*150 mm)

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 30 | 70 | 30 |
| 4.00 | 30 | 70 | 30 |
| 24.00 | 90 | 10 | 30 |

### Example 21 N-((7S,10S,13S)-1-(((1S,9S)-5-chloro-9-ethyl-9-hydroxy-4-methyl-10,13-dio xo-2,3,9,10,13,15-hexahydro-1H,12H-benzo[de]pyrano[3',4':6,7]indolizino[1,2-b]quinolin-1-yl)a mino)-7,1,13-trimethyl-1,6,9,12,15-pentaoxo-3,18,21,24,27,30,33,36,39-nonaoxa-5,8,11,14-tetra azabut-4-yl)-2,6-bis(2-(methylsulfonyl)pyrimidin-5-yl)isonicotinamide (C-23)

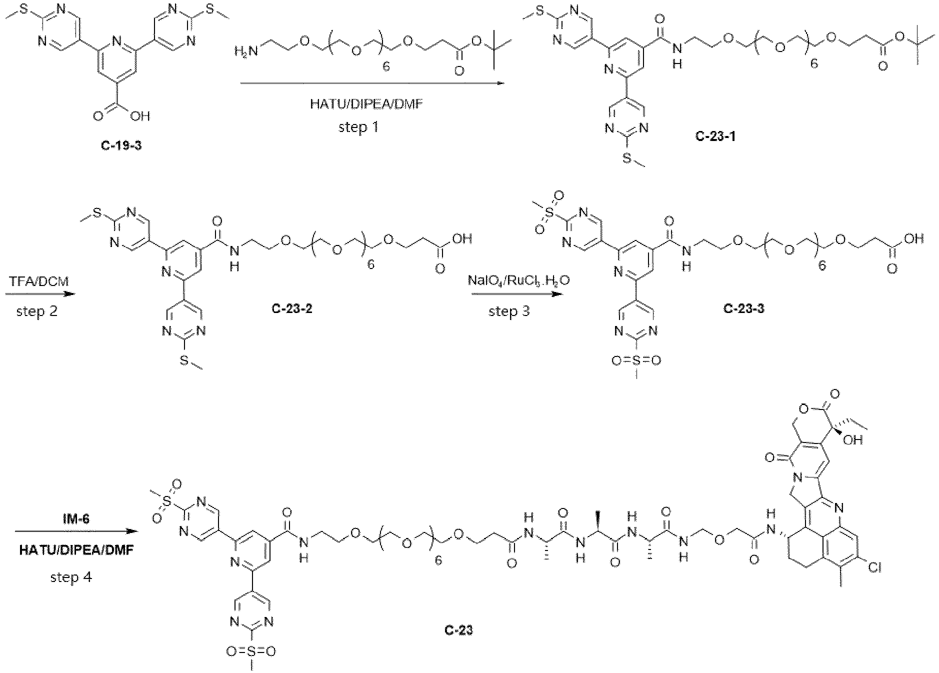

### Step 1:

2,6-bis(2-(methylthio)pyrimidin-5-yl)isonicotinic acid (1.50 g, 4.04 mmol) and tert-butyl 1-amino-3,6,9,12,15,18,21,24-octaoxaheptacosan-27-oate (2.01 g, 4.04 mmol) were added to DMF (20.0 mL), followed by addition of HOBt (1.64 g, 12.1 mmol), EDCI (2.32 g, 12.1 mmol and DIEA (2.09 g, 16.2 mmol). The reaction solution was heated to 60 °C and stirred for 2 h, then cooled to room temperature. Water (10.0 mL) and ethyl acetate (20.0 mL) were added to the reaction solution, and the aqueous phase was extracted twice with ethyl acetate (25.0 mL x 2). The organic phases were combined and dried with anhydrous sodium sulfate, filtered and concentrated to obtain the crude product tert-butyl 1-(2,6-bis(2-(methylthio)pyrimidin-5-yl)pyridin-4-yl)-1-oxo-5,8,11,14,17,20,23,26-octaoxa-2-azanonacosan-29-oate (3.00 g), which was directly used in the next reaction.

### Step 2:

Tert-butyl 1-(2,6-bis(2-(methylthio)pyrimidin-5-yl)pyridin-4-yl)-1-oxo-5,8,11,14,17,20,23, 26-octaoxa-2-azanonacosan-29-oate (3.00 g, 3.53 mmol) was added to dichloromethane (1 0.0 mL), then TFA (15.4 g, 134 mmol) was added, and the resulting solution was stirre d for 12 h at 25 °C. The reaction solution was directly concentrated to obtain a crude p roduct. The crude product was purified by preparative high performance liquid chromatog raphy and then lyophilized to obtain 1-(2,6-bis(2-(methylthio)pyrimidin-5-yl)pyridin-4-yl)-1-oxo-5,8,11,14,17,20,23,26-octaoxa-2-azanonacosan-29-oic acid (1.20 g).

The structural characterization data were as follows:
ESI-MS (m/z):795.3 [M+H]⁺.

The preparation method was as follows:
Chromatographic column: Welch Ultimate C18 (150 mm*25mm*5 µm)

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 34 | 66 | 25 |
| 10.00 | 64 | 36 | 25 |

Step 3: 1-(2,6-bis(2-(methylthio)pyrimidin-5-yl)pyridin-4-yl)-1-oxo-5,8,11,14,17,20,23,26 -octaoxa-2-azanonacosan-29-oic acid (500 mg, 0.63 mmol) was added to acetonitrile (10 mL) and water (5 mL), followed by addition of ruthenium trichloride hydrate (13.0 mg, 0.063 mmol) and sodium periodate (1.35 g, 6.29 mmol). The system was reacted at 25 ° C for 1 h and was extracted with water (10 ml) and ethyl acetate (40 ml). The organic phase was concentrated to obtain a crude product, which was purified by column chroma tography (MeOH/DCM=10~20%) and concentrated to obtain 1-(2,6-bis(2-(methylsulfonyl)p yrimidin-5-yl)pyridin-4-yl)-1-oxo-5,8,11,14,17,20,23,26-octaoxa-2-azanonacosan-29-oic acid (350 mg).

The structural characterization data were as follows:
ESI-MS (m/z):859.3 [M+H]⁺.

### Step 4:

(2S)-2-amino-N-((2S)-1-(((2S)-1-(((2-(((9S)-5-chloro-9-ethyl-9-hydroxy-4-methyl-10,13-di oxo-2,3,9,10,13,15-hexahydro-1H,12H-benzo[de]pyrano[3',4':6,7]indolizino[1,2-b]quinolin-1-yl) amino)-2-oxoethoxy)methyl)amino)-1-oxopropan-2-yl)amino)-1-oxopropan-2-yl)propionamide (IM-6,20.0 mg, 0.025 mmol) and 1-(2,6-bis(2-(methylsulfonyl)pyrimidin-5-yl)pyridin-4-yl)-1 -oxo-5,8,11,14,17,20,23,26-octaoxa-2-azanonacosan-29-oic acid (21.5 mg, 0.025 mmol) wer e dissolved in DMF (1 mL), followed by addition of HATU (19.0 mg, 0.050 mmol) and DIPEA (12.9 mg, 0.100 mmol), and the resulting solution was reacted for 2 h at room temperature. The reaction solution was directly purified by preparative high performance 1 iquid chromatography and then lyophilized to obtain N-((7S,10S,13S)-1-(((1S,9S)-5-chloro-9-ethyl-9-hydroxy-4-methyl-10,13-dioxo-2,3,9,10,13,15-hexahydro-1H,12H-benzo[de]pyrano[3', 4':6,7]indolizino[1,2-b]quinolin-1-yl)amino)-7,1,13-trimethyl-1,6,9,12,15-pentaoxo-3,18,21,24,2 7,30,33,36,39-nonaoxa-5,8,11,14-tetraazabut-4-yl)-2,6-bis(2-(methylsulfonyl)pyrimidin-5-yl)iso nicotinamide (17.0 mg).

The structural characterization data were as follows:
ESI-MS (m/z):1592.6 [M+H]⁺.

The preparation method was as follows:
Chromatographic column: Waters XBridge Prep C18OBD (5µm*19mm*150 mm)

### Mobile phase A: acetonitrile: Mobile phase B: water (0.05% formic acid)

| Time [min] | Mobile phase A [%] | Mobile phase B [%] | Flow rate [mL/min] |
|---|---|---|---|
| 0.00 | 30 | 70 | 28 |
| 4.00 | 30 | 70 | 28 |
| 24.00 | 90 | 10 | 28 |

### Example 22 2,2"-bis(methylsulfonyl)-[5,4':6',5"-tripyrimidine]-2'-carboxylic acid (II-9)

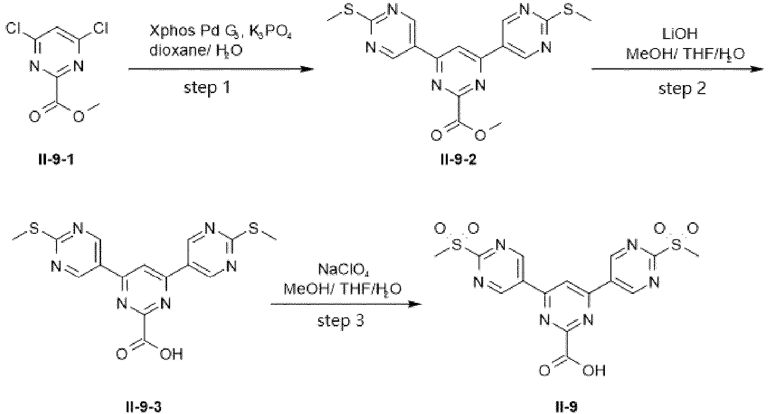

### Step 1:

Methyl 4,6-dichloropyrimidine-2-carboxylate (II-9-1, 1.00 g, 4.83 mmol), (2-methylthiopyrimidin-5-yl)boronic acid (1.81 g, 10.6 mmol), Xphos-Pd-G3 (409 mg, 483 µmol) and K₃PO₄ (3.08 g, 14.5 mmol) were added in 1,4-dioxane (9.00 mL) and water (3.00 mL). After nitrogen replacement for 3 times, the temperature was raised to 100 °C and stirred for 5 h. After the reaction solution was cooled to room temperature, water (60.0 mL) was added into the reaction solution. The reaction solution was continuely stirred for 1 h, and then filtered. The filtrate was concentrated to obtain a crude product. The crude product was pulped by petroleum ether/ethyl acetate (1:1, 40 mL), and filtered again. The filter cake was vacuum dried to obtain methyl 2,2"-bis(methylthio)-[5,4':6',5"-tripyrimidine]-2'-carboxylate (1.39 g, 3.60 mmol).

### Step 2:

Methyl 2,2"-bis(methylthio)-[5,4':6',5"-tripyrimidine]-2'-carboxylate (1.39 g, 3.60 mmol) was dissolved in THF (10.0 mL), methanol (10.0 mL) and water (10.0 mL), then lithium hydroxide (258 mg, 11 mmol) was added, and the resulting solution was stirred for 2 h at 25 °C. The pH of the reaction solution was adjusted to 2 with 1N hydrochloric acid aqueous solution, and solids were precipitated. The reaction solution was filtered, and the filter cake was dried in vacuum to obtain crude product 2,2"-bis(methylthio)-[5,4':6',5"-tripyrimidine]-2'-carboxylic acid (1.06 g).

The structural characterization data were as follows:
ESI-MS (m/z):373.1 [M+H]⁺.

### Step 3:

The same procedure as that for preparing C-23-3 in Example 21 was adopted, in which C-23-2 was replaced by II-9-3 (2,2"-bis(methylthio)-[5,4':6',5"-tripyrimidine]-2'-carboxylic acid) to obtain Compound II-9.

The structural characterization data were as follows:
ESI-MS (m/z):437.0 [M+H]⁺.

### Example 23 1-(2,2"-bis(methylsulfonyl)-[5,4':6',5"-tripyrimidin]-2'-yl)-1-oxo-5,8,11-trioxa -2-azatetradecan-14-oic acid (C-29-3)

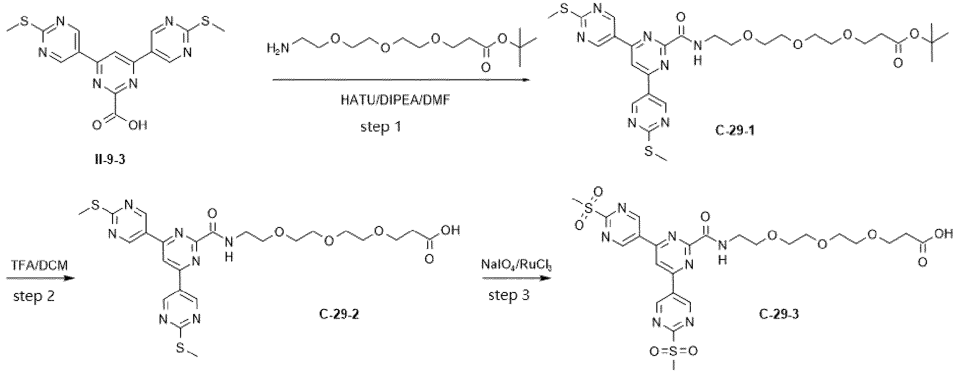

### Step 1:

2,2"-bis(methylthio)-[5,4':6',5"-tripyrimidine]-2'-carboxylic acid (350 mg, 940 µmol) was dissolved in DMF (5.00 mL), followed by addition of T3P (1.91 g, 3.00 mmol, 1.79 mL), DIPEA (365 mg, 2.82 mmol, 491 µL) and tert-butyl 3-[2-[2-(2-aminoethoxy)ethoxy]ethoxy]-propionate (261 mg, 940 µmol), and the resulting solution was stirred at 25 °C for 6 h. Water (60.0 mL) was added to the reaction solution, and extraction with ethyl acetate was carried out for three times (40 mL x 3). The organic phases were combined and dried with anhydrous sodium sulfate, filtered and concentrated to obtain crude product tert-butyl 1-(2,2"-bis(methylthio)-[5,4':6',5"-tripyrimidine]-2'-yl)-1-oxo-5,8,11-trioxa-2-azatetradecan-14-oate (850 mg).

### Step 2:

Tert-butyl 1-(2,2"-bis(methylthio)-[5,4':6',5"-tripyrimidine]-2'-yl)-1-oxo-5,8,11-trioxa-2-azatetradecan-14-oate (800 mg, 1.27 mmol) was dissolved in dichloromethane (2.00 mL), then TFA (3.07 g, 26.9 mmol, 2.00 mL) was added, and the resulting solution was stirred for 2 h at 25 °C. The reaction solution was directly concentrated, and purified by preparative high performance liquid chromatography and then lyophilized to obtain1-(2,2"-bis(methylthio)-[5,4':6',5"-tripyrimidin]-2'-yl)-1-oxo-5,8,11-trioxa-2-azatetradecan-14-oic acid (145 mg, 249 µmol).

The structural characterization data were as follows:
ESI-MS (m/z):576.2 [M+H]⁺.

The preparation method was as follows:
Chromatographic column: Phenomenex luna C18 (150mm*25 mm*10µm)

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 27 | 73 | 30 |
| 10.00 | 57 | 43 | 30 |

### Step 3:

The same procedure as that for preparing C-23-3 in Example 21 was adopted, in which C-23-2 was replaced by C-29-2 (1-(2,2"-bis(methylthio)-[5,4':6',5"-tripyrimidin]-2'-yl)-1-oxo-5,8,11-trioxa-2-azatetradecan-14-oic acid) to obtain Compound C-29-3.

The structural characterization data were as follows:
ESI-MS (m/z):640.1 [M+H]⁺.

### Example 24 1-(2,2"-bis(methylsulfonyl)-[5,4':6',5"-tripyrimidine]-2'-yl)-1-oxo-5,8,11,14,1 7,20,23,26-octaoxa-2-azanonan-29-oic acid (C-30-3)

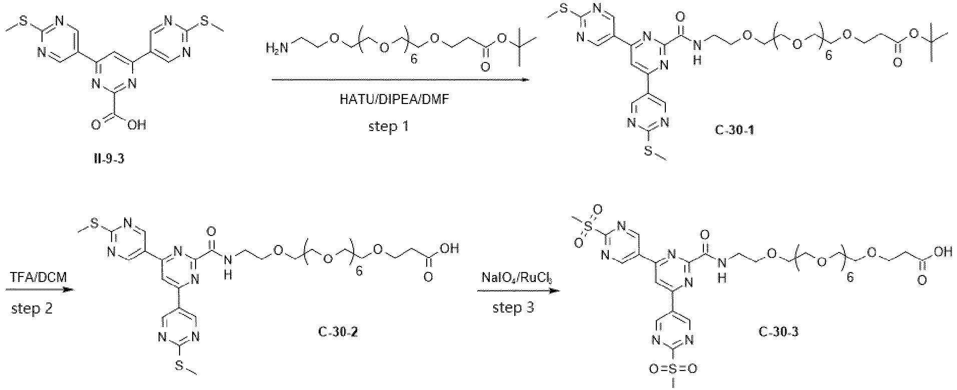

### Step 1:

4,6-bis(2-methylthiopyrimidin-5-yl)pyrimidine-2-carboxylic acid (0.35 g, 940 µmol) was dissolved in DMF (6.00 mL), followed by addition of T3P (2.04 g, 3.21 mmol, 1.91 mL), DIPEA (364 mg, 2.82 mmol, 491 µL) and tert-butyl 1-amino-3,6,9,12,15,18,21,24-octaoxaheptan -27-oate (468 mg, 940 µmol). The reaction solution was stirred at 25 °C for 12 hours. Water (60.00 mL) was added to the reaction solution and extraction with ethyl acetate was carried out for three times (40.00 mL x 3). The organic phases were combined and dried with anhydrous sodium sulfate, filtered and concentrated to obtain crude product tert-butyl 1-(2,2"-bis(methylthio)-[5,4':6',5"-tripyrimidine]-2'-yl)-1-oxo-5,8,11,14,17,20,23,26-octaoxa-2-azanonan-29-oate (800 mg).

### Step 2:

Tert-butyl 1-(2,2"-bis(methylthio)-[5,4':6',5"-tripyrimidine]-2'-yl)-1-oxo-5,8,11,14,17,20,23,26-octaoxa-2-azanonan-29-oate (800 mg, 939 µmol) was dissolved in dichloromethane (10.0 mL), then TFA (3.07 g, 26.9 mmol, 2 mL) was added, and the resulting solution was stirred for 2 h at 25 °C. The reaction solution was directly concentrated, and purified by preparative high performance liquid chromatography and then lyophilized to obtain 1-(2,2"-bis(methylthio)-[5,4':6',5"-tripyrimidine]-2'-yl)-1-oxo-5,8,11,14,17,20,23,26-octaoxa-2-azanonan-29-oic acid (389 mg, 483 µmol).

The structural characterization data were as follows:
ESI-MS (m/z):796.3 [M+H]⁺.

The preparation method was as follows:
Chromatographic column: Phenomenex luna C18 (150 mm*25 mm*10 µm)

### Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 29 | 71 | 25 |
| 10.00 | 59 | 41 | 25 |

### Step 3:

The same procedure as that for preparing C-23-3 in Example 21 was adopted, in w hich C-23-2 was replaced by C-30-2 (1-(2,2"-bis(methylthio)-[5,4':6',5"-tripyrimidine]-2'-yl)-1-oxo-5,8,11,14,17,20,23,26-octaoxa-2-azanonan-29-oic acid) to obtain Compound C-30-3.

The structural characterization data were as follows:
ESI-MS (m/z):860.3 [M+H]⁺.

### 3. Preparation of Bioactive Conjugate

### 3.1 Preparation of bioactive conjugates of anti-ROR1 antibody

### Conjugation method A:

0.5 mL of antibody (anti-ROR1 antibody 19F6_Hu35V1, 3-20 mg/mL, abbreviated as 19F6 in Table 1) was diluted with 0.1 M disodium edetate solution (pH 7.60), followed by addition of 1 M Na₂HPO₄ solution to adjust pH to 7.60; 10 mM TCEP (Tris (2-carboxyethyl) phosphine) solution (pH 7.60) was then added and mixed well, and the resulting solution was placed at room temperature for 2 hrs. The payload-linker (10 mM) dissolved in DMSO was added into the above solution system in a molar ratio of 5-10 times to the antibody and mixed well, and let the resulting solution stand for 20 h at room temperature. At the end of the reaction, the buffer was replaced with a 10 mM histidine buffer solution at pH 6.0 using an NAP-5 gel column (Cytiva) to obtain an ADC product, as shown in Table 1.

### Conjugation method B:

0.5 mL of antibody (anti-ROR1 antibody 19F6_Hu35V1, 3-20 mg/mL, abbreviated as 19F6 in Table 1) was diluted with 0.1 M disodium edetate solution (pH 7.60), followed by addition of 1 M Na₂HPO₄ solution to adjust pH to 7.60; 10 mM TCEP (Tris(2-carboxyethyl)phosphine)solution (pH 7.60) was then added and well mixed, and the resulting solution was placed at room temperature for 2 hrs. TCEP was removed with NAP-5 gel column (Cytiva), and the payload-linker (10 mM) dissolved in DMSO was added into the resulting solution system at a molar ratio of 5-10 times to the antibody and mixed well, and let the resulting solution stand at room temperature for 20 hrs. At the end of the reaction, the buffer was replaced with 10 mM histidine buffer solution at pH 6.0 using NAP-5 gel column (Cytiva) to obtain an ADC product, as shown in Table 1.

### Conjugation method C: (Suitable for preparation of 19F6-MC-VC-PABC-MMAE)

1.8 mL of antibody 19F6_Hu35V1 (22.7 g/L) was diluted with 90 uL of 20 mM PB + 0.1 M EDTA (pH 7.60), followed by addition of 1M Na₂HPO₄ to adjust pH to 7.66; and 10 mM TCEP (Tris(2-carboxyethyl)phosphine, 66.2 uL, pH 7.60) solution was added and mixed well, and let the resulting solution stand at room temperature for 1.5 hrs. A payload-linker (MC-VC-PABC-MMAE, 10 mM) solution was added into the above solution system at a molar ratio of 4.8 times and mixed well, and let the resulting solution stand still at room temperature for 2 h. At the end of the reaction, the buffer was replaced with a 10 mM histidine-histidine hydrochloride buffer solution at pH 6.0 using an NAP-25 gel column (Cytiva). An antibody-drug conjugate, ADC (19F6-MC-VC-PABC-MMAE) was obtained. The DAR determined by mass spectrometry was 4.15.

**Table 1: ADC preparation method, number and DAR**

| Coupling reaction substrates | Coupling method | ADC Name | Drug-linker/antibody molar ratio | Average DAR(n₁) | Percentage of DAR4 |
|---|---|---|---|---|---|
| 19F6+ Drug-linker 7 | Method B | 19F6-ADC III-5-A | 6.0 | 3.87 | 56.7% |
| 19F6+ Drug-linker 10 | Method B | 19F6-ADC III-6-A | 7.0 | 4.42 | 53.2% |
| 19F6+ Drug-linker 12 | Method A | 19F6-ADC IV-1-A | 6.5 | 3.74 | 79.1% |
| 19F6+ Drug-linker 12 | Method A | 19F6-ADC IV-1-B | 8.0 | 3.75 | 80.0% |
| 19F6+ Drug-linker 12 | Method B | 19F6-ADC IV-1-C | 6.5 | 3.82 | 88.5% |
| 19F6+ Drug-linker 12 | Method B | 19F6-ADC IV-1-D | 8.0 | 3.87 | 91.4% |
| 19F6+ Drug-linker 16 | Method A | 19F6-ADC IV-7-A | 10.0 | 3.98 | 57.6% |

| | | | | | |
|---|---|---|---|---|---|
| Determination of Drug-to-Antibody Ratio (DAR) of Bioactive Conjugates | | | | | |

The molecular weight of ADC samples was determined by SEC-MS, and the drug-to-antibody ratio (DAR) was calculated.

Chromatography conditions:
Chromatographic column: ACQUITY UPLC Protein BEH SEC Column,BTQR-18-016;
Sample room temperature: 8 °C; Column temperature: not controlled; UV: 280 nm;
Mobile phase: 20mM ammonium acetate, Flow rate: 0. 1ml/min, 20 min, Sample load: 50 ug;
Mass chromatography conditions:
   Mass spectrometer model: AB Sciex Triple TOF 5600+;
   GS1 55; GS2 55; CUR 30; TEM 450; ISVF 5500; DP 75; CE 5;
   m/z 900-7000; Time bins to sum 100.
      (1) The molecular weight of 19F6-ADC III-5-A was determined by SEC-MS, and the drug-to-antibody ratio was calculated.

SEC-MS molecular weight analysis of the conjugated 19F6-ADC III-5-A was shown in Table 2 and DAR was 3.87.

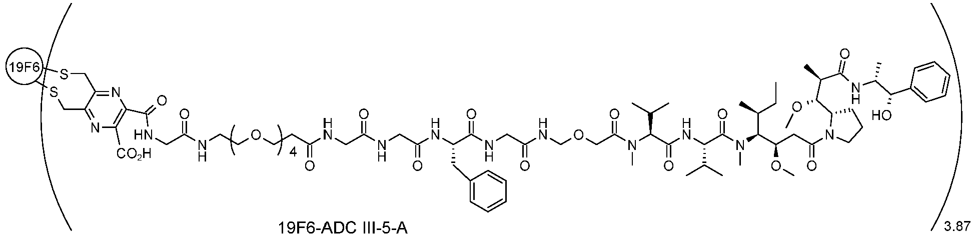

**Table 2: Measured molecular weight of 19F6-ADC III-5-A and DAR calculation**

| Load | Exp. | Maximum signal strength | Ratio | DAR |
|---|---|---|---|---|
| Naked antibody | 145050.00 | 0.00 | 0.000 | 3.87 |
| DAR2 | N/A | 0.00 | 0.000 | |
| DAR3 | 149863.68 | 18.50 | 0.282 | |
| DAR4 | 151472.75 | 37.25 | 0.567 | |
| DAR5 | 153078.63 | 9.92 | 0.151 | |

(2) The molecular weight of 19F6-ADC III-6-A was determined by SEC-MS, and the drug-to-antibody ratio was calculated.

SEC-MS molecular weight analysis of the conjugated 19F6-ADC III-6-A was shown in Table 3 and DAR was 4.42.

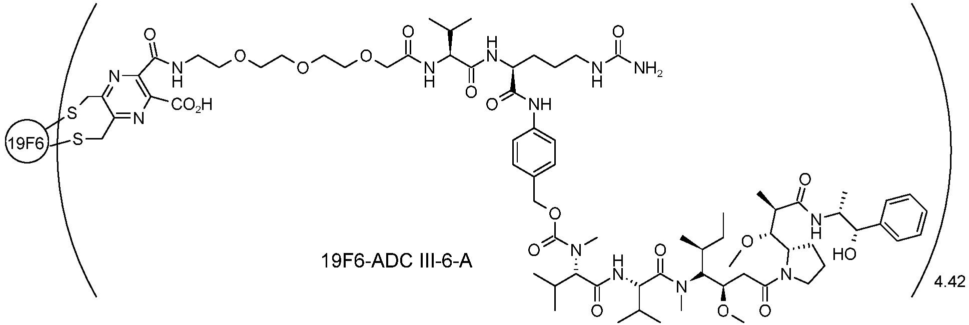

**Table 3: Measured molecular weight of 19F6-ADC III-6-A and DAR calculation**

| Load | MW_{Exp.} | Maximum signal strength | Ratio | DAR |
|---|---|---|---|---|
| Naked antibody | 145050.00 | 0.00 | 0.000 | 4.42 |
| DAR2 | N/A | 0.00 | 0.000 | |
| DAR3 | 149510.90 | 4.85 | 0.052 | |
| DAR4 | 151001.13 | 49.64 | 0.532 | |
| DAR5 | 152483.55 | 33.24 | 0.356 | |
| DAR6 | 153972.11 | 5.54 | 0.059 | |

(3) The molecular weight of 19F6-ADC IV-1-A was determined by SEC-MS, and the drug-to-antibody ratio was calculated.

SEC-MS molecular weight analysis of the conjugated 19F6-ADC IV-1-A was shown in Table 4 and DAR was 3.74.

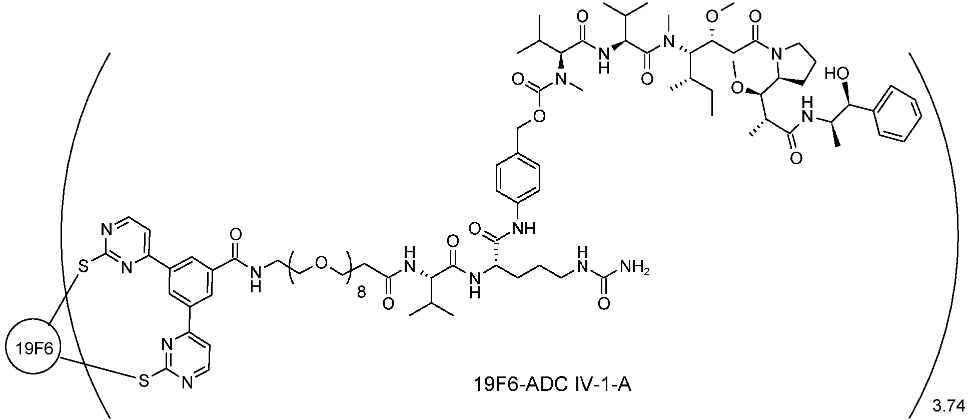

**Table 4: Measured molecular weight of 19F6-ADC IV-1-A and DAR calculation**

| Load | MW_{Exp.} | Maximum signal strength | Ratio | DAR |
|---|---|---|---|---|
| Naked antibody | 145054.77 | 0.00 | 0.000 | 3.74 |
| DAR1 | 146863.97 | 2.09 | 0.017 | |
| DAR2 | 148669.83 | 1.75 | 0.014 | |
| DAR3 | 150468.31 | 21.98 | 0.178 | |
| DAR4 | 152274.07 | 97.91 | 0.791 | |

(4) The molecular weight of 19F6-ADC IV-1-B was determined by SEC-MS, and the drug-to-antibody ratio was calculated.

SEC-MS molecular weight analysis of the conjugated 19F6-ADC IV-1-B was shown in Table 5 and DAR was 3.75.

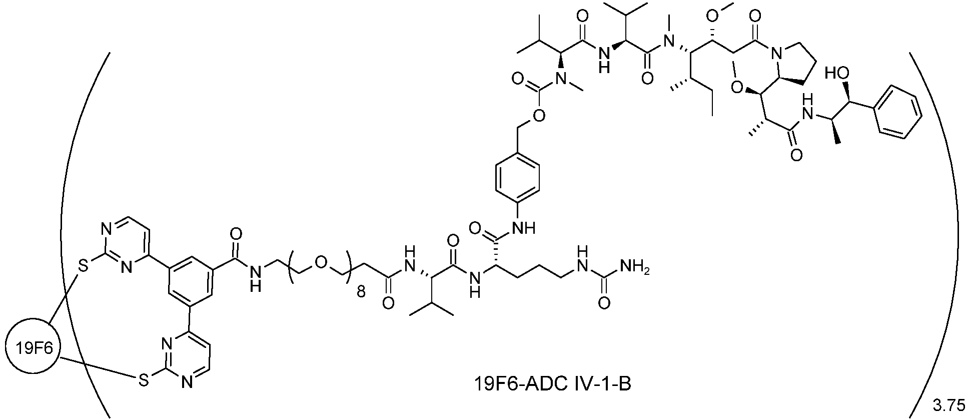

**Table 5: Measured molecular weight of 19F6-ADC IV-1-B and DAR calculation**

| Load | Exp. | Maximum signal strength | Ratio | DAR |
|---|---|---|---|---|
| Naked antibody | 145054.77 | 0.00 | 0.000 | 3.75 |
| DAR1 | 146861.63 | 1.96 | 0.015 | |
| DAR2 | 148662.46 | 2.54 | 0.020 | |
| DAR3 | 150467.51 | 21.19 | 0.165 | |
| DAR4 | 152273.42 | 102.47 | 0.800 | |

(5) The molecular weight of 19F6-ADC IV-1-C was determined by SEC-MS, and the drug-to-antibody ratio was calculated.

SEC-MS molecular weight analysis of the conjugated 19F6-ADC IV-1-C was shown in Table 6 and DAR was 3.82.

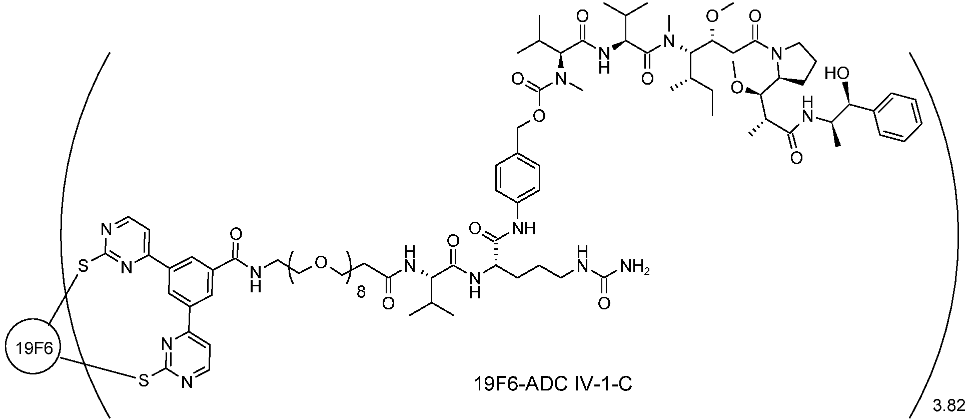

**Table 6: Measured molecular weight of 19F6-ADC IV-1-C and DAR calculation**

| Load | MW_{Exp.} | Maximum signal strength | Ratio | DAR |
|---|---|---|---|---|
| Naked antibody | 145054.77 | 0.00 | 0.000 | 3.82 |
| DAR1 | 146862.39 | 1.68 | 0.025 | |
| DAR2 | 148667.57 | 0.96 | 0.014 | |
| DAR3 | 150470.67 | 5.12 | 0.076 | |
| DAR4 | 152273.43 | 60.04 | 0.885 | |

(6) The molecular weight of 19F6-ADC IV-1-D was determined by SEC-MS, and the drug-to-antibody ratio was calculated.

SEC-MS molecular weight analysis of the conjugated 19F6-ADC IV-1-D was shown in Table 7 and DAR was 3.87.

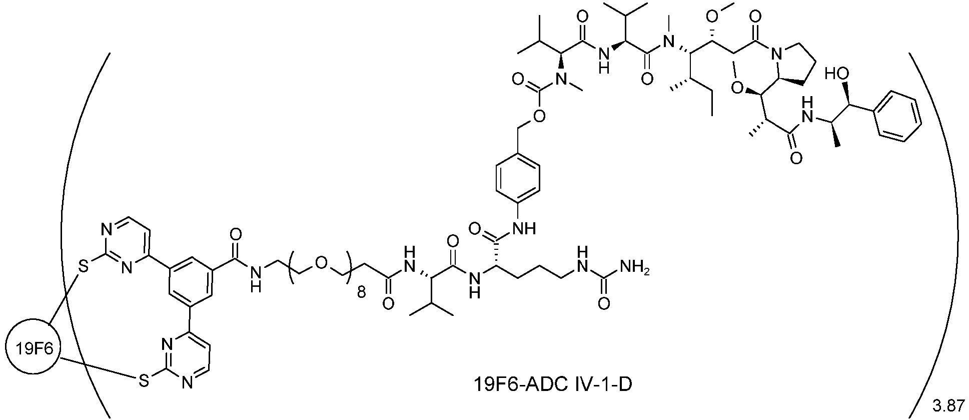

**Table 7: Measured molecular weight of 19F6-ADC IV-1-D and DAR calculation**

| Load | MW_{Exp.} | Maximum signal strength | Ratio | DAR |
|---|---|---|---|---|
| Naked antibody | 145054.77 | 0.00 | 0.000 | 3.87 |
| DAR1 | 146855.00 | 1.79 | 0.017 | |
| DAR2 | 148660.10 | 1.00 | 0.009 | |
| DAR3 | 150470.36 | 6.44 | 0.060 | |
| DAR4 | 152273.33 | 98.45 | 0.914 | |

(7) The molecular weight of 19F6-ADC IV-7-A was determined by SEC-MS, and the drug-to-antibody ratio was calculated.

SEC-MS molecular weight analysis of the conjugated 19F6-ADC IV-7-A was shown in Table 8 and DAR was 3.98.

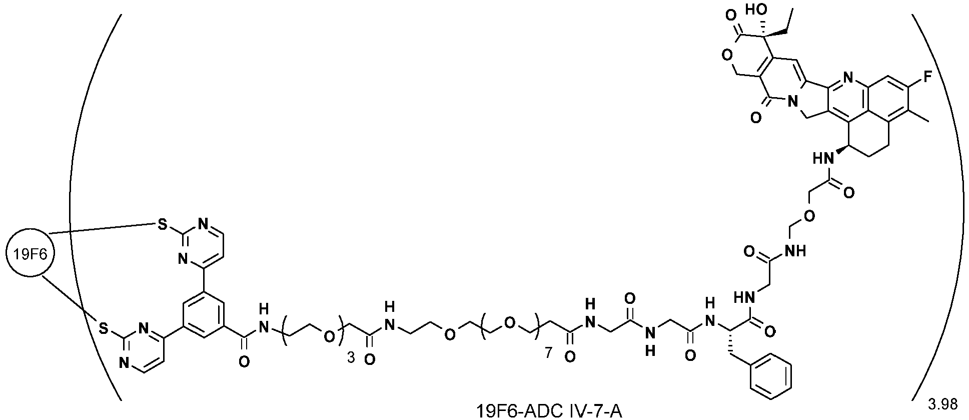

**Table 8: Measured molecular weight of 19F6-ADC IV-7-A and DAR calculation**

| Load | MW_{Exp.} | Maximum signal strength | Ratio | DAR |
|---|---|---|---|---|
| Naked antibody | 145054.69 | 1.75 | 0.013 | 3.98 |
| DAR1 | 146760.24 | 2.02 | 0.016 | |
| DAR2 | 148460.32 | 5.75 | 0.044 | |
| DAR3 | 150178.32 | 11.66 | 0.090 | |
| DAR4 | 151894.99 | 74.75 | 0.576 | |
| DAR5 | 153762.19 | 33.88 | 0.261 | |

### 3.2 Preparation of bioactive conjugates of antibody B7-H3

### 3.2.1 Preparation of 2#8890ADC C-10 (DAR 4)

0.2274 ml of antibody 2#8890 (10.994 mg/mL) was diluted with 11.4 uL 20 mM PB + 0.1M EDTA (pH 7.60), followed by addition of 1M Na₂HPO₄ solution to adjust pH to 7.60, and 10 mM TCEP(Tris(2-carboxyethyl)phosphine, 9.5 uL, pH 7.60) solution was added and mixed well, and the resulting solution was placed at room temperature for 1.5 hrs. Then the solution of C-10 (14 uL, 10 mM) dissolved in dimethyl sulfoxide was slowly added in 8 times molar ratio and mixed well, and let the resulting solution stand overnight at room temperature. After the completion of the reaction, the buffer solution was replaced with 20 mM histidine buffer solution at pH 6.0 using NAP-5 gel column (Cytiva) to obtain an antibody-drug conjugate 2#8890ADC C-10. The DAR determined by mass spectrometry was 4.17.

LC-MS molecular weight analysis was performed on the conjugated ADC samples.

Chromatography conditions:
Liquid chromatography column: ACQUITY UPLC MAbPac BEH SEC;
Mobile phase A:20 mM NH4Ac;
Flow rate: 0.1 ml/min; Sample room temperature: 8°C; Column temperature: 60°C; Sample load:2 µl;

| | | | | | | |
|---|---|---|---|---|---|---|
| Time (Min) | 2 | 20 | 22 | 25 | 26 | 30 |
| Mobile phase A (vol%) | 75 | 20 | 5 | 5 | 75 | 75 |
| Mobile phase B (vol%) | 25 | 80 | 95 | 95 | 25 | 25 |

Mass chromatography conditions:
Mass spectrometer model: AB Sciex Triple TOF 5600+;
GS1 55; GS2 55; CUR 30; TEM 450; ISVF 5500; DP 75; CE 5; Accumulation time 0.5 s;
m/z 900-7000; Time bins to sum 40.

### 3.2.2 Preparation of 2#8890ADC C-17 (DAR 4)

0.292 mL of antibody 2#8890 (8.565 mg/mL) was diluted with 14.6 uL 20 mM PB + 0.1M EDTA (pH 7.60), followed by addition of 1M Na₂HPO₄ solution to adjust pH to 7.60, and 20 mM TCEP (Tris(2-carboxyethyl)phosphine, 4.77 uL, pH 7.60) solution was added and mixed well and the resulting solution was placed at room temperature for 1.5 hrs. C-17 (8.76 uL, 10 mM) solution dissolved in dimethyl sulfoxide was slowly added in 5 times molar ratio and mixed well, and let the resulting solution stand overnight at room temperature. After the completion of the reaction, the buffer solution was replaced with 20 mM histidine buffer solution at pH 6.0 by NAP-5 gel column (Cytiva) to obtain an antibody-drug conjugate 2#8890ADC C-17. The DAR determined by mass spectrometry was 3.86.

**Table 9: Measured molecular weight of 2#8890ADC C-17 and DAR calculation**

| Load | MW_{Exp.} | Maximum signal strength | Ratio | DAR |
|---|---|---|---|---|
| Naked antibody | 143589.05 | 4.28 | 2.89% | 3.86 |
| DAR1 | 144812.48 | 3.51 | 2.37% | |
| DAR2 | 146030.36 | 6.03 | 4.08% | |
| DAR3 | 147256.26 | 15.31 | 10.34% | |
| DAR4 | 148472.07 | 86.98 | 58.78% | |
| DAR5 | 149698.77 | 29.18 | 19.72% | |
| DAR6 | 150923.77 | 2.69 | 1.82% | |
| DAR7 | 152146.77 | 0.00 | 0.00% | |
| DAR8 | 152428.06 | 0.00 | 0.00% | |

### 3.2.3 Preparation of 2#8890ADC C-19 (DAR 4)

0.584 mL antibody 2#8890 (8.565 mg/mL) was diluted with 29.2 uL 20 mM PB + 0.1M EDTA (pH 7.60), followed by addition of 1M Na₂HPO₄ solution to adjust pH to 7.60; 20 mM TCEP (Tris (2-carboxyethyl) phosphine, 9.54 uL, pH 7.60) solution was added and mixed well and the resulting solution was placed at room temperature for 1.5 hrs. C-19 (17.9 uL, 10 mM) solution dissolved in dimethyl sulfoxide was slowly added in 5.5 times molar ratio and mixed well, and let the resulting solution stand overnight at room temperature. After the completion of the reaction, the buffer solution was replaced with 20 mM histidine buffer solution at pH 6.0 using NAP-5 gel column (Cytiva) to obtain an antibody-drug conjugate 2#8890ADC C-19. The DAR determined by mass spectrometry was 4.05.

**Table 10: Measured molecular weight of 2#8890ADC C-19 and DAR calculation**

| Load | MW_{Exp.} | Maximum signal strength | Ratio | DAR |
|---|---|---|---|---|
| Naked antibody | 143573.53 | 0.00 | 0.00% | 4.05 |
| DAR1 | 144803.53 | 0.00 | 0.00% | |
| DAR2 | 146023.53 | 5.46 | 4.74% | |
| DAR3 | 147249.84 | 11.84 | 10.27% | |
| DAR4 | 148487.65 | 76.03 | 65.95% | |
| DAR5 | 149720.90 | 15.88 | 13.77% | |
| DAR6 | 150951.98 | 2.92 | 5.27% | |
| DAR7 | 152177.98 | 0.00 | 0.00% | |
| DAR8 | 152277.10 | 0.00 | 0.00% | |

### 3.2.4 Preparation of 2#8890ADC C-21 (DAR 4)

0.584 mL antibody 2#8890 (8.565 mg/mL) was diluted with 29.2 uL 20 mM PB + 0.1M EDTA (pH 7.60), followed by addition of 1M Na₂HPO₄ solution to adjust pH to 7.60, and 20 mM TCEP(Tris(2-carboxyethyl)phosphine, 9.54 uL, pH 7.60) solution was added and mixed well and the resulting solution was placed at room temperature for 1.5 hrs. C-21 (17.9 uL, 10 mM) solution dissolved in dimethyl sulfoxide was slowly added in 5.5 times molar ratio and mixed well, and let the resulting solution stand at room temperature. After the completion of the reaction, the buffer solution was replaced with 20 mM histidine buffer solution at pH 6.0 by using NAP-5 gel column (Cytiva) to obtain an antibody-drug conjugate 2#8890ADC C-21. The DAR determined by mass spectrometry was 3.92.

**Table 11: Measured molecular weight of 2#8890ADC C-21 and DAR calculation**

| Load | MW_{Exp.} | Maximum signal strength | Ratio | DAR |
|---|---|---|---|---|
| Naked antibody | 143563.00 | 0.00 | 0.00% | 3.92 |
| DAR1 | 145014.00 | 0.00 | 0.00% | |
| DAR2 | 146465.00 | 8.13 | 3.15% | |
| DAR3 | 147916.87 | 39.28 | 15.23% | |
| DAR4 | 149349.10 | 176.57 | 68.45% | |
| DAR5 | 150783.40 | 33.96 | 13.17% | |
| DAR6 | 152234.40 | 0.00 | 0.00% | |
| DAR7 | 153685.40 | 0.00 | 0.00% | |
| DAR8 | 1451.00 | 0.00 | 0.00% | |

### 4. Detection of the inhibitory effect of the bioactive conjugates on in vitro cell viability

### 4.1 Inhibitory effect of ADC on cell proliferation

(1) Cell plating: First, NCI-N87 tumor cells were cultured in a corresponding culture medium, digested with trypsin, centrifuged, followed by resuspension and counting. The cell suspension was adjusted to an appropriate concentration for plating. The source of tumor cells is shown in Table 9.

**Table 9: Source of tumor cells**

| Cell name | Tumor type | Source |
|---|---|---|
| NCI-N87 | Human gastric cancer cell | ATCC |

Co-incubation of the ADC of the present disclosure and tumor cells: After the cells adhered to the wall, the culture medium was removed, and the diluted bioactive molecule (the ADC of the present disclosure) was added to wells of the above plate and incubated for 96 h.

In vitro cell viability detection: After the incubation, 50 µL of Cell Counting-Lite^{™} 2.0 reagent (Vazyme) was added to each well, shaken and mixed in dark. After 10 minutes of reaction, the detection was carried out, and reads were given by a microplate reader (manufacturer: BMG, model: PHERAStar-FS). The background RLU was obtained by using Cell Counting-Lite^{™} in the culture medium without cells, and the vehicle RLU was obtained by using Cell Counting-Lite^{™} in the culture medium containing cells. Cell inhibition rate = 1-(sample RLU-background RLU)/(vehicle RLU-background RLU)×100%, and the IC₅₀ of the compounds was calculated according to a four-parameter model fitting curve.

(2) Data results: The test results are shown in Table 10.

**Table 10: Killing results of ADCs on NCI-N87 cell line**

| ADC number | IC₅₀ (µg/mL) |
|---|---|
| | NCI-N87 (96 h) |
| 19F6-ADC III-6-A | 8.48 |
| 19F6-ADC IV-1-D | 2.57 |
| 19F6-MC-VC-PABC-MMAE | 24.4 |

It is shown that the ADCs (19F6-ADCIII-6-A and 19F6-ADCIV-1-D) prepared by the new conjugation method can kill tumor cells, and the new conjugation method is effective in preparation of ADC molecules. And compared with the randomly conjugated ADC (19F6-MC-VC-PABC-MMAE), the ADCs (19F6-ADCIII-6-A and 19F6-ADCIV-1-D) prepared by the new conjugation method showed better cell killing activity.

### 4.2 Inhibitory effect of ADC on HT29 cell proliferation

(1) Cell plating: First, HT29 tumor cells were cultured in a corresponding culture medium, digested with trypsin, centrifuged, followed by resuspension and counting. The cell suspension was adjusted to an appropriate concentration for plating. The source of tumor cells is shown in Table 11.

**Table 11: Source of tumor cells**

| Cell name | Tumor type | Source |
|---|---|---|
| HT29 | human colon cancer cell | Nanjing Co-bioer Bioscience |

Co-incubation of the ADC of the present disclosure and tumor cells: After the cells adhered to the wall, the culture medium was removed, and the diluted bioactive molecule (the ADC of the present disclosure) was added to the wells of the above plate and incubated for 96 h.

In vitro cell viability detection: After the incubation, 50 µL of Cell Counting-Lite^{™} 2.0 reagent (Vazyme) was added to each well, shaken and mixed in dark. After 10 minutes of reaction, the detection was carried out, and reads were given by a microplate reader (manufacturer: BMG, model: PHERAStar-FS). The background RLU was obtained by using Cell Counting-Lite^{™} in the culture medium without cells, and the vehicle RLU was obtained by using Cell Counting-Lite^{™} in the culture medium containing cells. Cell inhibition rate = 1-(sample RLU-background RLU)/(vehicle RLU-background RLU)×100%, and the IC₅₀ of the compounds was calculated according to a four-parameter model fitting curve.

(2) Data results: The test results are shown in Table 12.

**Table 12: Killing results of ADCs on HT29 cell line**

| ADC number | IC₅₀ (µg/mL) |
|---|---|
| | HT29 (96 h) |
| 19F6-ADC IV-1-D | 19.47 |

It is shown that the ADC (19F6-ADC IV-1-D) prepared by the new conjugation method can kill tumor cells, and the new conjugation method is effective in preparation of ADC molecules.

### 4.3 Inhibitory effect of ADC on NCI-H1975 cell proliferation

(1) Cell plating: First, NCI-H1975 tumor cells were cultured in a corresponding culture medium, digested with trypsin, centrifuged, followed by resuspension and counting. The cell suspension was adjusted to an appropriate concentration for plating. The source of tumor cells is shown in Table 13.

**Table 13: Source of tumor cells**

| Cell name | Tumor type | Source |
|---|---|---|
| NCI-H1975 | human lung adenocarcinoma cell | Nanjing Co-bioer Bioscience |

Co-incubation of the ADC of the present disclosure and tumor cells: After the cells adhered to the wall, the culture medium was removed, and the diluted bioactive molecule (the ADC of the present disclosure) was added to wells of the above plate and incubated for 96 h.

In vitro cell viability detection: After the incubation, 50 µL of Cell Counting-Lite^{™} 2.0 reagent (Vazyme) was added to each well, shaken and mixed in dark. After 10 minutes of reaction, the detection was carried out, and reads were given by a microplate reader (manufacturer: BMG, model: PHERAStar-FS). The background RLU was obtained by using Cell Counting-Lite^{™} in the culture medium without cells, and the vehicle RLU was obtained by using Cell Counting-Lite^{™} in the culture medium containing cells. Cell inhibition rate = 1-(sample RLU-background RLU)/(vehicle RLU-background RLU)×100%, and the IC₅₀ of the compounds was calculated according to a four-parameter model fitting curve.

(2) Data results: The test results are shown in Table 14.

**Table 14: Killing results of ADCs on NCI-H1975 cell line**

| ADC number | IC₅₀ (µg/mL) |
|---|---|
| | NCI-H1975 (96 h) |
| 19F6-ADC IV-1-D | 1.79 |

It is shown that the ADC (19F6-ADC IV-1-D) prepared by the new conjugation method can kill tumor cells, and the new conjugation method is effective in preparation of ADC molecules.

In addition to those described herein, various modifications of the present disclosure will be obvious to those skilled in the art based on the foregoing description. Such modifications are also intended to fall within the scope of the appended claims.

## Claims

1. A compound or a pharmaceutically acceptable salt, ester, stereoisomer, tautomer, polymorph, solvate, N-oxide or isotope labeled compound thereof, wherein the compound has a structure of formula I: 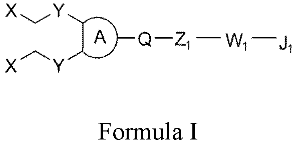 wherein
X is a leaving group, such as F, Cl, Br, I, OMs, OTs, OTf, p-nitrophenol ester, fluorophenol ester, C₁₋₆ alkyl sulfonyl or 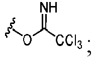
Y is absent or is selected from substituted or unsubstituted C₁₋₆ alkylene, sulfonyl and carbonyl, and when being substituted, the C₁₋₆ alkylene is substituted by substituent(s) selected from hydrogen, halogen, hydroxyl, -CN, -C₁₋₆ alkyl, -C₁₋₆ haloalkyl, -OC₁₋₆ alkyl, and -OC₁₋₆ haloalkyl;
Ring A is selected from substituted or unsubstituted C₆₋₁₀ aromatic ring, substituted or unsubstituted 5- to 12-membered heteroaromatic ring and substituted or unsubstituted 5- to 12-membered heterocycle, and when being substituted, each of the C₆₋₁₀ aromatic ring, the 5- to 12-membered heteroaromatic ring and the 5- to 12-membered heterocycle is substituted by substituent(s) independently selected from hydrogen, halogen, hydroxyl, -CN, -C₁₋₆ alkyl, -C₁₋₆ haloalkyl, -OC₁₋₆ alkyl, -OC₁₋₆ haloalkyl, carboxyl, polyethylene glycol, amino acid, phosphoric acid, sulfonic acid, amino, azido and alkynyl;
Q is absent or is composed of one or more of the following substituted or unsubstituted groups: -NH-, -O-, -CH₂-, hydroxyl, carbonyl, amido, sulfonyl, sulfonylurea group, carbamoyl, oximido, -NH-S(=O)₂-NH-C(=O)O-, -C(=O)NH-, - NHC(=O)- or C₂₋₆ alkynylene, and when being substituted, each of the groups is substituted by substituent(s) independently selected from hydrogen, halogen, hydroxyl, -CN, -C₁₋₆ alkyl, -C₁₋₆ haloalkyl, -OC₁₋₆ alkyl, and -OC₁₋₆ haloalkyl;
Z₁ is absent or is selected from substituted or unsubstituted phenyl, substituted or unsubstituted 5- to 6-membered heteroaryl, amido, substituted or unsubstituted -CH₂- and substituted or unsubstituted C₂₋₆ alkynylene, and when being substituted, each of the phenyl, the 5- to 6-membered heteroaryl, the -CH₂- and the C₂₋₆ alkynylene is substituted by substituent(s) independently selected from hydrogen, halogen, hydroxyl, -CN, -C₁₋₆ alkyl, -C₁₋₆ haloalkyl, -OC₁₋₆ alkyl and -OC₁₋₆ haloalkyl;
W₁ is absent or is one or more groups selected from substituted or unsubstituted C₁₋₁₀ alkylene, -(CH₂CH₂O)ₚ- and -(OCH_{2C}H₂)ₚ-, wherein p is an integer from 1 to 20, and when being substituted, the C₁₋₁₀ alkylene is substituted by substituent(s) selected from hydrogen, halogen, hydroxyl, -CN, -C₁₋₆ alkyl, -C₁₋₆ haloalkyl, -OC₁₋₆ alkyl and - OC₁₋₆ haloalkyl; and
J₁ is selected from -COOH, -NH₂, substituted -NH₂, 3- to 10-membered nitrogen-containing heterocycle, substituted 3- to 10-membered nitrogen-containing heterocycle, alkynyl, 8- to 16-membered alkynyl group-containing cyclic group, substituted 8- to 16-membered alkynyl group-containing cyclic group, azido, tetrazinyl, hydroxylamine, aldehyde, keto, sulfonylurea group, isocyanate, thioisocyanate, maleimide and hydroxyl, and the term "substituted" means substitution by substituent(s) independently selected from hydrogen, halogen, hydroxyl, -CN, -C₁₋₆ alkyl, -C₁₋₆ haloalkyl, -OC₁₋₆ alkyl and -OC₁₋₆ haloalkyl.

2. The compound or a pharmaceutically acceptable salt, ester, stereoisomer, tautomer, polymorph, solvate, N-oxide or isotope labeled compound thereof according to claim 1, wherein
X is a leaving group, such as Cl, Br, I, OMs, OTs, OTf or 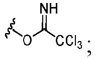
Y is absent or carbonyl;
Ring A is selected from substituted or unsubstituted C₆₋₁₀ aromatic ring, substituted or unsubstituted 5- to 12-membered heteroaromatic ring and substituted or unsubstituted 5- to 12-membered heterocycle, and when being substituted, each of the C₆₋₁₀ aromatic ring, the 5- to 12-membered heteroaromatic ring and the 5- to 12-membered heterocycle is substituted by substituent(s) independently selected from hydrogen, halogen, hydroxyl, -CN, -C₁₋₆ alkyl, -C₁₋₆ haloalkyl, -OC₁₋₆ alkyl, and -OC₁₋₆ haloalkyl;
Q is absent or is substituted or unsubstituted -C(=O)NH-, and when being substituted, the -C(=O)NH- is substituted by substituent(s) selected from hydrogen, halogen, hydroxyl, -CN, -C₁₋₆ alkyl, -C₁₋₆ haloalkyl, -OC₁₋₆ alkyl, and -OC₁₋₆ haloalkyl;
Z₁ is absent or is substituted or unsubstituted -CH₂- or substituted or unsubstituted C₂₋₆ alkynylene, and when being substituted, the -CH₂- or the C₂₋₆ alkynylene is substituted by substituent(s) independently selected from hydrogen, halogen, hydroxyl, -CN, -C₁₋₆ alkyl, -C₁₋₆ haloalkyl, -OC₁₋₆ alkyl and -OC₁₋₆ haloalkyl;
W₁ is absent or is one or more groups selected from substituted or unsubstituted C₁₋₁₀ alkylene, -(CH₂CH₂O)ₚ- and -(OCH_{2C}H₂)ₚ-, wherein p is an integer from 1 to 10, and when being substituted, the C₁₋₁₀ alkylene is substituted by substituent(s) selected from hydrogen, halogen, hydroxyl, -CN, -C₁₋₆ alkyl, -C₁₋₆ haloalkyl, -OC₁₋₆ alkyl and - OC₁₋₆ haloalkyl; and
J₁ is selected from -COOH, -NH₂, 3- to 10-membered nitrogen-containing heterocycle, sulfonylurea group and hydroxyl.

3. The compound of formula I according to claim 1 or 2, wherein the structure is selected from the group consisting of: 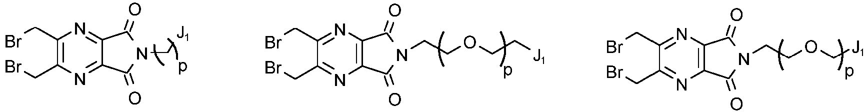 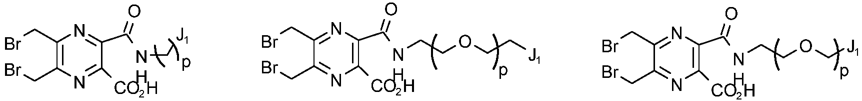 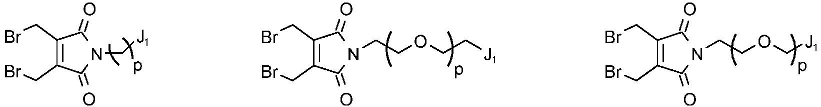 wherein p is an integer from 1 to 10 and J₁ is -COOH or -NH₂.

4. The compound of formula I according to any one of claims 1-3, wherein the structure is selected from the group consisting of:

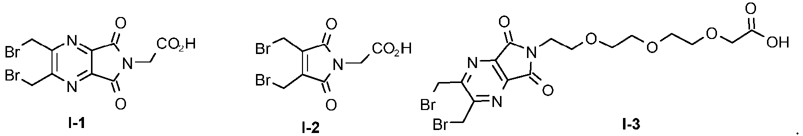

5. A compound of formula II or a pharmaceutically acceptable salt, ester, stereoisomer, tautomer, polymorph, solvate, N-oxide or isotope labeled compound thereof, wherein the compound of formula II has the following structure: 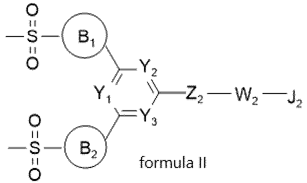
wherein each of B₁ and B₂ is independently a single bond or a substituted or unsubstituted 5- to 12-membered nitrogen-containing heteroaromatic ring, and when being substituted, the 5- to 12-membered nitrogen-containing heteroaromatic ring is substituted by substituent(s) composed of one or more of the following groups: hydrogen, halogen, hydroxyl,-CN, substituted or unsubstituted C₁₋₁₀ alkylene, -C₁₋₆ haloalkyl, -OC₁₋₆ alkyl, -OC₁₋₆ haloalkyl, carboxyl, substituted or unsubstituted amido, substituted or unsubstituted carbamoyl, substituted or unsubstituted polyethylene glycol, alkynyl and azido, and when being substituted, each of the C₁₋₁₀ alkylene, the amido, the carbamoyl and the polyethylene glycol is substituted by substituent(s) independently selected from hydrogen, halogen, hydroxyl, -CN, -C₁₋₆ alkyl, -C₁₋₆ haloalkyl, -OC₁₋₆ alkyl and -OC₁₋₆ haloalkyl;
Y₁, Y₂ and Y₃ are each independently selected from C(R) and N;
Z₂ is absent or is a group composed of one or more of the following substituted or unsubstituted functional groups: -NH-, -O-, -CH₂-, hydroxyl, carbonyl, amido, sulfonyl, sulfonylurea group, carbamoyl, oximido, -NH-S(=O)₂-NH-C(=O)O-, -C(=O)NH-, - NHC(=O)- or C₂₋₆ alkynylene, and when being substituted, each of the functional groups is substituted by substituent(s) independently selected from hydrogen, halogen, hydroxyl, -CN, -C₁₋₆ alkyl, -C₁₋₆ haloalkyl, -OC₁₋₆ alkyl, and -OC₁₋₆ haloalkyl;
W₂ is absent or is one or more groups selected from substituted or unsubstituted C₁₋₁₀ alkylene, -(CH₂CH₂O)ₚ- and -(OCH_{2C}H₂)ₚ-, and when being substituted, the C₁₋₁₀ alkylene is substituted by substituent(s) selected from hydrogen, halogen, hydroxyl, -CN, -C₁₋₆ alkyl, -C₁₋₆ haloalkyl, -OC₁₋₆ alkyl and -OC₁₋₆ haloalkyl; p is an integer from 1 to 10;
J₂ is selected from -COOH, -N(R)(R'), substituted or unsubstituted 3- to 10-membered nitrogen-containing heterocycle, sulfonylurea group, alkynyl, substituted or unsubstituted 8- to 16-membered alkynyl group-containing cyclic group, azido, tetrazinyl, substituted or unsubstituted hydroxylamine, aldehyde, keto, isocyanate, thioisocyanate, maleimide and hydroxyl, and when being substituted, each of the 3- to 10-membered nitrogen-containing heterocycle, the 8- to 16-membered alkynyl group-containing cyclic group and the hydroxylamine is substituted by substituent(s) independently selected from hydrogen, halogen, hydroxyl, -CN, -C₁₋₆ alkyl, -C₁₋₆ haloalkyl, -OC₁₋₆ alkyl and -OC₁₋₆ haloalkyl;
each of R and R' is independently selected from hydrogen, halogen, hydroxyl, - CN, -C₁₋₆ alkyl, -C₁₋₆ haloalkyl, -OC₁₋₆ alkyl, and -OC₁₋₆ haloalkyl.

6. The compound of formula II according to claim 5, wherein
each of B₁ and B₂ is independently a single bond or a substituted or unsubstituted 5- to 6-membered nitrogen-containing heteroaromatic ring, and when being substituted, the 5- to 6-membered nitrogen-containing heteroaromatic ring is substituted by substituent(s) selected from the group consisting of: hydrogen, halogen, hydroxyl, -CN, substituted or unsubstituted C₁₋₁₀ alkylene, -C₁₋₆ haloalkyl, -OC₁₋₆ alkyl, -OC₁₋₆ haloalkyl, carboxyl, substituted or unsubstituted amido, substituted or unsubstituted carbamoyl, substituted or unsubstituted polyethylene glycol, alkynyl and azido, and when being substituted, each of the C₁₋₁₀ alkylene, the amido, the carbamoyl and the polyethylene glycol is substituted by substituent(s) independently selected from hydrogen, halogen, hydroxyl, -CN, -C₁₋₆ alkyl, -C₁₋₆ haloalkyl, -OC₁₋₆ alkyl and -OC₁₋₆ haloalkyl; preferably, each of B₁ and B₂ is independently a single bond or a substituted or unsubstituted pyrimidine ring, and when being substituted, the pyrimidine ring is substituted by substituent(s) selected from hydrogen, halogen, hydroxyl, -CN, -C₁₋₆ alkyl, -C₁₋₆ haloalkyl, -OC₁₋₆ alkyl and -OC₁₋₆ haloalkyl;
Y₁, Y₂ and Y₃ are each independently selected from CH and N;
Z₂ is absent or is a group composed of one or more of the following substituted or unsubstituted functional groups: -NH-, -CH₂-, carbonyl, -C(=O)NH-, -NHC(=O)- or C₂₋₆ alkynylene, and when being substituted, each of the functional groups is substituted by substituent(s) independently selected from hydrogen, halogen, hydroxyl, -CN, -C₁₋₆ alkyl, -C₁₋₆ haloalkyl, -OC₁₋₆ alkyl, and -OC₁₋₆ haloalkyl;
W₂ is absent or is one or more groups selected from substituted or unsubstituted C₁₋₁₀ alkylene, -(CH₂CH₂O)ₚ- and -(OCH_{2C}H₂)ₚ-, and when being substituted, the C₁₋₁₀ alkylene is substituted by substituent(s) selected from hydrogen, halogen, hydroxyl, -CN, -C₁₋₆ alkyl, -C₁₋₆ haloalkyl, -OC₁₋₆ alkyl and -OC₁₋₆ haloalkyl;
J₂ is selected from -COOH, -NH₂, 3- to 10-membered nitrogen-containing heterocycle, sulfonylurea group and hydroxyl; and
p is an integer from 1 to 10.

7. The compound of formula II according to claim 5 or 6, wherein
each of B₁ and B₂ is independently a substituted or unsubstituted 5- to 6-membered nitrogen-containing heteroaromatic ring; when being substituted, the 5- to 6-membered nitrogen-containing heteroaromatic ring is substituted by substituent(s) composed of one or more of the following groups: hydrogen, C₁₋₁₀ alkylene, carboxyl, amido, carbamoyl, polyethylene glycol, alkynyl, and azido;
Y₁, Y₂ and Y₃ are each independently selected from CH and N;
Z₂ is absent or is a group composed of one or more of the following functional groups: -NH-, -CH₂-, carbonyl and C₂₋₆ alkynylene;
W₂ is absent or is one or more groups selected from C₁₋₁₀ alkylene, -(CH₂CH₂O)ₚ- and -(OCH₂CH₂)ₚ-;
J₂ is selected from -COOH, -NH₂, 3- to 10-membered nitrogen-containing heterocycle, sulfonylurea group and hydroxyl; and
p is an integer from 1 to 10.

8. The compound of formula II according to any one of claims 5-7, wherein each of B₁ and B₂ is independently selected from substituted or unsubstituted pyridinyl and pyrimidyl; when being substituted, each of the pyridinyl and the pyrimidyl is substituted by substituent(s) composed of one or more of the following groups: hydrogen, C₁₋₁₀ alkylene, carboxyl, amido, carbamoyl, polyethylene glycol, alkynyl, and azido;
Y₁, Y₂ and Y₃ are each independently selected from CH and N;
Z₂ is absent or is a group composed of one or two of the following functional groups: -NH- and carbonyl;
W₂ is absent or is one or more groups selected from C₁₋₁₀ alkylene, -(CH₂CH₂O)ₚ- and -(OCH₂CH₂)ₚ-;
J₂ is -COOH or -NH₂; and
p is an integer from 1 to 10.

9. The compound or a pharmaceutically acceptable salt, ester, stereoisomer, tautomer, polymorph, solvate, N-oxide or isotope labeled compound thereof according to claim 5 or 6, wherein
each of B₁ and B₂ is independently a substituted or unsubstituted 5- to 12-membered nitrogen-containing heteroaromatic ring, and when being substituted, the 5-to 12-membered nitrogen-containing heteroaromatic ring is substituted by substituent(s) composed of one or more of the following groups: hydrogen, C₁₋₁₀ alkylene, carboxyl, amido, carbamoyl, polyethylene glycol, alkynyl, and azido;
and/or
Z₂ is absent or is a group composed of one or more of the following functional groups: -NH-, -CH₂- and carbonyl;
and/or
J₂ is -COOH or -NH₂;
and/or
p is an integer from 3 to 8.

10. The compound of formula II according to any one of claims 5-9, wherein
each of B₁ and B₂ is independently selected from substituted or unsubstituted pyridinyl and pyrimidyl, and when being substituted, the pyridinyl or the pyrimidyl is substituted by substituent(s) composed of one or more of the following groups: hydrogen, C₁₋₁₀ alkylene, carboxyl, amido, carbamoyl, polyethylene glycol, alkynyl, and azido.

11. The compound of formula II according to any one of claims 5-10, wherein
each of B₁ and B₂ is independently pyridinyl and pyrimidyl.

12. The compound of formula II according to any one of claims 5-11, wherein
Z₂ is absent or is a group composed of one or more of the following substituted or unsubstituted functional groups: -NH-, -CH₂-, carbonyl or C₂₋₆ alkynylene, and when being substituted, each of the functional groups is substituted by substituent(s) selected from hydrogen, halogen, hydroxyl, -CN, -C₁₋₆ alkyl, -C₁₋₆ haloalkyl, -OC₁₋₆ alkyl, or - OC₁₋₆ haloalkyl.

13. The compound of formula II according to any one of claims 5-12, wherein
Z₂ is absent or is a group composed of one or two of the following functional groups: -NH- and carbonyl.

14. The compound of formula II according to any one of claims 5-13, wherein
Z₂ is absent or is -C(=O)NH-.

15. The compound of formula II according to any one of claims 5-14, wherein
J₂ is -COOH.

16. The compound of formula II according to any one of claims 5-15, which has the following structure: 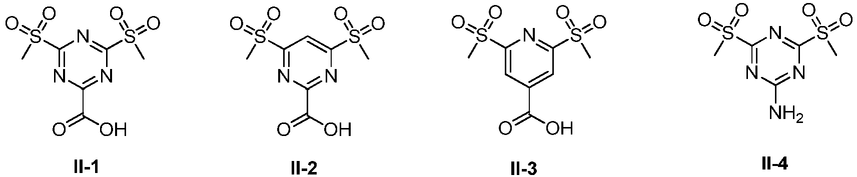 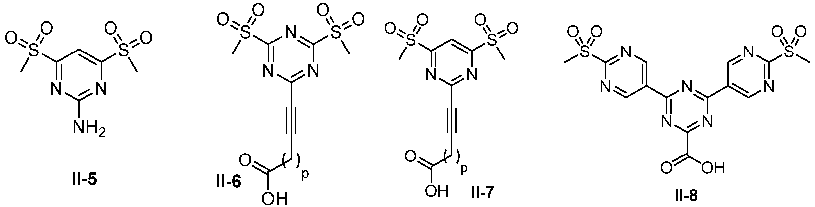 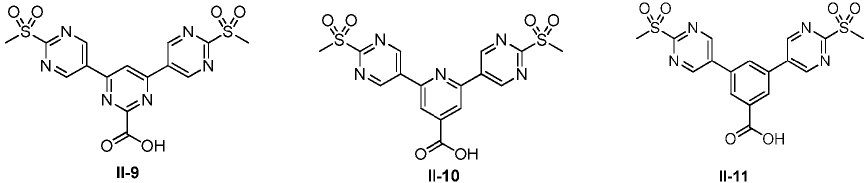 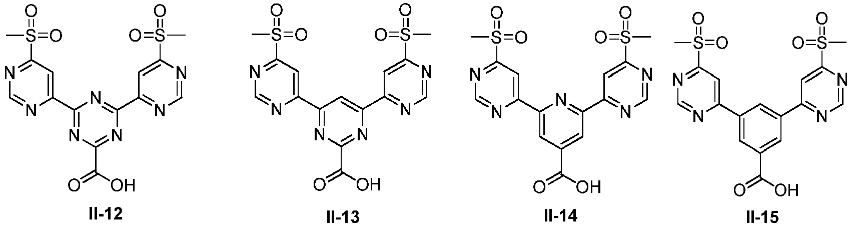 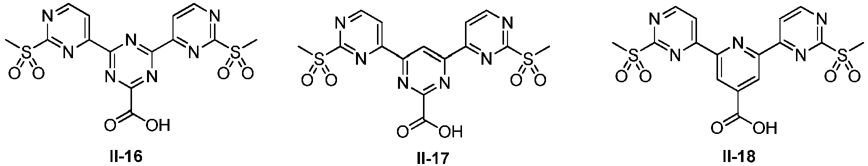 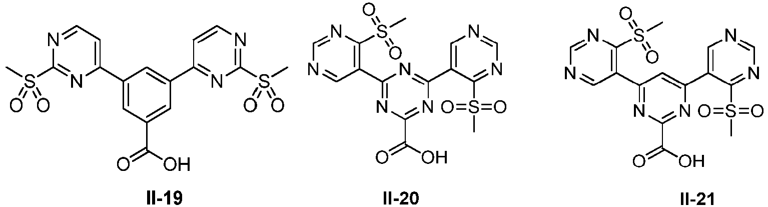 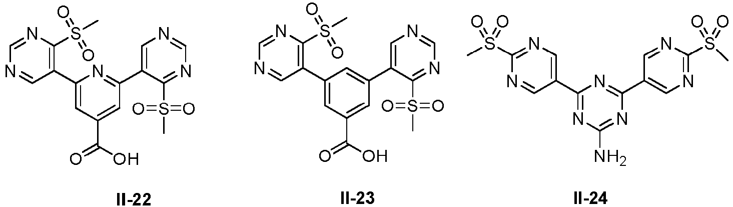 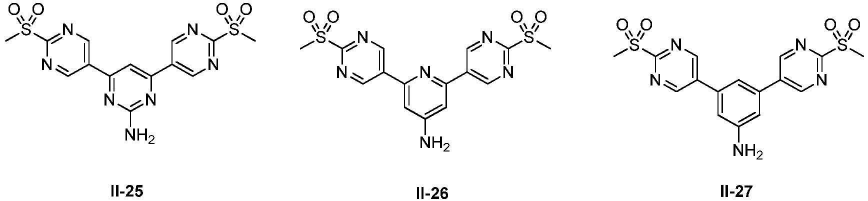 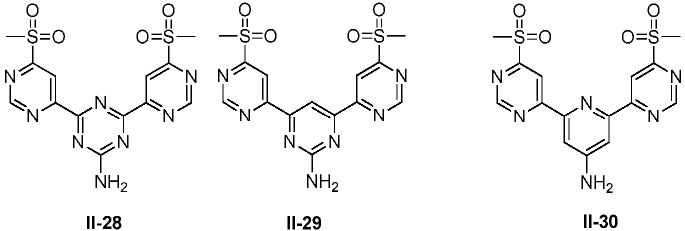 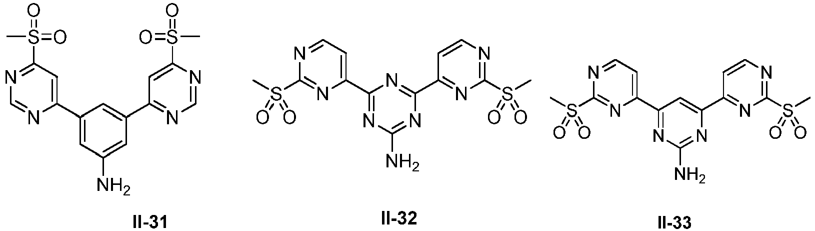 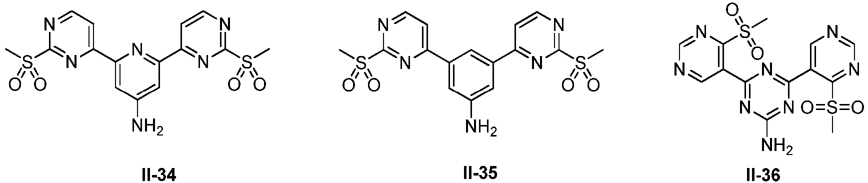 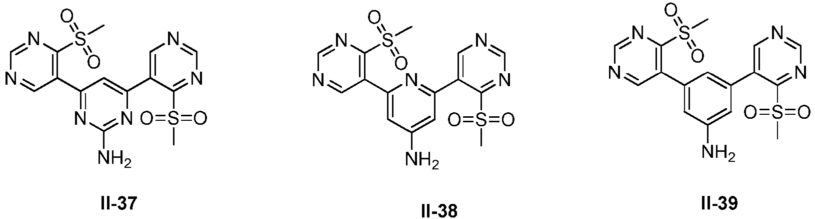 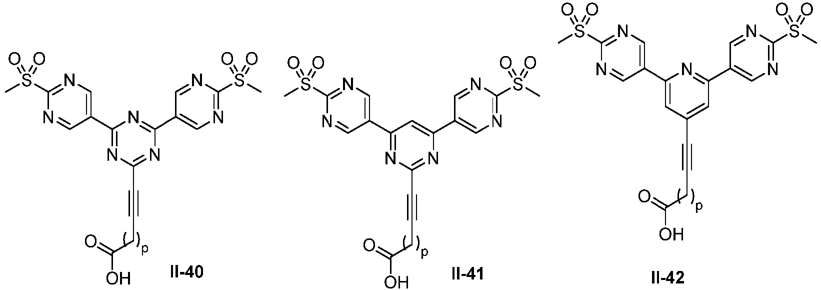 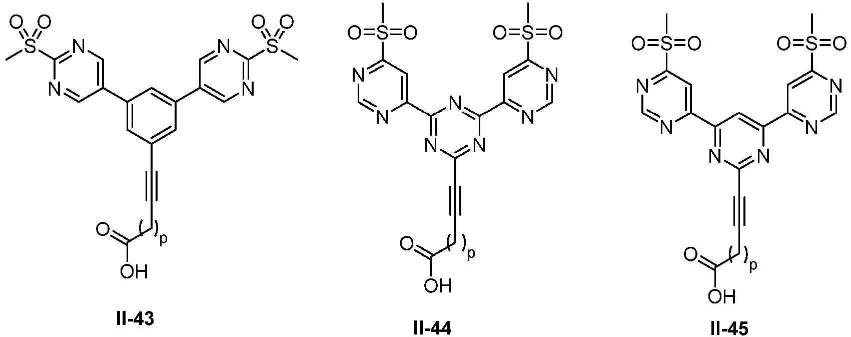 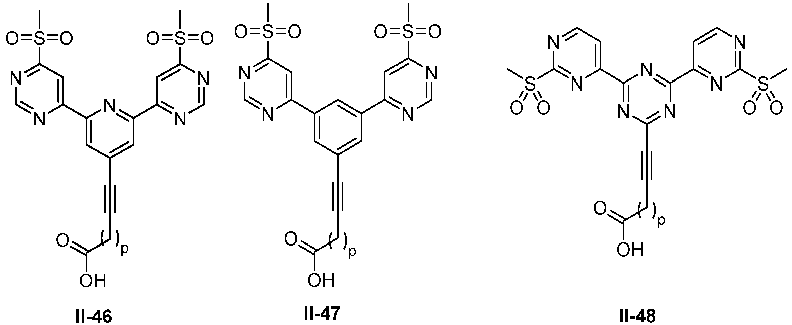 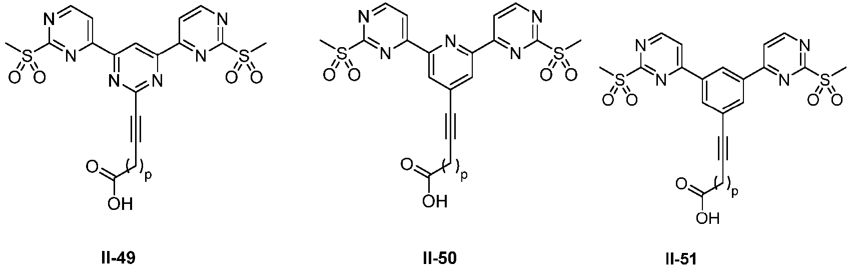 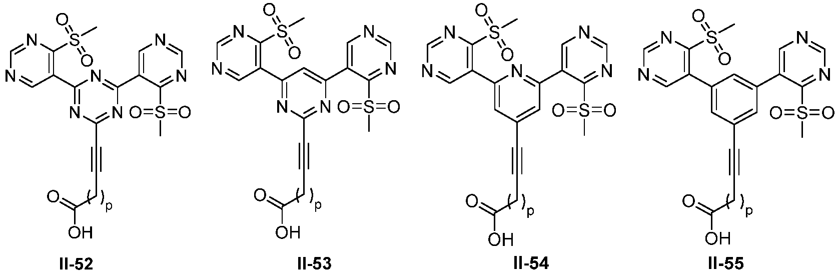 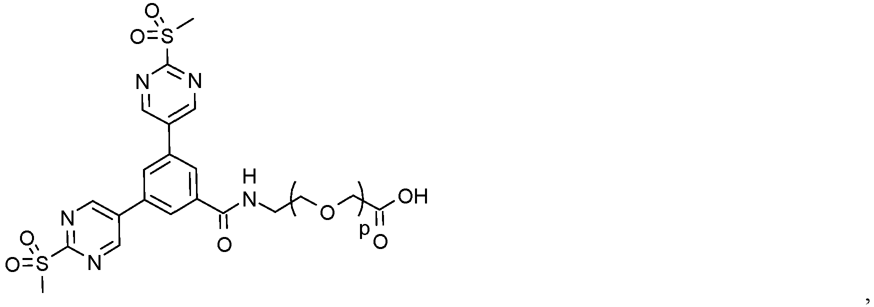 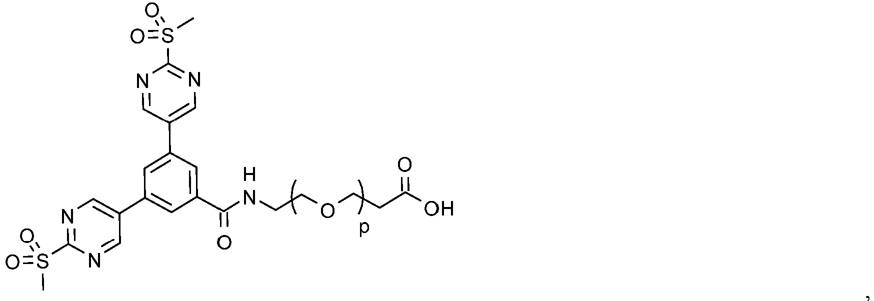 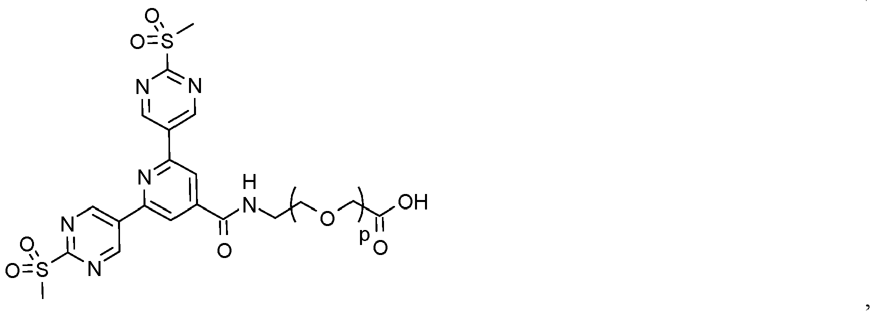 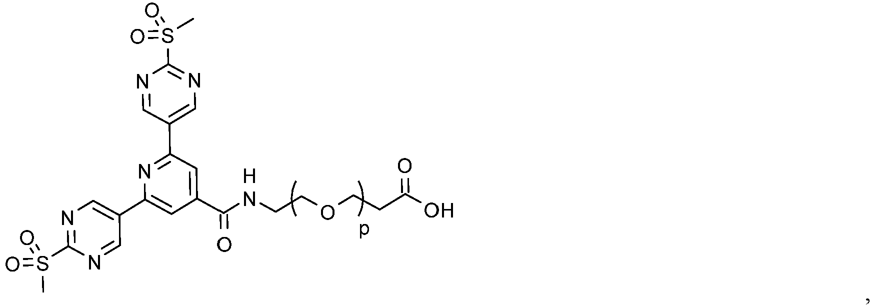 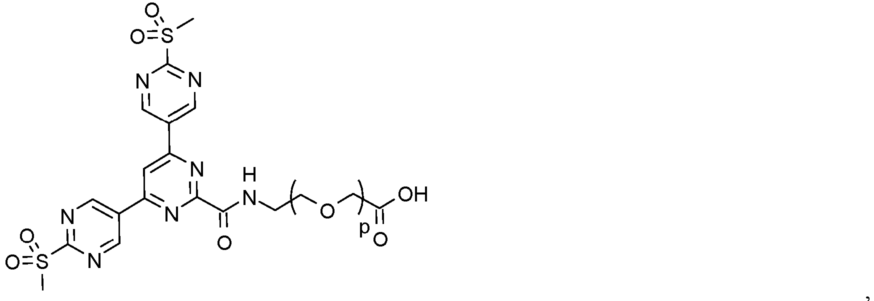 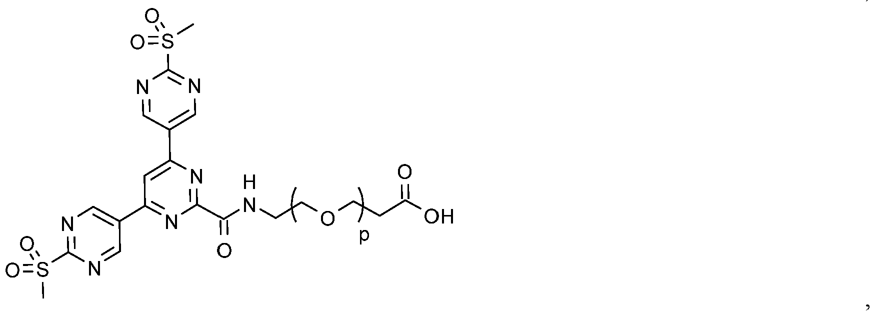 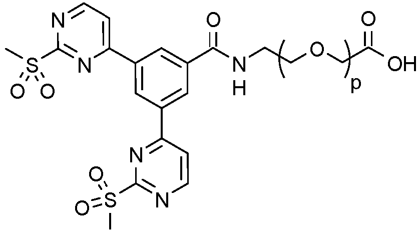 or 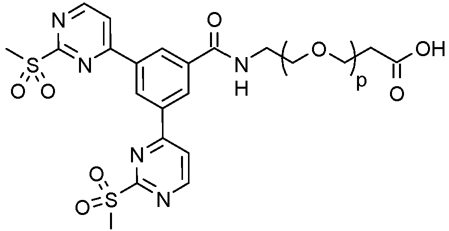 p is an integer from 1 to 10.

17. The compound of formula II according to any one of claims 5-16, wherein the compound has the following structure: 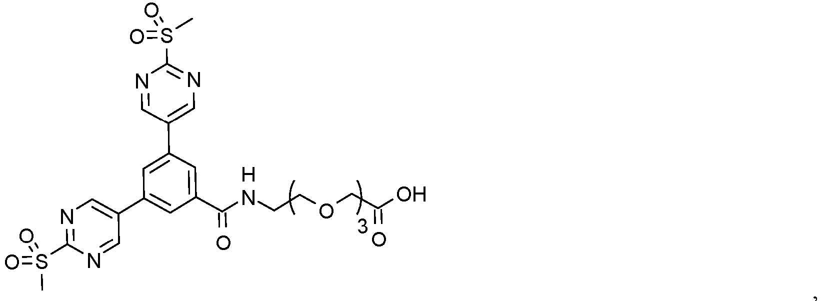 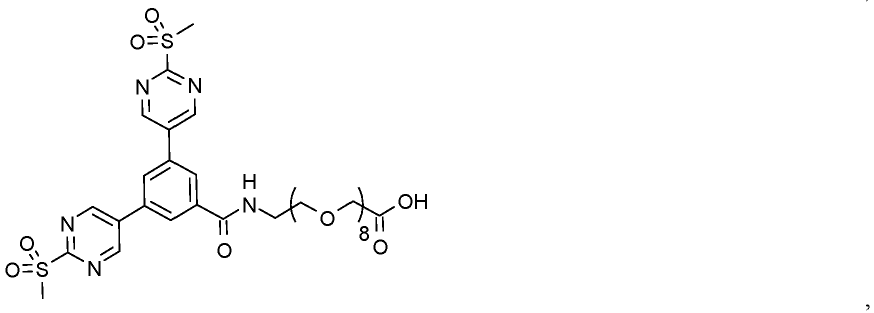 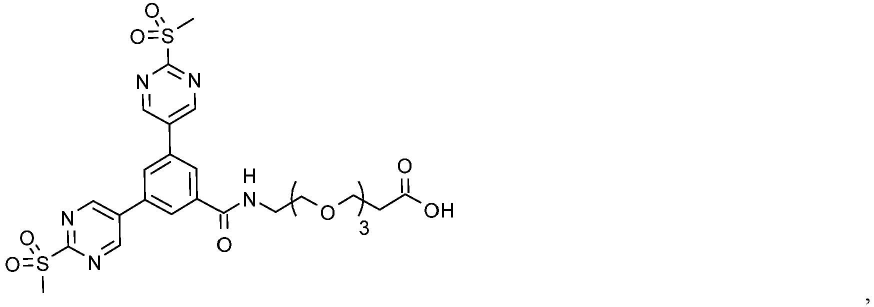 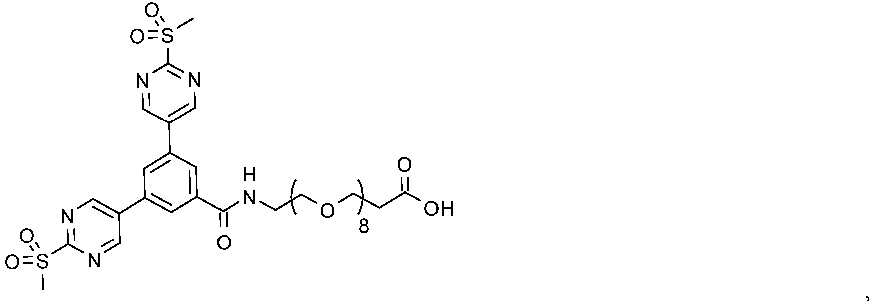 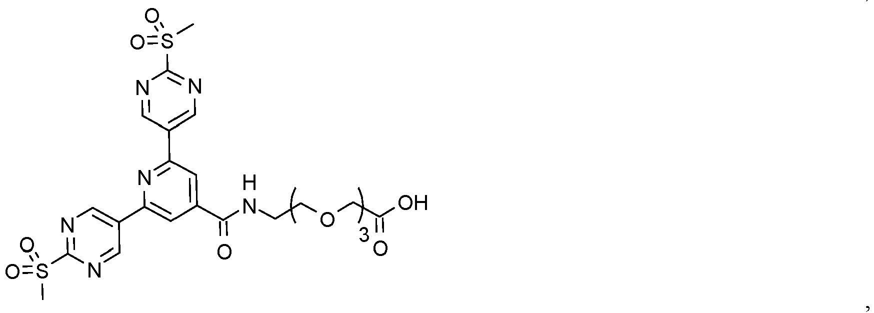 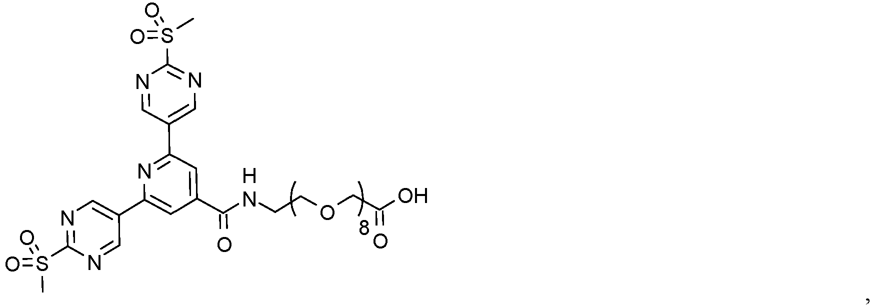 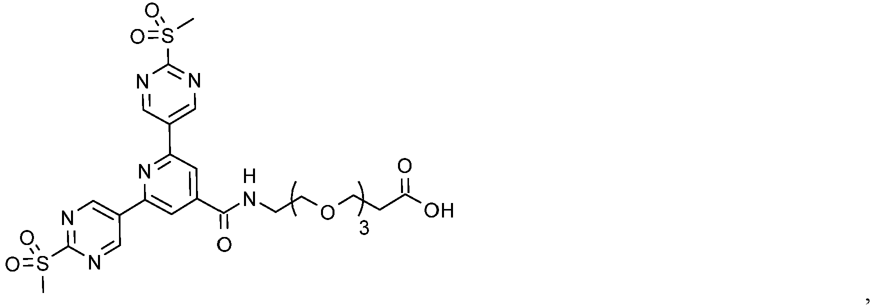 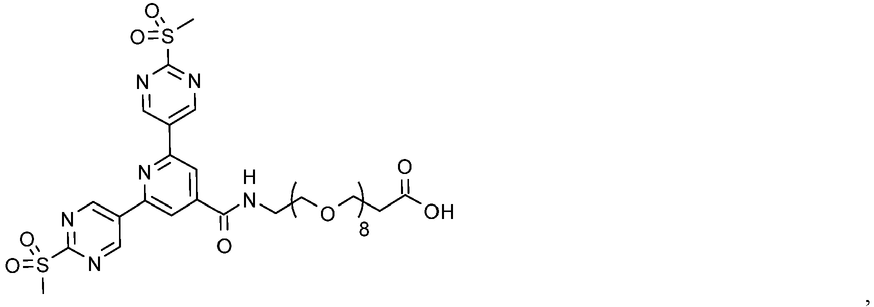 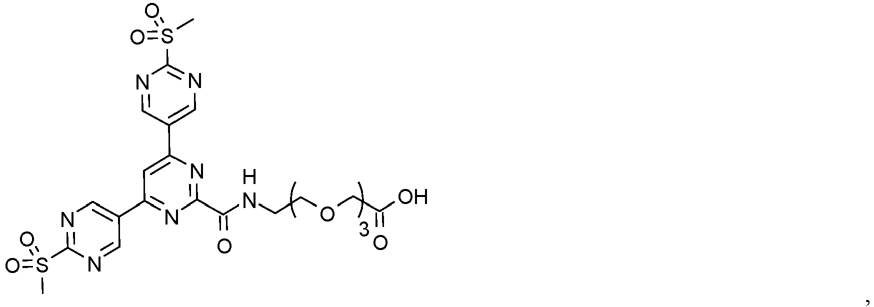 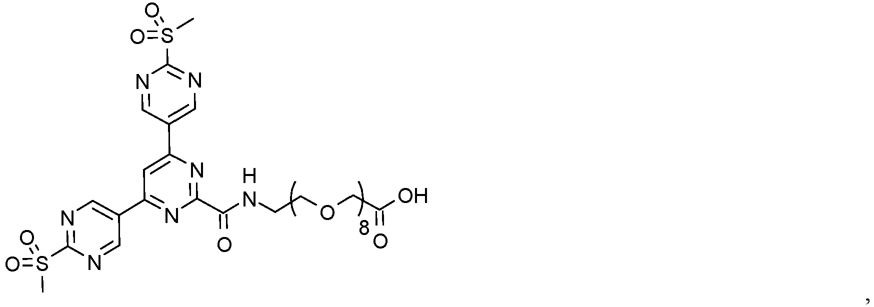 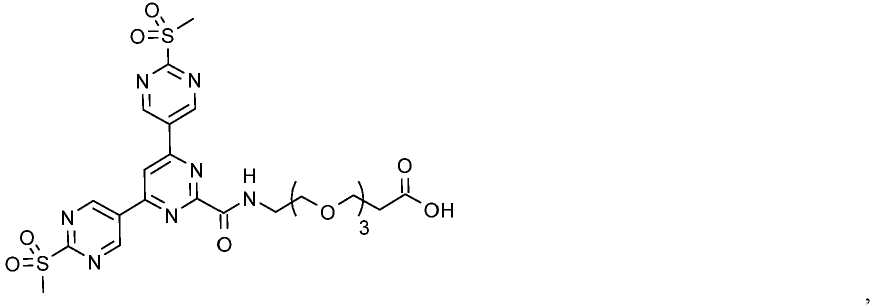 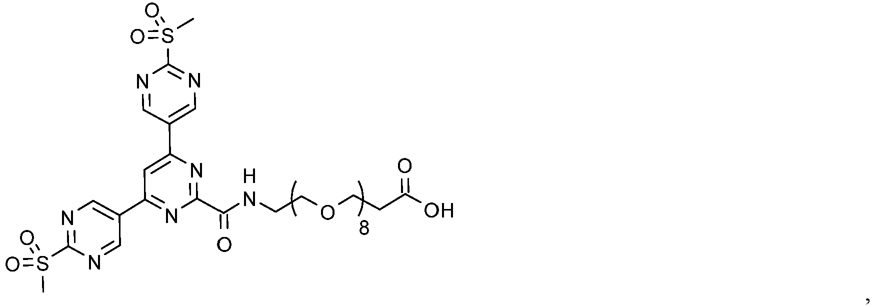 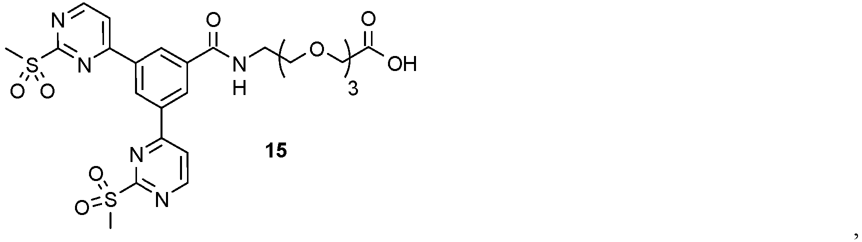 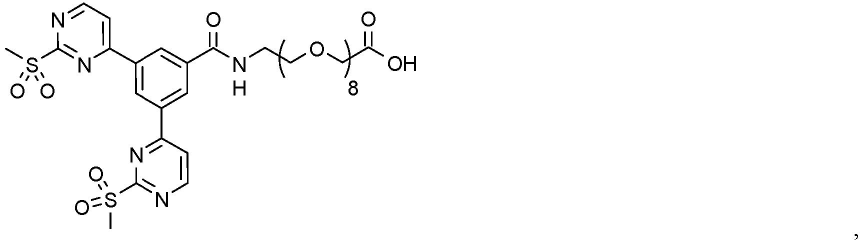 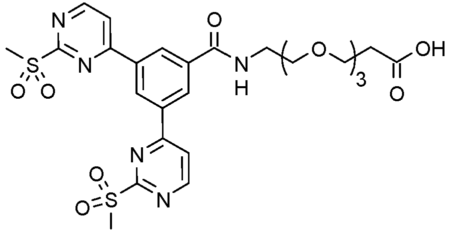 or

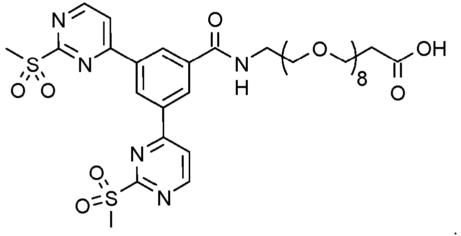

18. The compound of formula II according to any one of claims 5-17, wherein the compound has the following structure:

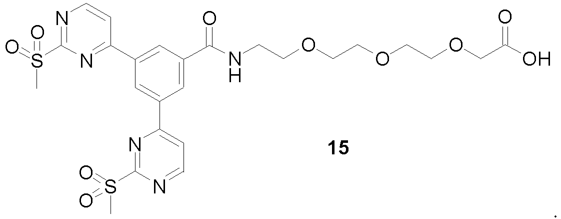

19. A compound or a pharmaceutically acceptable salt, ester, stereoisomer, tautomer, polymorph, solvate, N-oxide or isotope labeled compound thereof, wherein the compound is selected from the group consisting of:
(1) 3,5-bis(2-(methylthio)pyrimidin-4-yl)benzoic acid;
(2) 3,5-bis(2-(methylsulfonyl)pyrimidin-4-yl)benzoic acid;
(3) tert-butyl 1-(3,5-bis(2-(methylsulfonyl)pyrimidin-4-yl)phenyl)-1-oxo-5,8,11-trioxa-2-azatridecan-13-oleate;
(4) 3,5-bis(2-(methylthio)pyrimidin-5-yl)benzoic acid;
(5) 3,5-bis(2-(methylsulfonyl)pyrimidin-5-yl)benzoic acid;
(6) 1-(3,5-bis(2-(methylsulfonyl)pyrimidin-5-yl)phenyl)-1-oxo-5,8,11-trioxa-2-azatridecan-13-oic acid;
(7) 1-(3,5-bis(2-(methylthio)pyrimidin-5-yl)phenyl)-1-oxo-5,8,11-trioxa-2-azatetradecan-14-oic acid;
(8) 1-(3,5-bis(2-(methylsulfonyl)pyrimidin-5-yl)phenyl)-1-oxo-5,8,11-trioxa-2-azatetradecan-14-oic acid;
(9) 1-(3,5-bis(2-(methylthio)pyrimidin-5-yl)phenyl)-1-oxo-5,8,11,14,17,20,23,26-octaoxa-2-azanonacosan-29-oic acid;
(10) 1-(3,5-bis(2-(methylsulfonyl)pyrimidin-5-yl)phenyl)-1-oxo-5,8,11,14,17,20,23,26-octaoxa-2-azanonacosan-29-oic acid;
(11) 2,6-bis(2-(methylthio)pyrimidin-5-yl)isonicotinic acid;
(12) 1-(2,6-bis(2-(methylthio)pyrimidin-5-yl)pyridin-4-yl)-1-oxo-5,8,11-trioxa-2-azatetradecan-14-oic acid;
(13) 1-(2,6-bis(2-(methylsulfonyl)pyrimidin-5-yl)pyridin-4-yl)-1-oxo-5,8,11-trioxa-2-azatetradecan-14-oic acid;
(14) 1-(2,6-bis(2-(methylthio)pyrimidin-5-yl)pyridin-4-yl)-1-oxo-5,8,11,14,17,20,23,26-octaoxa-2-azanonacosan-29-oic acid;
(15) 1-(2,6-bis(2-(methylsulfonyl)pyrimidin-5-yl)pyridin-4-yl)-1-oxo-5,8,11,14,17,20,23,26-octaoxa-2-azanonacosan-29-oic acid;
(16) 2,2"-bis(methylthio)-[5,4':6',5"-tripyrimidine]-2'-carboxylic acid;
(17) 2,2"-bis(methylsulfonyl)-[5,4':6',5"-tripyrimidine]-2'-carboxylic acid;
(18) 1-(2,2"-bis(methylthio)-[5,4':6',5"-tripyrimidin]-2'-yl)-1-oxo-5,8,11-trioxa-2-azatetradecan-14-oic acid; or
(19) 1-(2,2"-bis(methylsulfonyl)-[5,4':6',5"-tripyrimidin]-2'-yl)-1-oxo-5,8,11-trioxa-2-azatetradecan-14-oic acid.

20. A compound or a pharmaceutically acceptable salt, ester, stereoisomer, tautomer, polymorph, solvate, N-oxide or isotope labeled compound thereof, wherein the compound has the structure of formula V: 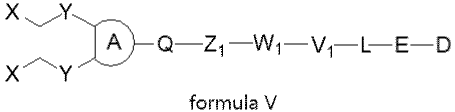 wherein
E is selected from a single bond, -NH-CH₂-, 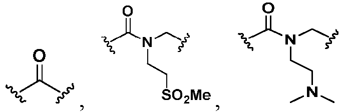 and 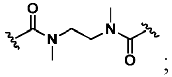
D is a fragment of a bioactive molecule (e.g., a cytotoxic drug);
X, Y, A, Q, Z₁ and W₁ are as defined in any one of claims 1-4;
V₁ is a group formed when J₁ in the compound of formula I according to any one of claims 1-4 is linked to L; preferably, V₁ is selected from -C(=O)-, -N(R₁)-, -O-, 3- to 10-membered nitrogen-containing heterocycle and sulfonylurea group, wherein R₁ is H, C₁₋₆ alkyl or C₂₋₆ alkoxyalkyl; further preferably, V₁ is -C(=O)- or -N(R₁)-, wherein R₁ is H, C₁₋₆ alkyl or C₂₋₆ alkoxyalkyl;
L is a linker that links V₁ and E.

21. The compound of formula V according to claim 20, wherein the compound has the following structure: 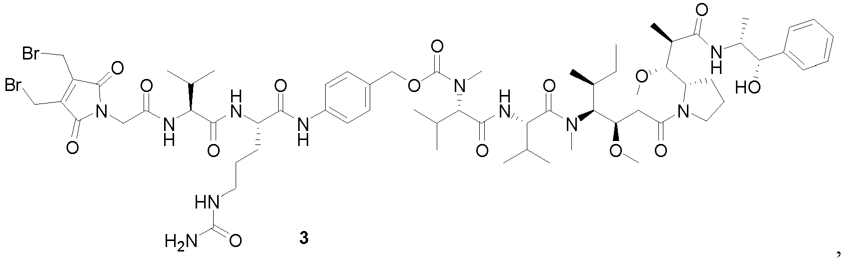 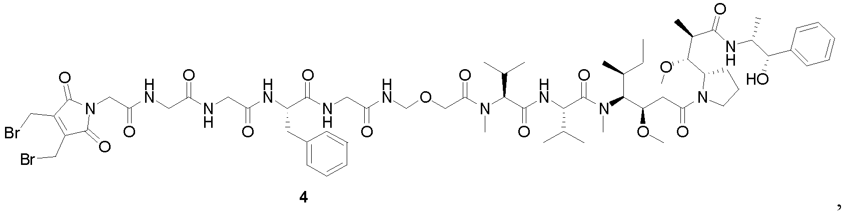 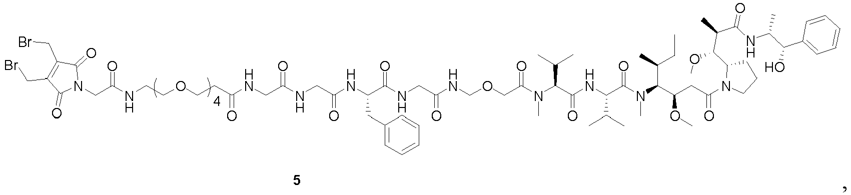 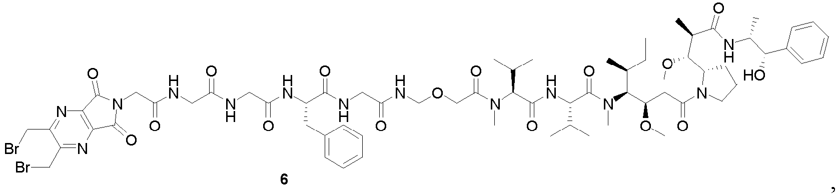 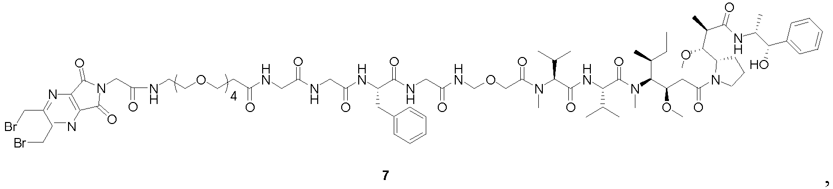 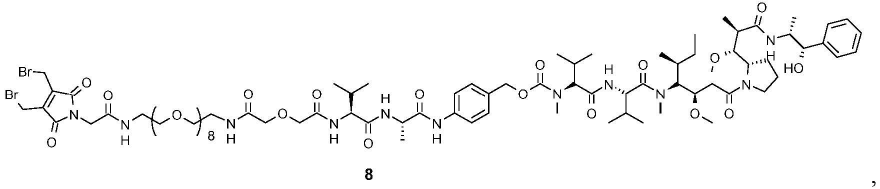 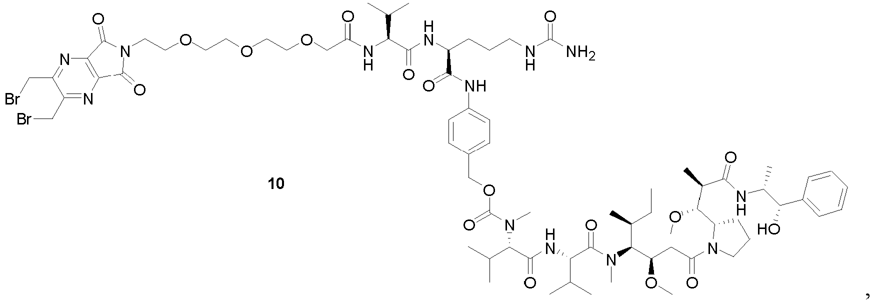 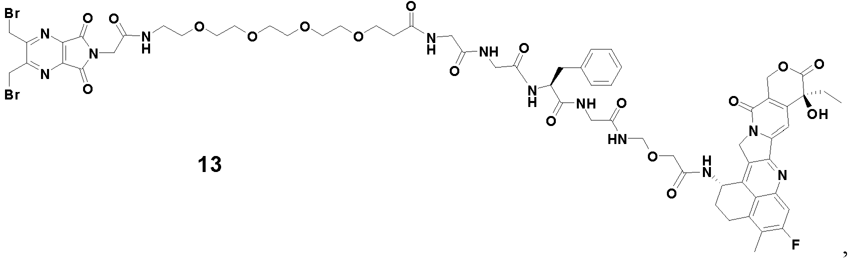 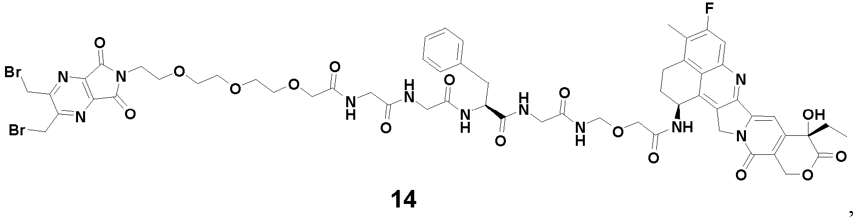 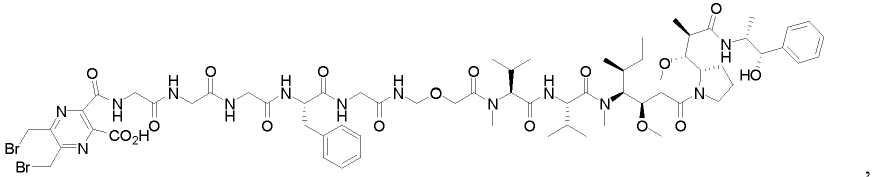 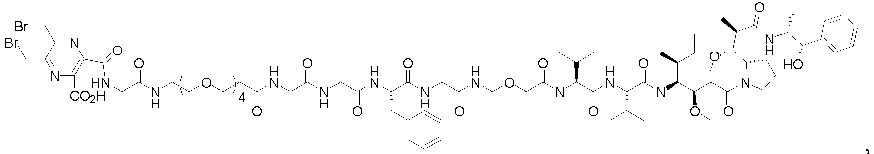 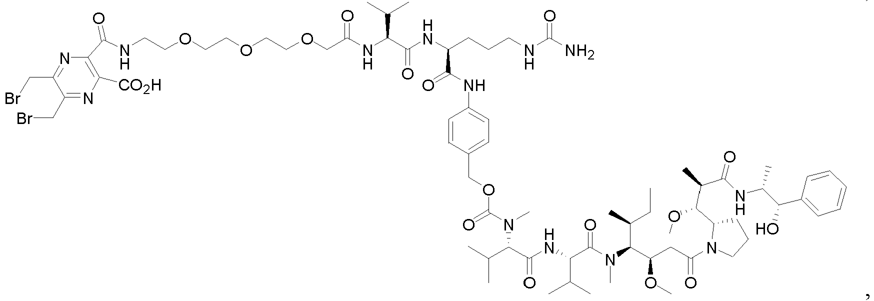 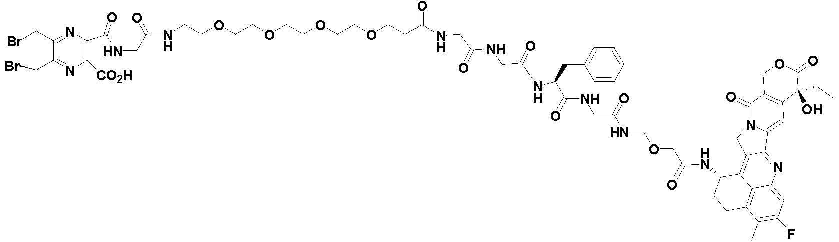 or

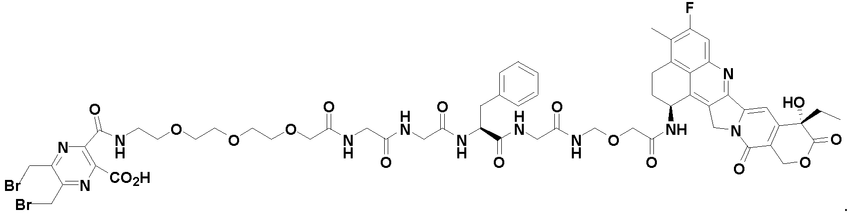

22. A compound or a pharmaceutically acceptable salt, ester, stereoisomer, tautomer, polymorph, solvate, N-oxide or isotope labeled compound thereof, wherein the compound has a structure of formula VI: 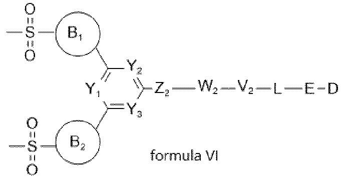
B₁, B₂, Y₁, Y₂, Y₃, Z₂ and W₂ are as defined according to any one of claims 5-18;
L is a linker that links V₂ and E;
V₂ is a group formed when J₂ in the compound of formula II according to any one of claims 5-18 is linked to L; preferably, V₂ is selected from -C(=O)-, -N(R₂)-, -O-, 3-to 10-membered nitrogen-containing heterocycle and sulfonylurea group, wherein R₂ is H, C₁₋₆ alkyl or C₂₋₆ alkoxyalkyl; further preferably, V₂ is -C(=O)- or -N(R₂)-, wherein R₂ is H, C₁₋₆ alkyl or C₂₋₆ alkoxyalkyl;
E is selected from a single bond, -NH-CH₂-, 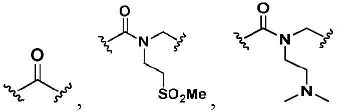 and 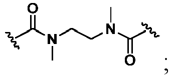
D is a fragment of a bioactive molecule (e.g., a cytotoxic drug).

23. The compound according to claim 22, wherein
B₁ and B₂ are pyrimidyl;
Y₁ is CH or C, Y₂ and Y₃ are both CH;
Z₂ is -C(=O)NH-;
W₂ is -(CH₂CH₂O)ₚ-C₁₋₁₀ alkylene- or -(OCH₂CH₂)ₚ-C₁₋₁₀ alkylene-, p is an integer from 1 to 10;
V₂ is -C(=O)-;
L is 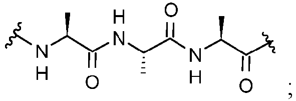 E is -NH-CH₂-;
D is selected from 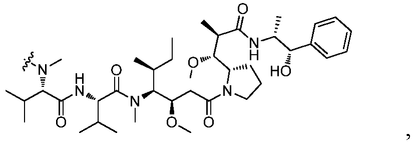 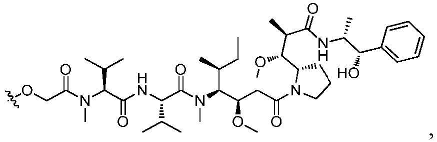 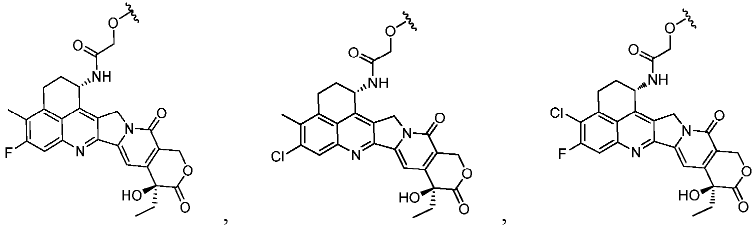 and

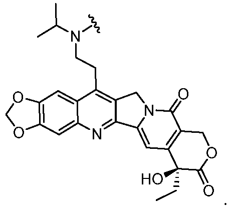

24. The compound according to claim 23, wherein W₂ is selected from - (CH₂CH₂O)₃-CH₂-, -(CH₂CH₂O)₃-(CH₂)₂- and -(OCH₂CH₂)₈-(CH₂)₂-.

25. The compound according to any one of claims 22-24, wherein D is

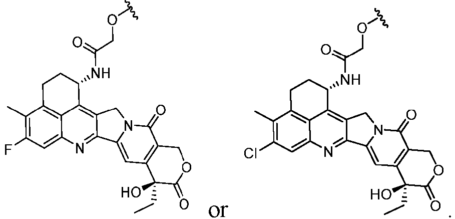

26. The compound according to any one of claims 22-25, wherein the structure of the compound is selected from: 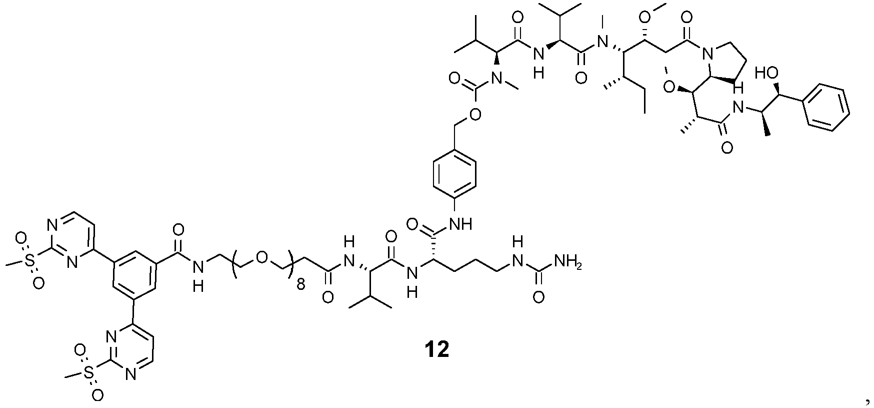 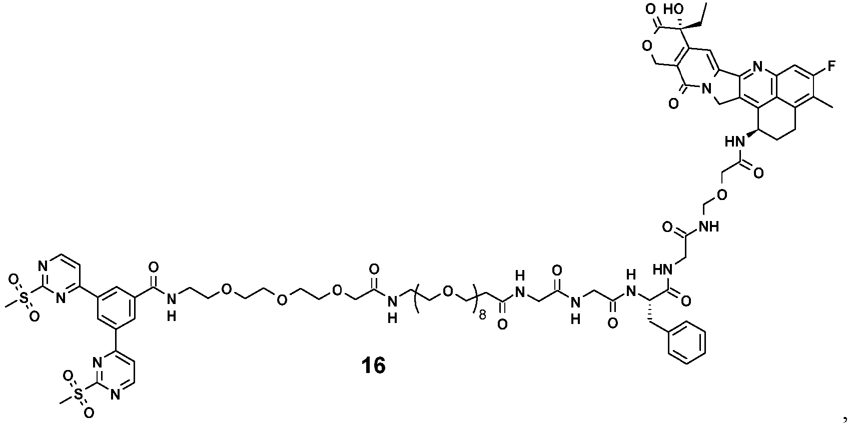 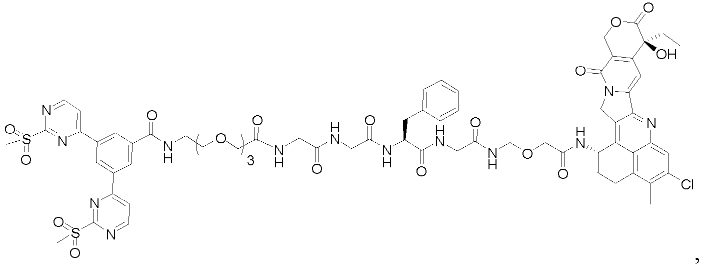 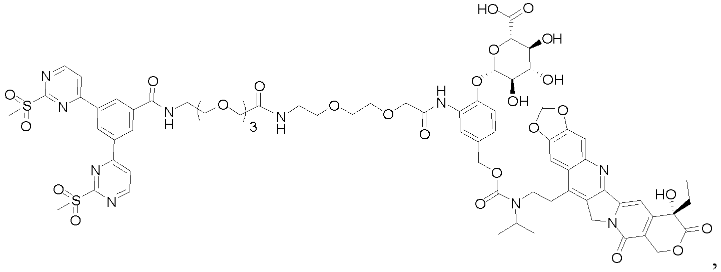 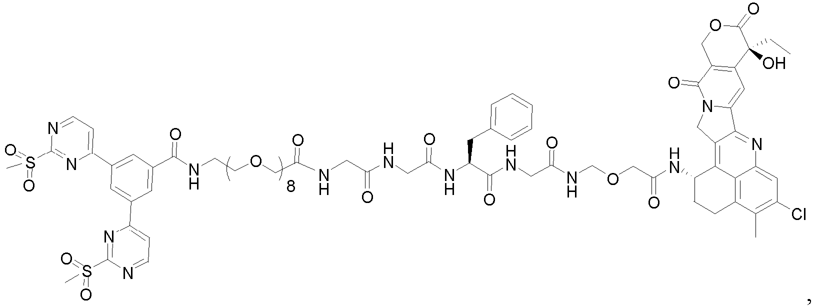 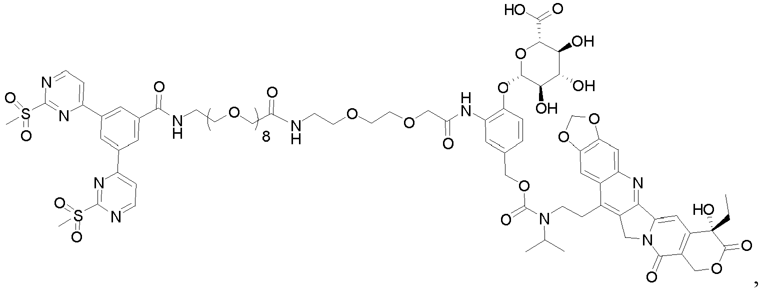 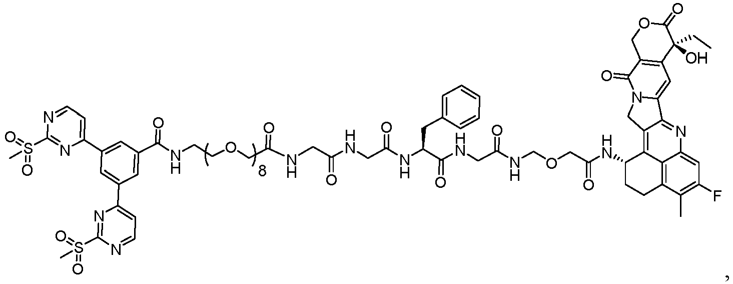 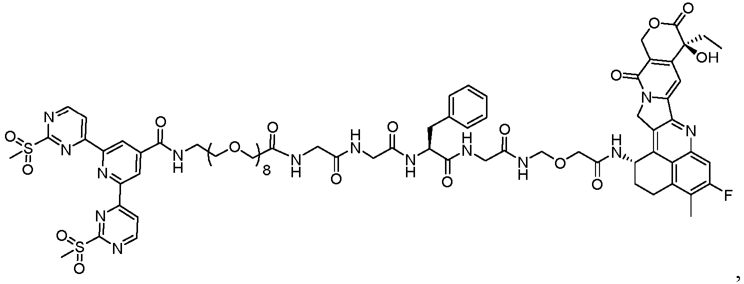 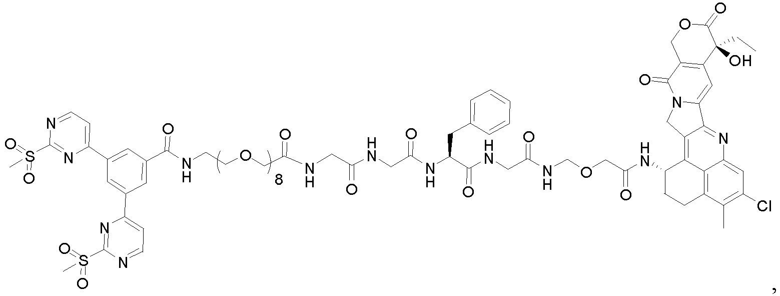 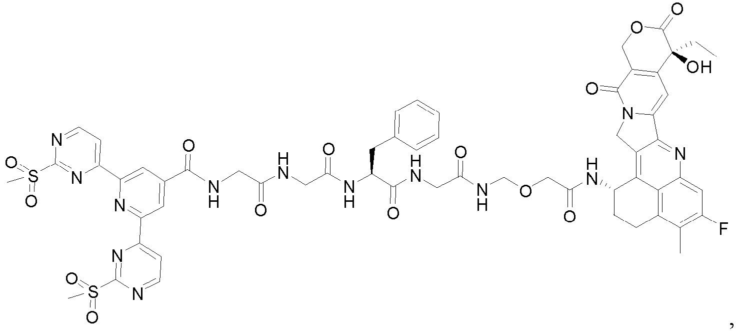 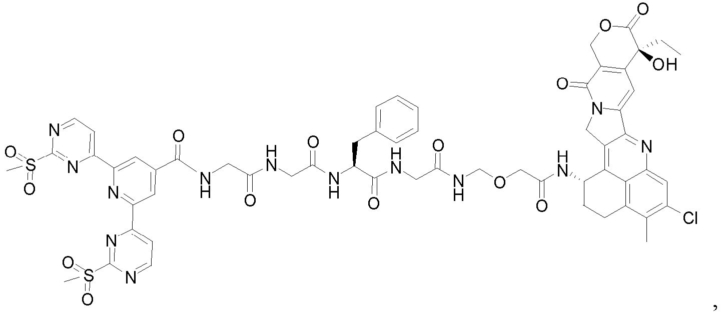 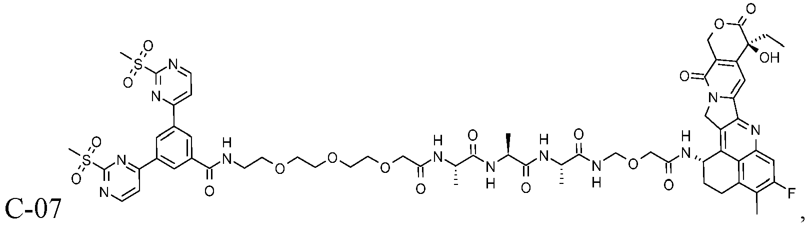 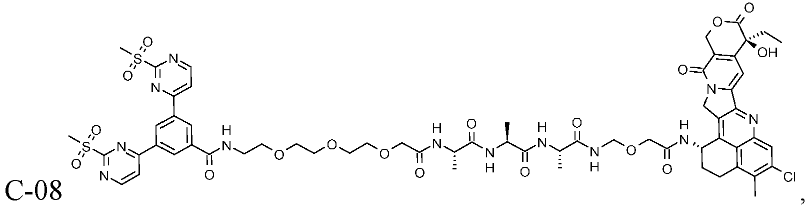 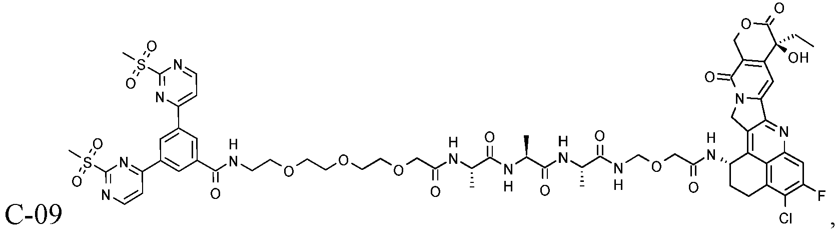 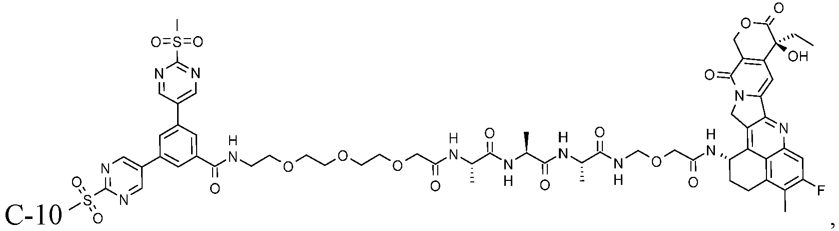 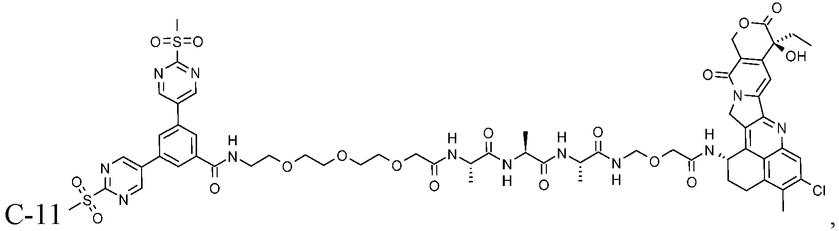 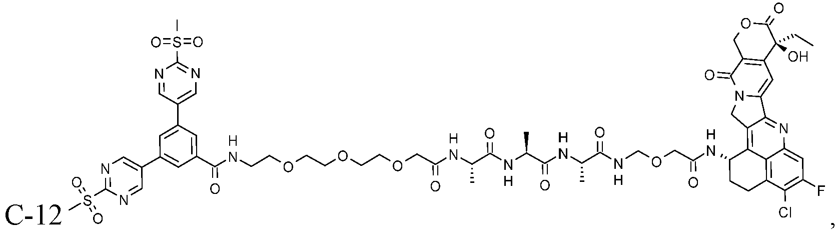 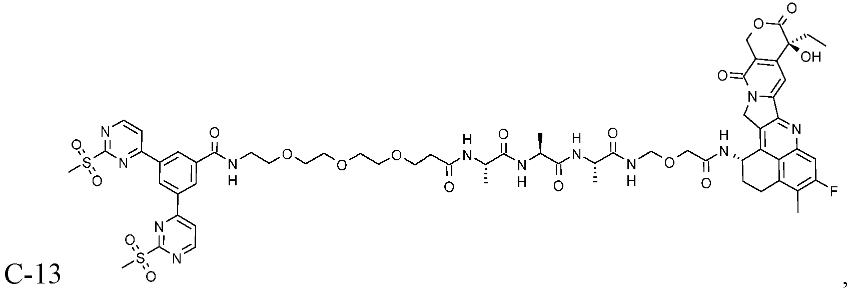 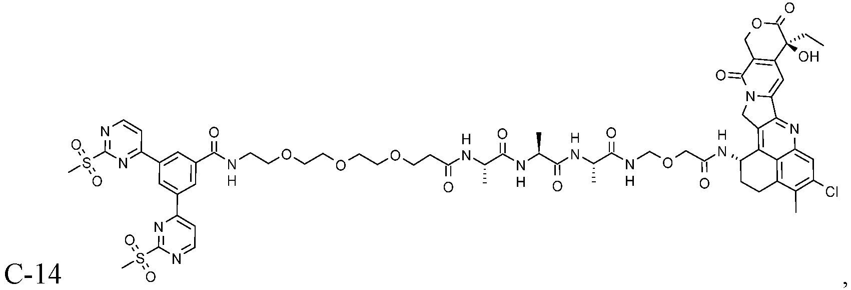 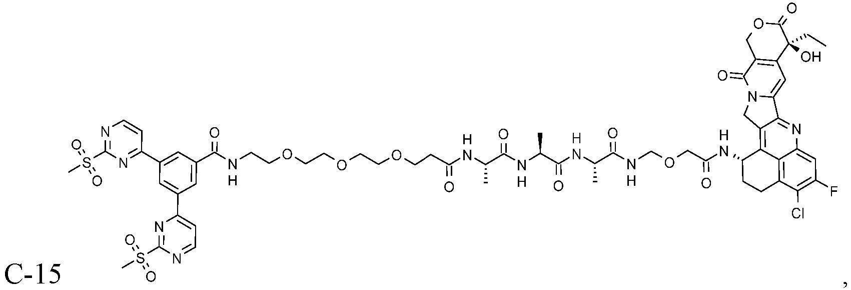 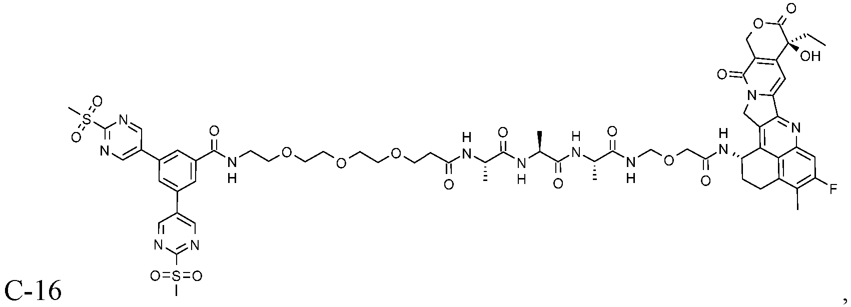 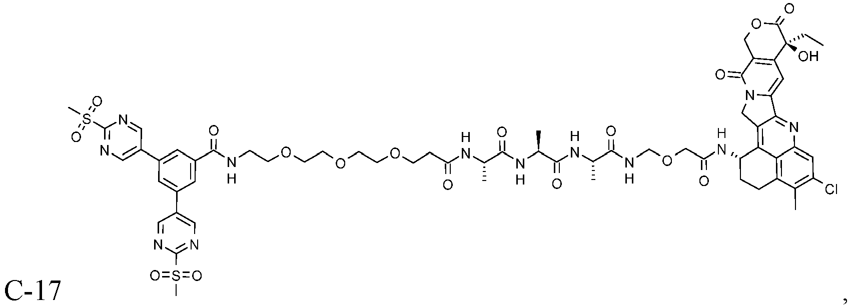 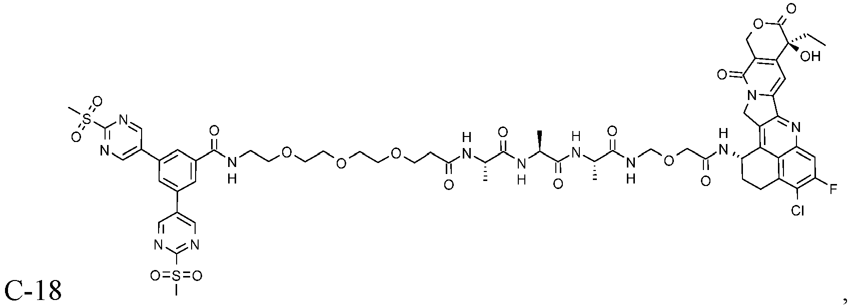 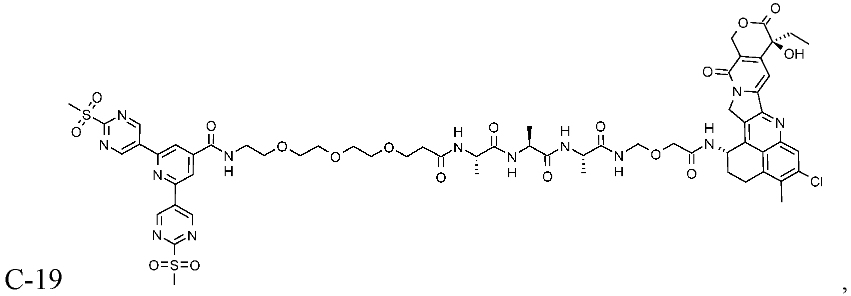 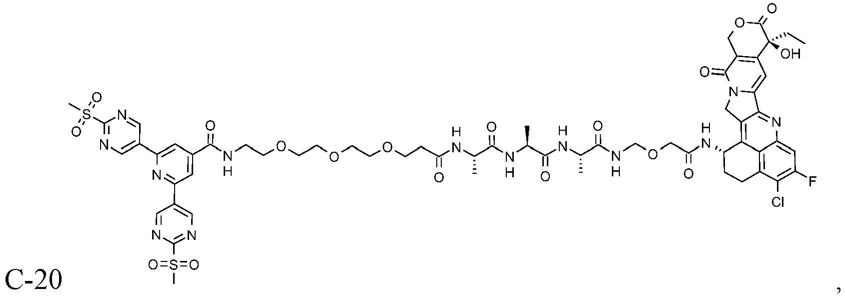 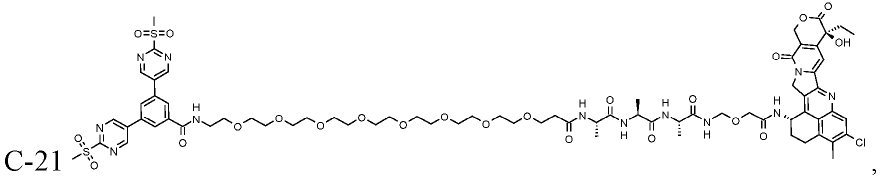 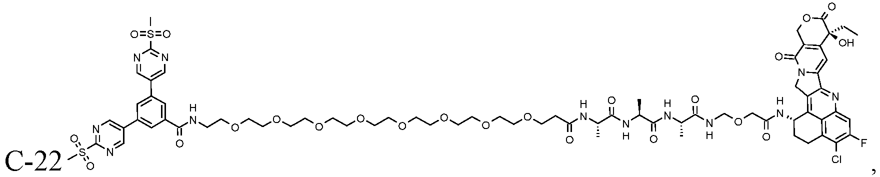 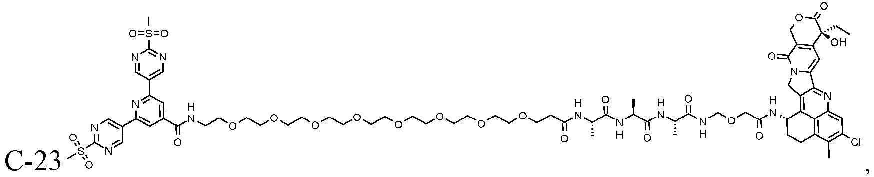 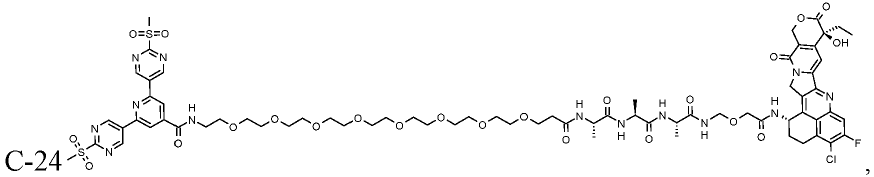 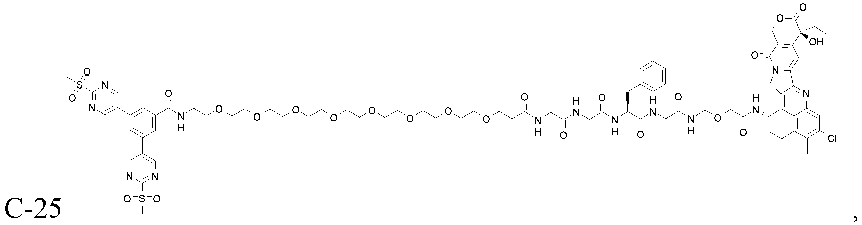 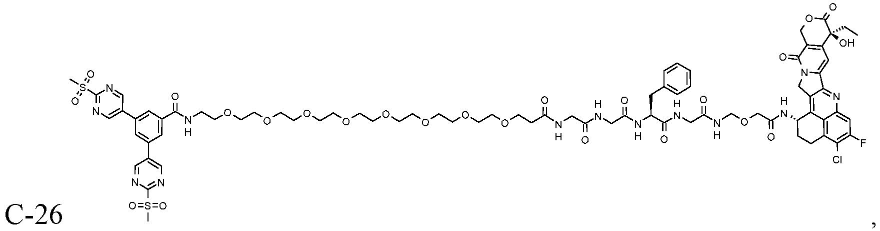 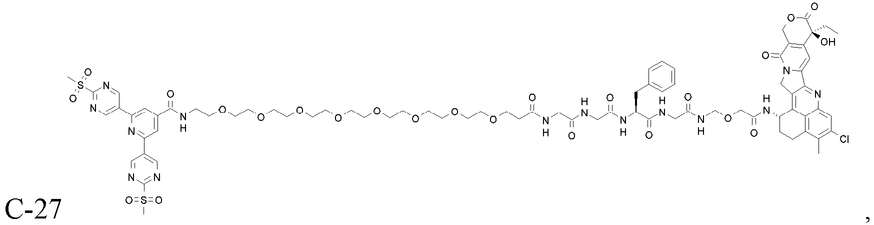 or

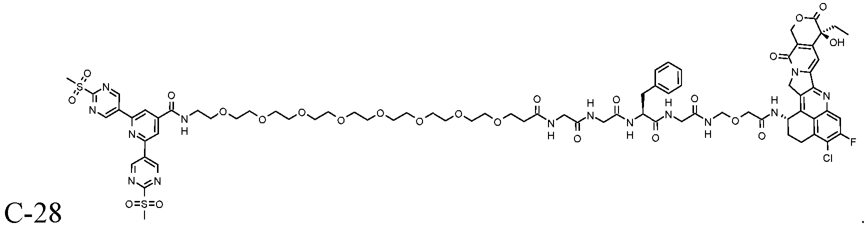

27. A bioactive conjugate, having a structure of formula VII: 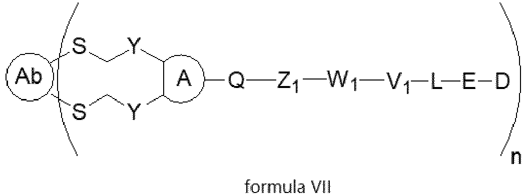
wherein Ab is a targeting moiety (such as a small molecular ligand, a protein (e.g., an antibody), a polypeptide, a non-protein reagent (e.g., sugar, RNA or DNA)); n is an integer or a decimal from 1 to 10;
V₁ is -C(O)- or -N(R₁)-, wherein R₁ is H, C₁₋₆ alkyl or C₂₋₆ alkoxyalkyl;
L is a linker that links V₁ and E;
E is a structural fragment that links L and D, preferably E is as defined in claim 20;
D is a fragment of a bioactive molecule (e.g., a cytotoxic drug); 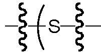
in the conjugate indicates, when the targeting moiety is an antibody, a specific linkage from a sulfhydryl in the antibody to the rest of the conjugate;
the remaining groups are as defined in any one of claims 1-4.

28. A bioactive conjugate, having a structure of formula VIII: 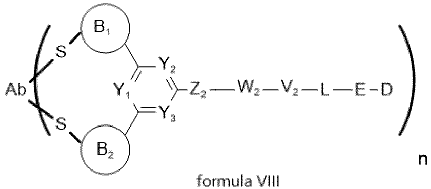
wherein Ab is a targeting moiety (such as a small molecular ligand, a protein (e.g., an antibody), a polypeptide, a non-protein reagent (e.g., sugar, RNA or DNA)); n is an integer or a decimal from 1 to 10;
V₂ is -C(O)- or -N(R₂)-, wherein R₂ is H, C₁₋₆ alkyl or C₂₋₆ alkoxyalkyl;
L is a linker that links V₂ and E;
E is a structural fragment that links L and D, preferably E is as defined in claim 22;
D is a fragment of a bioactive molecule (e.g., a cytotoxic drug); 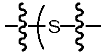 in the conjugate indicates, when the targeting moiety is an antibody, a specific linkage from a sulfhydryl in the antibody to the rest of the conjugate;
the remaining groups are as defined in any one of claims 5-18.

29. The bioactive conjugate according to claim 28, having a structure selected from the following group, wherein Ab is selected from an anti-Her2 antibody, an anti-Trop2 antibody, or an anti-ROR1 antibody, and n₁ ranges from 1 to 8:

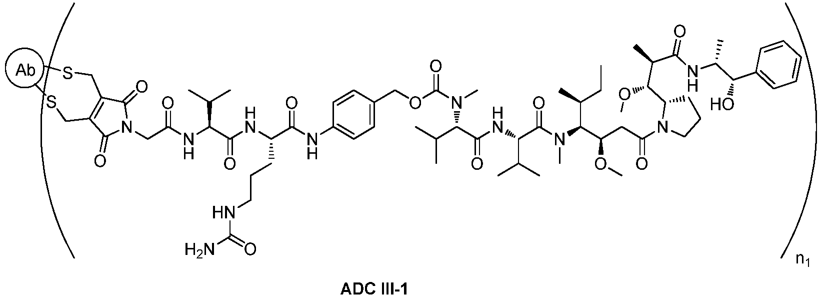

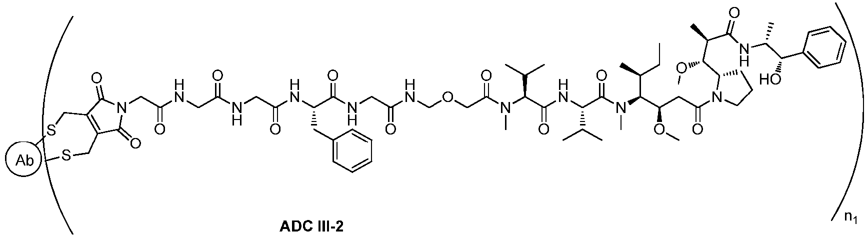

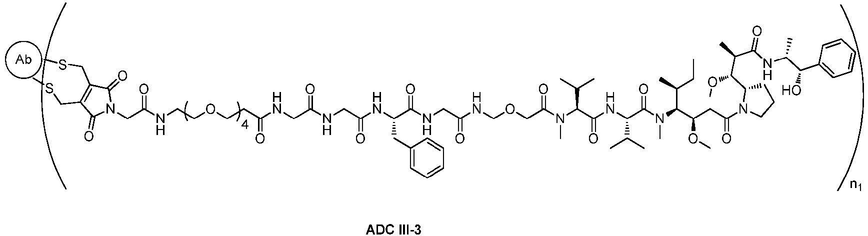

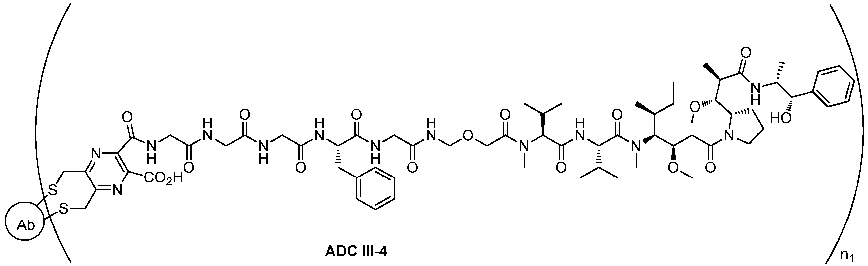

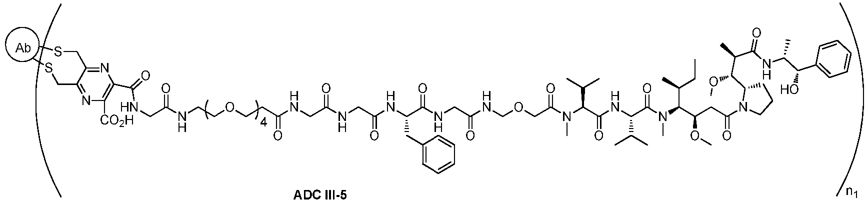

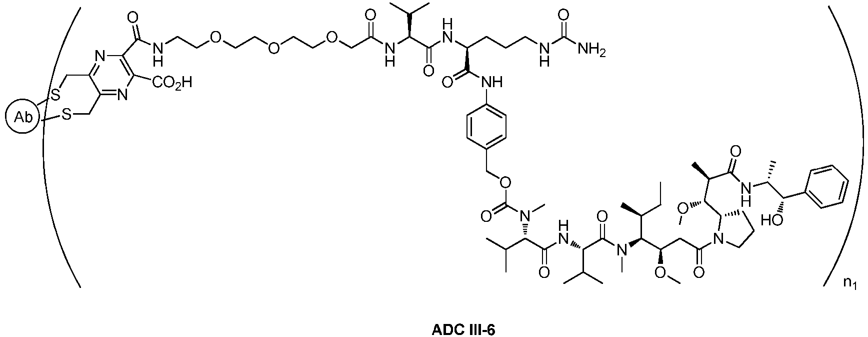

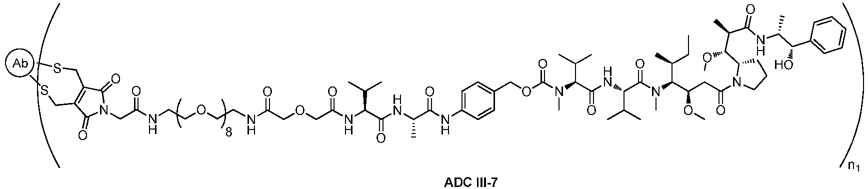

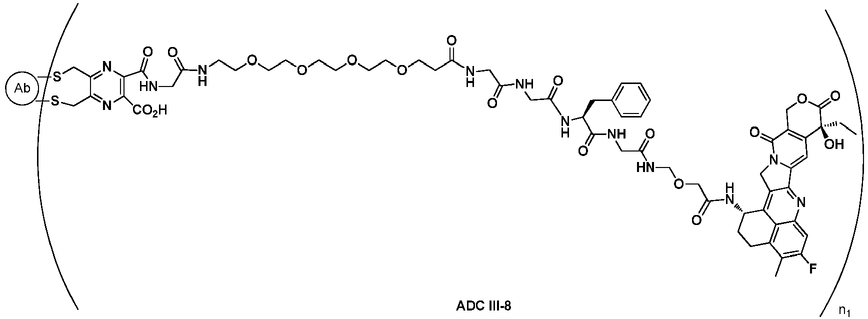

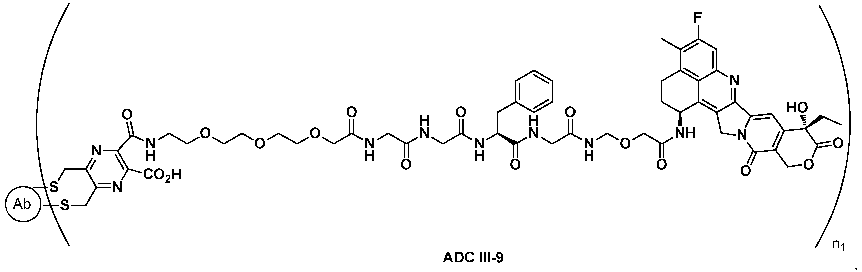

30. The bioactive conjugate according to claim 29, wherein
the anti-Her2 antibody is Trastuzumab; an antibody containing a heavy chain variable region having heavy chain complementary determining regions of Trastuzumab and a light chain variable region having light chain complementary determining regions of Trastuzumab; or an antibody containing a heavy chain variable region of Trastuzumab and a light chain variable region of Trastuzumab;
the anti-Trop2 antibody is Sacituzumab; an antibody containing a heavy chain variable region having heavy chain complementary determining regions of Sacituzumab and a light chain variable region having light chain complementary determining regions of Sacituzumab; or an antibody containing a heavy chain variable region of Sacituzumab and a light chain variable region of Sacituzumab; and/or
the anti-ROR1 antibody is:
i) an antibody containing a heavy chain variable region having CDR-H1 as set forth in SEQ ID NO: 3, CDR-H2 as set forth in SEQ ID NO: 4 and CDR-H3 as set forth in SEQ ID NO: 5, and a light chain variable region having CDR-L1 as set forth in SEQ ID NO: 6, CDR-L2 as set forth in SEQ ID NO: 7 and CDR-L3 as set forth in SEQ ID NO: 8, defined according to the Chothia numbering system;
ii) an antibody containing a heavy chain variable region having CDR-H1 as set forth in SEQ ID NO: 9, CDR-H2 as set forth in SEQ ID NO: 10 and CDR-H3 as set forth in SEQ ID NO: 5, and a light chain variable region having CDR-L1 as set forth in SEQ ID NO: 6, CDR-L2 as set forth in SEQ ID NO: 7 and CDR-L3 as set forth in SEQ ID NO: 8, defined according to the AbM numbering system;
iii) an antibody containing a heavy chain variable region having CDR-H1 as set forth in SEQ ID NO: 11, CDR-H2 as set forth in SEQ ID NO: 12 and CDR-H3 as set forth in SEQ ID NO: 5, and a light chain variable region having CDR-L1 as set forth in SEQ ID NO: 6, CDR-L2 as set forth in SEQ ID NO: 7 and CDR-L3 as set forth in SEQ ID NO: 8, defined according to the Kabat numbering system;
iv) an antibody containing a heavy chain variable region having CDR-H1 as set forth in SEQ ID NO: 13, CDR-H2 as set forth in SEQ ID NO: 14 and CDR-H3 as set forth in SEQ ID NO: 15, and a light chain variable region having CDR-L1 as set forth in SEQ ID NO: 16, CDR-L2 as set forth in SEQ ID NO: 17 and CDR-L3 as set forth in SEQ ID NO: 8, defined according to the IMGT numbering system;
v) an antibody containing a heavy chain variable region as set forth in SEQ ID NO: 1 and a light chain variable region as set forth in SEQ ID NO: 2; or
vi) 19F6_Hu35V1, containing a heavy chain variable region as set forth in SEQ ID NO: 1, a light chain variable region as set forth in SEQ ID NO: 2, a heavy chain constant region as set forth in SEQ ID NO: 18 and a light chain constant region as set forth in SEQ ID NO: 19.

31. The bioactive conjugate according to claim 29 or 30, wherein
n₁ ranges from 1 to 6, for example from 3 to 5.

32. The bioactive conjugate according to claim 28, having a structure selected from the following group, wherein Ab is selected from an anti-Her2 antibody, an anti-Trop2 antibody, or an anti-ROR1 antibody; n₁ ranges from 1 to 8, x ranges from 1 to 10:

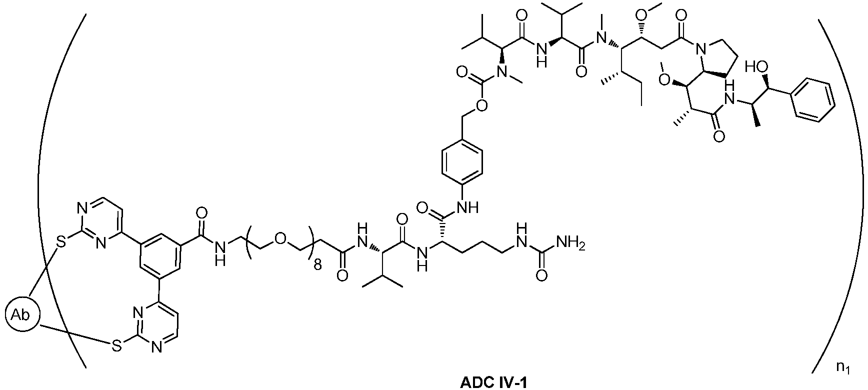

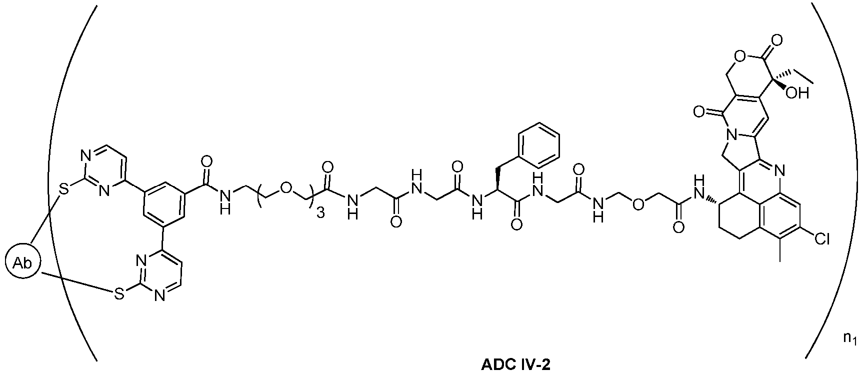

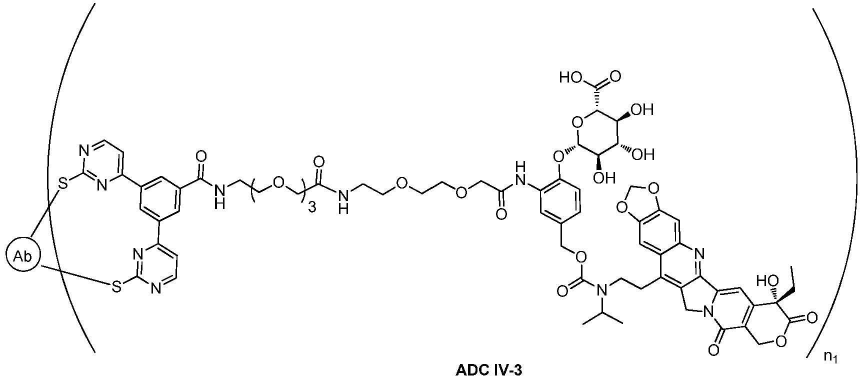

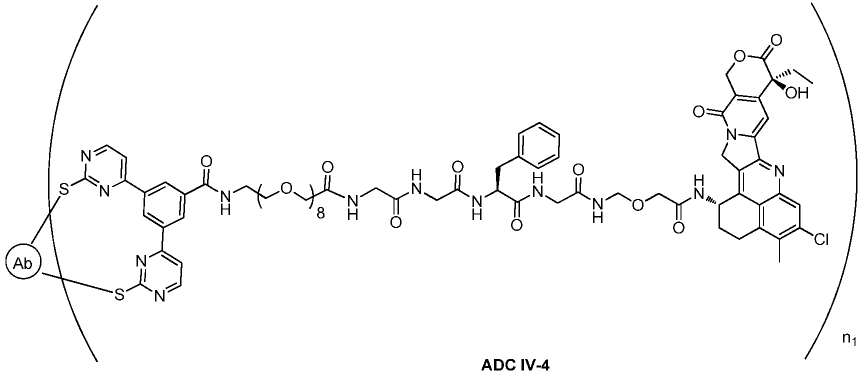

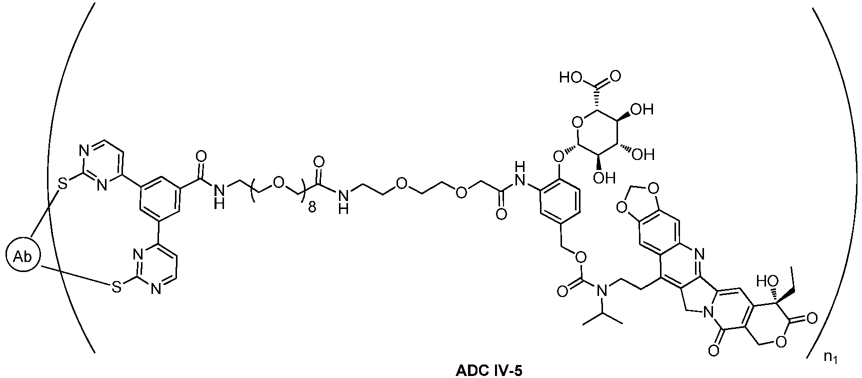

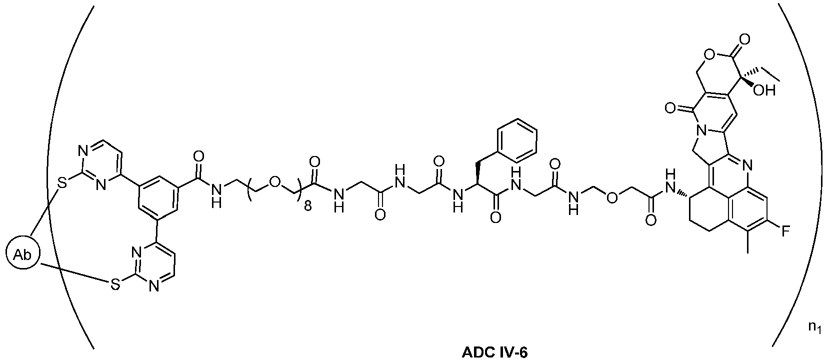

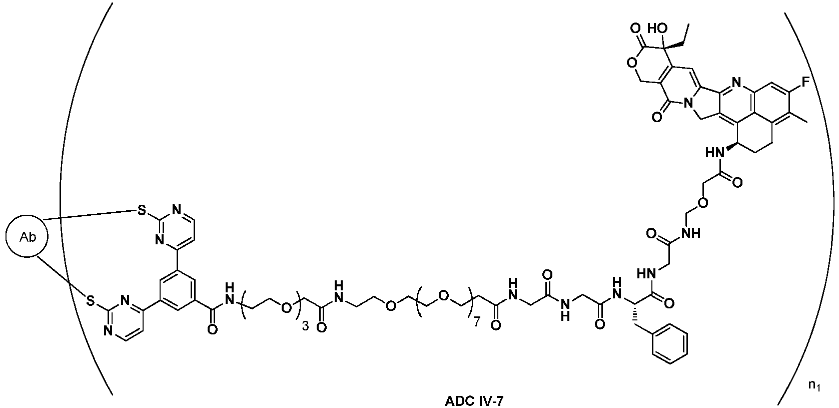

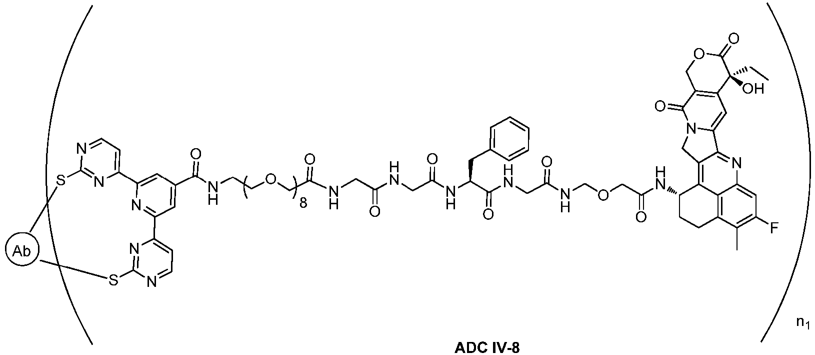

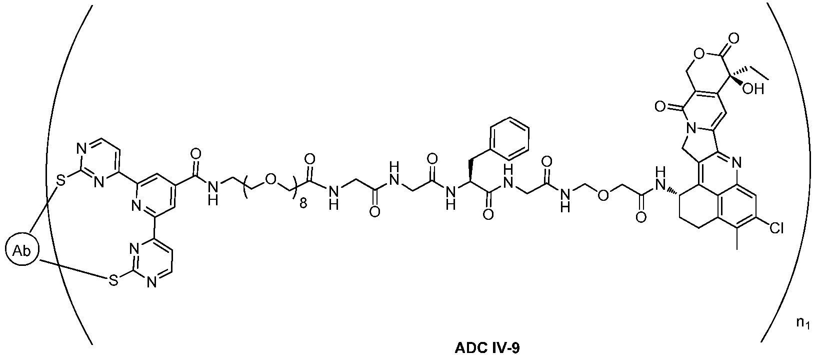

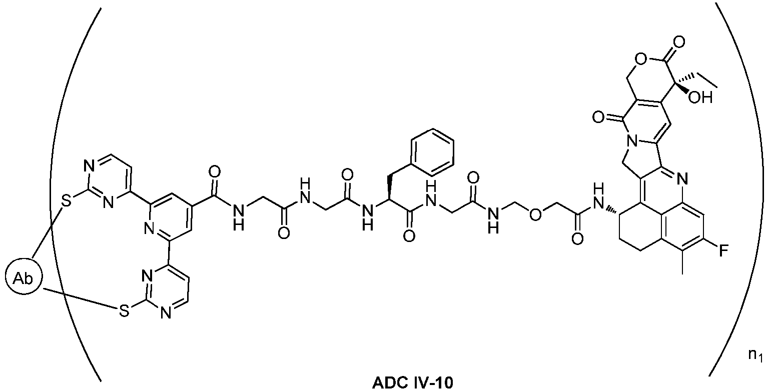

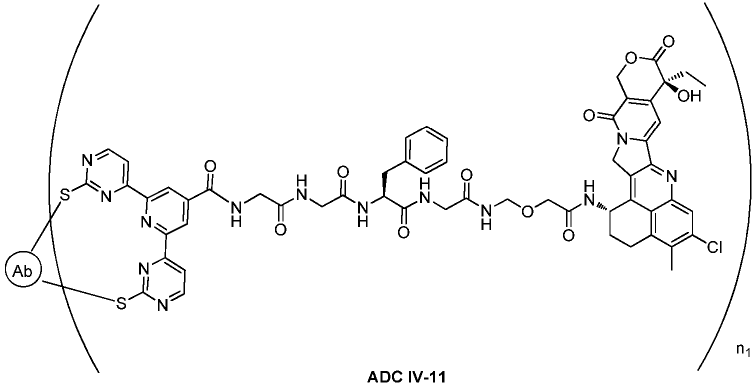

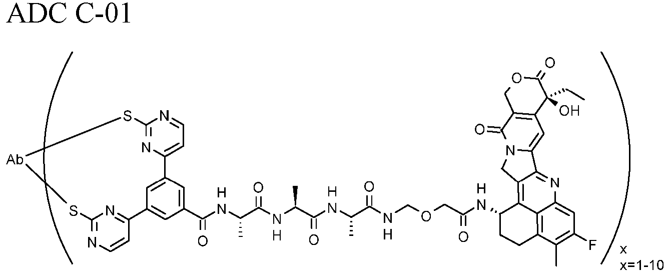

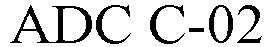

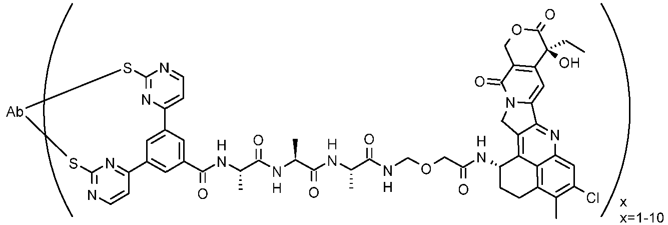

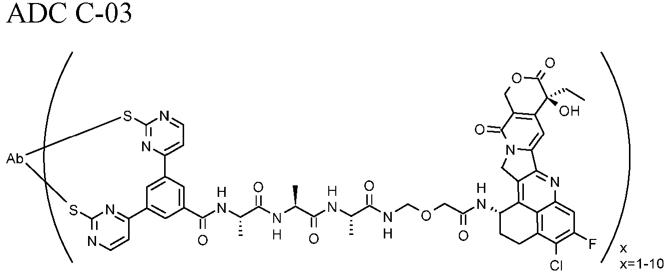

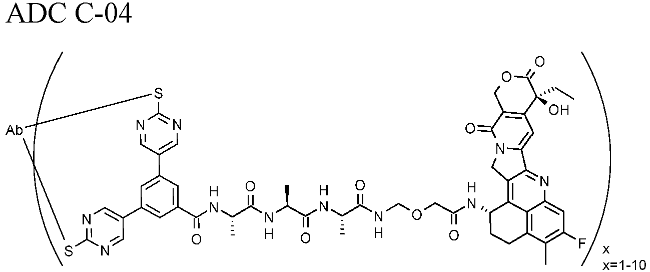

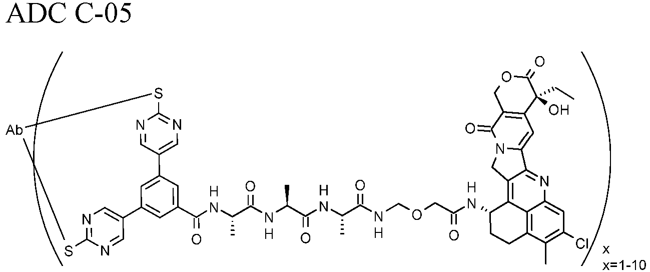

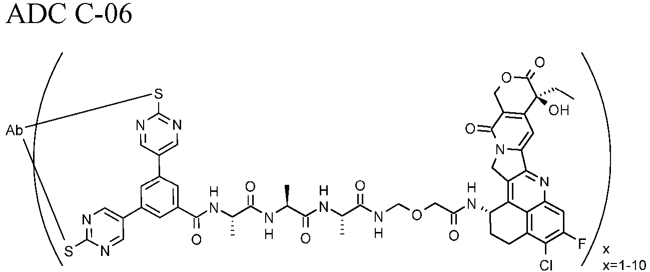

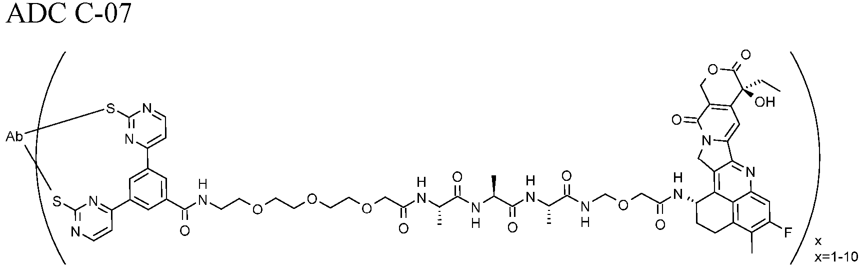

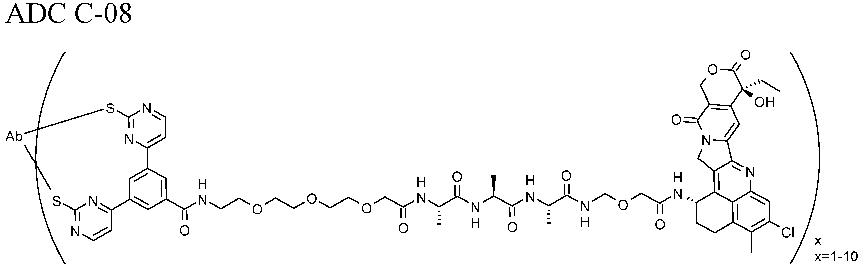

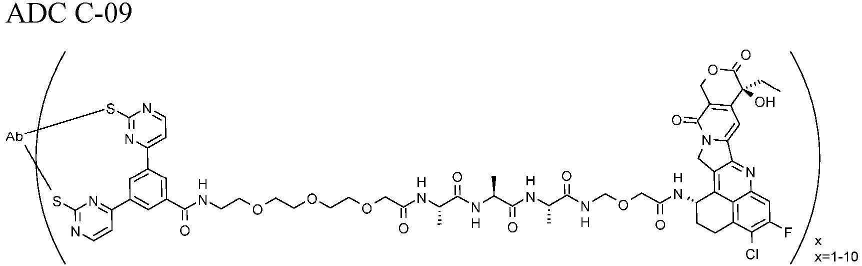

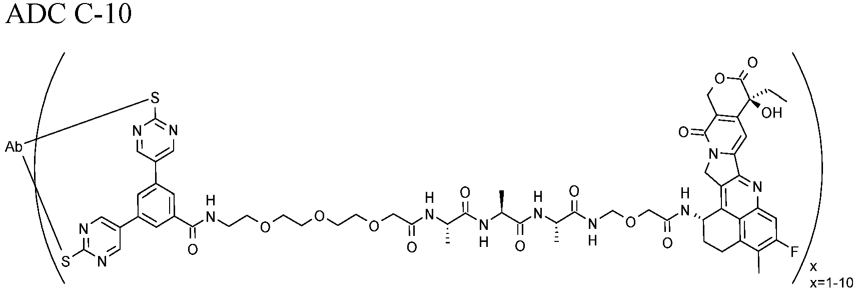

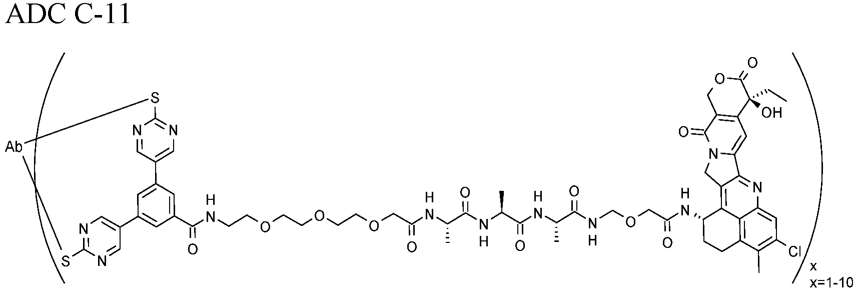

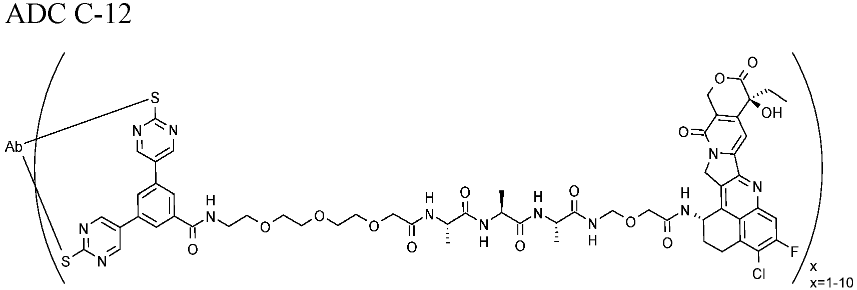

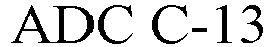

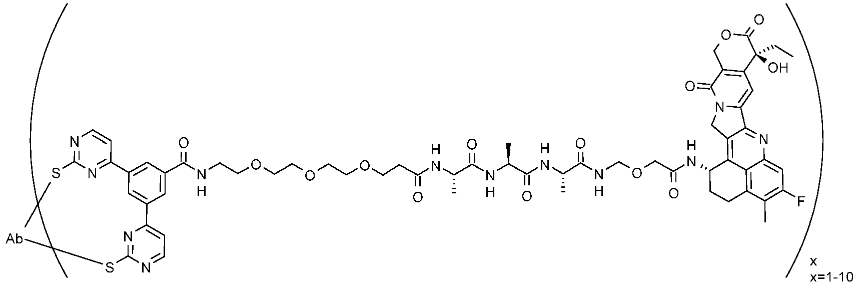

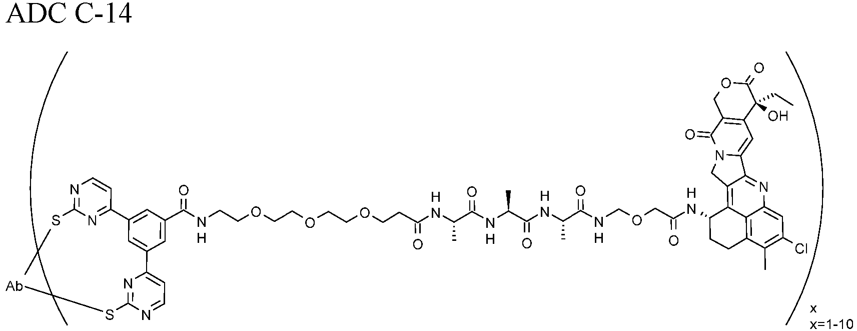

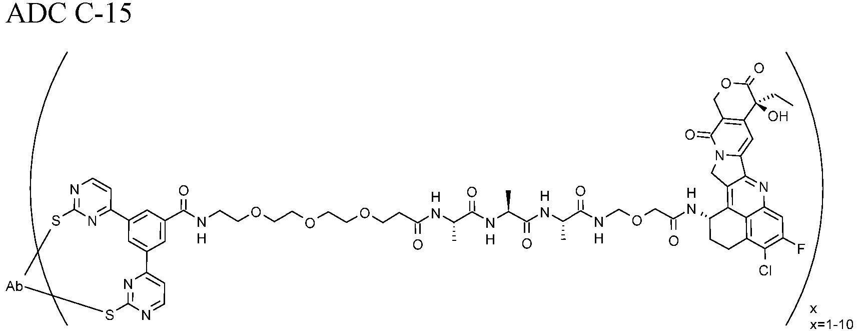

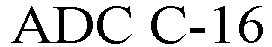

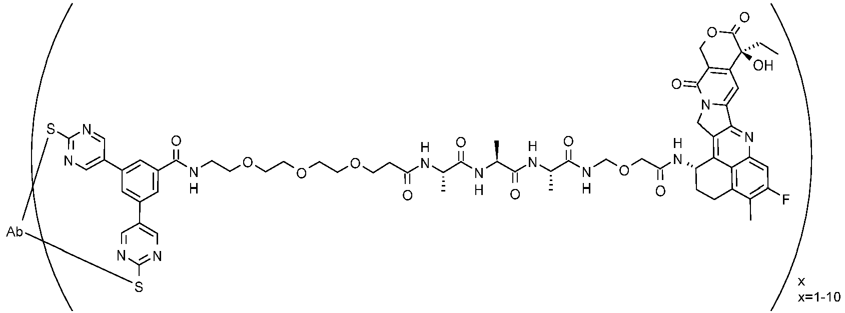

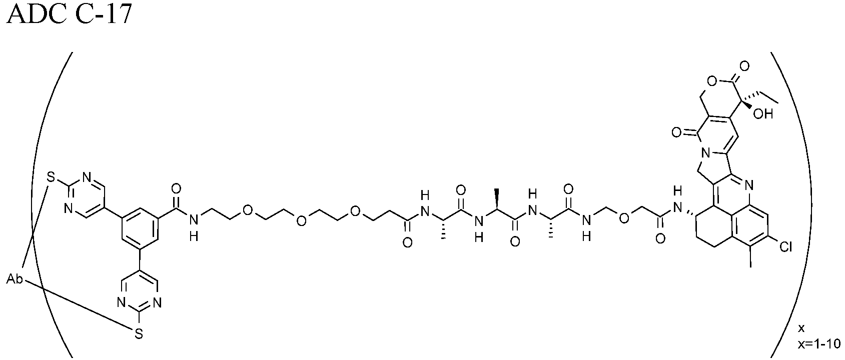

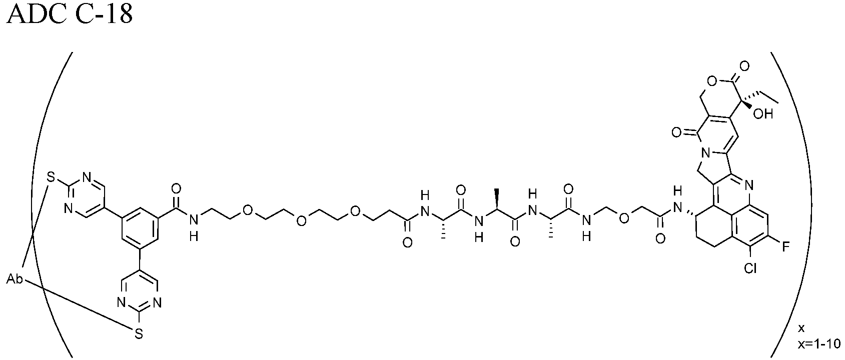

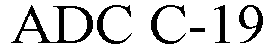

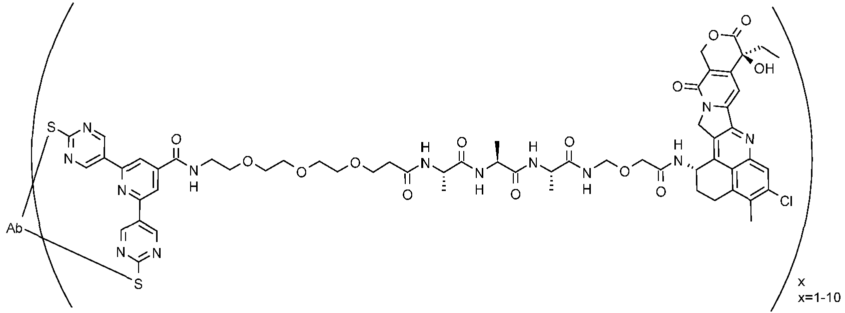

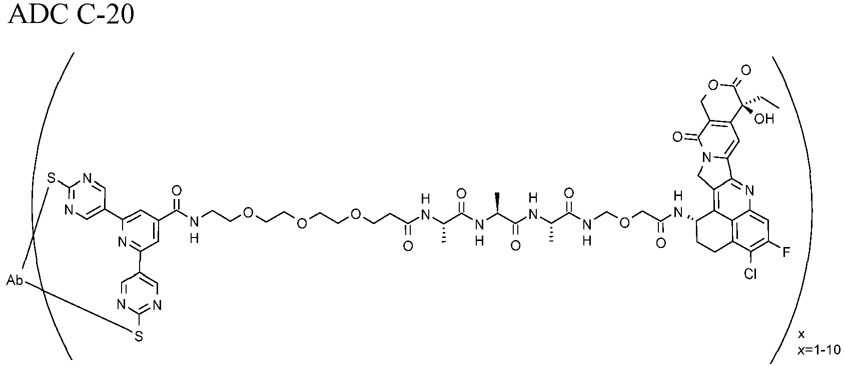

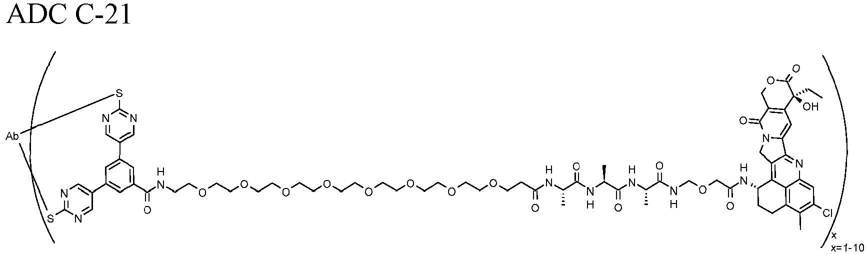

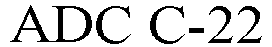

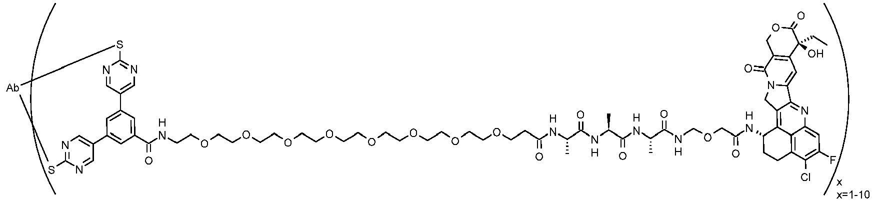

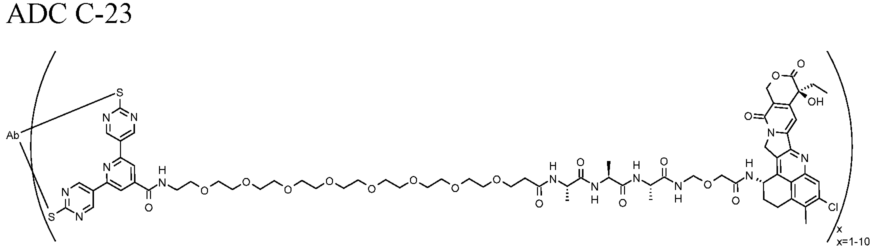

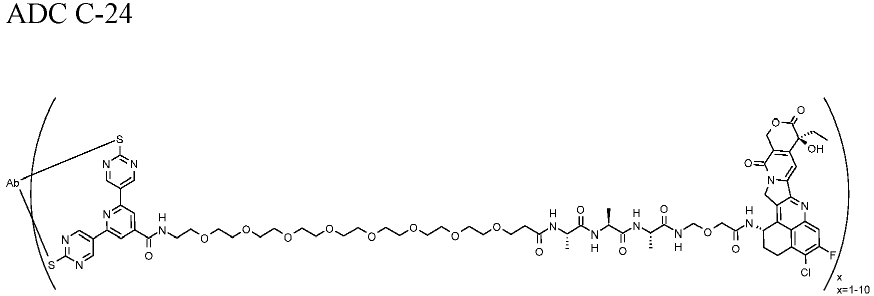

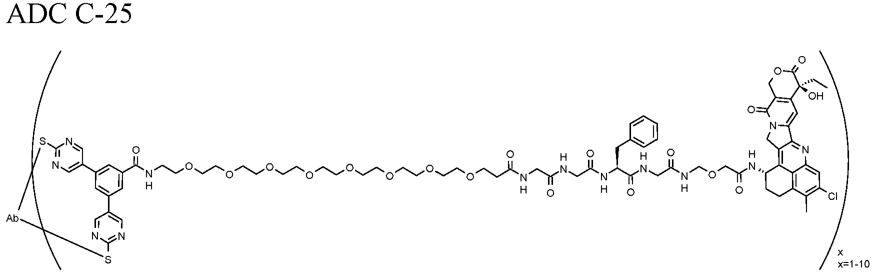

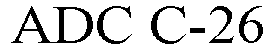

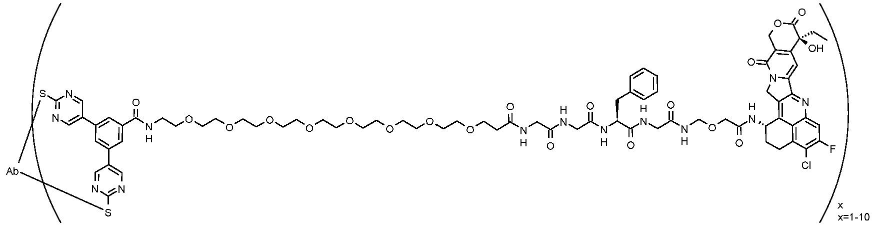

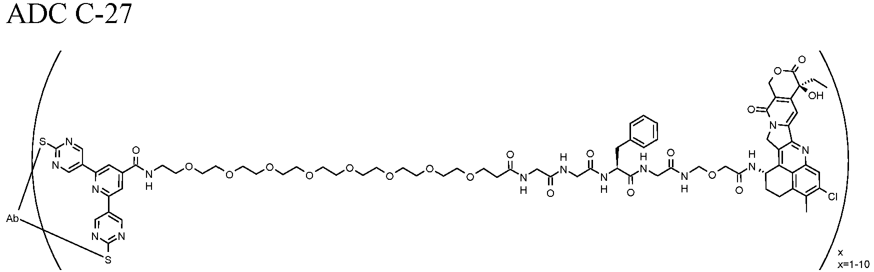

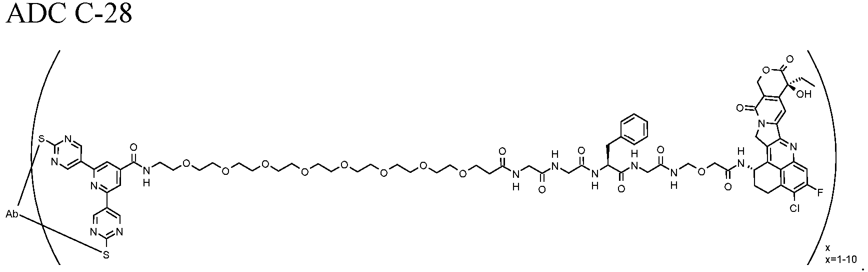

33. The bioactive conjugate according to claim 32, wherein
(1) the anti-Her2 antibody is Trastuzumab; an antibody containing a heavy chain variable region having heavy chain complementary determining regions of Trastuzumab and a light chain variable region having light chain complementary determining regions of Trastuzumab; or an antibody containing a heavy chain variable region of Trastuzumab and a light chain variable region of Trastuzumab;
(2) the anti-Trop2 antibody is Sacituzumab; an antibody containing a heavy chain variable region having heavy chain complementary determining regions of Sacituzumab and a light chain variable region having light chain complementary determining regions of Sacituzumab; or an antibody containing a heavy chain variable region of Sacituzumab and a light chain variable region of Sacituzumab;
(3) the anti-ROR1 antibody is:
i) an antibody containing a heavy chain variable region having CDR-H1 as set forth in SEQ ID NO: 3, CDR-H2 as set forth in SEQ ID NO: 4 and CDR-H3 as set forth in SEQ ID NO: 5, and a light chain variable region having CDR-L1 as set forth in SEQ ID NO: 6, CDR-L2 as set forth in SEQ ID NO: 7 and CDR-L3 as set forth in SEQ ID NO: 8, defined according to the Chothia numbering system;
ii) an antibody containing a heavy chain variable region having CDR-H1 as set forth in SEQ ID NO: 9, CDR-H2 as set forth in SEQ ID NO: 10 and CDR-H3 as set forth in SEQ ID NO: 5, and a light chain variable region having CDR-L1 as set forth in SEQ ID NO: 6, CDR-L2 as set forth in SEQ ID NO: 7 and CDR-L3 as set forth in SEQ ID NO: 8, defined according to the AbM numbering system;
iii) an antibody containing a heavy chain variable region having CDR-H1 as set forth in SEQ ID NO: 11, CDR-H2 as set forth in SEQ ID NO: 12 and CDR-H3 as set forth in SEQ ID NO: 5, and a light chain variable region having CDR-L1 as set forth in SEQ ID NO: 6, CDR-L2 as set forth in SEQ ID NO: 7 and CDR-L3 as set forth in SEQ ID NO: 8, defined according to the Kabat numbering system;
iv) an antibody containing a heavy chain variable region having CDR-H1 as set forth in SEQ ID NO: 13, CDR-H2 as set forth in SEQ ID NO: 14 and CDR-H3 as set forth in SEQ ID NO: 15, and a light chain variable region having CDR-L1 as set forth in SEQ ID NO: 16, CDR-L2 as set forth in SEQ ID NO: 17 and CDR-L3 as set forth in SEQ ID NO: 8, defined according to the IMGT numbering system;
v) an antibody containing a heavy chain variable region as set forth in SEQ ID NO: 1 and a light chain variable region as set forth in SEQ ID NO: 2; or
vi) 19F6_Hu35V1, an antibody containing a heavy chain variable region as set forth in SEQ ID NO: 1, a light chain variable region as set forth in SEQ ID NO: 2, a heavy chain constant region as set forth in SEQ ID NO: 18 and a light chain constant region as set forth in SEQ ID NO: 19.

34. The bioactive conjugate according to claim 31 or 32, wherein
n₁ ranges from 1 to 6, for example from 3 to 5.

35. The bioactive conjugate according to any one of claims 31-33, wherein
x ranges from 1 to 6, for example from 3 to 5.

36. A pharmaceutical composition comprising the compound or a pharmaceutically acceptable salt, ester, stereoisomer, tautomer, polymorph, solvate, N-oxide or isotope labeled compound thereof according to any one of claims 20-26, or the bioactive conjugate according to any one of claims 27-35, and one or more pharmaceutically acceptable carriers;
preferably, the drug-to-antibody ratio (DAR) of the pharmaceutical composition is 1.0-6.0, such as 1, 2, 3, 4, 5 or 6, and such as 1.0-1.5, 1.0-2.0, 1.0-2.5, 1.0-3.0, 1.0-3.5, 1.0-4.0, 1.0-4.5, 1.0-5.0, 1.0-5.5, 1.0-6.0, 1.5-2.0, 1.5-2.5, 1.5-3.0, 1.5-3.5, 1.5-4.0, 1.5-4.5, 1.5-5.0, 1.5-5.5, 1.5-6.0, 2.0-2.5, 2.0-3.0, 2.0-3.5, 2.0-4.0, 2.0-4.5, 2.0-5.0, 2.0-5.5, 2.0-6.0, 2.5-3.0, 2.5-3.5, 2.5-4.0, 2.5-4.5, 2.5-5.0, 2.5-5.5, 2.5-6.0, 3.0-3.5, 3.0-4.0, 3.0-4.5, 3.0-5.0, 3.0-5.5, 3.0-6.0, 3.5-4.0, 3.5-4.5, 3.5-5.0, 3.5-5.5, 3.5-6.0, 4.0-4.5, 4.0-5.0, 4.0-5.5, 4.0-6.0, 4.5-5.0, 4.5-5.5, 4.5-6.0, 5.0-5.5, 5.0-6.0 or 5.5-6.0.

37. A kit product, containing the compound or a pharmaceutically acceptable salt, ester, stereoisomer, tautomer, polymorph, solvate, N-oxide or isotope labeled compound thereof according to any one of claims 20-26, or the bioactive conjugate according to any one of claims 27-35, or the pharmaceutical composition according to claim 36, and an optional drug specification.

38. Use of the compound or a pharmaceutically acceptable salt, ester, stereoisomer, tautomer, polymorph, solvate, N-oxide or isotope labeled compound thereof according to any one of claims 20-26, the bioactive conjugate according to any one of claims 27-35, or the pharmaceutical composition according to claim 36 in the preparation of a medicament for the prevention or treatment of a tumor disease.

39. The compound or a pharmaceutically acceptable salt, ester, stereoisomer, tautomer, polymorph, solvate, N-oxide or isotope labeled compound thereof according to any one of claims 20-26, the bioactive conjugate according to any one of claims 27-35, or the pharmaceutical composition according to claim 36 for use in preventing or treating a tumor disease.

40. A method for preventing or treating a tumor disease, comprising administration of an effective amount of the compound or a pharmaceutically acceptable salt, ester, stereoisomer, tautomer, polymorph, solvate, N-oxide or isotope labeled compound thereof according to any one of claims 20-26, the bioactive conjugate according to any one of claims 27-35, or the pharmaceutical composition according to claim 36 to a subject in need thereof.

41. A method for the synthesis of a compound, **characterized in that**, the method comprises the following steps: 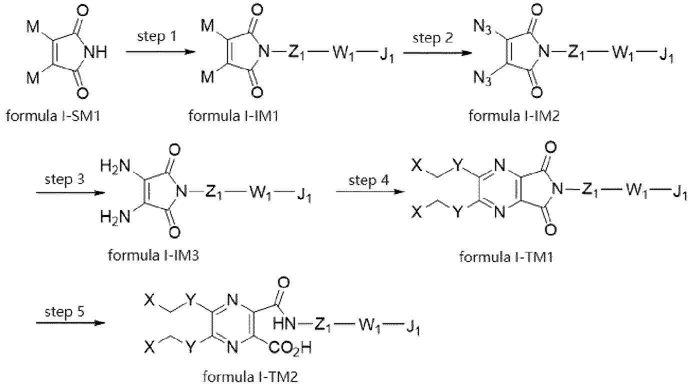 wherein
X, Z₁, W₁ and J₁ are as defined in any one of claims 1-4; Y is absent;
M is a leaving group for substitution reaction, comprising but not limited to halogen, trifluoromethane sulfonate, and p-toluene sulfonate, preferably halogen;
or, comprises the following steps: 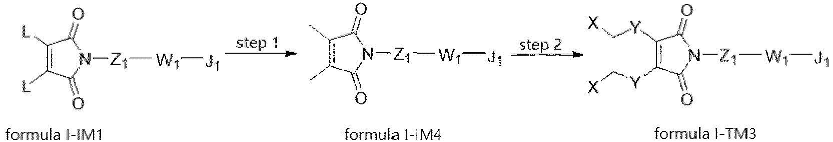
wherein
X, Z₁, W₁ and J₁ are as defined in any one of claims 1-4;
L is a leaving group for substitution reaction, comprising but not limited to halogen, trifluoromethane sulfonate and p-toluene sulfonate, preferably halogen and OTf;
or, comprises the following steps: 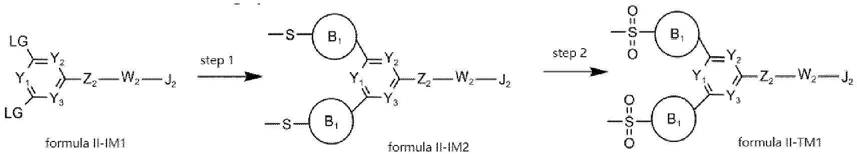
wherein
Y₁, Y₂, Y₃, B₁, B₂, Z₂, W₂ and J₂ are as defined in any one of claims 5-18;
LG is a leaving group for coupling reaction, comprising but not limited to halogen and trifluoromethane sulfonate, preferably halogen.

42. Use of the compound in any one of claims 1-4 for the preparation of a payload-linker compound, wherein
the payload-linker compound is prepared from the compound according to any one of claims 1-4 by the following steps: 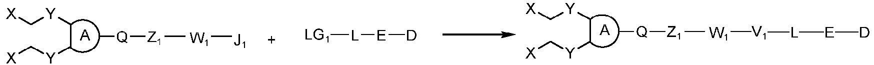
wherein X, Y, A, Q, Z₁, J₁, W₁, V₁, L, E and D are as defined in any one of claims 1-4, 20 and 21; LG₁ is selected from groups that condensate with J₁.

43. The use according to claim 42, wherein
the payload-linker compound is selected from the compound or a pharmaceutically acceptable salt, ester, stereoisomer, tautomer, polymorph, solvate, N-oxide or isotope labeled compound thereof according to claim 20 or 21.

44. The use according to claim 43, wherein
LG₁ is -COOH or -NH(R₁), wherein R₁ is H, C₁₋₆ alkyl or C₂₋₆ alkoxyalkyl.

45. Use of the compound according to any one of claim 5-18 for the preparation of a payload-linker compound; wherein
the payload-linker compound is prepared from the compound according to any one of claims 5-18 through the following steps: 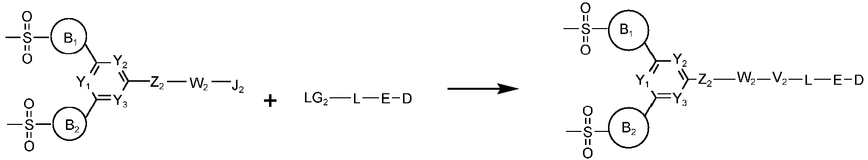
wherein B₁, B₂, Y₁, Y₂, Y₃, Z₂, J₂, W₂, V₂, L, E and D are as defined in any one of claims 5-18, 22-26; LG₂ is selected from groups that condensate with J₂.

46. The use according to claim 45, wherein
the payload-linker is selected from the compound or a pharmaceutically acceptable salt, ester, stereoisomer, tautomer, polymorph, solvate, N-oxide or isotope labeled compound thereof according to any one of claims 22-26.

47. The use according to claim 45, wherein
LG₂ is -COOH or -NH(R₂), wherein R₂ is H, C₁₋₆ alkyl or C₂₋₆ alkoxyalkyl.

48. Use of the compound according to any one of claims 1-4 for the preparation of a bioactive conjugate; wherein
the bioactive conjugate is prepared from the compound according to any one of claims 1-4 through steps 1a and 1b below:
step 1a: 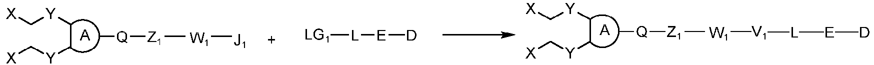
step 1b: 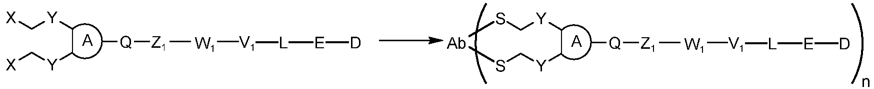
wherein X, Y, A, Q, Z₁, J₁, W₁, V₁, L, E, D, Ab and n are as defined in any one of claims 1-4, 20, 21, 27, 29-31; LG₁ is selected from groups that condensate with J₁.

49. The use according to claim 48, wherein
the bioactive conjugate is selected from the bioactive conjugates according to any one of claims 27, 29-31.

50. The use according to claim 48 or 49, wherein
LG₁ is -COOH or -NH(R₁), wherein R₁ is H, C₁₋₆ alkyl or C₂₋₆ alkoxyalkyl.

51. Use of the compound according to any one of claims 5-18 for the preparation of a bioactive conjugate; wherein
the bioactive conjugate is prepared from the compound according to any one of claims 5-18 through steps 2a and 2b below:
step 2a: 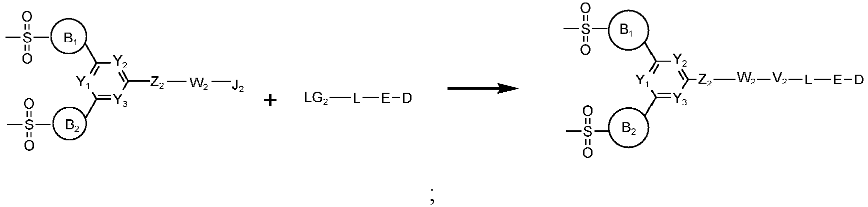
step 2b: 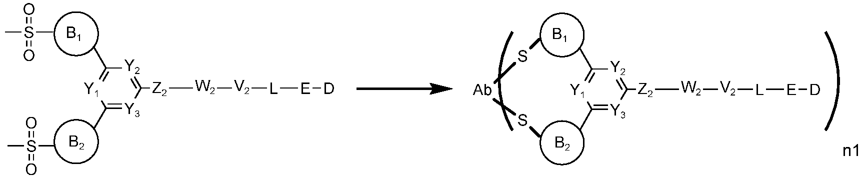
wherein B₁, B₂, Y₁, Y₂, Y₃, Z₂, J₂, W₂, V₂, L, E, D, Ab and n1 are as defined in any one of claims 5-18, 22-26, 28, 32-35; LG₂ is selected from groups that condensate with J₂.

52. The use according to claim 51, wherein
the bioactive conjugate is selected from the bioactive conjugates according to any one of claims 28, 32-35.

53. The use according to claim 51 or 52, wherein
LG₂ is -COOH or -NH(R₂), wherein R₂ is H, C₁₋₆ alkyl or C₂₋₆ alkoxyalkyl.

54. A method for preparing a compound, wherein the method comprises the following steps: 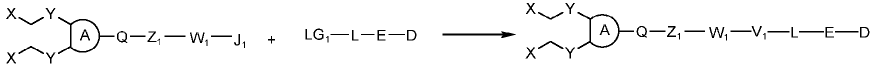 wherein X, Y, A, Q, Z₁, J₁, W₁, V₁, L, E and D are as defined in any one of claims 1-4, 20, 21; LG₁ is selected from groups that condensate with J₁.

55. The method according to claim 54, wherein
LG₁ is -COOH or -NH(R₁), wherein R₁ is H, C₁₋₆ alkyl or C₂₋₆ alkoxyalkyl.

56. A method for preparing a compound, wherein the method comprises the following steps: 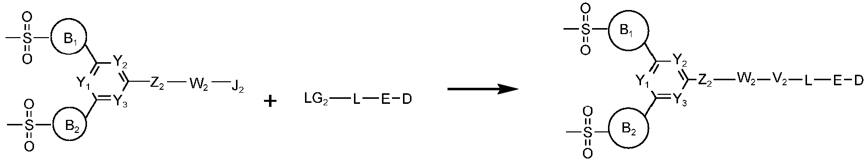 wherein B₁, B₂, Y₁, Y₂, Y₃, Z₂, J₂, W₂, V₂, L, E and D are as defined in any one of claims 5-18, 22-26; LG₂ is selected from groups that condensate with J₂.

57. The method according to claim 56, wherein
LG₂ is -COOH or -NH(R₂), wherein R₂ is H, C₁₋₆ alkyl or C₂₋₆ alkoxyalkyl.

58. A method for preparing an antibody-drug conjugate, wherein the method comprises the following steps:
step 1a: 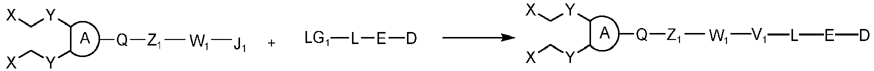
step 1b: 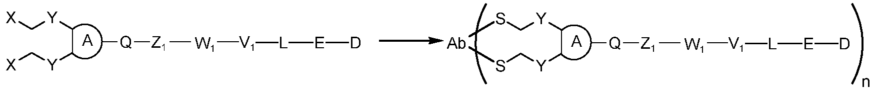
wherein X, Y, A, Q, Z₁, J₁, W₁, V₁, L, E, D, Ab and n are as defined in any one of claims 1-4, 20, 21, 27, 29-31; LG₁ is selected from groups that condensate with J₁.

59. The method according to claim 58, wherein
LG₁ is -COOH or -NH(R₁), wherein R₁ is H, C₁₋₆ alkyl or C₂₋₆ alkoxyalkyl.

60. A method for preparing an antibody-drug conjugate, wherein the method comprises the following steps:
step 2a: 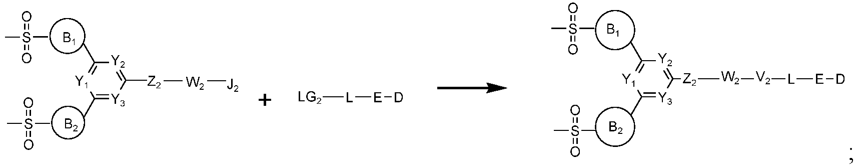
step 2b: 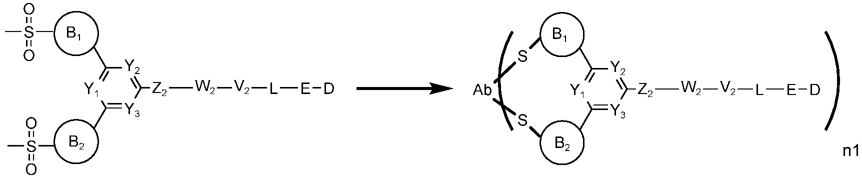
wherein B₁, B₂, Y₁, Y₂, Y₃, Z₂, J₂, W₂, V₂, L, E, D, Ab and n1 are as defined in any one of claims 5-18, 22-26, 28, 32-35; LG₂ is selected from groups that condensate with J₂.

61. The method according to claim 60, wherein
LG₂ is -COOH or -NH(R₂), wherein R₂ is H, C₁₋₆ alkyl or C₂₋₆ alkoxyalkyl.
